# EUROPEAN PATENT APPLICATION

(11) **EP 4 186 922 A1**
(43) Date of publication of application: **31.05.2023**
(21) Application number: 21845495.7
(22) Date of filing: 23.07.2021
(51) Int. Cl.: C07K 16/10, G01N 33/569, A61P 31/14, A61K 39/00

(54) **BINDING MOLECULE HAVING NEUTRALIZING ACTIVITY AGAINST CORONAVIRUS SUPERFAMILY**

(30) Priority: 24.07.2020 KR 20200092285; 11.09.2020 KR 20200116817; 13.11.2020 KR 20200152158; 10.12.2020 KR 20200171999; 10.02.2021 KR 20210019481; 22.03.2021 KR 20210036902; 30.03.2021 KR 20210041251; 09.06.2021 KR 20210075095
(71) Applicant: Celltrion, Inc., Incheon 22014 (KR); Korea Disease Control and Prevention Agency, Chungcheongbuk-go 28159 (KR)
(72) Inventor: KIM, Cheol-Min, Incheon 22014 (KR); SEO, Ji-Min, Incheon 22014 (KR); AN, Yong-Jin, Incheon 22014 (KR); KIM, Min-Soo, Incheon 22014 (KR); LEE, Soo-Young, Incheon 22014 (KR); LIM, Hee-Young, Cheongju-si, Chungcheongbuk-do 28159 (KR); LEE, Joo-Yeon, Cheongju-si, Chungcheongbuk-do 28159 (KR); KIM, Kyung-Chang, Cheongju-si, Chungcheongbuk-do 28159 (KR); YANG, Jeong-Sun, Cheongju-si, Chungcheongbuk-do 28159 (KR); LEE, Han-Saem, Cheongju-si, Chungcheongbuk-do 28159 (KR); WOO, Hye-Min, Cheongju-si, Chungcheongbuk-do 28159 (KR); KIM, Jun-Won, Cheongju-si, Chungcheongbuk-do 28159 (KR)
(74) Representative: Cabinet Laurent & Charras
(86) International application number: PCT/KR2021/009567
(87) International publication number: WO 2022/019711

(57) **Abstract**

The present invention relates to a binding molecule having neutralizing activity against the coronavirus superfamily. More specifically, the binding molecule of the present invention has excellent binding affinity and excellent neutralizing efficacy against various coronavirus species that may infect humans, including not only wild-type SARS-coronavirus-2 (SARS-CoV-2) and mutant viruses that may occur in the future, but also SARS-coronavirus-1 (SARS-CoV-1), Middle East respiratory syndrome coronavirus (MERS-CoV), etc., which infect humans, causing fatal diseases. Thus, the binding molecule is very useful for the diagnosis, prevention or treatment of diseases caused by coronaviruses.

## Description

### Technical Field

The present invention relates to a binding molecule having neutralizing activity against the coronavirus superfamily, and more specifically, to a binding molecule having excellent binding affinity and neutralizing efficacy against various coronavirus species that may infect humans, including not only wild type SARS-coronavirus-2 (SARS-CoV-2) and mutant viruses that may occur in the future, but also SARS-coronavirus-1 (SARS-CoV-1), Middle East respiratory syndrome coronavirus (MERS-CoV), etc., which infect humans, causing fatal diseases.

### Background Art

Coronaviruses refer to viruses belonging to the family Coronaviridae and are generally found not only in birds but also in various mammals including humans. It is known that there are various coronavirus species, and coronaviruses cause both respiratory and digestive infectious diseases depending on the characteristics of the virus and on the host. Coronaviruses are viruses that cause respiratory illnesses of varying severity from the common cold to fatal pneumonia. Viruses belonging to the family Coronaviridae generally have a single-stranded, infectious, positive sense RNA genome with a length of 27 to 32 kb, and contain a cap at the 5' end of the genome and a poly A tail at the 3' end. Coronaviruses have an envelope, and contain major outer membrane proteins such as spike (S) protein and envelope (E) protein. The spike protein is involved in viral infection and pathogenicity, such as neutralizing antibody induction, receptor binding, and membrane fusion, and the envelope (E) protein is involved in morphogenesis of viral particles and extracellular release of the virus after infection.

Seven types of coronavirus are known to cause disease in humans, and infection with three types among them may cause even more severe or fatal pneumonia in humans. The three types that are fatal to humans include SARS-coronavirus-1 (SARS-CoV-1), which was identified as the cause of the 2003 severe acute respiratory syndrome outbreak which was a worldwide problem, MERS-coronavirus (MERS-CoV), which was identified as the cause of the 2012 Middle East respiratory syndrome outbreak, and the new coronavirus severe acute respiratory syndrome coronavirus 2 (SARS-CoV-2), which was first found in Wuhan, China in late 2019 and identified as the cause of coronavirus infection 2019 (COVID-19).

Severe acute respiratory syndrome (SARS) coronavirus 1 (SARS-CoV-1) is surrounded by a spherical envelope, like other coronaviruses, and has crown-like spikes (spike protein, S protein) on its surface. Severe acute respiratory syndrome caused by SARS coronavirus was first identified in China, and resulted in more than 8,000 cases and more than 800 deaths worldwide by mid-2003. So far, there is no antiviral agent that can treat human SARS coronavirus infection. However, ribavirin is used in the early stage of the infection, and high-concentration steroids may be used to suppress immune-mediated damage to infected tissues.

Middle East respiratory syndrome (MERS) coronavirus (MERS-CoV) has a single positive-stranded RNA genome, and the genes thereof are arranged in the order of RNA polymerase gene, structural protein genes, envelope protein, membrane protein, and nucleocapsid protein. The virus that causes Middle East respiratory syndrome (MERS) is a coronavirus similar to the virus that causes severe acute respiratory syndrome (SARS). There is no specific treatment for MERS, and nonsteroidal anti-inflammatory drugs (NSAIDs) such as acetaminophen or ibuprofen are administered to relieve fever and muscle pain.

Severe acute respiratory syndrome (SARS) coronavirus 2 (SARS-CoV-2) is a positive-sense single-stranded RNA coronavirus when subjected to DNA sequencing. SARS-CoV-2 is contagious to humans and is the cause of coronavirus disease 2019 (COVID-19). The first outbreak of COVID-19 occurred in Wuhan, Hubei Province, China.

People infected with SARS-CoV-2 may exhibit mild to severe symptoms such as fever, cough, shortness of breath, and diarrhea. People with complications or illnesses and the elderly are more likely to die.

In particular, early detection and treatment are very important because people with underlying diseases such as heart disease and diabetes are more susceptible to infection and more likely to suffer from complications or organ damage. From December 8, 2019 to March 20, 2020, there were 245,550 patients, among whom 10,049 died, which is a mortality rate of 4.09% (WHO). The virus spread to 177 countries, including Korea, by 2020.

Currently, there is no therapeutic agent for coronavirus disease 2019 (COVID-19), and therapeutic effects are expected to be exhibited by administering existing therapeutic agents to patients. Ebola therapeutic agents or candidates, for example, antiviral drugs such as favipiravir, remdesivir, and galidesivir, and a hepatitis C drug such as ribavirin, are being used as therapeutic agents for COVID-19. In addition, the antimalarial drug chloroquine has also been shown to be effective against COVID-19, and thus is in open-label clinical trials. However, ribavirin, which is a hepatitis C therapeutic drug, may have severe side effects such as anemia, and interferon, which is an antiviral drug, is also recommended to be used with caution because of the various side effects thereof.

Although these drugs have been used in the treatment of COVID-19 patients and show therapeutic effects thereon, it has not yet been clearly proven on what mechanism they are effective. In China, it was announced that plasma therapy of injecting the plasma of patients who had recovered from COVID-19 was effective in the treatment of severely ill patients, but the therapeutic effect thereof is unclear and is highly uncertain.

In Korea, the COVID-19 Central Clinical Trial TF (Task Force) established a treatment principle for COVID-19 on February 13, 2020, and announced that Kaletra, which is an AIDS therapeutic agent, and chloroquine and hydroxychloroquine, which are antimalarial drugs, are recommended as the first-line therapeutic agents, and ribavirin and interferon are not recommended as the first-line therapeutic agents due to side effects thereof. As for mild cases or young patients, and 10 days after the onset of the disease, it was judged that the symptoms would improve without administration of an antiviral drug, and it was agreed to administer antiviral drugs to the elderly, patients with underlying diseases, and severely ill patients.

The US CDC i) announced that COVID-19 could become endemic like MERS rather than a seasonal pandemic virus and cause infection, and ii) mentioned the need to strengthen surveillance so that data-based conclusions can be drawn although there is no evidence that the coronavirus is lurking in the real community due to the spread of the virus to the community at some point this year or next year (February 13, 2020).

The Korea Disease Control and Prevention Agency (formerly Korea Centers for Disease Control and Prevention) announced that i) COVID-19 could be a long-term epidemic like influenza and thus it should be included in monitoring programs such as that used for influenza, and ii) coronaviruses (4 types) that are prevalent among people are also prevalent from winter to spring, leaving the possibility that COVID-19 could also become endemic (February 17, 2020).

Unlike SARS and MERS, there are concerns about the realization of the COVID-19 pandemic, but there is the possibility of a lull after spring (April), so there are many experts who take a careful approach depending on the progress thereof. Due to the lack of information on COVID-19, experts have different opinions on future developments, but few experts predict that it will be resolved in a short time. Although further progress of COVID-19 will be influenced by the accuracy of analysis of prevalence and characteristics of COVID-19 and by how long the crisis from COVID-19 will last, there are concerns about the possibility of endemic disease if it is spread worldwide through asymptomatic infected people. There is an urgent need to prepare countermeasures for the likelihood of recurrence of COVID-19 worldwide.

In addition, recently, there have been more than 50,000 confirmed cases of COVID-19 per day in the United States alone, and it is the opinion of the academic community that the occurrence of about 200,000 confirmed cases of COVID-19 per day around the world is due to mutation of the virus. Research results that coronaviruses mutate into a contagious form and that mutation occurs in the spike protein are amplifying concerns, and if mutation occurs in a part playing an important role in viral infection, such as the spike protein, it may also influence the development of vaccines and therapeutic agents. As various mutant SARS-CoV-2 viruses have been reported, it is urgent to provide a method capable of acting against not only wild-type viruses but also mutant viruses.

A rapid diagnostic test (RDT) is also referred to by various names such as "immunochromatographic analysis", "rapid kit analysis", and the like. A user can simply detect an analyte from a biological or chemical sample by the rapid diagnostic test. The rapid diagnostic test is a method capable of qualitatively and quantitatively testing analytes within a short time using the properties of biological or chemical materials that specifically adhere to each other.

The rapid diagnostic test is the most advanced assay kit among the detection methods developed until recently, in view of simplicity and quickness, and is usefully used to diagnose various disease-causing substances such as antibodies or antigens of infectious pathogens, cancer factors, heart disease markers, and the like.

There is no specific preventive agent, therapeutic agent, or diagnostic kit for coronaviruses including SARS-CoV-2, or SARS-CoV-1 and MERS-CoV which are likely to re-emerge. Accordingly, the present inventors have made efforts to develop a therapeutic antibody capable of acting against mutant viruses and coronaviruses that are likely to occur later.

Accordingly, the present applicant has conducted extensive studies, and as a result, developed an antibody capable of neutralizing not only wild-type COVID-19 and mutant COVID-19 viruses, but also viruses belonging to the family Coronaviridae, such as SARS or MERS, thereby completing the present invention.

### DISCLOSURE

### Technical Problem

In order to solve the above-described problems, the present inventors have developed a binding molecule that is able to bind to various coronavirus species that may infect humans, including not only wild type SARS-coronavirus-2 (SARS-CoV-2) and mutant viruses that may occur in the future, but also SARS-coronavirus-1 (SARS-CoV-1), Middle East respiratory syndrome coronavirus (MERS-CoV), etc., which infect humans, causing fatal diseases, and have found that the binding molecule has excellent binding affinity and/or neutralizing efficacy, thereby completing the present invention.

An object of the present invention is to provide a neutralizing binding molecule that binds to a spike protein (S protein) on the surface of coronavirus.

Another object of the present invention is to provide a composition for diagnosing, preventing or treating a disease caused by coronavirus, the composition comprising the binding molecule.

Still another object of the present invention is to provide a kit for diagnosing, preventing or treating a disease caused by coronavirus, the kit comprising the binding molecule.

Yet another object of the present invention is to provide a method of diagnosing, preventing or treating a disease caused by coronavirus by administering the composition to a subject having the disease caused by coronavirus.

### Technical Solution

To achieve the above objects, the present invention provides a binding molecule, particularly a neutralizing binding molecule, which binds to a spike protein (S protein) on the surface of coronavirus.

The present invention also provides an immunoconjugate in which at least one tag is additionally conjugated to the binding molecule.

The present invention also provides a nucleic acid molecule encoding the binding molecule.

The present invention also provides an expression vector into which the nucleic acid molecule has been inserted.

The present invention also provides a cell line transformed with the expression vector.

The present invention also provides a composition for diagnosing, preventing or treating a disease caused by coronavirus, the composition comprising the binding molecule.

The present invention also provides a kit for diagnosing, preventing or treating a disease caused by coronavirus, the kit comprising the binding molecule.

The present invention also provides a method for diagnosing, preventing or treating a disease caused by coronavirus, the method comprising a step of administering a therapeutically effective amount of the composition to a subject having the disease caused by coronavirus.

The present invention also provides a strip for immunochromatographic analysis, the strip comprising the binding molecule.

The present invention also provides a diagnostic kit for diagnosing a disease caused by coronavirus, the diagnostic kit comprising the strip for immunochromatographic analysis.

The present invention also provides a method of detecting a disease caused by coronavirus using the diagnostic kit.

The present invention also provides a method of diagnosing a disease caused by coronavirus using the diagnostic kit.

### Advantageous Effects

The binding molecule of the present invention has excellent binding affinity and excellent neutralizing efficacy against various coronavirus species that may infect humans, including not only wild type SARS-coronavirus-2 (SARS-CoV-2) and mutant viruses that may occur in the future, but also SARS-coronavirus-1 (SARS-CoV-1), Middle East respiratory syndrome coronavirus (MERS-CoV), etc., which infect humans, causing fatal diseases. Thus, the binding molecule is very useful for the diagnosis, prevention or treatment of a disease caused by coronavirus.

### Brief Description of Drawings

FIG. 1 shows the results of evaluating the mechanism of action of antibody No. 32 according to an embodiment of the present invention by performing biolayer interference (BLI) analysis using Octet.
FIG. 2a shows the results of evaluating the average body weight of each group every day for 6 days before and after infection with SARS-CoV-2 virus during a mouse animal experiment conducted using the binding molecule of the present invention.
FIG. 2b shows the results of measuring the virus titer of mouse lung tissue using Vero cells after inoculation with SARS-CoV-2 virus during a mouse animal experiment conducted using the binding molecule of the present invention.
FIG. 2c shows the results of measuring the virus titer of mouse nasal lavage using Vero cells after inoculation with SARS-CoV-2 virus during a mouse animal experiment conducted using the binding molecule of the present invention.

### Best Mode

Hereinafter, the present invention will be described in more detail.

The scope of the present invention is not limited by the following description, and in particular, may include all aspects that may vary depending on the experimental conditions described in the following Examples and the like. Since the scope of the present invention will be defined by the appended claims, terms used herein for further understanding are used only for the purpose of describing embodiments of the present invention in detail, and the scope of the present invention is not limited thereby.

Unless otherwise defined herein, all technical and scientific terms used in the present specification are to be interpreted as having the same meanings as those commonly understood by those of ordinary skill in the art. All of the references mentioned in the present specification are incorporated herein by reference in their entirety to describe the invention of the present specification.

The present invention includes a **binding molecule** having ability to bind to or to neutralize all or part of a spike protein (S protein) on the surface of coronavirus, particularly SARS-coronavirus-2 (SARS-CoV-2), SARS-coronavirus-1 (SARS-CoV-1), and/or Middle East respiratory syndrome coronavirus (MERS-CoV), an **immunoconjugate** in which at least one tag is additionally conjugated to the binding molecule, a **nucleic acid molecule** encoding the binding molecule, an **expression vector** into which the nucleic acid molecule has been inserted, and/or a **cell line** transformed with the expression vector; a **composition** for diagnosing, ameliorating, preventing and/or treating a disease caused by coronavirus and a **kit** for diagnosing, ameliorating, preventing and/or treating a disease caused by coronavirus, which comprise at least one of the foregoing; a **method** of diagnosing, ameliorating, preventing and/or treating a disease caused by coronavirus using at least one of the foregoing, the **use** thereof for the diagnosis, amelioration, prevention or treatment of a disease caused by coronavirus, and the **use thereof for the preparation** of the binding molecule, the immunoconjugate, the nucleic acid molecule, the expression vector, the cell line, the composition or the kit for diagnosing, preventing and/or treating a disease caused by coronavirus, but the scope of the present invention is not limited thereto, and all analogues thereof may be included within the scope intended by the present invention.

The present invention is directed to a binding molecule and a neutralizing binding molecule, which bind to a spike protein (S protein) on the surface of coronavirus, particularly at least one selected from the group consisting of a receptor binding domain (RBD), S1 domain and S2 domain in the spike protein. The coronavirus may be at least one selected from the group consisting of SARS-coronavirus-2 (SARS-CoV-2), SARS-coronavirus-1 (SARS-CoV-1), and Middle East respiratory syndrome coronavirus (MERS-CoV).

The spike protein (S protein) on the surface of SARS-coronavirus-2 (SARS-CoV-2) of the present invention may consist of or comprise the sequence of SEQ ID NO: 1,521, and may include derivatives and/or variants thereof, without being limited thereto.

The receptor binding domain (RBD) of the spike protein on the surface of SARS-coronavirus-2 of the present invention may consist of or comprise the sequence of SEQ ID NO: 1,522, and may include derivatives and/or variants thereof, without being limited thereto.

In an embodiment of the present invention, the coronavirus may be:
i) SARS-coronavirus-2 (SARS-CoV-2) and SARS-coronavirus-1 (SARS-CoV-1);
ii) SARS-coronavirus-2 (SARS-CoV-2) and Middle East respiratory syndrome coronavirus (MERS-CoV); or
iii) SARS-coronavirus-2 (SARS-CoV-2), SARS-coronavirus-1 (SARS-CoV-1) and Middle East respiratory syndrome coronavirus (MERS-CoV).

The binding molecule may bind to a spike protein on the surface of SARS-coronavirus-2 (SARS-CoV-2), particularly the receptor binding domain (RBD), S1 domain and S2 domain; and/or a spike protein on SARS-coronavirus-1 (SARS-CoV-1); and/or a spike protein on MERS-coronavirus (MERS-CoV).

In one embodiment of the present invention, the neutralizing binding molecule of the present invention may have excellent binding affinity and/or excellent neutralizing activity against the spike protein of coronavirus, in particular, SARS-coronavirus-2 (SARS-CoV-2). The neutralizing binding molecule of the present invention may have excellent binding affinity for the spike protein of SARS-coronavirus-2 (SARS-CoV-2), and/or exhibit excellent neutralizing activity against wild-type SARS-coronavirus-2 (SARS-CoV-2). In addition, the neutralizing binding molecule of the present invention may also exhibit excellent neutralizing activity against a mutant virus having a mutation in the spike protein of SARS-coronavirus-2 (SARS-CoV-2). As an example, the neutralizing binding molecule of the present invention may also exhibit neutralizing activity against a virus having a mutation in S protein domains other than the RBD of the spike protein of SARS-coronavirus-2 (SARS-CoV-2), without being limited thereto.

In one embodiment of the present invention, the neutralizing binding molecule of the present invention may bind to the spike protein of SARS-coronavirus-2 (SARS-CoV-2), particularly the receptor binding domain (RBD), S1 domain and S2 domain; and/or the spike protein of SARS-coronavirus-1 (SARS-CoV-1); and/or the spike protein of MERS-coronavirus (MERS-CoV), and exhibit neutralizing activity against various coronavirus species, including SARS-coronavirus-2 (SARS-CoV-2), SARS-coronavirus-1 (SARS-CoV-1) and/or MERS-coronavirus (MERS-CoV).

Coronaviruses are RNA viruses belonging to the subfamily Coronavirinae of the family Coronaviridae, and are divided according to the host into four genera: alpha-, beta-, delta-, and gamma-coronaviruses. Not only human corona viruses but also mammalian coronaviruses found in cats, pigs, cattle, and bats mainly belong to the alpha- and beta-coronavirus genera, and most viruses infecting birds belong to the genus gamma-coronavirus. It is known that there are various coronavirus species, and coronaviruses cause both respiratory and digestive infectious diseases depending on the characteristics of the virus and on the host. Coronaviruses are viruses that cause respiratory illnesses of varying severity from the common cold to fatal pneumonia. Viruses belonging to the family Coronaviridae generally have a single-stranded, infectious, positive sense RNA genome with a length of 27 to 32 kb, and contain a cap at the 5' end of the genome and a poly A tail at the 3' end. Coronaviruses have an envelope, and contain major outer membrane proteins such as spike (S) protein and envelope (E) protein. The spike protein is involved in viral infection and pathogenicity, such as neutralizing antibody induction, receptor binding, and membrane fusion, and the envelope (E) protein is involved in morphogenesis of viral particles and extracellular release of the virus after infection.

Seven types of coronavirus are known to cause disease in humans, and infection with three types among them may cause even more severe or fatal pneumonia in humans. The three types that are fatal to humans include SARS-coronavirus-1 (SARS-CoV-1), which was identified as the cause of the 2002 severe acute respiratory syndrome outbreak which was a worldwide problem, MERS-coronavirus (MERS-CoV), which was identified as the cause of the 2012 Middle East respiratory syndrome outbreak, and the new coronavirus severe acute respiratory syndrome coronavirus 2 (SARS-CoV-2), which was first found in Wuhan, China in late 2019 and identified as the cause of coronavirus infection 2019 (COVID-19). Severe acute respiratory syndrome (SARS) coronavirus 2, SARS-CoV-2) is the cause of coronavirus infection 2019 (COVID-19), and is a novel coronavirus first found in Wuhan, China in late 2019.

SARS-coronavirus-1 (SARS-CoV-1) is a new infectious disease that has spread to countries worldwide, including Hong Kong, Singapore, and Canada, within a few months after its first outbreak in China in the winter of 2002, and the causative agent of SARS is SARS coronavirus-1. SARS-CoV-1 is presumed to have crossed the interspecies barrier from animals to infect humans by coronavirus variants that use animals as hosts. It is known that the main symptoms of SARS-CoV-1 are fever and cough, a sharp decrease in blood oxygen saturation, dyspnea, pneumonia, etc., and most of SARS-CoV-1 patients recover, but SARS-CoV-1 can be the cause of death in the elderly or children. Since there is no vaccine or therapeutic agent for prevention, it has been recommended to prescribe flu drugs, antiviral drugs, and the like.

MERS-coronavirus (MERS-CoV) was first detected in Jordan and Saudi Arabia in 2012, and most MERS-CoV infections occurred in Saudi Arabia, and occurred in people who traveled to the Middle East from other countries or worked in the Middle East. MERS-coronavirus is spread through close contact with MERS patients or through airborne droplets released by an infected person coughing or sneezing, and most people infected with MERS experience fever, chills, muscle pain, and cough. So far, there is no MERS vaccine and therapeutic agent in Korea and other countries. For treatment of MERS-CoV, it is generally recommended to use drugs such as the immunomodulator interferon and the antiviral agent ribavirin or lopinavir. However, it has been reported that interferon and ribavirin cause side effects such as bone marrow function deterioration, anemia and viral mutations, and thus there are concerns about the safety thereof. In addition, co-administration of interferon and ribavirin showed a therapeutic effect against MERS in the monkey model, but it did not show a great effect on MERS patients in actual clinical practice, and thus there has been a need to develop a safer and more effective agent for treatment of MERS. In Korea, there has been no additional occurrence after the last occurrence of MERS-coronavirus infection, but MERS-coronavirus infection has continuously occurred in Middle Eastern countries, and thus it is necessary to prepare countermeasures against the re-introduction of MERS-coronavirus.

The World Health Organization (WHO) classifies SARS-coronavirus-2 (SARS-CoV-2) into six types based on amino acid changes due to differences in the gene sequence. SARS-CoV-2 was first classified into S and L types, and was then further divided into L, V, and G types, and G was subdivided into GH and GR, resulting in a total of six types: S, L, V, G, GH, and GR. At the beginning of the outbreak of COVID-19, S and V types were prevalent in Asian regions including Wuhan, China, and then different types were discovered in each continent, and the SARS-coronavirus-2 of the present invention includes these types. Among these, it has been reported that the GH type is likely to have high transmissibility. In Korea, based on the results of classifying genes collected from patients with coronavirus infection, most of them were found to be GH type, which is a variant of the G type prevalent in Europe and the United States, and this type is known to be highly transmissible. In particular, the G-type virus, in which the amino acid at position 614 of the spike protein, which plays an important role in the intracellular invasion of the virus, changed from aspartic acid (D) to glycine (G), has increased rapidly in Europe and the United States since March, and now appears in almost all regions, and the SARS-coronavirus-2 of the present invention includes the same. A recent report showed that more than 70 coronavirus variants occurred, among which 8 variants (D614G, etc.) having increased transmissibility, 10 variants (A841V, etc.) avoiding neutralizing antibodies, and 17 variants (I472V, etc.) on which plasma treatment effects are low, were identified, and the SARS-coronavirus-2 of the present invention includes these variants.

In one embodiment, the neutralizing binding molecule of the present invention may exhibit neutralizing activity against strains such as an S type (the amino acid at position 614 of the S protein is D), L type, V type, G type (the amino acid at position 614 of the S protein is G), GH type, or GR type, based on the amino acid mutation in SARS-coronavirus-2 (SARS-CoV-2), without being limited thereto. Examples of the SARS-CoV-2 virus S-type include, but are not limited to, a BetaCoV/Korea/KCDC03/2020 strain. Examples of the SARS-CoV-2 virus G-type include, but are not limited to, hCoV-19/South Korea/KUMC 17/2020 strain. In one embodiment, the neutralizing binding molecule of the present invention may also exhibit neutralizing activity against a mutant virus having D614G mutation at amino acid position 614 of the spike protein of SARS-coronavirus-2 (SARS-CoV-2).

In one embodiment of the present invention, the neutralizing binding molecule of the present invention may have neutralizing activity against any one or more mutant viruses selected from the group consisting of the following mutant viruses 1) to 39), without being limited thereto.
1) a mutant virus having a mutation at one or more of amino acid positions 69 and 70 of the spike protein of SARS-coronavirus-2;
2) a mutant virus having a mutation at amino acid position 80 of the spike protein of SARS-coronavirus-2;
3) a mutant virus having a mutation at amino acid position 222 of the spike protein of SARS-coronavirus-2;
4) a mutant virus having a mutation at amino acid position 234 of the spike protein of SARS-coronavirus-2;
5) a mutant virus having a mutation at amino acid position 372 of the spike protein of SARS-coronavirus-2;
6) a mutant virus having a mutation at amino acid position 384 of the spike protein of SARS-coronavirus-2;
7) a mutant virus having a mutation at amino acid position 406 of the spike protein of SARS-coronavirus-2;
8) a mutant virus having a mutation at amino acid position 417 of the spike protein of SARS-coronavirus-2;
9) a mutant virus having a mutation at amino acid position 420 of the spike protein of SARS-coronavirus-2;
10) a mutant virus having a mutation at amino acid position 439 of the spike protein of SARS-coronavirus-2;
11) a mutant virus having a mutation at amino acid position 440 of the spike protein of SARS-coronavirus-2;
12) a mutant virus having a mutation at amino acid position 444 of the spike protein of SARS-coronavirus-2;
13) a mutant virus having a mutation at amino acid position 445 of the spike protein of SARS-coronavirus-2;
14) a mutant virus having a mutation at amino acid position 446 of the spike protein of SARS-coronavirus-2;
15) a mutant virus having a mutation at amino acid position 449 of the spike protein of SARS-coronavirus-2;
16) a mutant virus having a mutation at amino acid position 452 of the spike protein of SARS-coronavirus-2;
17) a mutant virus having a mutation at amino acid position 453 of the spike protein of SARS-coronavirus-2;
18) a mutant virus having a mutation at amino acid position 455 of the spike protein of SARS-coronavirus-2;
19) a mutant virus having a mutation at amino acid position 456 of the spike protein of SARS-coronavirus-2;
20) a mutant virus having a mutation at amino acid position 460 of the spike protein of SARS-coronavirus-2;
21) a mutant virus having a mutation at amino acid position 473 of the spike protein of SARS-coronavirus-2;
22) a mutant virus having a mutation at amino acid position 475 of the spike protein of SARS-coronavirus-2;
23) a mutant virus having a mutation at amino acid position 476 of the spike protein of SARS-coronavirus-2;
24) a mutant virus having a mutation at amino acid position 477 of the spike protein of SARS-coronavirus-2;
25) a mutant virus having a mutation at amino acid position 478 of the spike protein of SARS-coronavirus-2;
26) a mutant virus having a mutation at amino acid position 484 of the spike protein of SARS-coronavirus-2;
27) a mutant virus having a mutation at amino acid position 486 of the spike protein of SARS-coronavirus-2;
28) a mutant virus having a mutation at amino acid position 489 of the spike protein of SARS-coronavirus-2;
29) a mutant virus having a mutation at amino acid position 490 of the spike protein of SARS-coronavirus-2;
30) a mutant virus having a mutation at amino acid position 493 of the spike protein of SARS-coronavirus-2;
31) a mutant virus having a mutation at amino acid position 494 of the spike protein of SARS-coronavirus-2;
32) a mutant virus having a mutation at amino acid position 498 of the spike protein of SARS-coronavirus-2;
33) a mutant virus having a mutation at amino acid position 501 of the spike protein of SARS-coronavirus-2;
34) a mutant virus having a mutation at amino acid position 614 of the spike protein of SARS-coronavirus-2;
35) a mutant virus having a mutation at amino acid position 677 of the spike protein of SARS-coronavirus-2;
36) a mutant virus having a mutation at amino acid position 681 of the spike protein of SARS-coronavirus-2;
37) a mutant virus having a mutation at amino acid position 685 of the spike protein of SARS-coronavirus-2;
38) a mutant virus having a mutation at amino acid position 701 of the spike protein of SARS-coronavirus-2; and
39) a mutant virus having a mutation at amino acid position 1,176 of the spike protein of SARS-coronavirus-2.

In one embodiment of the present invention, the neutralizing binding molecule of the present invention may have neutralizing activity against any one or more mutant viruses selected from the group consisting of the following mutant viruses 1) to 39), without being limited thereto.
1) a mutant virus having HV69-70del mutation at amino acid positions 69 and 70 of the spike protein of SARS-coronavirus-2;
2) a mutant virus having D80A mutation at amino acid position 80 of the spike protein of SARS-coronavirus-2;
3) a mutant virus having A222V mutation at amino acid position 222 of the spike protein of SARS-coronavirus-2;
4) a mutant virus having N234Q mutation at amino acid position 234 of the spike protein of SARS-coronavirus-2;
5) a mutant virus having A372V mutation at amino acid position 372 of the spike protein of SARS-coronavirus-2;
6) a mutant virus having P384L mutation at amino acid position 384 of the spike protein of SARS-coronavirus-2;
7) a mutant virus having E406Q mutation or E406W mutation at amino acid position 406 of the spike protein of SARS-coronavirus-2;
8) a mutant virus having K417N mutation, K417T mutation or K417E mutation at amino acid position 417 of the spike protein of SARS-coronavirus-2;
9) a mutant virus having D420N mutation at amino acid position 420 of the spike protein of SARS-coronavirus-2;
10) a mutant virus having N439K mutation at amino acid position 439 of the spike protein of SARS-coronavirus-2;
11) a mutant virus having N440D mutation at amino acid position 440 of the spike protein of SARS-coronavirus-2;
12) a mutant virus having K444Q mutation at amino acid position 444 of the spike protein of SARS-coronavirus-2;
13) a mutant virus having V445A mutation at amino acid position 445 of the spike protein of SARS-coronavirus-2;
14) a mutant virus having G446V mutation or G446S mutation at amino acid position 446 of the spike protein of SARS-coronavirus-2;
15) a mutant virus having Y449N mutation at amino acid position 449 of the spike protein of SARS-coronavirus-2;
16) a mutant virus having L452R mutation at amino acid position 452 of the spike protein of SARS-coronavirus-2;
17) a mutant virus having Y453F mutation at amino acid position 453 of the spike protein of SARS-coronavirus-2;
18) a mutant virus having L455F mutation at amino acid position 455 of the spike protein of SARS-coronavirus-2;
19) a mutant virus having F456L mutation at amino acid position 456 of the spike protein of SARS-coronavirus-2;
20) a mutant virus having N460T mutation at amino acid position 460 of the spike protein of SARS-coronavirus-2;
21) a mutant virus having Y473F mutation at amino acid position 473 of the spike protein of SARS-coronavirus-2;
22) a mutant virus having A475V mutation at amino acid position 475 of the spike protein of SARS-coronavirus-2;
23) a mutant virus having G476S mutation at amino acid position 476 of the spike protein of SARS-coronavirus-2;
24) a mutant virus having S477N mutation or S477R mutation at amino acid position 477 of the spike protein of SARS-coronavirus-2;
25) a mutant virus having T478K mutation at amino acid position 478 of the spike protein of SARS-coronavirus-2;
26) a mutant virus having E484K mutation, E484G mutation or E484Q mutation at amino acid position 484 of the spike protein of SARS-coronavirus-2;
27) a mutant virus having F486V mutation, F486I mutation, F486S mutation or F486L mutation at amino acid position 486 of the spike protein of SARS-coronavirus-2;
28) a mutant virus having Y489H mutation at amino acid position 489 of the spike protein of SARS-coronavirus-2;
29) a mutant virus having F490S mutation at amino acid position 490 of the spike protein of SARS-coronavirus-2;
30) a mutant virus having Q493K mutation, Q493R mutation, Q493M mutation, Q493Y mutation or Q493A mutation at amino acid position 493 of the spike protein of SARS-coronavirus-2;
31) a mutant virus having S494P mutation, S494Q mutation or S494L mutation at amino acid position 494 of the spike protein of SARS-coronavirus-2;
32) a mutant virus having Q498H mutation at amino acid position 498 of the spike protein of SARS-coronavirus-2;
33) a mutant virus having N501Y mutation, N501T mutation orN501F mutation at amino acid position 501 of the spike protein of SARS-coronavirus-2;
34) a mutant virus having D614G mutation at amino acid position 614 of the spike protein of SARS-coronavirus-2;
35) a mutant virus having Q677H mutation at amino acid position 677 of the spike protein of SARS-coronavirus-2;
36) a mutant virus having P681H mutation or P681R mutation at amino acid position 681 of the spike protein of SARS-coronavirus-2;
37) a mutant virus having R685H mutation at amino acid position 685 of the spike protein of SARS-coronavirus-2;
38) a mutant virus having A701V mutation at amino acid position 701 of the spike protein of SARS-coronavirus-2; and
39) a mutant virus having VI 176F mutation at amino acid position 1,176 of the spike protein of SARS-coronavirus-2.

In one embodiment thereof, the neutralizing binding molecule of the present invention has binding affinity and/or neutralizing activity against SARS-coronavirus-2 strains isolated to date, for example, the UNKNOWN-LR757996 strain and the SARS-CoV-2/Hu/DP/Kng/19-027 strain, the isolation date and location of which are unknown; Wuhan-Hu-1 strain isolated in China in December 2019; BetaCoV/Wuhan/IPBCAMS-WH-01/2019 strain first isolated in China on December 23, 2019; BetaCoV/Wuhan/IPBCAMS-WH-02/2019 strain, BetaCoV/Wuhan/IPBCAMS-WH-03/2019 strain, BetaCoV/Wuhan/IPBCAMS-WH-04/2019 strain, WIV02 strain, WIV04 strain, WIV05 strain, WIV06 strain, and WIV07 strain isolated in China on December 30, 2019; 2019-nCoV/Japan/TY/WK-521/2020 strain, 2019-nCoV/Japan/TY/WK-501/2020 strain, 2019-nCoV/Japan/TY/WK-012/2020 strain, and 2019-nCoV/Japan/KY/V-029/2020 strain isolated in Japan in January 2020; SNU01 strain isolated in Korea in January 2020; BetaCoV/Korea/KCDC03/2020 strain isolated in Korea; BetaCoV/Wuhan/IPBCAMS-WH-05/2020 strain isolated in China in January 1, 2020; 2019-nCoV WHU02 strain, and 2019-nCoV WHU01 strain isolated in China on January 2, 2020; SARS-CoV-2/WH-09/human/2020/CHN strain isolated in China in January 8, 2020; 2019-nCoV_HKU-SZ-002a_2020 strain isolated in China on January 10, 2020; 2019-nCoV_HKU-SZ-005b_2020 strain isolated in China on January 11, 2020; SARS-CoV-2/Yunnan-01/human/2020/CHN strain isolated in China on January 17, 2020; 2019-nCoV/USA-WA1/2020 strain isolated in the United States on January 19, 2020; HZ-1 strain isolated in China on January 20, 2020; 2019-nCoV/LTSA-II1/2020 strain isolated in the United States on January 21, 2020; 2019-nCoV/USA-CA2/2020 strain, and 2019-nCoV/USA-AZ1/2020 strain isolated in the United States on January 22, 2020; 2019-nCoV/USA-CA1/2020 strain isolated in the United States on January 23, 2020; Australia/VIC01/2020 strain isolated in Australia on January 25, 2020; 2019-nCoV/USA-WA1-F6/2020 strain, and 2019-nCoV/USA-WA-A12/2020 strain isolated in the United States on January 25, 2020; 2019-nCoV/USA-CA6/2020 strain isolated in the United States on January 27, 2020; 2019-nCoV/USA-IL2/2020 strain isolated in the United States on January 28, 2020; 2019-nCoV/USA-MA1/2020 strain, 2019-nCoV/USA-CA5/2020 strain, 2019-nCoV/USA-CA4/2020 strain, and 2019-nCoV/USA-CA3/2020 strain isolated in the United States on January 29, 2020; nCoV-FIN-29-Jan-2020 strain isolated in Finland on January 29, 2020; SARS-CoV-2/IQTC02/human/2020/CHN strain isolated in China on January 29, 2020; 2019-nCoV/LTSA-WI1/2020 strain isolated in the United States on January 31, 2020; SARS-CoV-2/NTU01/2020/TWN strain isolated in Taiwan on January 31, 2020; SARS-CoV-2/NTU02/2020/TWN strain isolated in Taiwan on February 5, 2020; 2019-nCoV/USA-CA7/2020 strain isolated in the United States on February 6, 2020; SARS-CoV-2/01/human/2020/SWE strain isolated in Sweden on February 7, 2020; 2019-nCoV/USA-CA8/2020 strain isolated in the United States on February 10, 2020; 2019-nCoV/USA-TX1/2020 strain isolated in the United States on February 11, 2020; 2019-nCoV/USA-CA9/2020 strain isolated in the United States on February 23, 2020; SARS-CoV-2/SP02/human/2020/BRA strain isolated in Brazil on February 28, 2020; UNKNOWN-LR757995, UNKNOWN-LR757997, UNKNOWN-LR757998, and SARS-CoV-2/Hu/DP/Kng/19-020, the isolation date and location of which are unknown; 2019-nCoV/Japan/AVI-004/2020 isolated in Japan in January 2020; SARS0CoV-2/61-TW/human/2020/ NPL isolated in Nepal on January 13, 2020; SARS-CoV-2/IQTC01/human/2020/CHN strain isolated in China on February 5, 2020; hCoV-19/South Korea/KUMC17/2020 strain isolated in Korea, and SARS-coronavirus-2 strain to be isolated in the future, without being limited thereto.

In one embodiment, the neutralizing binding molecule of the present invention has binding affinity and/or neutralizing activity against SARS-coronavirus-1 (SARS-CoV-1) strains, for example, SIN2500, SIN2677, SIN2679, SIN2748 and SIN2744 strains isolated in Singapore; icSARS-CoV (SARS-CoV Urbani strain) strain isolated in Hanoi; Urbani v2163 strain; TW-1 strain; TOR-2 strain isolated in Canada; CUHK-W1 and HKU-39849 strains isolated in Hong Kong; and GZ01 strain isolated in Guangzhou; and BJ01, BJ02, BJ03 and BJ04 strains isolated in Beijing, without being limited thereto.

In one embodiment, the neutralizing binding molecule of the present invention has binding affinity and/or neutralizing activity against Middle East respiratory syndrome coronavirus (MERS-CoV) strains, for example, EMC/2012 strain isolated in Saudi Arabia; MERS-HCoV/Jordan/01 strain isolated in Jordan; 2c England-Qatar/2012 strain isolated in England; MERS-CoV/Korea/KNIH/002_05_2015 and KOREA/Seoul/168-2-2015 strains isolated in Korea; Jeddah_C9313/KSA/2014 strain; Florida/USA-2_Saudi Arabia_2014 strain isolated in Saudi Arabia; and icMERS-CoV-T1015N strain, without being limited thereto.

Coronaviruses can cause not only the common cold in humans, but also direct viral bronchitis or secondary bacterial bronchitis. Seven types of human coronavirus have been reported, such as human coronavirus 299E (HCoV-229E), human coronavirus OC43 (HCoV-OC43), human coronavirus NL63 (HCoV-NL63), human coronavirus HKU1 (HCoV-HKU1), Middle East respiratory syndrome coronavirus (MERS-CoV), severe acute respiratory syndrome coronavirus 1 (SARS-CoV-1), and SARS-coronavirus-2 (SARS-CoV-2). Infection with four types (HCoV-229E, HCoV-OC43, HCoV-NL63, and HCoV-HKU1) results in mild upper respiratory tract disease causing symptoms of the common cold, but three types (SARS-CoV-1, MERS-CoV, and SARS-CoV-2) can cause severe and fatal pneumonia. The human coronavirus that first occurred in China in the winter of 2002 is severe acute respiratory syndrome coronavirus 1 (SARS-CoV-1), which causes severe acute respiratory syndrome, and the main symptoms thereof are fever, malaise, muscle pain, headache, chills, etc., and cough and shortness of breath may appear. SARS-CoV-1 causes upper and lower respiratory tract infections. Currently, there is no fundamental treatment method for SARS-CoV-1, and an antibacterial agent against the causative agent of common pneumonia is administered. The main clinical symptoms of Middle East respiratory syndrome coronavirus (MERS-CoV) found in 2012 are fever, cough, and shortness of breath. Most MERS-CoV patients have severe acute respiratory tract disease (pneumonia), but some patients have mild acute upper respiratory tract disease or are asymptomatic. In particular, MERS-Cov has a high infection rate and poor prognosis in people with underlying diseases (diabetes, renal failure, chronic lung disease, or immunodeficiency disease). As of 2020, MERS-CoV outbreaks have been reported in about 27 countries. Currently, a vaccine for preventing MERS-CoV infection has not been developed worldwide, and a therapeutic agent for MERS-CoV has not been developed due to limited information on the disease.

In one embodiment of the present invention, the present invention is directed to binding molecules that are able to bind to and neutralize the coronavirus superfamily against which a preventive or therapeutic agent has not yet been developed, and the binding molecules of the present invention may also have binding affinity and/or neutralizing activity against coronaviruses, including wild-type and mutant SARS-CoV-2, and/or SARS-CoV-1, and/or MERS-CoV.

In one embodiment of the present invention, the binding molecule comprises any one binding molecule among binding molecule Nos. 1 to 190 shown in Table 1 below, or comprises a binding molecule derived therefrom. For example, for each of binding molecule Nos. 1 to 190 shown in Table 1 below, the scope of the present invention includes a binding molecule that comprises all of three light-chain CDR regions (LC CDR1, LC CDR2, and LC CDR3) and three heavy-chain CDR regions (HC CDR1, HC CDR2, and HC CDR3), or comprises a light-chain CDR region comprising at least one CDR region among the three light-chain CDR regions (LC CDR1, LC CDR2, and LC CDR3) and/or a heavy-chain CDR region comprising at least one CDR region among the three heavy-chain CDR regions (HC CDR1, HC CDR2, and HC CDR3), as long as this binding molecule achieves the purposes and effects of the present invention. In Table 1 below, No. represents each binding molecule number.

The "binding molecule that achieves the purposes and effects of the present invention" may be one satisfying the following: the minimum concentration value of the binding molecule that neutralizes 100 TCID50 of virus for SARS-coronavirus-2 (SARS-CoV-2) is preferably 2 µg/ml or less, more preferably 1 µg/ml or less, even more preferably 0.5 µg/ml or less, even more preferably 0.25 µg/ml or less, even more preferably 0.125 µg/ml or less; and/or

the binding molecule is able to bind to a receptor-binding domain (RBD) of the spike protein of SARS-coronavirus-2 (SARS-CoV-2) with an **equilibrium dissociation constant** (K_{D}) of preferably 1.0×10⁻⁸ M or less, more preferably 1.0 × 10⁻⁹ M or less, even more preferably 1.0 × 10⁻¹⁰ M or less; and/or

the binding molecule has an **IC₅₀ value** of preferably 200 ng/ml or less, more preferably 100 ng/ml or less, even more preferably 50 ng/ml or less, even more preferably 25 ng/ml or less, even more preferably 10 ng/ml or less, as evaluated according to a plaque reduction neutralization test (PRNT) method for SARS-coronavirus-2 (SARS-CoV-2), without being limited thereto. Here, **IC₅₀ value** is the antibody concentration at which the antibody exhibits 50% of neutralizing activity against the virus.

In another embodiment of the present invention, the binding molecule comprises any one binding molecule selected from the group consisting of binding molecule Nos. 2, 3, 12, 15, 16, 18, 19, 20, 22, 23, 24, 26, 31, 32, 33, 34, 37, 43, 45, 48, 50, 51, 53, 54, 55, 56, 59, 60, 61, 66, 69, 74, 75, 76, 81, 82, 83, 85, 86, 87, 93, 94, 95, 96, 98, 102, 103, 104 and 108 among the binding molecules shown in Table 1 below, or a binding molecule derived therefrom. That is, for each of the above binding molecule numbers, the scope of the present invention includes a binding molecule that comprises all of three light-chain CDR regions (LC CDR1, LC CDR2, and LC CDR3) and three heavy-chain CDR regions (HC CDR1, HC CDR2, and HC CDR3), or comprises a light-chain CDR region comprising at least one CDR region among the three light-chain CDR regions (LC CDR1, LC CDR2, and LC CDR3) and/or a heavy-chain CDR region comprising at least one CDR region among the three heavy-chain CDR regions (HC CDR1, HC CDR2, and HC CDR3), as long as this binding molecule achieves the purposes and effects of the present invention.

In another embodiment, the binding molecule comprises any one binding molecule selected from the group consisting of binding molecule Nos. 2, 32, 59, 95, 102 and 103 among the binding molecules shown in Table 1 below, or a binding molecule derived therefrom. That is, for each of the above binding molecule numbers, the scope of the present invention includes a binding molecule that comprises all of three light-chain CDR regions (LC CDR1, LC CDR2, and LC CDR3) and three heavy-chain CDR regions (HC CDR1, HC CDR2, and HC CDR3), or comprises a light-chain CDR region comprising at least one CDR region among the three light-chain CDR regions (LC CDR1, LC CDR2, and LC CDR3) and/or a heavy-chain CDR region comprising at least one CDR region among the three heavy-chain CDR regions (HC CDR1, HC CDR2, and HC CDR3), as long as this binding molecule achieves the purposes and effects of the present invention.

In another embodiment, the binding molecule comprises any one binding molecule selected from the group consisting of binding molecule Nos. 32, 33, 34, 37, 45, 48, 53, 54, 59, 61 and 81 among the binding molecules shown in Table 1 below, or a binding molecule derived therefrom. That is, for each of the above binding molecule numbers, the scope of the present invention includes a binding molecule that comprises all of three light-chain CDR regions (LC CDR1, LC CDR2, and LC CDR3) and three heavy-chain CDR regions (HC CDR1, HC CDR2, and HC CDR3), or comprises a light-chain CDR region comprising at least one CDR region among the three light-chain CDR regions (LC CDR1, LC CDR2, and LC CDR3) and/or a heavy-chain CDR region comprising at least one CDR region among the three heavy-chain CDR regions (HC CDR1, HC CDR2, and HC CDR3), as long as this binding molecule achieves the purposes and effects of the present invention.

In another embodiment, the binding molecule comprises any one binding molecule selected from the group consisting of binding molecule Nos. 32, 34, 37, 45 and 54 among the binding molecules shown in Table 1 below, or a binding molecule derived therefrom. That is, for each of the above binding molecule numbers, the scope of the present invention includes a binding molecule that comprises all of three light-chain CDR regions (LC CDR1, LC CDR2, and LC CDR3) and three heavy-chain CDR regions (HC CDR1, HC CDR2, and HC CDR3), or comprises a light-chain CDR region comprising at least one CDR region among the three light-chain CDR regions (LC CDR1, LC CDR2, and LC CDR3) and/or a heavy-chain CDR region comprising at least one CDR region among the three heavy-chain CDR regions (HC CDR1, HC CDR2, and HC CDR3), as long as this binding molecule achieves the purposes and effects of the present invention.

In another embodiment, the binding molecule comprises any one binding molecule selected from the group consisting of binding molecule Nos. 32 and 54 among the binding molecules shown in Table 1 below, or a binding molecule derived therefrom. That is, for each of the above binding molecule numbers, the scope of the present invention includes a binding molecule that comprises all of three light-chain CDR regions (LC CDR1, LC CDR2, and LC CDR3) and three heavy-chain CDR regions (HC CDR1, HC CDR2, and HC CDR3), or comprises a light-chain CDR region comprising at least one CDR region among the three light-chain CDR regions (LC CDR1, LC CDR2, and LC CDR3) and/or a heavy-chain CDR region comprising at least one CDR region among the three heavy-chain CDR regions (HC CDR1, HC CDR2, and HC CDR3), as long as this binding molecule achieves the purposes and effects of the present invention.

In another embodiment, the binding molecule may be the binding molecule of binding molecule No. 32 among the binding molecules shown in Table 1 below, or a binding molecule derived therefrom. That is, for binding molecule No. 32, the scope of the present invention includes a binding molecule that comprises all of three light-chain CDR regions (LC CDR1, LC CDR2, and LC CDR3) and three heavy-chain CDR regions (HC CDR1, HC CDR2, and HC CDR3), or comprises a light-chain CDR region comprising at least one CDR region among the three light-chain CDR regions (LC CDR1, LC CDR2, and LC CDR3) and/or a heavy-chain CDR region comprising at least one CDR region among the three heavy-chain CDR regions (HC CDR1, HC CDR2, and HC CDR3), as long as this binding molecule achieves the purposes and effects of the present invention.

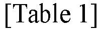

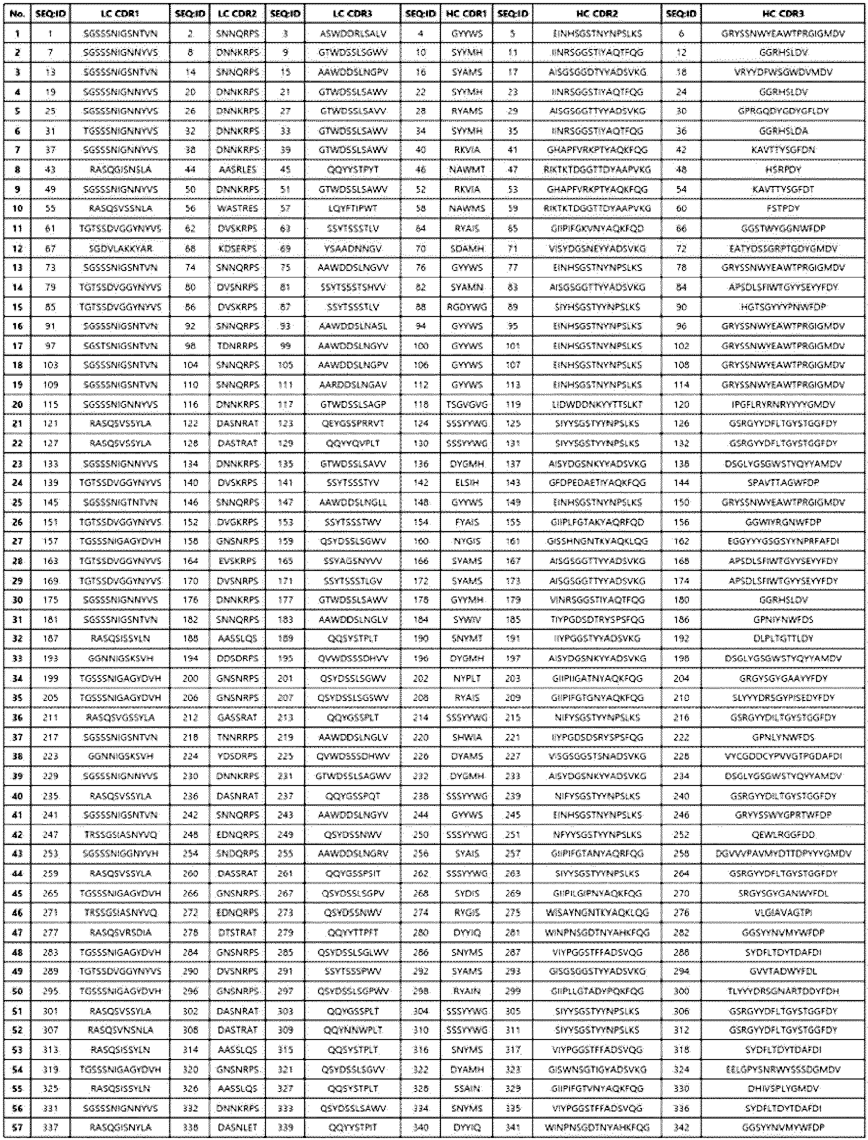

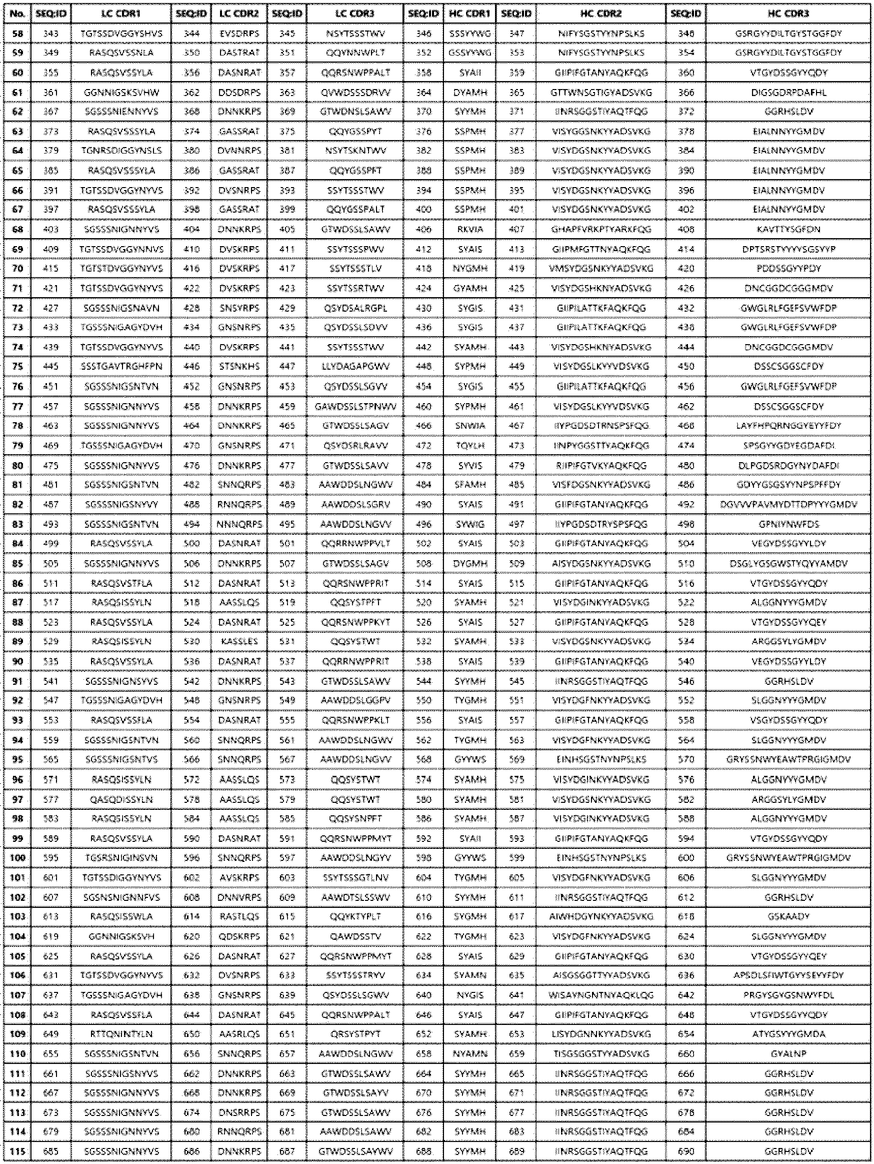

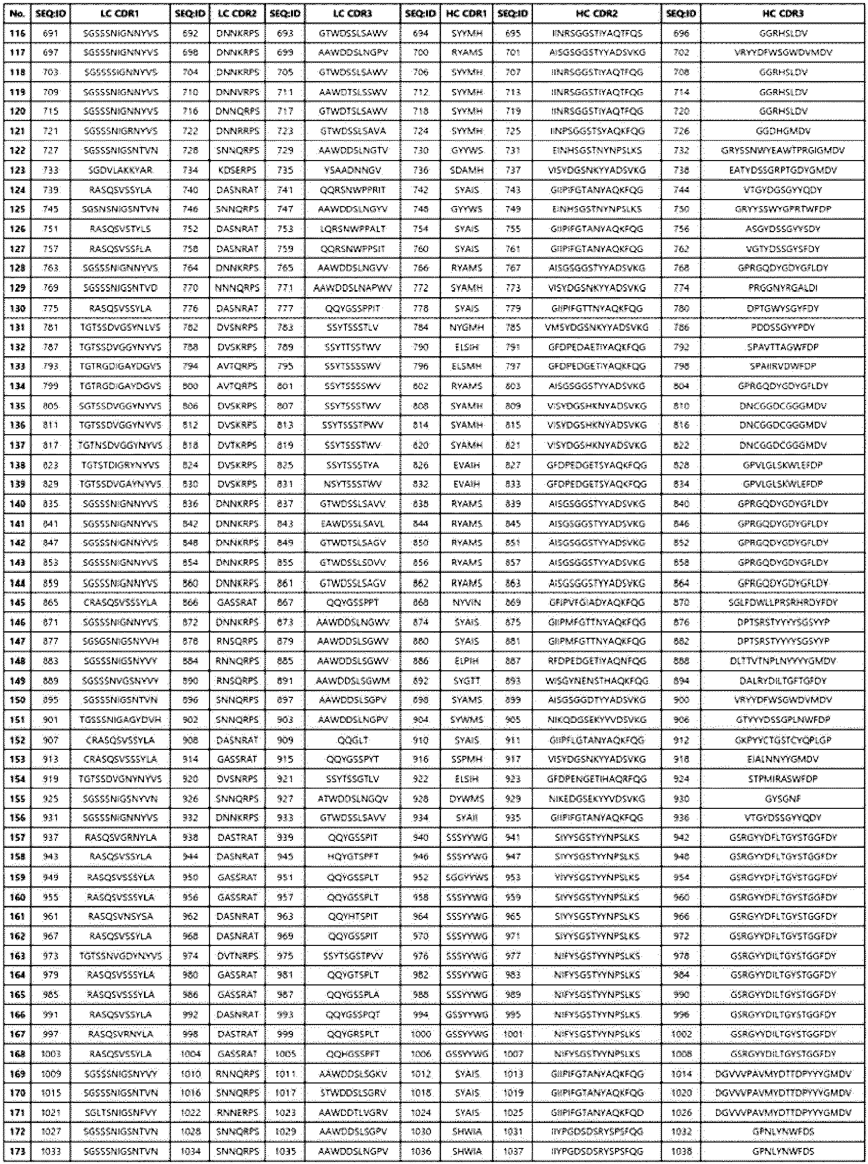

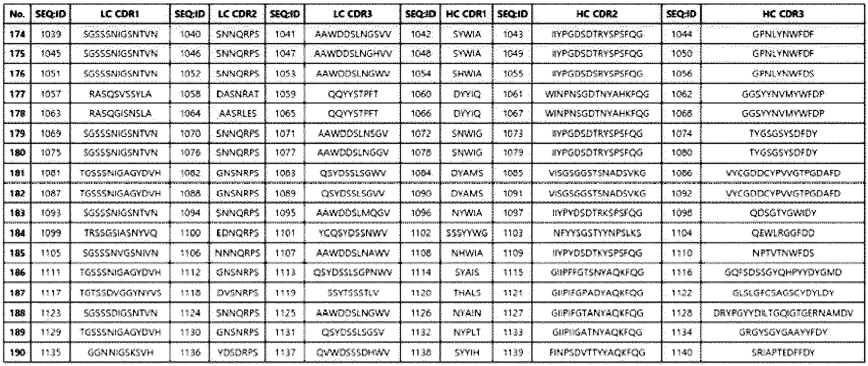

In the present invention, CDRs of variable regions were determined through a conventional method using a system devised by Kabat et al. (see Kabat et al., Sequences of Proteins of Immunological Interest (5th), National Institutes of Health, Bethesda, MD. (1991)). The CDR numbering used in the present invention is determined using the Kabat method, but the present invention also encompasses binding molecules comprising CDRs determined through other methods such as an IMGT method, Chothia method, AbM method, or the like. For example, as shown in Table 2 below, as binding molecules comprising three light-chain CDR regions (LC CDR1, LC CDR2, and LC CDR3) contained in the light-chain (LC) variable region and three heavy-chain CDR regions (HC CDR1, HC CDR2, and HC CDR3) contained in the heavy-chain (HC) variable region, binding molecules comprising CDRs determined through a Kabat method, an IMGT method, a Chothia method and/or an AbM method are included within the scope of the present invention.

In one embodiment of the present invention, the binding molecule comprises any one binding molecule selected from the group consisting of binding molecules Nos. 1 to 190 shown in Table 2 below, or comprises a binding molecule derived therefrom. In Table 2 below, No. represents each binding molecule number.

In another embodiment of the present invention, the binding molecule may be any one selected from the group consisting of binding molecule Nos. 2, 3, 12, 15, 16, 18, 19, 20, 22, 23, 24, 26, 31, 32, 33, 34, 37, 43, 45, 48, 50, 51, 53, 54, 55, 56, 59, 60, 61, 66, 69, 74, 75, 76, 81, 82, 83, 85, 86, 87, 93, 94, 95, 96, 98, 102, 103, 104 and 108 among the binding molecules shown in Table 2 below, or may be a binding molecule comprising a sequence derived therefrom.

In another embodiment of the present invention, the binding molecule may be any one selected from the group consisting of binding molecule Nos. 2, 32, 59, 95, 102 and 103 among the binding molecules shown in Table 2 below, or may be a binding molecule comprising a sequence derived therefrom.

In another embodiment of the present invention, the binding molecule may be any one selected from the group consisting of binding molecule Nos. 32, 33, 34, 37, 45, 48, 53, 54, 59, 61 and 81 among the binding molecules shown in Table 2 below, or may be a binding molecule comprising a sequence derived therefrom.

In another embodiment of the present invention, the binding molecule may be any one selected from the group consisting of binding molecule Nos. 32, 34, 37, 45 and 54 among the binding molecules shown in Table 2 below, or may be a binding molecule comprising a sequence derived therefrom.

In another embodiment of the present invention, the binding molecule may be any one selected from the group consisting of binding molecule Nos. 32 and 54 among the binding molecules shown in Table 2 below, or may be a binding molecule comprising the same.

In another embodiment of the present invention, the binding molecule may be the binding molecule of binding molecule No. 32 among the binding molecules shown in Table 2 below.

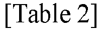

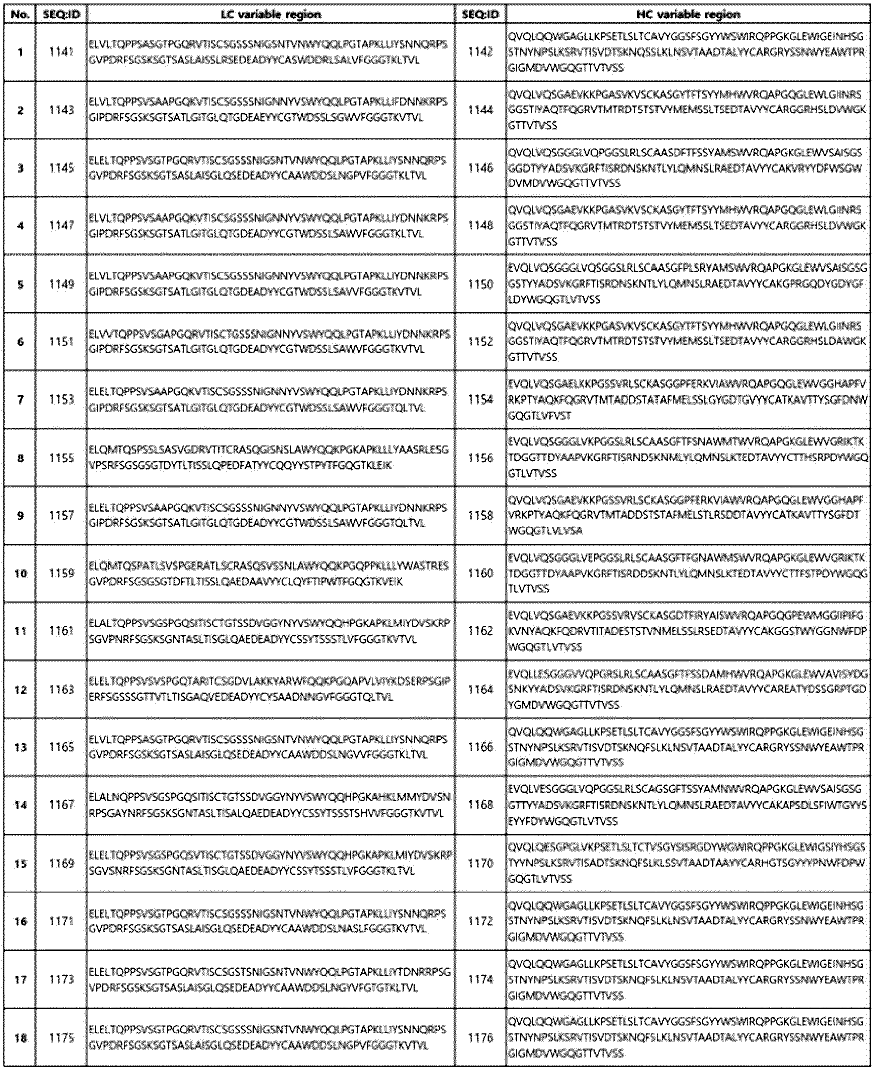

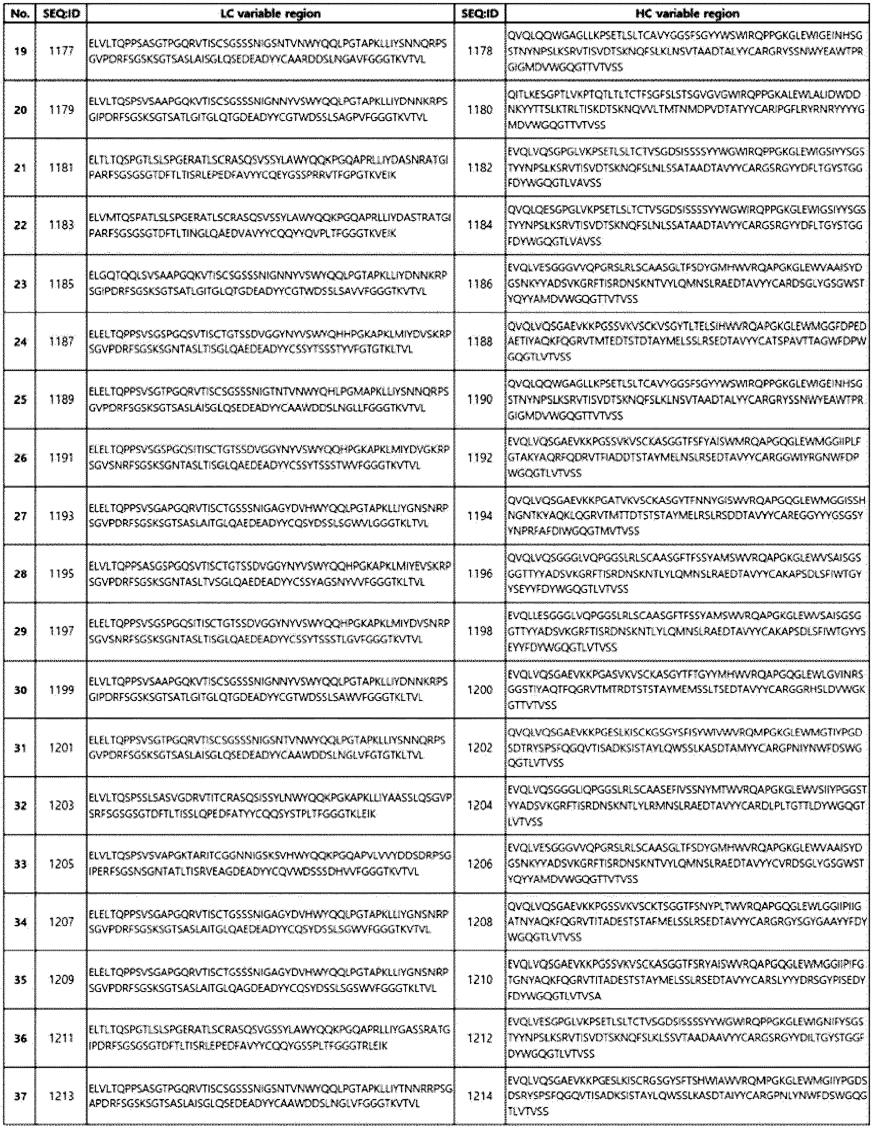

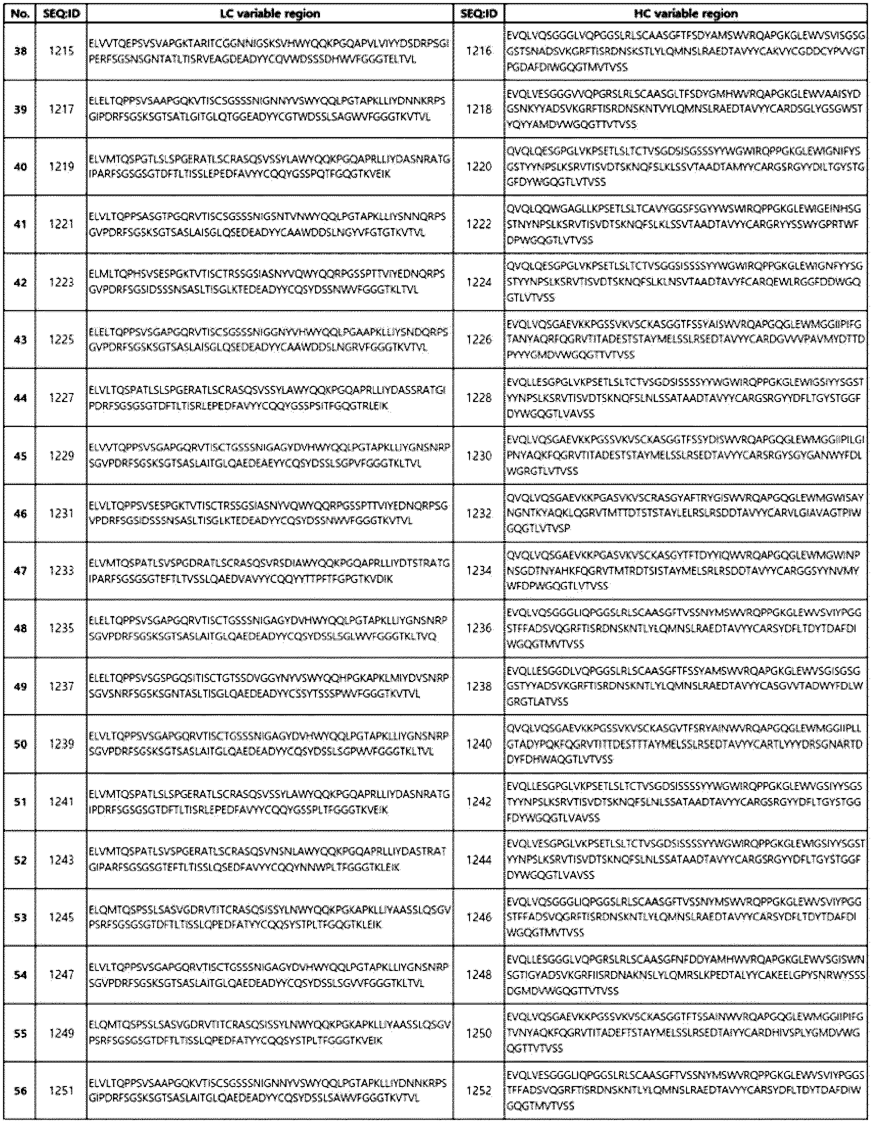

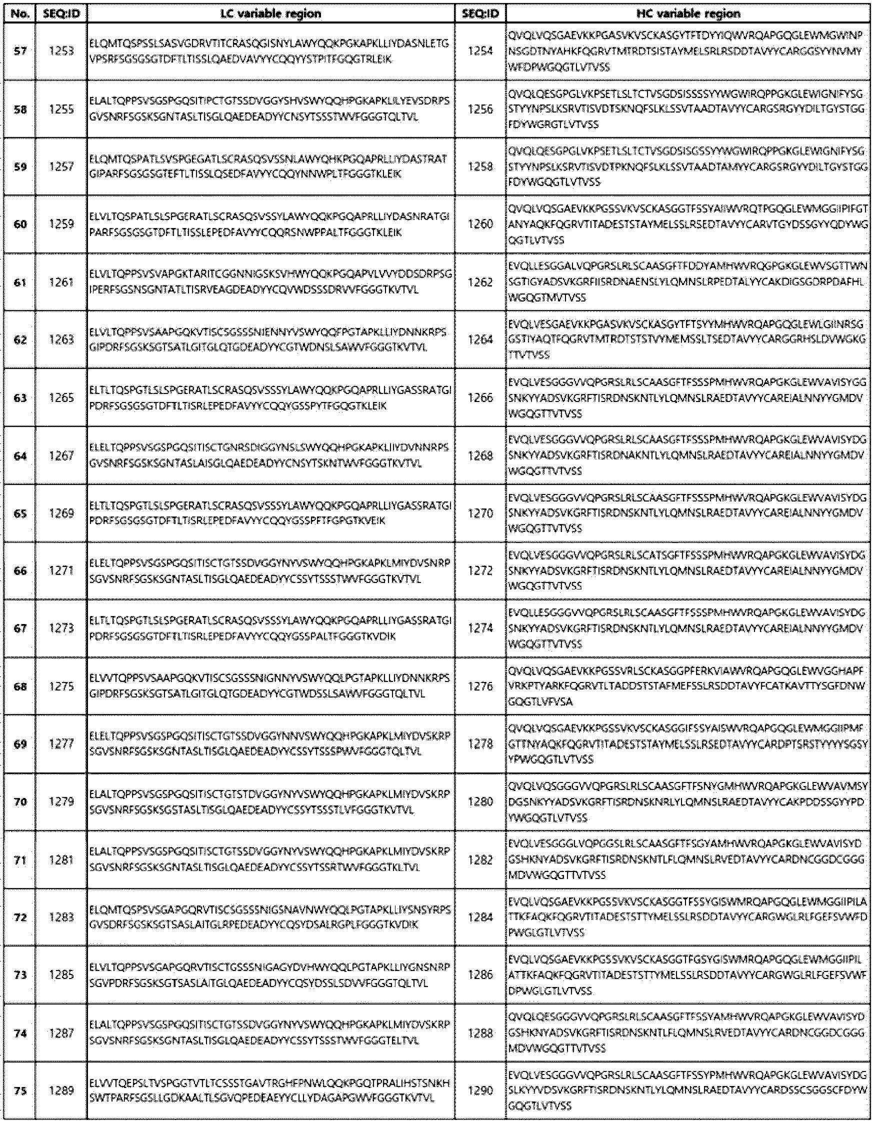

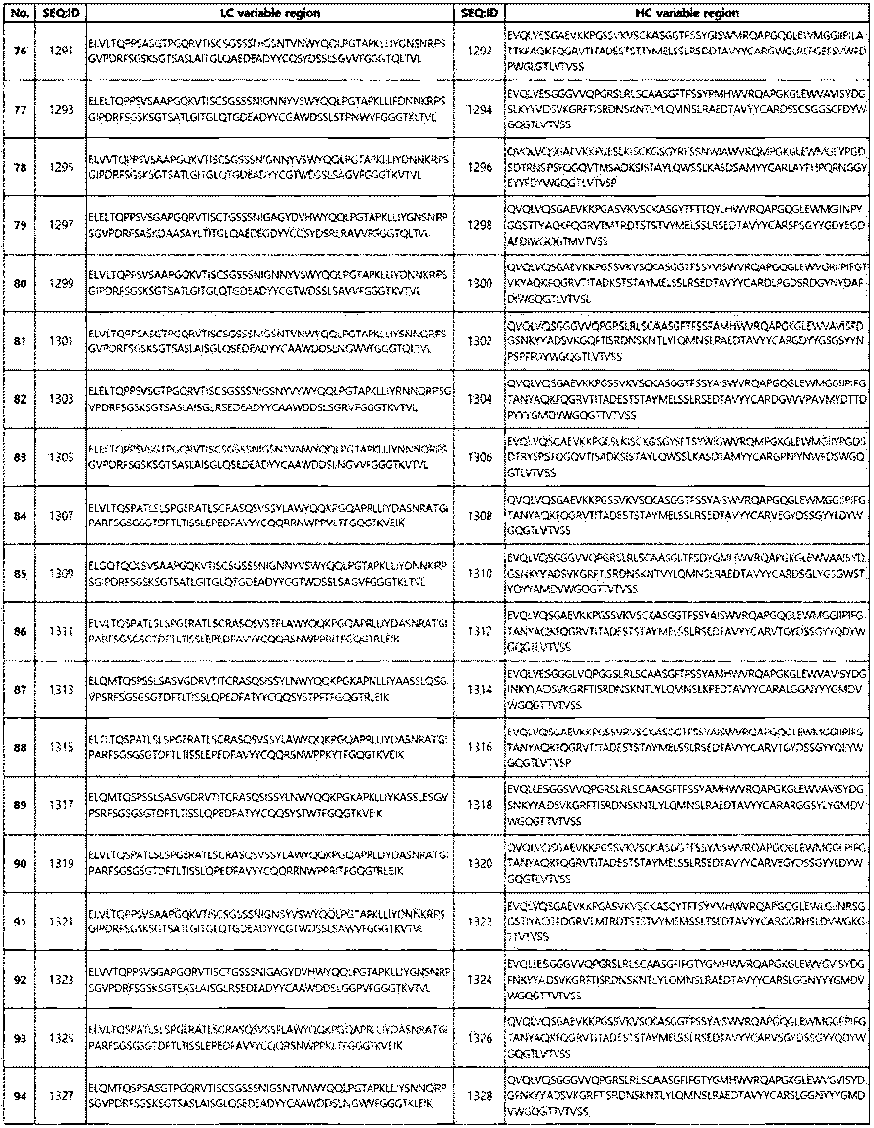

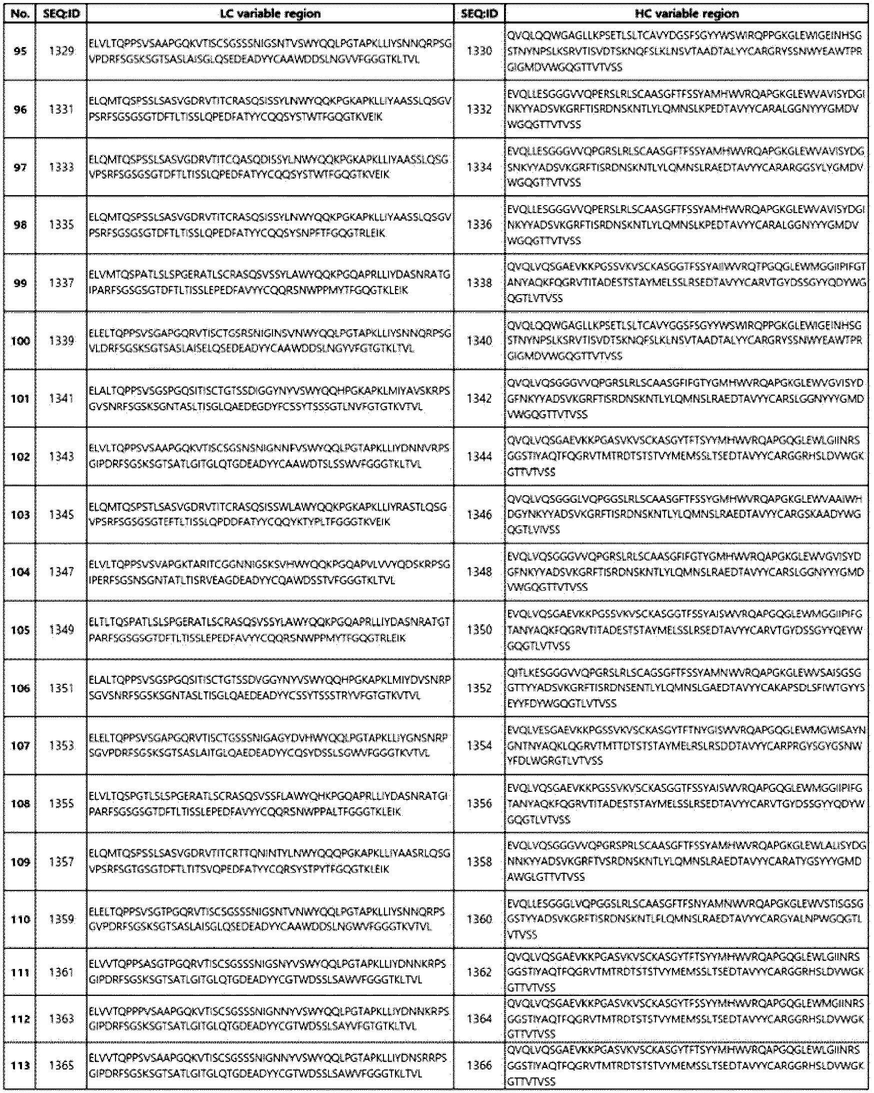

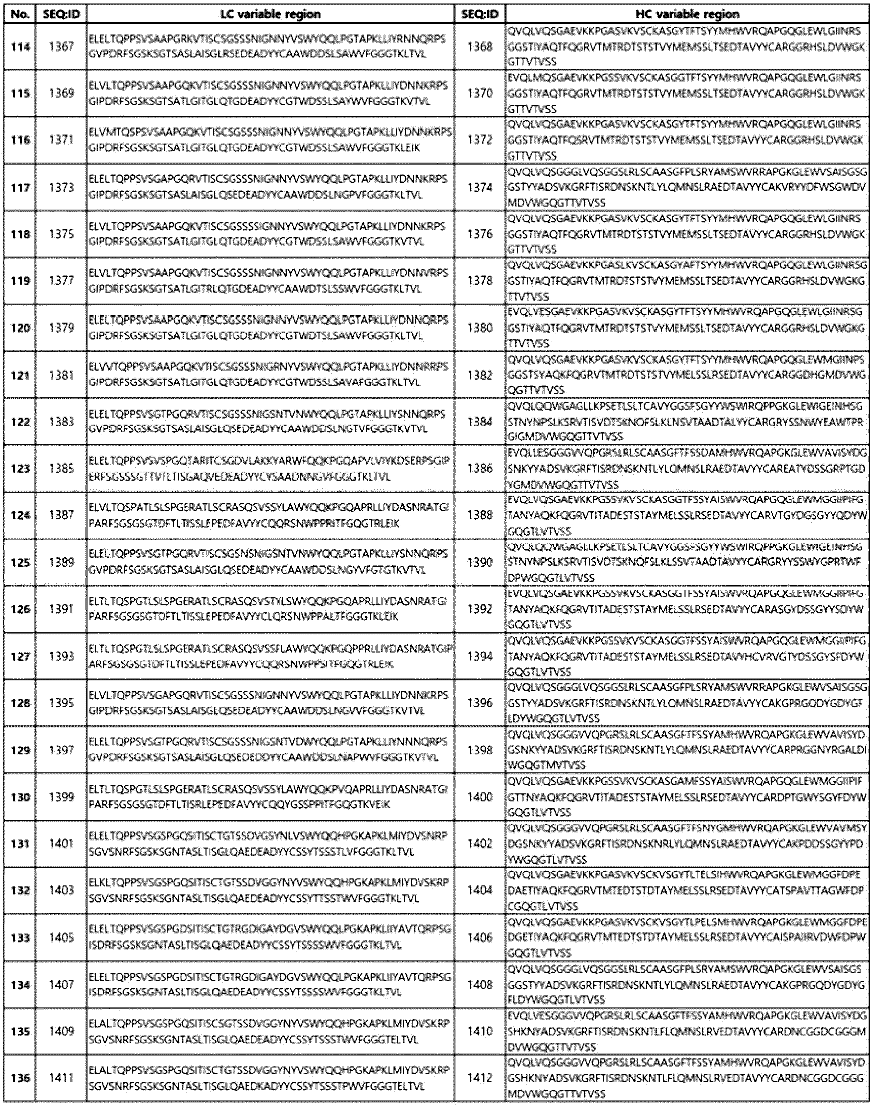

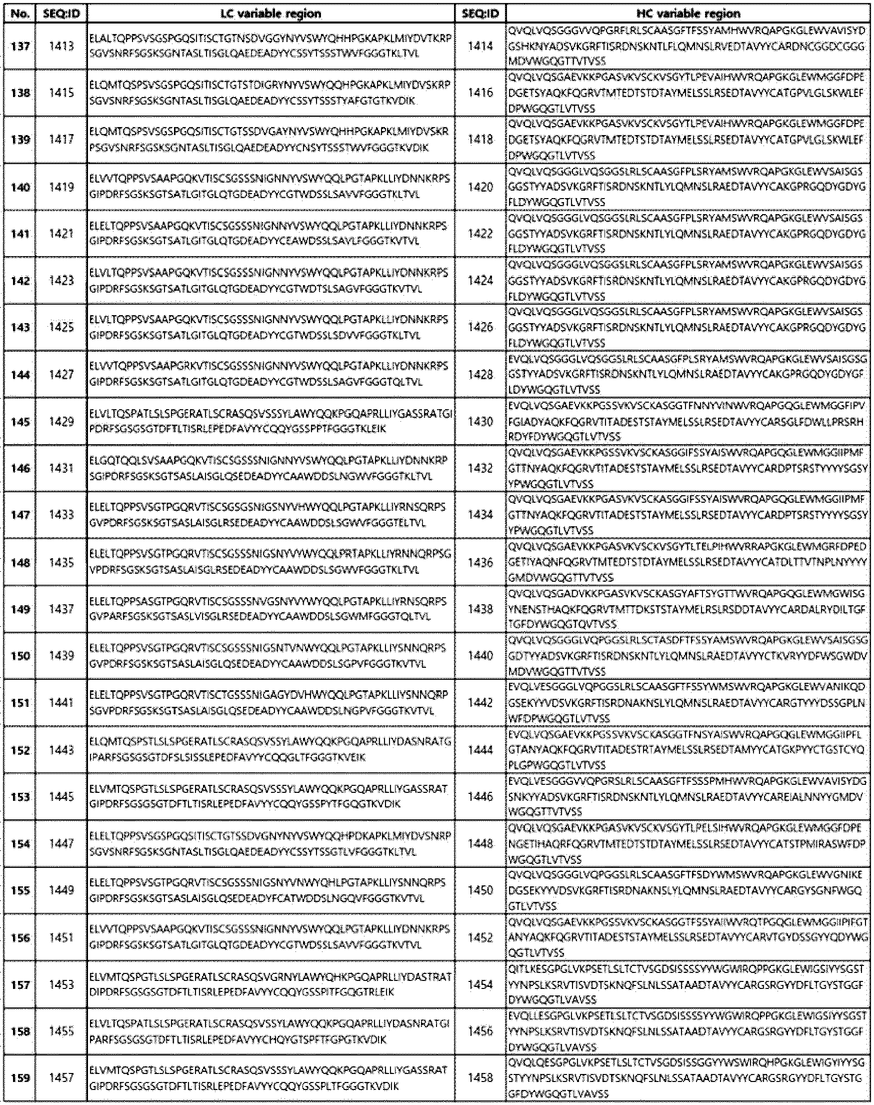

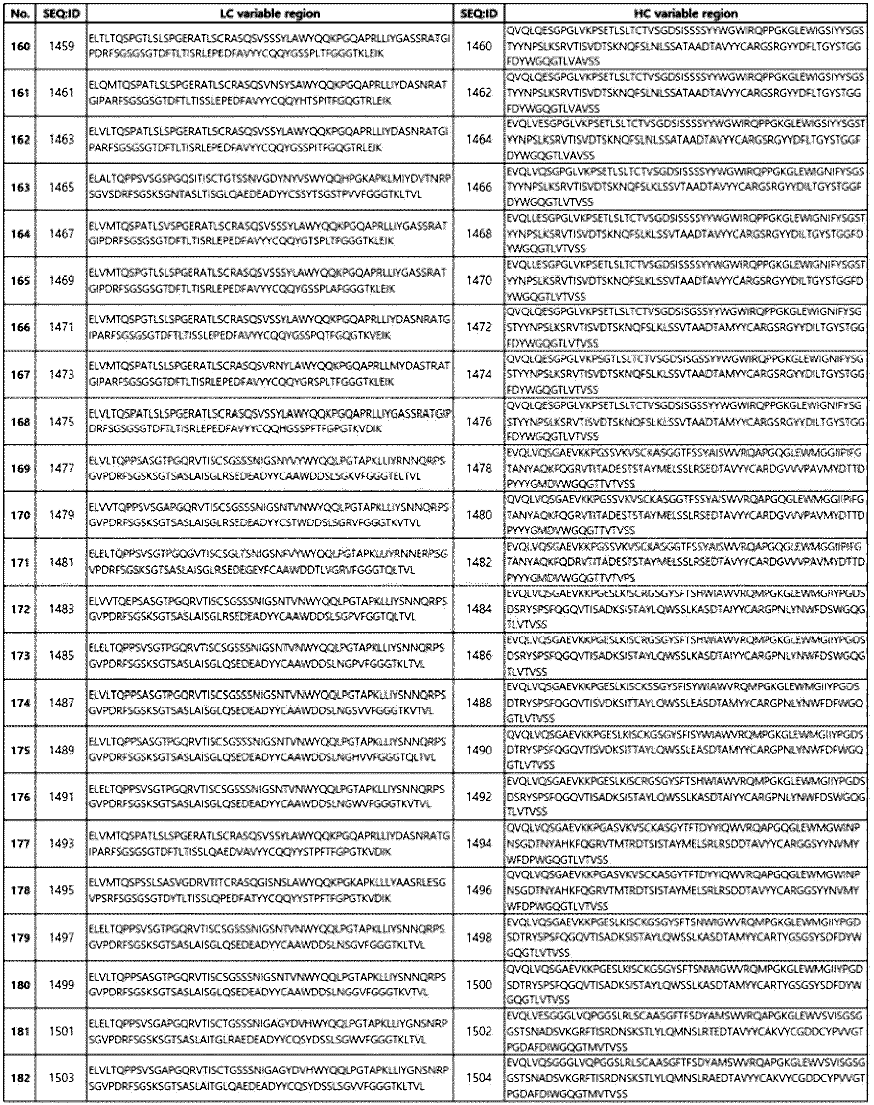

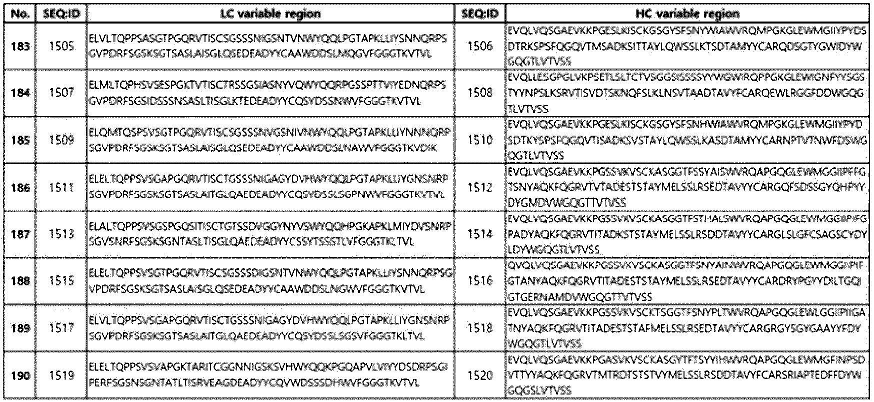

In another embodiment of the present invention, the binding molecule comprises a binding molecule having a sequence identity of 80% to 99%, preferably 85 to 99%, more preferably 90 to 99% to any one binding molecule selected from the group consisting of binding molecule Nos. 1 to 190 shown in Table 2 above, and this binding molecule is included within the scope of the present invention as long as it achieves the purposes and effects of the present invention.

In another embodiment of the present invention, the binding molecule comprises a binding molecule having a sequence identity of 80% to 99%, preferably 85 to 99%, more preferably 90 to 99% to any one binding molecule selected from the group consisting of binding molecule Nos. 2, 3, 12, 15, 16, 18, 19, 20, 22, 23, 24, 26, 31, 32, 33, 34, 37, 43, 45, 48, 50, 51, 53, 54, 55, 56, 59, 60, 61, 66, 69, 74, 75, 76, 81, 82, 83, 85, 86, 87, 93, 94, 95, 96, 98, 102, 103, 104 and 108 shown in Table 2 above, and this binding molecule is included within the scope of the present invention as long as it achieves the purposes and effects of the present invention.

In another embodiment of the present invention, the binding molecule comprises a binding molecule having a sequence identity of 80% to 99%, preferably 85 to 99%, more preferably 90 to 99% to any one binding molecule selected from the group consisting of binding molecule Nos. 2, 32, 59, 95, 102 and 103 shown in Table 2 above, and this binding molecule is included within the scope of the present invention as long as it achieves the purposes and effects of the present invention.

In another embodiment of the present invention, the binding molecule comprises a binding molecule having a sequence identity of 80% to 99%, preferably 85 to 99%, more preferably 90 to 99% to any one binding molecule selected from the group consisting of binding molecule Nos. 32, 33, 34, 37, 45, 48, 53, 54, 59, 61 and 81 shown in Table 2 above, and this binding molecule is included within the scope of the present invention as long as it achieves the purposes and effects of the present invention.

In another embodiment of the present invention, the binding molecule comprises a binding molecule having a sequence identity of 80% to 99%, preferably 85 to 99%, more preferably 90 to 99% to any one binding molecule selected from the group consisting of binding molecule Nos. 32, 34, 37, 45 and 54 shown in Table 2 above, and this binding molecule is included within the scope of the present invention as long as it achieves the purposes and effects of the present invention.

In another embodiment of the present invention, the binding molecule comprises a binding molecule having a sequence identity of 80% to 99%, preferably 85 to 99%, more preferably 90 to 99% to any one binding molecule selected from the group consisting of binding molecule Nos. 32 and 54 shown in Table 2 above, and this binding molecule is included within the scope of the present invention as long as it achieves the purposes and effects of the present invention.

In another embodiment of the present invention, the binding molecule comprises a binding molecule having a sequence identity of 80% to 99%, preferably 85 to 99%, more preferably 90 to 99% to the binding molecule of binding molecule No. 32 shown in Table 2 above, and this binding molecule is included within the scope of the present invention as long as it achieves the purposes and effects of the present invention.

In one embodiment of the present invention, the binding molecule may be a binding molecule comprising three light-chain CDR regions (LC CDR1, LC CDR2, and LC CDR3) contained in a light-chain (LC) variable region set forth in any one selected from the group consisting of SEQ ID NOS: 1141, 1143, 1145, 1147, 1149, 1151, 1153, 1155, 1157, 1159, 1161, 1163, 1165, 1167, 1169, 1171, 1173, 1175, 1177, 1179, 1181, 1183, 1185, 1187, 1189, 1191, 1193, 1195, 1197, 1199, 1201, 1203, 1205, 1207, 1209, 1211, 1213, 1215, 1217, 1219, 1221, 1223, 1225, 1227, 1229, 1231, 1233, 1235, 1237, 1239, 1241, 1243, 1245, 1247, 1249, 1251, 1253, 1255, 1257, 1259, 1261, 1263, 1265, 1267, 1269, 1271, 1273, 1275, 1277, 1279, 1281, 1283, 1285, 1287, 1289, 1291, 1293, 1295, 1297, 1299, 1301, 1303, 1305, 1307, 1309, 1311, 1313, 1315, 1317, 1319, 1321, 1323, 1325, 1327, 1329, 1331, 1333, 1335, 1337, 1339, 1341, 1343, 1345, 1347, 1349, 1351, 1353, 1355, 1357, 1359, 1361, 1363, 1365, 1367, 1369, 1371, 1373, 1375, 1377, 1379, 1381, 1383, 1385, 1387, 1389, 1391, 1393, 1395, 1397, 1399, 1401, 1403, 1405, 1407, 1409, 1411, 1413, 1415, 1417, 1419, 1421, 1423, 1425, 1427, 1429, 1431, 1433, 1435, 1437, 1439, 1441, 1443, 1445, 1447, 1449, 1451, 1453, 1455, 1457, 1459, 1461, 1463, 1465, 1467, 1469, 1471, 1473, 1475, 1477, 1479, 1481, 1483, 1485, 1487, 1489, 1491, 1493, 1495, 1497, 1499, 1501, 1503, 1505, 1507, 1509, 1511, 1513, 1515, 1517, and 1519, and three heavy-chain CDR regions (HC CDR1, HC CDR2, and HC CDR3) contained in a heavy-chain (HC) variable region set forth in any one selected from the group consisting of SEQ ID NOS: 1142, 1144, 1146, 1148, 1150, 1152, 1154, 1156, 1158, 1160, 1162, 1164, 1166, 1168, 1170, 1172, 1174, 1176, 1178, 1180, 1182, 1184, 1186, 1188, 1190, 1192, 1194, 1196, 1198, 1200, 1202, 1204, 1206, 1208, 1210, 1212, 1214, 1216, 1218, 1220, 1222, 1224, 1226, 1228, 1230, 1232, 1234, 1236, 1238, 1240, 1242, 1244, 1246, 1248, 1250, 1252, 1254, 1256, 1258, 1260, 1262, 1264, 1266, 1268, 1270, 1272, 1274, 1276, 1278, 1280, 1282, 1284, 1286, 1288, 1290, 1292, 1294, 1296, 1298, 1300, 1302, 1304, 1306, 1308, 1310, 1312, 1314, 1316, 1318, 1320, 1322, 1324, 1326, 1328, 1330, 1332, 1334, 1336, 1338, 1340, 1342, 1344, 1346, 1348, 1350, 1352, 1354, 1356, 1358, 1360, 1362, 1364, 1366, 1368, 1370, 1372, 1374, 1376, 1378, 1380, 1382, 1384, 1386, 1388, 1390, 1392, 1394, 1396, 1398, 1400, 1402, 1404, 1406, 1408, 1410, 1412, 1414, 1416, 1418, 1420, 1422, 1424, 1426, 1428, 1430, 1432, 1434, 1436, 1438, 1440, 1442, 1444, 1446, 1448, 1450, 1452, 1454, 1456, 1458, 1460, 1462, 1464, 1466, 1468, 1470, 1472, 1474, 1476, 1478, 1480, 1482, 1484, 1486, 1488, 1490, 1492, 1494, 1496, 1498, 1500, 1502, 1504, 1506, 1508, 1510, 1512, 1514, 1516, 1518 and 1520.

In another embodiment of the present invention, the binding molecule may be a binding molecule comprising three light-chain CDR regions (LC CDR1, LC CDR2, and LC CDR3) contained in a light-chain (LC) variable region set forth in any one selected from the group consisting of SEQ ID NOS: 1143, 1145, 1163, 1169, 1171, 1175, 1177, 1179, 1183, 1185, 1187, 1191, 1201, 1203, 1205, 1207, 1213, 1225, 1229, 1235, 1239, 1241, 1245, 1247, 1249, 1251, 1257, 1259, 1261, 1271, 1277, 1287, 1289, 1291, 1301, 1303, 1305, 1309, 1311, 1313, 1325, 1327, 1329, 1331, 1335, 1343, 1345, 1347 and 1355, and three heavy-chain CDR regions (HC CDR1, HC CDR2, and HC CDR3) contained in a heavy-chain (HC) variable region set forth in any one selected from the group consisting of SEQ ID NOS: 1144, 1146, 1164, 1170, 1172, 1176, 1178, 1180, 1184, 1186, 1188, 1192, 1202, 1204, 1206, 1208, 1214, 1226, 1230, 1236, 1240, 1242, 1246, 1248, 1250, 1252, 1258, 1260, 1262, 1272, 1278, 1288, 1290, 1292, 1302, 1304, 1306, 1310, 1312, 1314, 1326, 1328, 1330, 1332, 1336, 1344, 1346, 1348 and 1356.

In another embodiment of the present invention, the binding molecule may be a binding molecule comprising three light-chain CDR regions (LC CDR1, LC CDR2, and LC CDR3) contained in a light-chain (LC) variable region set forth in any one selected from the group consisting of SEQ ID NOS: 1143, 1203, 1257, 1329, 1343 and 1345, and three heavy-chain CDR regions (HC CDR1, HC CDR2, and HC CDR3) contained in a heavy-chain (HC) variable region set forth in any one selected from the group consisting of SEQ ID NOS: 1144, 1204, 1258, 1330, 1344 and 1346.

In another embodiment of the present invention, the binding molecule may be a binding molecule comprising three light-chain CDR regions (LC CDR1, LC CDR2, and LC CDR3) contained in a light-chain (LC) variable region set forth in any one selected from the group consisting of SEQ ID NOS: 1203, 1205, 1207, 1213, 1229, 1235, 1245, 1247, 1257, 1261 and 1301, and three heavy-chain CDR regions (HC CDR1, HC CDR2, and HC CDR3) contained in a heavy-chain (HC) variable region set forth in any one selected from the group consisting of SEQ ID NOS: 1204, 1206, 1208, 1214, 1230, 1236, 1246, 1248, 1258, 1262 and 1302.

In another embodiment of the present invention, the binding molecule may be a binding molecule comprising three light-chain CDR regions (LC CDR1, LC CDR2, and LC CDR3) contained in a light-chain (LC) variable region set forth in any one selected from the group consisting of SEQ ID NOS: 1203, 1207, 1213, 1229 and 1247, and three heavy-chain CDR regions (HC CDR1, HC CDR2, and HC CDR3) contained in a heavy-chain (HC) variable region set forth in any one selected from the group consisting of SEQ ID NOS: 1204, 1208, 1214, 1230 and 1248.

In another embodiment of the present invention, the binding molecule may be a binding molecule comprising three light-chain CDR regions (LC CDR1, LC CDR2, and LC CDR3) contained in a light-chain (LC) variable region set forth in any one selected from the group consisting of SEQ ID NOS: 1203 and 1247, and three heavy-chain CDR regions (HC CDR1, HC CDR2, and HC CDR3) contained in a heavy-chain (HC) variable region set forth in any one selected from the group consisting of SEQ ID NOS: 1204 and 1248.

In another embodiment of the present invention, the binding molecule may be a binding molecule comprising three light-chain CDR regions (LC CDR1, LC CDR2, and LC CDR3) contained in a light-chain (LC) variable region set forth in SEQ ID NO: 1203, and three heavy-chain CDR regions (HC CDR1, HC CDR2, and HC CDR3) contained in a heavy-chain (HC) variable region set forth in SEQ ID NO: 1204.

In one embodiment, the LC CDR1 may comprises any one selected from the group consisting of SEQ ID NOS: 1, 7, 13, 19, 25, 31, 37, 43, 49, 55, 61, 67, 73, 79, 85, 91, 97, 103, 109, 115, 121, 127, 133, 139, 145, 151, 157, 163, 169, 175, 181, 187, 193, 199, 205, 211, 217, 223, 229, 235, 241, 247, 253, 259, 265, 271, 277, 283, 289, 295, 301, 307, 313, 319, 325, 331, 337, 343, 349, 355, 361, 367, 373, 379, 385, 391, 397, 403, 409, 415, 421, 427, 433, 439, 445, 451, 457, 463, 469, 475, 481, 487, 493, 499, 505, 511, 517, 523, 529, 535, 541, 547, 553, 559, 565, 571, 577, 583, 589, 595, 601, 607, 613, 619, 625, 631, 637, 643, 649, 655, 661, 667, 673, 679, 685, 691, 697, 703, 709, 715, 721, 727, 733, 739, 745, 751, 757, 763, 769, 775, 781, 787, 793, 799, 805, 811, 817, 823, 829, 835, 841, 847, 853, 859, 865, 871, 877, 883, 889, 895, 901, 907, 913, 919, 925, 931, 937, 943, 949, 955, 961, 967, 973, 979, 985, 991, 997, 1003, 1009, 1015, 1021, 1027, 1033, 1039, 1045, 1051, 1057, 1063, 1069, 1075, 1081, 1087, 1093, 1099, 1105, 1111, 1117, 1123, 1129 and 1135, or a sequence derived therefrom, or
comprise any one selected from the group consisting of SEQ ID NOS: 7, 13, 67, 85, 91, 103, 109, 115, 127, 133, 139, 151, 181, 187, 193, 199, 217, 253, 265, 283, 295, 301, 313, 319, 325, 331, 349, 355, 361, 391, 409, 439, 445, 451, 481, 487, 493, 505, 511, 517, 553, 559, 565, 571, 583, 607, 613, 619 and 643, or a sequence derived therefrom, or
comprise any one selected from the group consisting of SEQ ID NOS: 7, 187, 349, 565, 607 and 613, or a sequence derived therefrom, or
comprise any one selected from the group consisting of SEQ ID NOS: 187, 193, 199, 217, 265, 283, 313, 319, 349, 361 and 481, or a sequence derived therefrom, or
comprise any one selected from the group consisting of SEQ ID NOS: 187, 199, 217, 265 and 319, or a sequence derived therefrom, or
comprise any one selected from the group consisting of SEQ ID NOS: 187 and 319, or a sequence derived therefrom.

The LC CDR2 may comprise any one selected from the group consisting of SEQ ID NOS: 2, 8, 14, 20, 26, 32, 38, 44, 50, 56, 62, 68, 74, 80, 86, 92, 98, 104, 110, 116, 122, 128, 134, 140, 146, 152, 158, 164, 170, 176, 182, 188, 194, 200, 206, 212, 218, 224, 230, 236, 242, 248, 254, 260, 266, 272, 278, 284, 290, 296, 302, 308, 314, 320, 326, 332, 338, 344, 350, 356, 362, 368, 374, 380, 386, 392, 398, 404, 410, 416, 422, 428, 434, 440, 446, 452, 458, 464, 470, 476, 482, 488, 494, 500, 506, 512, 518, 524, 530, 536, 542, 548, 554, 560, 566, 572, 578, 584, 590, 596, 602, 608, 614, 620, 626, 632, 638, 644, 650, 656, 662, 668, 674, 680, 686, 692, 698, 704, 710, 716, 722, 728, 734, 740, 746, 752, 758, 764, 770, 776, 782, 788, 794, 800, 806, 812, 818, 824, 830, 836, 842, 848, 854, 860, 866, 872, 878, 884, 890, 896, 902, 908, 914, 920, 926, 932, 938, 944, 950, 956, 962, 968, 974, 980, 986, 992, 998, 1004, 1010, 1016, 1022, 1028, 1034, 1040, 1046, 1052, 1058, 1064, 1070, 1076, 1082, 1088, 1094, 1100, 1106, 1112, 1118, 1124, 1130 and 1136, or a sequence derived therefrom, or
comprise any one selected from the group consisting of SEQ ID NOS: 8, 14, 68, 86, 92, 104, 110, 116, 128, 134, 140, 152, 182, 188, 194, 200, 218, 254, 266, 284, 296, 302, 314, 320, 326, 332, 350, 356, 362, 392, 410, 440, 446, 452, 482, 488, 494, 506, 512, 518, 554, 560, 566, 572, 584, 608, 614, 620 and 644, or a sequence derived therefrom, or
comprise any one selected from the group consisting of SEQ ID NOS: 8, 188, 350, 566, 608 and 614, or a sequence derived therefrom, or
comprise any one selected from the group consisting of SEQ ID NOS: 188, 194, 200, 218, 266, 284, 314, 320, 350, 362 and 482, or a sequence derived therefrom, or
comprise any one selected from the group consisting of SEQ ID NOS: 188, 200, 218, 266 and 320, or a sequence derived therefrom, or
comprise any one selected from the group consisting of SEQ ID NOS: 188 and 320, or a sequence derived therefrom.

The LC CDR3 may comprise any one selected from the group consisting of SEQ ID NOS: 3, 9, 15, 21, 27, 33, 39, 45, 51, 57, 63, 69, 75, 81, 87, 93, 99, 105, 111, 117, 123, 129, 135, 141, 147, 153, 159, 165, 171, 177, 183, 189, 195, 201, 207, 213, 219, 225, 231, 237, 243, 249, 255, 261, 267, 273, 279, 285, 291, 297, 303, 309, 315, 321, 327, 333, 339, 345, 351, 357, 363, 369, 375, 381, 387, 393, 399, 405, 411, 417, 423, 429, 435, 441, 447, 453, 459, 465, 471, 477, 483, 489, 495, 501, 507, 513, 519, 525, 531, 537, 543, 549, 555, 561, 567, 573, 579, 585, 591, 597, 603, 609, 615, 621, 627, 633, 639, 645, 651, 657, 663, 669, 675, 681, 687, 693, 699, 705, 711, 717, 723, 729, 735, 741, 747, 753, 759, 765, 771, 777, 783, 789, 795, 801, 807, 813, 819, 825, 831, 837, 843, 849, 855, 861, 867, 873, 879, 885, 891, 897, 903, 909, 915, 921, 927, 933, 939, 945, 951, 957, 963, 969, 975, 981, 987, 993, 999, 1005, 1011, 1017, 1023, 1029, 1035, 1041, 1047, 1053, 1059, 1065, 1071, 1077, 1083, 1089, 1095, 1101, 1107, 1113, 1119, 1125, 1131 and 1137, or a sequence derived therefrom, or
comprise any one selected from the group consisting of SEQ ID NOS: 9, 15, 69, 87, 93, 105, 111, 117, 129, 135, 141, 153, 183, 189, 195, 201, 219, 255, 267, 285, 297, 303, 315, 321, 327, 333, 351, 357, 363, 393, 411, 441, 447, 453, 483, 489, 495, 507, 513, 519, 555, 561, 567, 573, 585, 609, 615, 621 and 645, or a sequence derived therefrom, or
comprise any one selected from the group consisting of SEQ ID NOS: 9, 189, 351, 567, 609 and 615, or a sequence derived therefrom, or
comprise any one selected from the group consisting of SEQ ID NOS: 189, 195, 201, 219, 267, 285, 315, 321, 351, 363 and 483, or a sequence derived therefrom, or
comprise any one selected from the group consisting of SEQ ID NOS: 189, 201, 219, 267 and 321, or a sequence derived therefrom, or
comprise any one selected from the group consisting of SEQ ID NOS: 189 and 321, or a sequence derived therefrom.

The HC CDR1 may comprise any one selected from the group consisting of SEQ ID NOS: 4, 10, 16, 22, 28, 34, 40, 46, 52, 58, 64, 70, 76, 82, 88, 94, 100, 106, 112, 118, 124, 130, 136, 142, 148, 154, 160, 166, 172, 178, 184, 190, 196, 202, 208, 214, 220, 226, 232, 238, 244, 250, 256, 262, 268, 274, 280, 286, 292, 298, 304, 310, 316, 322, 328, 334, 340, 346, 352, 358, 364, 370, 376, 382, 388, 394, 400, 406, 412, 418, 424, 430, 436, 442, 448, 454, 460, 466, 472, 478, 484, 490, 496, 502, 508, 514, 520, 526, 532, 538, 544, 550, 556, 562, 568, 574, 580, 586, 592, 598, 604, 610, 616, 622, 628, 634, 640, 646, 652, 658, 664, 670, 676, 682, 688, 694, 700, 706, 712, 718, 724, 730, 736, 742, 748, 754, 760, 766, 772, 778, 784, 790, 796, 802, 808, 814, 820, 826, 832, 838, 844, 850, 856, 862, 868, 874, 880, 886, 892, 898, 904, 910, 916, 922, 928, 934, 940, 946, 952, 958, 964, 970, 976, 982, 988, 994, 1000, 1006, 1012, 1018, 1024, 1030, 1036, 1042, 1048, 1054, 1060, 1066, 1072, 1078, 1084, 1090, 1096, 1102, 1108, 1114, 1120, 1126, 1132 and 1138, or a sequence derived therefrom, or
comprise any one selected from the group consisting of SEQ ID NOS: 10, 16, 70, 88, 94, 106, 112, 118, 130, 136, 142, 154, 184, 190, 196, 202, 220, 256, 268, 286, 298, 304, 316, 322, 328, 334, 352, 358, 364, 394, 412, 442, 448, 454, 484, 490, 496, 508, 514, 520, 556, 562, 568, 574, 586, 610, 616, 622 and 646, or a sequence derived therefrom, or
comprise any one selected from the group consisting of SEQ ID NOS: 10, 190, 352, 568, 610 and 616, or a sequence derived therefrom, or
comprise any one selected from the group consisting of SEQ ID NOS: 190, 196, 202, 220, 268, 286, 316, 322, 352, 364 and 484, or a sequence derived therefrom, or
comprise any one selected from the group consisting of SEQ ID NOS: 190, 202, 220, 268 and 322, or a sequence derived therefrom, or
comprise any one selected from the group consisting of SEQ ID NOS: 190 and 322, or a sequence derived therefrom.

The HC CDR2 may any one selected from the group consisting of SEQ ID NOS: 5, 11, 17, 23, 29, 35, 41, 47, 53, 59, 65, 71, 77, 83, 89, 95, 101, 107, 113, 119, 125, 131, 137, 143, 149, 155, 161, 167, 173, 179, 185, 191, 197, 203, 209, 215, 221, 227, 233, 239, 245, 251, 257, 263, 269, 275, 281, 287, 293, 299, 305, 311, 317, 323, 329, 335, 341, 347, 353, 359, 365, 371, 377, 383, 389, 395, 401, 407, 413, 419, 425, 431, 437, 443, 449, 455, 461, 467, 473, 479, 485, 491, 497, 503, 509, 515, 521, 527, 533, 539, 545, 551, 557, 563, 569, 575, 581, 587, 593, 599, 605, 611, 617, 623, 629, 635, 641, 647, 653, 659, 665, 671, 677, 683, 689, 695, 701, 707, 713, 719, 725, 731, 737, 743, 749, 755, 761, 767, 773, 779, 785, 791, 797, 803, 809, 815, 821, 827, 833, 839, 845, 851, 857, 863, 869, 875, 881, 887, 893, 899, 905, 911, 917, 923, 929, 935, 941, 947, 953, 959, 965, 971, 977, 983, 989, 995, 1001, 1007, 1013, 1019, 1025, 1031, 1037, 1043, 1049, 1055, 1061, 1067, 1073, 1079, 1085, 1091, 1097, 1103, 1109, 1115, 1121, 1127, 1133 and 1139, or a sequence derived therefrom, or
comprise any one selected from the group consisting of SEQ ID NOS: 11, 17, 71, 89, 95, 107, 113, 119, 131, 137, 143, 155, 185, 191, 197, 203, 221, 257, 269, 287, 299, 305, 317, 323, 329, 335, 353, 359, 365, 395, 413, 443, 449, 455, 485, 491, 497, 509, 515, 521, 557, 563, 569, 575, 587, 611, 617, 623 and 647, or a sequence derived therefrom, or
comprise any one selected from the group consisting of SEQ ID NOS: 11, 191, 353, 569, 611 and 617, or a sequence derived therefrom, or
comprise any one selected from the group consisting of SEQ ID NOS: 191, 197, 203, 221, 269, 287, 317, 323, 353, 365 and 485, or a sequence derived therefrom, or
comprise any one selected from the group consisting of SEQ ID NOS: 191, 203, 221, 269 and 323, or a sequence derived therefrom, or
comprise any one selected from the group consisting of SEQ ID NOS: 191 and 323, or a sequence derived therefrom.

The HC CDR3 may comprise any one selected from the group consisting of SEQ ID NOS: 6, 12, 18, 24, 30, 36, 42, 48, 54, 60, 66, 72, 78, 84, 90, 96, 102, 108, 114, 120, 126, 132, 138, 144, 150, 156, 162, 168, 174, 180, 186, 192, 198, 204, 210, 216, 222, 228, 234, 240, 246, 252, 258, 264, 270, 276, 282, 288, 294, 300, 306, 312, 318, 324, 330, 336, 342, 348, 354, 360, 366, 372, 378, 384, 390, 396, 402, 408, 414, 420, 426, 432, 438, 444, 450, 456, 462, 468, 474, 480, 486, 492, 498, 504, 510, 516, 522, 528, 534, 540, 546, 552, 558, 564, 570, 576, 582, 588, 594, 600, 606, 612, 618, 624, 630, 636, 642, 648, 654, 660, 666, 672, 678, 684, 690, 696, 702, 708, 714, 720, 726, 732, 738, 744, 750, 756, 762, 768, 774, 780, 786, 792, 798, 804, 810, 816, 822, 828, 834, 840, 846, 852, 858, 864, 870, 876, 882, 888, 894, 900, 906, 912, 918, 924, 930, 936, 942, 948, 954, 960, 966, 972, 978, 984, 990, 996, 1002, 1008, 1014, 1020, 1026, 1032, 1038, 1044, 1050, 1056, 1062, 1068, 1074, 1080, 1086, 1092, 1098, 1104, 1110, 1116, 1122, 1128, 1134 and 1140, or a sequence derived therefrom, or
comprise any one selected from the group consisting of SEQ ID NOS: 12, 18, 72, 90, 96, 108, 114, 120, 132, 138, 144, 156, 186, 192, 198, 204, 222, 258, 270, 288, 300, 306, 318, 324, 330, 336, 354, 360, 366, 396, 414, 444, 450, 456, 486, 492, 498, 510, 516, 522, 558, 564, 570, 576, 588, 612, 618, 624 and 648, or a sequence derived therefrom, or
comprise any one selected from the group consisting of SEQ ID NOS: 12, 192, 354, 570, 612 and 618, or a sequence derived therefrom, or
comprise any one selected from the group consisting of SEQ ID NOS: 192, 198, 204, 222, 270, 288, 318, 324, 354, 366 and 486, or a sequence derived therefrom, or
comprise any one selected from the group consisting of SEQ ID NOS: 192, 204, 222, 270 and 324, or a sequence derived therefrom, or
comprise any one selected from the group consisting of SEQ ID NOS: 192 and 324, or a sequence derived therefrom.

In one embodiment of the present invention, the binding molecule is a binding molecule that binds to a spike protein (S protein) on the surface of SARS-coronavirus-2 (SARS-CoV-2), and comprises a binding molecule that competes with any one binding molecule selected from the group consisting of the following binding molecules 1) to 190), and this binding molecule is included within the scope of the present invention as long as it achieves the purposes and effects of the present invention:
1) a binding molecule comprising a) a light-chain variable region comprising a CDR1 region of SEQ ID NO: 1, a CDR2 region of SEQ ID NO: 2, and a CDR3 region of SEQ ID NO: 3, and b) a heavy-chain variable region comprising a CDR1 region of SEQ ID NO: 4, a CDR2 region of SEQ ID NO: 5, and a CDR3 region of SEQ ID NO: 6;
2) a binding molecule comprising a) a light-chain variable region comprising a CDR1 region of SEQ ID NO: 7, a CDR2 region of SEQ ID NO: 8, and a CDR3 region of SEQ ID NO: 9, and b) a heavy-chain variable region comprising a CDR1 region of SEQ ID NO: 10, a CDR2 region of SEQ ID NO: 11, and a CDR3 region of SEQ ID NO: 12;
3) a binding molecule comprising a) a light-chain variable region comprising a CDR1 region of SEQ ID NO: 13, a CDR2 region of SEQ ID NO: 14, and a CDR3 region of SEQ ID NO: 15, and b) a heavy-chain variable region comprising a CDR1 region of SEQ ID NO: 16, a CDR2 region of SEQ ID NO: 17, and a CDR3 region of SEQ ID NO: 18;
4) a binding molecule comprising a) a light-chain variable region comprising a CDR1 region of SEQ ID NO: 19, a CDR2 region of SEQ ID NO: 20, and a CDR3 region of SEQ ID NO: 21, and b) a heavy-chain variable region comprising a CDR1 region of SEQ ID NO: 22, a CDR2 region of SEQ ID NO: 23, and a CDR3 region of SEQ ID NO: 24;
5) a binding molecule comprising a) a light-chain variable region comprising a CDR1 region of SEQ ID NO: 25, a CDR2 region of SEQ ID NO: 26, and a CDR3 region of SEQ ID NO: 27, and b) a heavy-chain variable region comprising a CDR1 region of SEQ ID NO: 28, a CDR2 region of SEQ ID NO: 29, and a CDR3 region of SEQ ID NO: 30;
6) a binding molecule comprising a) a light-chain variable region comprising a CDR1 region of SEQ ID NO: 31, a CDR2 region of SEQ ID NO: 32, and a CDR3 region of SEQ ID NO: 33, and b) a heavy-chain variable region comprising a CDR1 region of SEQ ID NO: 34, a CDR2 region of SEQ ID NO: 35, and a CDR3 region of SEQ ID NO: 36;
7) a binding molecule comprising a) a light-chain variable region comprising a CDR1 region of SEQ ID NO: 37, a CDR2 region of SEQ ID NO: 38, and a CDR3 region of SEQ ID NO: 39, and b) a heavy-chain variable region comprising a CDR1 region of SEQ ID NO: 40, a CDR2 region of SEQ ID NO: 41, and a CDR3 region of SEQ ID NO: 42;
8) a binding molecule comprising a) a light-chain variable region comprising a CDR1 region of SEQ ID NO: 43, a CDR2 region of SEQ ID NO: 44, and a CDR3 region of SEQ ID NO: 45, and b) a heavy-chain variable region comprising a CDR1 region of SEQ ID NO: 46, a CDR2 region of SEQ ID NO: 47, and a CDR3 region of SEQ ID NO: 48;
9) a binding molecule comprising a) a light-chain variable region comprising a CDR1 region of SEQ ID NO: 49, a CDR2 region of SEQ ID NO: 50, and a CDR3 region of SEQ ID NO: 51, and b) a heavy-chain variable region comprising a CDR1 region of SEQ ID NO: 52, a CDR2 region of SEQ ID NO: 53, and a CDR3 region of SEQ ID NO: 54;
10) a binding molecule comprising a) a light-chain variable region comprising a CDR1 region of SEQ ID NO: 55, a CDR2 region of SEQ ID NO: 56, and a CDR3 region of SEQ ID NO: 57, and b) a heavy-chain variable region comprising a CDR1 region of SEQ ID NO: 58, a CDR2 region of SEQ ID NO: 59, and a CDR3 region of SEQ ID NO: 60;
11) a binding molecule comprising a) a light-chain variable region comprising a CDR1 region of SEQ ID NO: 61, a CDR2 region of SEQ ID NO: 62, and a CDR3 region of SEQ ID NO: 63, and b) a heavy-chain variable region comprising a CDR1 region of SEQ ID NO: 64, a CDR2 region of SEQ ID NO: 65, and a CDR3 region of SEQ ID NO: 66;
12) a binding molecule comprising a) a light-chain variable region comprising a CDR1 region of SEQ ID NO: 67, a CDR2 region of SEQ ID NO: 68, and a CDR3 region of SEQ ID NO: 69, and b) a heavy-chain variable region comprising a CDR1 region of SEQ ID NO: 70, a CDR2 region of SEQ ID NO: 71, and a CDR3 region of SEQ ID NO: 72;
13) a binding molecule comprising a) a light-chain variable region comprising a CDR1 region of SEQ ID NO: 73, a CDR2 region of SEQ ID NO: 74, and a CDR3 region of SEQ ID NO: 75, and b) a heavy-chain variable region comprising a CDR1 region of SEQ ID NO: 76, a CDR2 region of SEQ ID NO: 77, and a CDR3 region of SEQ ID NO: 78;
14) a binding molecule comprising a) a light-chain variable region comprising a CDR1 region of SEQ ID NO: 79, a CDR2 region of SEQ ID NO: 80, and a CDR3 region of SEQ ID NO: 81, and b) a heavy-chain variable region comprising a CDR1 region of SEQ ID NO: 82, a CDR2 region of SEQ ID NO: 83, and a CDR3 region of SEQ ID NO: 84;
15) a binding molecule comprising a) a light-chain variable region comprising a CDR1 region of SEQ ID NO: 85, a CDR2 region of SEQ ID NO: 86, and a CDR3 region of SEQ ID NO: 87, and b) a heavy-chain variable region comprising a CDR1 region of SEQ ID NO: 88, a CDR2 region of SEQ ID NO: 89, and a CDR3 region of SEQ ID NO: 90;
16) a binding molecule comprising a) a light-chain variable region comprising a CDR1 region of SEQ ID NO: 91, a CDR2 region of SEQ ID NO: 92, and a CDR3 region of SEQ ID NO: 93, and b) a heavy-chain variable region comprising a CDR1 region of SEQ ID NO: 94, a CDR2 region of SEQ ID NO: 95, and a CDR3 region of SEQ ID NO: 96;
17) a binding molecule comprising a) a light-chain variable region comprising a CDR1 region of SEQ ID NO: 97, a CDR2 region of SEQ ID NO: 98, and a CDR3 region of SEQ ID NO: 99, and b) a heavy-chain variable region comprising a CDR1 region of SEQ ID NO: 100, a CDR2 region of SEQ ID NO: 101, and a CDR3 region of SEQ ID NO: 102;
18) a binding molecule comprising a) a light-chain variable region comprising a CDR1 region of SEQ ID NO: 103, a CDR2 region of SEQ ID NO: 104, and a CDR3 region of SEQ ID NO: 105, and b) a heavy-chain variable region comprising a CDR1 region of SEQ ID NO: 106, a CDR2 region of SEQ ID NO: 107, and a CDR3 region of SEQ ID NO: 108;
19) a binding molecule comprising a) a light-chain variable region comprising a CDR1 region of SEQ ID NO: 109, CDR2 region of SEQ ID NO: 110, and CDR3 region of SEQ ID NO: 111, and b) a heavy-chain variable region comprising a CDR1 region of SEQ ID NO: 112, a CDR2 region of SEQ ID NO: 113, and a CDR3 region of SEQ ID NO: 114;
20) a binding molecule comprising a) a light-chain variable region comprising a CDR1 region of SEQ ID NO: 115, a CDR2 region of SEQ ID NO: 116, and a CDR3 region of SEQ ID NO: 117, and b) a heavy-chain variable region comprising a CDR1 region of SEQ ID NO: 118, a CDR2 region of SEQ ID NO: 119, and a CDR3 region of SEQ ID NO: 120;
21) a binding molecule comprising a) a light-chain variable region comprising a CDR1 region of SEQ ID NO: 121, a CDR2 region of SEQ ID NO: 122, and a CDR3 region of SEQ ID NO: 123, and b) a heavy-chain variable region comprising a CDR1 region of SEQ ID NO: 124, a CDR2 region of SEQ ID NO: 125, and a CDR3 region of SEQ ID NO: 126;
22) a binding molecule comprising a) a light-chain variable region comprising a CDR1 region of SEQ ID NO: 127, a CDR2 region of SEQ ID NO: 128, and a CDR3 region of SEQ ID NO: 129, and b) a heavy-chain variable region comprising a CDR1 region of SEQ ID NO: 130, a CDR2 region of SEQ ID NO: 131, and a CDR3 region of SEQ ID NO: 132;
23) a binding molecule comprising a) a light-chain variable region comprising a CDR1 region of SEQ ID NO: 133, a CDR2 region of SEQ ID NO: 134, and a CDR3 region of SEQ ID NO: 135, and b) a heavy-chain variable region comprising a CDR1 region of SEQ ID NO: 136, a CDR2 region of SEQ ID NO: 137, and a CDR3 region of SEQ ID NO: 138;
24) a binding molecule comprising a) a light-chain variable region comprising a CDR1 region of SEQ ID NO: 139, a CDR2 region of SEQ ID NO: 140, and a CDR3 region of SEQ ID NO: 141, and b) a heavy-chain variable region comprising a CDR1 region of SEQ ID NO: 142, a CDR2 region of SEQ ID NO: 143, and a CDR3 region of SEQ ID NO: 144;
25) a binding molecule comprising a) a light-chain variable region comprising a CDR1 region of SEQ ID NO: 145, a CDR2 region of SEQ ID NO: 146, and a CDR3 region of SEQ ID NO: 147, and b) a heavy-chain variable region comprising a CDR1 region of SEQ ID NO: 148, a CDR2 region of SEQ ID NO: 149, and a CDR3 region of SEQ ID NO: 150;
26) a binding molecule comprising a) a light-chain variable region comprising a CDR1 region of SEQ ID NO: 151, a CDR2 region of SEQ ID NO: 152, and a CDR3 region of SEQ ID NO: 153, and b) a heavy-chain variable region comprising a CDR1 region of SEQ ID NO: 154, a CDR2 region of SEQ ID NO: 155, and a CDR3 region of SEQ ID NO: 156;
27) a binding molecule comprising a) a light-chain variable region comprising a CDR1 region of SEQ ID NO: 157, a CDR2 region of SEQ ID NO: 158, and a CDR3 region of SEQ ID NO: 159, and b) a heavy-chain variable region comprising a CDR1 region of SEQ ID NO: 160, a CDR2 region of SEQ ID NO: 161, and a CDR3 region of SEQ ID NO: 162;
28) a binding molecule comprising a) a light-chain variable region comprising a CDR1 region of SEQ ID NO: 163, a CDR2 region of SEQ ID NO: 164, and a CDR3 region of SEQ ID NO: 165, and b) a heavy-chain variable region comprising a CDR1 region of SEQ ID NO: 166, a CDR2 region of SEQ ID NO: 167, and a CDR3 region of SEQ ID NO: 168;
29) a binding molecule comprising a) a light-chain variable region comprising a CDR1 region of SEQ ID NO: 169, a CDR2 region of SEQ ID NO: 170, and a CDR3 region of SEQ ID NO: 171, and b) a heavy-chain variable region comprising a CDR1 region of SEQ ID NO: 172, a CDR2 region of SEQ ID NO: 173, and a CDR3 region of SEQ ID NO: 174;
30) a binding molecule comprising a) a light-chain variable region comprising a CDR1 region of SEQ ID NO: 175, a CDR2 region of SEQ ID NO: 176, and a CDR3 region of SEQ ID NO: 177, and b) a heavy-chain variable region comprising a CDR1 region of SEQ ID NO: 178, a CDR2 region of SEQ ID NO: 179, and a CDR3 region of SEQ ID NO: 180;
31) a binding molecule comprising a) a light-chain variable region comprising a CDR1 region of SEQ ID NO: 181, a CDR2 region of SEQ ID NO: 182, and a CDR3 region of SEQ ID NO: 183, and b) a heavy-chain variable region comprising a CDR1 region of SEQ ID NO: 184, a CDR2 region of SEQ ID NO: 185, and a CDR3 region of SEQ ID NO: 186;
32) a binding molecule comprising a) a light-chain variable region comprising a CDR1 region of SEQ ID NO: 187, a CDR2 region of SEQ ID NO: 188, and a CDR3 region of SEQ ID NO: 189, and b) a heavy-chain variable region comprising a CDR1 region of SEQ ID NO: 190, a CDR2 region of SEQ ID NO: 191, and a CDR3 region of SEQ ID NO: 192;
33) a binding molecule comprising a) a light-chain variable region comprising a CDR1 region of SEQ ID NO: 193, a CDR2 region of SEQ ID NO: 194, and a CDR3 region of SEQ ID NO: 195, and b) a heavy-chain variable region comprising a CDR1 region of SEQ ID NO: 196, a CDR2 region of SEQ ID NO: 197, and a CDR3 region of SEQ ID NO: 198;
34) a binding molecule comprising a) a light-chain variable region comprising a CDR1 region of SEQ ID NO: 199, a CDR2 region of SEQ ID NO: 200, and a CDR3 region of SEQ ID NO: 201, and b) a heavy-chain variable region comprising a CDR1 region of SEQ ID NO: 202, a CDR2 region of SEQ ID NO: 203, and a CDR3 region of SEQ ID NO: 204;
35) a binding molecule comprising a) a light-chain variable region comprising a CDR1 region of SEQ ID NO: 205, a CDR2 region of SEQ ID NO: 206, and a CDR3 region of SEQ ID NO: 207, and b) a heavy-chain variable region comprising a CDR1 region of SEQ ID NO: 208, a CDR2 region of SEQ ID NO: 209, and a CDR3 region of SEQ ID NO: 210;
36) a binding molecule comprising a) a light-chain variable region comprising a CDR1 region of SEQ ID NO: 211, a CDR2 region of SEQ ID NO: 212, and a CDR3 region of SEQ ID NO: 213, and b) a heavy-chain variable region comprising a CDR1 region of SEQ ID NO: 214, a CDR2 region of SEQ ID NO: 215, and a CDR3 region of SEQ ID NO: 216;
37) a binding molecule comprising a) a light-chain variable region comprising a CDR1 region of SEQ ID NO: 217, a CDR2 region of SEQ ID NO: 218, and a CDR3 region of SEQ ID NO: 219, and b) a heavy-chain variable region comprising a CDR1 region of SEQ ID NO: 220, a CDR2 region of SEQ ID NO: 221, and a CDR3 region of SEQ ID NO: 222;
38) a binding molecule comprising a) a light-chain variable region comprising a CDR1 region of SEQ ID NO: 223, a CDR2 region of SEQ ID NO: 224, and a CDR3 region of SEQ ID NO: 225, and b) a heavy-chain variable region comprising a CDR1 region of SEQ ID NO: 226, a CDR2 region of SEQ ID NO: 227, and a CDR3 region of SEQ ID NO: 228;
39) a binding molecule comprising a) a light-chain variable region comprising a CDR1 region of SEQ ID NO: 229, a CDR2 region of SEQ ID NO: 230, and a CDR3 region of SEQ ID NO: 231, and b) a heavy-chain variable region comprising a CDR1 region of SEQ ID NO: 232, a CDR2 region of SEQ ID NO: 233, and a CDR3 region of SEQ ID NO: 234;
40) a binding molecule comprising a) a light-chain variable region comprising a CDR1 region of SEQ ID NO: 235, a CDR2 region of SEQ ID NO: 236, and a CDR3 region of SEQ ID NO: 237, and b) a heavy-chain variable region comprising a CDR1 region of SEQ ID NO: 238, a CDR2 region of SEQ ID NO: 239, and a CDR3 region of SEQ ID NO: 240;
41) a binding molecule comprising a) a light-chain variable region comprising a CDR1 region of SEQ ID NO: 241, a CDR2 region of SEQ ID NO: 242, and a CDR3 region of SEQ ID NO: 243, and b) a heavy-chain variable region comprising a CDR1 region of SEQ ID NO: 244, a CDR2 region of SEQ ID NO: 245, and a CDR3 region of SEQ ID NO: 246;
42) a binding molecule comprising a) a light-chain variable region comprising a CDR1 region of SEQ ID NO: 247, a CDR2 region of SEQ ID NO: 248, and a CDR3 region of SEQ ID NO: 249, and b) a heavy-chain variable region comprising a CDR1 region of SEQ ID NO: 250, a CDR2 region of SEQ ID NO: 251, and a CDR3 region of SEQ ID NO: 252;
43) a binding molecule comprising a) a light-chain variable region comprising a CDR1 region of SEQ ID NO: 253, a CDR2 region of SEQ ID NO: 254, and a CDR3 region of SEQ ID NO: 255, and b) a heavy-chain variable region comprising a CDR1 region of SEQ ID NO: 256, a CDR2 region of SEQ ID NO: 257, and a CDR3 region of SEQ ID NO: 258;
44) a binding molecule comprising a) a light-chain variable region comprising a CDR1 region of SEQ ID NO: 259, a CDR2 region of SEQ ID NO: 260, and a CDR3 region of SEQ ID NO: 261, and b) a heavy-chain variable region comprising a CDR1 region of SEQ ID NO: 262, a CDR2 region of SEQ ID NO: 263, and a CDR3 region of SEQ ID NO: 264;
45) a binding molecule comprising a) a light-chain variable region comprising a CDR1 region of SEQ ID NO: 265, a CDR2 region of SEQ ID NO: 266, and a CDR3 region of SEQ ID NO: 267, and b) a heavy-chain variable region comprising a CDR1 region of SEQ ID NO: 268, a CDR2 region of SEQ ID NO: 269, and a CDR3 region of SEQ ID NO: 270;
46) a binding molecule comprising a) a light-chain variable region comprising a CDR1 region of SEQ ID NO: 271, a CDR2 region of SEQ ID NO: 272, and a CDR3 region of SEQ ID NO: 273, and b) a heavy-chain variable region comprising a CDR1 region of SEQ ID NO: 274, a CDR2 region of SEQ ID NO: 275, and a CDR3 region of SEQ ID NO: 276;
47) a binding molecule comprising a) a light-chain variable region comprising a CDR1 region of SEQ ID NO: 277, a CDR2 region of SEQ ID NO: 278, and a CDR3 region of SEQ ID NO: 279, and b) a heavy-chain variable region comprising a CDR1 region of SEQ ID NO: 280, a CDR2 region of SEQ ID NO: 281, and a CDR3 region of SEQ ID NO: 282;
48) a binding molecule comprising a) a light-chain variable region comprising a CDR1 region of SEQ ID NO: 283, a CDR2 region of SEQ ID NO: 284, and a CDR3 region of SEQ ID NO: 285, and b) a heavy-chain variable region comprising a CDR1 region of SEQ ID NO: 286, a CDR2 region of SEQ ID NO: 287, and a CDR3 region of SEQ ID NO: 288;
49) a binding molecule comprising a) a light-chain variable region comprising a CDR1 region of SEQ ID NO: 289, a CDR2 region of SEQ ID NO: 290, and a CDR3 region of SEQ ID NO: 291, and b) a heavy-chain variable region comprising a CDR1 region of SEQ ID NO: 292, a CDR2 region of SEQ ID NO: 293, and a CDR3 region of SEQ ID NO: 294;
50) a binding molecule comprising a) a light-chain variable region comprising a CDR1 region of SEQ ID NO: 295, a CDR2 region of SEQ ID NO: 296, and a CDR3 region of SEQ ID NO: 297, and b) a heavy-chain variable region comprising a CDR1 region of SEQ ID NO: 298, a CDR2 region of SEQ ID NO: 299, and a CDR3 region of SEQ ID NO: 300;
51) a binding molecule comprising a) a light-chain variable region comprising a CDR1 region of SEQ ID NO: 301, a CDR2 region of SEQ ID NO: 302, and a CDR3 region of SEQ ID NO: 303, and b) a heavy-chain variable region comprising a CDR1 region of SEQ ID NO: 304, a CDR2 region of SEQ ID NO: 305, and a CDR3 region of SEQ ID NO: 306;
52) a binding molecule comprising a) a light-chain variable region comprising a CDR1 region of SEQ ID NO: 307, a CDR2 region of SEQ ID NO: 308, and a CDR3 region of SEQ ID NO: 309, and b) a heavy-chain variable region comprising a CDR1 region of SEQ ID NO: 310, a CDR2 region of SEQ ID NO: 311, and a CDR3 region of SEQ ID NO: 312;
53) a binding molecule comprising a) a light-chain variable region comprising a CDR1 region of SEQ ID NO: 313, a CDR2 region of SEQ ID NO: 314, and a CDR3 region of SEQ ID NO: 315, and b) a heavy-chain variable region comprising a CDR1 region of SEQ ID NO: 316, a CDR2 region of SEQ ID NO: 317, and a CDR3 region of SEQ ID NO: 318;
54) a binding molecule comprising a) a light-chain variable region comprising a CDR1 region of SEQ ID NO: 319, a CDR2 region of SEQ ID NO: 320, and a CDR3 region of SEQ ID NO: 321, and b) a heavy-chain variable region comprising a CDR1 region of SEQ ID NO: 322, a CDR2 region of SEQ ID NO: 323, and a CDR3 region of SEQ ID NO: 324;
55) a binding molecule comprising a) a light-chain variable region comprising a CDR1 region of SEQ ID NO: 325, a CDR2 region of SEQ ID NO: 326, and a CDR3 region of SEQ ID NO: 327, and b) a heavy-chain variable region comprising a CDR1 region of SEQ ID NO: 328, a CDR2 region of SEQ ID NO: 329, and a CDR3 region of SEQ ID NO: 330;
56) a binding molecule comprising a) a light-chain variable region comprising a CDR1 region of SEQ ID NO: 331, a CDR2 region of SEQ ID NO: 332, and a CDR3 region of SEQ ID NO: 333, and b) a heavy-chain variable region comprising a CDR1 region of SEQ ID NO: 334, a CDR2 region of SEQ ID NO: 335, and a CDR3 region of SEQ ID NO: 336;
57) a binding molecule comprising a) a light-chain variable region comprising a CDR1 region of SEQ ID NO: 337, a CDR2 region of SEQ ID NO: 338, and a CDR3 region of SEQ ID NO: 339, and b) a heavy-chain variable region comprising a CDR1 region of SEQ ID NO: 340, a CDR2 region of SEQ ID NO: 341, and a CDR3 region of SEQ ID NO: 342;
58) a binding molecule comprising a) a light-chain variable region comprising a CDR1 region of SEQ ID NO: 343, a CDR2 region of SEQ ID NO: 344, and a CDR3 region of SEQ ID NO: 345, and b) a heavy-chain variable region comprising a CDR1 region of SEQ ID NO: 346, a CDR2 region of SEQ ID NO: 347, and a CDR3 region of SEQ ID NO: 348;
59) a binding molecule comprising a) a light-chain variable region comprising a CDR1 region of SEQ ID NO: 349, a CDR2 region of SEQ ID NO: 350, and a CDR3 region of SEQ ID NO: 351, and b) a heavy-chain variable region comprising a CDR1 region of SEQ ID NO: 352, a CDR2 region of SEQ ID NO: 353, and a CDR3 region of SEQ ID NO: 354;
60) a binding molecule comprising a) a light-chain variable region comprising a CDR1 region of SEQ ID NO: 355, a CDR2 region of SEQ ID NO: 356, and a CDR3 region of SEQ ID NO: 357, and b) a heavy-chain variable region comprising a CDR1 region of SEQ ID NO: 358, a CDR2 region of SEQ ID NO: 359, and a CDR3 region of SEQ ID NO: 360;
61) a binding molecule comprising a) a light-chain variable region comprising a CDR1 region of SEQ ID NO: 361, a CDR2 region of SEQ ID NO: 362, and a CDR3 region of SEQ ID NO: 363, and b) a heavy-chain variable region comprising a CDR1 region of SEQ ID NO: 364, a CDR2 region of SEQ ID NO: 365, and a CDR3 region of SEQ ID NO: 366;
62) a binding molecule comprising a) a light-chain variable region comprising a CDR1 region of SEQ ID NO: 367, a CDR2 region of SEQ ID NO: 368, and a CDR3 region of SEQ ID NO: 369, and b) a heavy-chain variable region comprising a CDR1 region of SEQ ID NO: 370, a CDR2 region of SEQ ID NO: 371, and a CDR3 region of SEQ ID NO: 372;
63) a binding molecule comprising a) a light-chain variable region comprising a CDR1 region of SEQ ID NO: 373, a CDR2 region of SEQ ID NO: 374, and a CDR3 region of SEQ ID NO: 375, and b) a heavy-chain variable region comprising a CDR1 region of SEQ ID NO: 376, a CDR2 region of SEQ ID NO: 377, and a CDR3 region of SEQ ID NO: 378;
64) a binding molecule comprising a) a light-chain variable region comprising a CDR1 region of SEQ ID NO: 379, a CDR2 region of SEQ ID NO: 380, and a CDR3 region of SEQ ID NO: 381, and b) a heavy-chain variable region comprising a CDR1 region of SEQ ID NO: 382, a CDR2 region of SEQ ID NO: 383, and a CDR3 region of SEQ ID NO: 384;
65) a binding molecule comprising a) a light-chain variable region comprising a CDR1 region of SEQ ID NO: 385, a CDR2 region of SEQ ID NO: 386, and a CDR3 region of SEQ ID NO: 387, and b) a heavy-chain variable region comprising a CDR1 region of SEQ ID NO: 388, a CDR2 region of SEQ ID NO: 389, and a CDR3 region of SEQ ID NO: 390;
66) a binding molecule comprising a) a light-chain variable region comprising a CDR1 region of SEQ ID NO: 391, a CDR2 region of SEQ ID NO: 392, and a CDR3 region of SEQ ID NO: 393, and b) a heavy-chain variable region comprising a CDR1 region of SEQ ID NO: 394, a CDR2 region of SEQ ID NO: 395, and a CDR3 region of SEQ ID NO: 396;
67) a binding molecule comprising a) a light-chain variable region comprising a CDR1 region of SEQ ID NO: 397, a CDR2 region of SEQ ID NO: 398, and a CDR3 region of SEQ ID NO: 399, and b) a heavy-chain variable region comprising a CDR1 region of SEQ ID NO: 400, a CDR2 region of SEQ ID NO: 401, and a CDR3 region of SEQ ID NO: 402;
68) a binding molecule comprising a) a light-chain variable region comprising a CDR1 region of SEQ ID NO: 403, a CDR2 region of SEQ ID NO: 404, and a CDR3 region of SEQ ID NO: 405, and b) a heavy-chain variable region comprising a CDR1 region of SEQ ID NO: 406, a CDR2 region of SEQ ID NO: 407, and a CDR3 region of SEQ ID NO: 408;
69) a binding molecule comprising a) a light-chain variable region comprising a CDR1 region of SEQ ID NO: 409, a CDR2 region of SEQ ID NO: 410, and a CDR3 region of SEQ ID NO: 411, and b) a heavy-chain variable region comprising a CDR1 region of SEQ ID NO: 412, a CDR2 region of SEQ ID NO: 413, and a CDR3 region of SEQ ID NO: 414;
70) a binding molecule comprising a) a light-chain variable region comprising a CDR1 region of SEQ ID NO: 415, a CDR2 region of SEQ ID NO: 416, and a CDR3 region of SEQ ID NO: 417, and b) a heavy-chain variable region comprising a CDR1 region of SEQ ID NO: 418, a CDR2 region of SEQ ID NO: 419, and a CDR3 region of SEQ ID NO: 420;
71) a binding molecule comprising a) a light-chain variable region comprising a CDR1 region of SEQ ID NO: 421, a CDR2 region of SEQ ID NO: 422, and a CDR3 region of SEQ ID NO: 423, and b) a heavy-chain variable region comprising a CDR1 region of SEQ ID NO: 424, a CDR2 region of SEQ ID NO: 425, and a CDR3 region of SEQ ID NO: 426;
72) a binding molecule comprising a) a light-chain variable region comprising a CDR1 region of SEQ ID NO: 427, a CDR2 region of SEQ ID NO: 428, and a CDR3 region of SEQ ID NO: 429, and b) a heavy-chain variable region comprising a CDR1 region of SEQ ID NO: 430, a CDR2 region of SEQ ID NO: 431, and a CDR3 region of SEQ ID NO: 432;
73) a binding molecule comprising a) a light-chain variable region comprising a CDR1 region of SEQ ID NO: 433, a CDR2 region of SEQ ID NO: 434, and a CDR3 region of SEQ ID NO: 435, and b) a heavy-chain variable region comprising a CDR1 region of SEQ ID NO: 436, a CDR2 region of SEQ ID NO: 437, and a CDR3 region of SEQ ID NO: 438;
74) a binding molecule comprising a) a light-chain variable region comprising a CDR1 region of SEQ ID NO: 439, a CDR2 region of SEQ ID NO: 440, and a CDR3 region of SEQ ID NO: 441, and b) a heavy-chain variable region comprising a CDR1 region of SEQ ID NO: 442, a CDR2 region of SEQ ID NO: 443, and a CDR3 region of SEQ ID NO: 444;
75) a binding molecule comprising a) a light-chain variable region comprising a CDR1 region of SEQ ID NO: 445, a CDR2 region of SEQ ID NO: 446, and a CDR3 region of SEQ ID NO: 447, and b) a heavy-chain variable region comprising a CDR1 region of SEQ ID NO: 448, a CDR2 region of SEQ ID NO: 449, and a CDR3 region of SEQ ID NO: 450;
76) a binding molecule comprising a) a light-chain variable region comprising a CDR1 region of SEQ ID NO: 451, a CDR2 region of SEQ ID NO: 452, and a CDR3 region of SEQ ID NO: 453, and b) a heavy-chain variable region comprising a CDR1 region of SEQ ID NO: 454, a CDR2 region of SEQ ID NO: 455, and a CDR3 region of SEQ ID NO: 456;
77) a binding molecule comprising a) a light-chain variable region comprising a CDR1 region of SEQ ID NO: 457, a CDR2 region of SEQ ID NO: 458, and a CDR3 region of SEQ ID NO: 459, and b) a heavy-chain variable region comprising a CDR1 region of SEQ ID NO: 460, a CDR2 region of SEQ ID NO: 461, and a CDR3 region of SEQ ID NO: 462;
78) a binding molecule comprising a) a light-chain variable region comprising a CDR1 region of SEQ ID NO: 463, a CDR2 region of SEQ ID NO: 464, and a CDR3 region of SEQ ID NO: 465, and b) a heavy-chain variable region comprising a CDR1 region of SEQ ID NO: 466, a CDR2 region of SEQ ID NO: 467, and a CDR3 region of SEQ ID NO: 468;
79) a binding molecule comprising a) a light-chain variable region comprising a CDR1 region of SEQ ID NO: 469, a CDR2 region of SEQ ID NO: 470, and a CDR3 region of SEQ ID NO: 471, and b) a heavy-chain variable region comprising a CDR1 region of SEQ ID NO: 472, a CDR2 region of SEQ ID NO: 473, and a CDR3 region of SEQ ID NO: 474;
80) a binding molecule comprising a) a light-chain variable region comprising a CDR1 region of SEQ ID NO: 475, a CDR2 region of SEQ ID NO: 476, and a CDR3 region of SEQ ID NO: 477, and b) a heavy-chain variable region comprising a CDR1 region of SEQ ID NO: 478, a CDR2 region of SEQ ID NO: 479, and a CDR3 region of SEQ ID NO: 480;
81) a binding molecule comprising a) a light-chain variable region comprising a CDR1 region of SEQ ID NO: 481, a CDR2 region of SEQ ID NO: 482, and a CDR3 region of SEQ ID NO: 483, and b) a heavy-chain variable region comprising a CDR1 region of SEQ ID NO: 484, a CDR2 region of SEQ ID NO: 485, and a CDR3 region of SEQ ID NO: 486;
82) a binding molecule comprising a) a light-chain variable region comprising a CDR1 region of SEQ ID NO: 487, a CDR2 region of SEQ ID NO: 488, and a CDR3 region of SEQ ID NO: 489, and b) a heavy-chain variable region comprising a CDR1 region of SEQ ID NO: 490, a CDR2 region of SEQ ID NO: 491, and a CDR3 region of SEQ ID NO: 492;
83) a binding molecule comprising a) a light-chain variable region comprising a CDR1 region of SEQ ID NO: 493, a CDR2 region of SEQ ID NO: 494, and a CDR3 region of SEQ ID NO: 495, and b) a heavy-chain variable region comprising a CDR1 region of SEQ ID NO: 496, a CDR2 region of SEQ ID NO: 497, and a CDR3 region of SEQ ID NO: 498;
84) a binding molecule comprising a) a light-chain variable region comprising a CDR1 region of SEQ ID NO: 499, a CDR2 region of SEQ ID NO: 500, and a CDR3 region of SEQ ID NO: 501, and b) a heavy-chain variable region comprising a CDR1 region of SEQ ID NO: 502, a CDR2 region of SEQ ID NO: 503, and a CDR3 region of SEQ ID NO: 504;
85) a binding molecule comprising a) a light-chain variable region comprising a CDR1 region of SEQ ID NO: 505, a CDR2 region of SEQ ID NO: 506, and a CDR3 region of SEQ ID NO: 507, and b) a heavy-chain variable region comprising a CDR1 region of SEQ ID NO: 508, a CDR2 region of SEQ ID NO: 509, and a CDR3 region of SEQ ID NO: 510;
86) a binding molecule comprising a) a light-chain variable region comprising a CDR1 region of SEQ ID NO: 511, a CDR2 region of SEQ ID NO: 512, and a CDR3 region of SEQ ID NO: 513, and b) a heavy-chain variable region comprising a CDR1 region of SEQ ID NO: 514, a CDR2 region of SEQ ID NO: 515, and a CDR3 region of SEQ ID NO: 516;
87) a binding molecule comprising a) a light-chain variable region comprising a CDR1 region of SEQ ID NO: 517, a CDR2 region of SEQ ID NO: 518, and a CDR3 region of SEQ ID NO: 519, and b) a heavy-chain variable region comprising a CDR1 region of SEQ ID NO: 520, a CDR2 region of SEQ ID NO: 521, and a CDR3 region of SEQ ID NO: 522;
88) a binding molecule comprising a) a light-chain variable region comprising a CDR1 region of SEQ ID NO: 523, a CDR2 region of SEQ ID NO: 524, and a CDR3 region of SEQ ID NO: 525, and b) a heavy-chain variable region comprising a CDR1 region of SEQ ID NO: 526, a CDR2 region of SEQ ID NO: 527, and a CDR3 region of SEQ ID NO: 528;
89) a binding molecule comprising a) a light-chain variable region comprising a CDR1 region of SEQ ID NO: 529, a CDR2 region of SEQ ID NO: 530, and a CDR3 region of SEQ ID NO: 531, and b) a heavy-chain variable region comprising a CDR1 region of SEQ ID NO: 532, a CDR2 region of SEQ ID NO: 533, and a CDR3 region of SEQ ID NO: 534;
90) a binding molecule comprising a) a light-chain variable region comprising a CDR1 region of SEQ ID NO: 535, a CDR2 region of SEQ ID NO: 536, and a CDR3 region of SEQ ID NO: 537, and b) a heavy-chain variable region comprising a CDR1 region of SEQ ID NO: 538, a CDR2 region of SEQ ID NO: 539, and a CDR3 region of SEQ ID NO: 540;
91) a binding molecule comprising a) a light-chain variable region comprising a CDR1 region of SEQ ID NO: 541, a CDR2 region of SEQ ID NO: 542, and a CDR3 region of SEQ ID NO: 543, and b) a heavy-chain variable region comprising a CDR1 region of SEQ ID NO: 544, a CDR2 region of SEQ ID NO: 545, and a CDR3 region of SEQ ID NO: 546;
92) a binding molecule comprising a) a light-chain variable region comprising a CDR1 region of SEQ ID NO: 547, a CDR2 region of SEQ ID NO: 548, and a CDR3 region of SEQ ID NO: 549, and b) a heavy-chain variable region comprising a CDR1 region of SEQ ID NO: 550, a CDR2 region of SEQ ID NO: 551, and a CDR3 region of SEQ ID NO: 552;
93) a binding molecule comprising a) a light-chain variable region comprising a CDR1 region of SEQ ID NO: 553, a CDR2 region of SEQ ID NO: 554, and a CDR3 region of SEQ ID NO: 555, and b) a heavy-chain variable region comprising a CDR1 region of SEQ ID NO: 556, a CDR2 region of SEQ ID NO: 557, and a CDR3 region of SEQ ID NO: 558;
94) a binding molecule comprising a) a light-chain variable region comprising a CDR1 region of SEQ ID NO: 559, a CDR2 region of SEQ ID NO: 560, and a CDR3 region of SEQ ID NO: 561, and b) a heavy-chain variable region comprising a CDR1 region of SEQ ID NO: 562, a CDR2 region of SEQ ID NO: 563, and a CDR3 region of SEQ ID NO: 564;
95) a binding molecule comprising a) a light-chain variable region comprising a CDR1 region of SEQ ID NO: 565, a CDR2 region of SEQ ID NO: 566, and a CDR3 region of SEQ ID NO: 567, and b) a heavy-chain variable region comprising a CDR1 region of SEQ ID NO: 568, a CDR2 region of SEQ ID NO: 569, and a CDR3 region of SEQ ID NO: 570;
96) a binding molecule comprising a) a light-chain variable region comprising a CDR1 region of SEQ ID NO: 571, a CDR2 region of SEQ ID NO: 572, and a CDR3 region of SEQ ID NO: 573, and b) a heavy-chain variable region comprising a CDR1 region of SEQ ID NO: 574, a CDR2 region of SEQ ID NO: 575, and a CDR3 region of SEQ ID NO: 576;
97) a binding molecule comprising a) a light-chain variable region comprising a CDR1 region of SEQ ID NO: 577, a CDR2 region of SEQ ID NO: 578, and a CDR3 region of SEQ ID NO: 579, and b) a heavy-chain variable region comprising a CDR1 region of SEQ ID NO: 580, a CDR2 region of SEQ ID NO: 581, and a CDR3 region of SEQ ID NO: 582;
98) a binding molecule comprising a) a light-chain variable region comprising a CDR1 region of SEQ ID NO: 583, a CDR2 region of SEQ ID NO: 584, and a CDR3 region of SEQ ID NO: 585, and b) a heavy-chain variable region comprising a CDR1 region of SEQ ID NO: 586, a CDR2 region of SEQ ID NO: 587, and a CDR3 region of SEQ ID NO: 588;
99) a binding molecule comprising a) a light-chain variable region comprising a CDR1 region of SEQ ID NO: 589, a CDR2 region of SEQ ID NO: 590, and a CDR3 region of SEQ ID NO: 591, and b) a heavy-chain variable region comprising a CDR1 region of SEQ ID NO: 592, a CDR2 region of SEQ ID NO: 593, and a CDR3 region of SEQ ID NO: 594;
100) a binding molecule comprising a) a light-chain variable region comprising a CDR1 region of SEQ ID NO: 595, a CDR2 region of SEQ ID NO: 596, and a CDR3 region of SEQ ID NO: 597, and b) a heavy-chain variable region comprising a CDR1 region of SEQ ID NO: 598, a CDR2 region of SEQ ID NO: 599, and a CDR3 region of SEQ ID NO: 600;
101) a binding molecule comprising a) a light-chain variable region comprising a CDR1 region of SEQ ID NO: 601, a CDR2 region of SEQ ID NO: 602, and a CDR3 region of SEQ ID NO: 603, and b) a heavy-chain variable region comprising a CDR1 region of SEQ ID NO: 604, a CDR2 region of SEQ ID NO: 605, and a CDR3 region of SEQ ID NO: 606;
102) a binding molecule comprising a) a light-chain variable region comprising a CDR1 region of SEQ ID NO: 607, a CDR2 region of SEQ ID NO: 608, and a CDR3 region of SEQ ID NO: 609, and b) a heavy-chain variable region comprising a CDR1 region of SEQ ID NO: 610, a CDR2 region of SEQ ID NO: 611, and a CDR3 region of SEQ ID NO: 612;
103) a binding molecule comprising a) a light-chain variable region comprising a CDR1 region of SEQ ID NO: 613, a CDR2 region of SEQ ID NO: 614, and a CDR3 region of SEQ ID NO: 615, and b) a heavy-chain variable region comprising a CDR1 region of SEQ ID NO: 616, a CDR2 region of SEQ ID NO: 617, and a CDR3 region of SEQ ID NO: 618;
104) a binding molecule comprising a) a light-chain variable region comprising a CDR1 region of SEQ ID NO: 619, a CDR2 region of SEQ ID NO: 620, and a CDR3 region of SEQ ID NO: 621, and b) a heavy-chain variable region comprising a CDR1 region of SEQ ID NO: 622, a CDR2 region of SEQ ID NO: 623, and a CDR3 region of SEQ ID NO: 624;
105) a binding molecule comprising a) a light-chain variable region comprising a CDR1 region of SEQ ID NO: 625, a CDR2 region of SEQ ID NO: 626, and a CDR3 region of SEQ ID NO: 627, and b) a heavy-chain variable region comprising a CDR1 region of SEQ ID NO: 628, a CDR2 region of SEQ ID NO: 629, and a CDR3 region of SEQ ID NO: 630;
106) a binding molecule comprising a) a light-chain variable region comprising a CDR1 region of SEQ ID NO: 631, a CDR2 region of SEQ ID NO: 632, and a CDR3 region of SEQ ID NO: 633, and b) a heavy-chain variable region comprising a CDR1 region of SEQ ID NO: 634, a CDR2 region of SEQ ID NO: 635, and a CDR3 region of SEQ ID NO: 636;
107) a binding molecule comprising a) a light-chain variable region comprising a CDR1 region of SEQ ID NO: 637, a CDR2 region of SEQ ID NO: 638, and a CDR3 region of SEQ ID NO: 639, and b) a heavy-chain variable region comprising a CDR1 region of SEQ ID NO: 640, a CDR2 region of SEQ ID NO: 641, and a CDR3 region of SEQ ID NO: 642;
108) a binding molecule comprising a) a light-chain variable region comprising a CDR1 region of SEQ ID NO: 643, a CDR2 region of SEQ ID NO: 644, and a CDR3 region of SEQ ID NO: 645, and b) a heavy-chain variable region comprising a CDR1 region of SEQ ID NO: 646, a CDR2 region of SEQ ID NO: 647, and a CDR3 region of SEQ ID NO: 648;
109) a binding molecule comprising a) a light-chain variable region comprising a CDR1 region of SEQ ID NO: 649, a CDR2 region of SEQ ID NO: 650, and a CDR3 region of SEQ ID NO: 651, and b) a heavy-chain variable region comprising a CDR1 region of SEQ ID NO: 652, a CDR2 region of SEQ ID NO: 653, and a CDR3 region of SEQ ID NO: 654;
110) a binding molecule comprising a) a light-chain variable region comprising a CDR1 region of SEQ ID NO: 655, a CDR2 region of SEQ ID NO: 656, and a CDR3 region of SEQ ID NO: 657, and b) a heavy-chain variable region comprising a CDR1 region of SEQ ID NO: 658, a CDR2 region of SEQ ID NO: 659, and a CDR3 region of SEQ ID NO: 660;
111) a binding molecule comprising a) a light-chain variable region comprising a CDR1 region of SEQ ID NO: 661, a CDR2 region of SEQ ID NO: 662, and a CDR3 region of SEQ ID NO: 663, and b) a heavy-chain variable region comprising a CDR1 region of SEQ ID NO: 664, a CDR2 region of SEQ ID NO: 665, and a CDR3 region of SEQ ID NO: 666;
112) a binding molecule comprising a) a light-chain variable region comprising a CDR1 region of SEQ ID NO: 667, a CDR2 region of SEQ ID NO: 668, and a CDR3 region of SEQ ID NO: 669, and b) a heavy-chain variable region comprising a CDR1 region of SEQ ID NO: 670, a CDR2 region of SEQ ID NO: 671, and a CDR3 region of SEQ ID NO: 672;
113) a binding molecule comprising a) a light-chain variable region comprising a CDR1 region of SEQ ID NO: 673, a CDR2 region of SEQ ID NO: 674, and a CDR3 region of SEQ ID NO: 675, and b) a heavy-chain variable region comprising a CDR1 region of SEQ ID NO: 676, a CDR2 region of SEQ ID NO: 677, and a CDR3 region of SEQ ID NO: 678;
114) a binding molecule comprising a) a light-chain variable region comprising a CDR1 region of SEQ ID NO: 679, a CDR2 region of SEQ ID NO: 680, and a CDR3 region of SEQ ID NO: 681, and b) a heavy-chain variable region comprising a CDR1 region of SEQ ID NO: 682, a CDR2 region of SEQ ID NO: 683, and a CDR3 region of SEQ ID NO: 684;
115) a binding molecule comprising a) a light-chain variable region comprising a CDR1 region of SEQ ID NO: 685, a CDR2 region of SEQ ID NO: 686, and a CDR3 region of SEQ ID NO: 687, and b) a heavy-chain variable region comprising a CDR1 region of SEQ ID NO: 688, a CDR2 region of SEQ ID NO: 689, and a CDR3 region of SEQ ID NO: 690;
116) a binding molecule comprising a) a light-chain variable region comprising a CDR1 region of SEQ ID NO: 691, a CDR2 region of SEQ ID NO: 692, and a CDR3 region of SEQ ID NO: 693, and b) a heavy-chain variable region comprising a CDR1 region of SEQ ID NO: 694, a CDR2 region of SEQ ID NO: 695, and a CDR3 region of SEQ ID NO: 696;
117) a binding molecule comprising a) a light-chain variable region comprising a CDR1 region of SEQ ID NO: 697, a CDR2 region of SEQ ID NO: 698, and a CDR3 region of SEQ ID NO: 699, and b) a heavy-chain variable region comprising a CDR1 region of SEQ ID NO: 700, a CDR2 region of SEQ ID NO: 701, and a CDR3 region of SEQ ID NO: 702;
118) a binding molecule comprising a) a light-chain variable region comprising a CDR1 region of SEQ ID NO: 703, a CDR2 region of SEQ ID NO: 704, and a CDR3 region of SEQ ID NO: 705, and b) a heavy-chain variable region comprising a CDR1 region of SEQ ID NO: 706, a CDR2 region of SEQ ID NO: 707, and a CDR3 region of SEQ ID NO: 708;
119) a binding molecule comprising a) a light-chain variable region comprising a CDR1 region of SEQ ID NO: 709, a CDR2 region of SEQ ID NO: 710, and a CDR3 region of SEQ ID NO: 711, and b) a heavy-chain variable region comprising a CDR1 region of SEQ ID NO: 712, a CDR2 region of SEQ ID NO: 713, and a CDR3 region of SEQ ID NO: 714;
120) a binding molecule comprising a) a light-chain variable region comprising a CDR1 region of SEQ ID NO: 715, a CDR2 region of SEQ ID NO: 716, and a CDR3 region of SEQ ID NO: 717, and b) a heavy-chain variable region comprising a CDR1 region of SEQ ID NO: 718, a CDR2 region of SEQ ID NO: 719, and a CDR3 region of SEQ ID NO: 720;
121) a binding molecule comprising a) a light-chain variable region comprising a CDR1 region of SEQ ID NO: 721, a CDR2 region of SEQ ID NO: 722, and a CDR3 region of SEQ ID NO: 723, and b) a heavy-chain variable region comprising a CDR1 region of SEQ ID NO: 724, a CDR2 region of SEQ ID NO: 725, and a CDR3 region of SEQ ID NO: 726;
122) a binding molecule comprising a) a light-chain variable region comprising a CDR1 region of SEQ ID NO: 727, a CDR2 region of SEQ ID NO: 728, and a CDR3 region of SEQ ID NO: 729, and b) a heavy-chain variable region comprising a CDR1 region of SEQ ID NO: 730, a CDR2 region of SEQ ID NO: 731, and a CDR3 region of SEQ ID NO: 732;
123) a binding molecule comprising a) a light-chain variable region comprising a CDR1 region of SEQ ID NO: 733, a CDR2 region of SEQ ID NO: 734, and a CDR3 region of SEQ ID NO: 735, and b) a heavy-chain variable region comprising a CDR1 region of SEQ ID NO: 736, a CDR2 region of SEQ ID NO: 737, and a CDR3 region of SEQ ID NO: 738;
124) a binding molecule comprising a) a light-chain variable region comprising a CDR1 region of SEQ ID NO: 739, a CDR2 region of SEQ ID NO: 740, and a CDR3 region of SEQ ID NO: 741, and b) a heavy-chain variable region comprising a CDR1 region of SEQ ID NO: 742, a CDR2 region of SEQ ID NO: 743, and a CDR3 region of SEQ ID NO: 744;
125) a binding molecule comprising a) a light-chain variable region comprising a CDR1 region of SEQ ID NO: 745, a CDR2 region of SEQ ID NO: 746, and a CDR3 region of SEQ ID NO: 747, and b) a heavy-chain variable region comprising a CDR1 region of SEQ ID NO: 748, a CDR2 region of SEQ ID NO: 749, and a CDR3 region of SEQ ID NO: 750;
126) a binding molecule comprising a) a light-chain variable region comprising a CDR1 region of SEQ ID NO: 751, a CDR2 region of SEQ ID NO: 752, and a CDR3 region of SEQ ID NO: 753, and b) a heavy-chain variable region comprising a CDR1 region of SEQ ID NO: 754, a CDR2 region of SEQ ID NO: 755, and a CDR3 region of SEQ ID NO: 756;
127) a binding molecule comprising a) a light-chain variable region comprising a CDR1 region of SEQ ID NO: 757, a CDR2 region of SEQ ID NO: 758, and a CDR3 region of SEQ ID NO: 759, and b) a heavy-chain variable region comprising a CDR1 region of SEQ ID NO: 760, a CDR2 region of SEQ ID NO: 761, and a CDR3 region of SEQ ID NO: 762;
128) a binding molecule comprising a) a light-chain variable region comprising a CDR1 region of SEQ ID NO: 763, a CDR2 region of SEQ ID NO: 764, and a CDR3 region of SEQ ID NO: 765, and b) a heavy-chain variable region comprising a CDR1 region of SEQ ID NO: 766, a CDR2 region of SEQ ID NO: 767, and a CDR3 region of SEQ ID NO: 768;
129) a binding molecule comprising a) a light-chain variable region comprising a CDR1 region of SEQ ID NO: 769, a CDR2 region of SEQ ID NO: 770, and a CDR3 region of SEQ ID NO: 771, and b) a heavy-chain variable region comprising a CDR1 region of SEQ ID NO: 772, a CDR2 region of SEQ ID NO: 773, and a CDR3 region of SEQ ID NO: 774;
130) a binding molecule comprising a) a light-chain variable region comprising a CDR1 region of SEQ ID NO: 775, a CDR2 region of SEQ ID NO: 776, and a CDR3 region of SEQ ID NO: 777, and b) a heavy-chain variable region comprising a CDR1 region of SEQ ID NO: 778, a CDR2 region of SEQ ID NO: 779, and a CDR3 region of SEQ ID NO: 780;
131) a binding molecule comprising a) a light-chain variable region comprising a CDR1 region of SEQ ID NO: 781, a CDR2 region of SEQ ID NO: 782, and a CDR3 region of SEQ ID NO: 783, and b) a heavy-chain variable region comprising a CDR1 region of SEQ ID NO: 784, a CDR2 region of SEQ ID NO: 785, and a CDR3 region of SEQ ID NO: 786;
132) a binding molecule comprising a) a light-chain variable region comprising a CDR1 region of SEQ ID NO: 787, a CDR2 region of SEQ ID NO: 788, and a CDR3 region of SEQ ID NO: 789, and b) a heavy-chain variable region comprising a CDR1 region of SEQ ID NO: 790, a CDR2 region of SEQ ID NO: 791, and a CDR3 region of SEQ ID NO: 792;
133) a binding molecule comprising a) a light-chain variable region comprising a CDR1 region of SEQ ID NO: 793, a CDR2 region of SEQ ID NO: 794, and a CDR3 region of SEQ ID NO: 795, and b) a heavy-chain variable region comprising a CDR1 region of SEQ ID NO: 796, a CDR2 region of SEQ ID NO: 797, and a CDR3 region of SEQ ID NO: 798;
134) a binding molecule comprising a) a light-chain variable region comprising a CDR1 region of SEQ ID NO: 799, a CDR2 region of SEQ ID NO: 800, and a CDR3 region of SEQ ID NO: 801, and b) a heavy-chain variable region comprising a CDR1 region of SEQ ID NO: 802, a CDR2 region of SEQ ID NO: 803, and a CDR3 region of SEQ ID NO: 804;
135) a binding molecule comprising a) a light-chain variable region comprising a CDR1 region of SEQ ID NO: 805, a CDR2 region of SEQ ID NO: 806, and a CDR3 region of SEQ ID NO: 807, and b) a heavy-chain variable region comprising a CDR1 region of SEQ ID NO: 808, a CDR2 region of SEQ ID NO: 809, and a CDR3 region of SEQ ID NO: 810;
136) a binding molecule comprising a) a light-chain variable region comprising a CDR1 region of SEQ ID NO: 811, a CDR2 region of SEQ ID NO: 812, and a CDR3 region of SEQ ID NO: 813, and b) a heavy-chain variable region comprising a CDR1 region of SEQ ID NO: 814, a CDR2 region of SEQ ID NO: 815, and a CDR3 region of SEQ ID NO: 816;
137) a binding molecule comprising a) a light-chain variable region comprising a CDR1 region of SEQ ID NO: 817, a CDR2 region of SEQ ID NO: 818, and a CDR3 region of SEQ ID NO: 819, and b) a heavy-chain variable region comprising a CDR1 region of SEQ ID NO: 820, a CDR2 region of SEQ ID NO: 821, and a CDR3 region of SEQ ID NO: 822;
138) a binding molecule comprising a) a light-chain variable region comprising a CDR1 region of SEQ ID NO: 823, a CDR2 region of SEQ ID NO: 824, and a CDR3 region of SEQ ID NO: 825, and b) a heavy-chain variable region comprising a CDR1 region of SEQ ID NO: 826, a CDR2 region of SEQ ID NO: 827, and a CDR3 region of SEQ ID NO: 828;
139) a binding molecule comprising a) a light-chain variable region comprising a CDR1 region of SEQ ID NO: 829, a CDR2 region of SEQ ID NO: 830, and a CDR3 region of SEQ ID NO: 831, and b) a heavy-chain variable region comprising a CDR1 region of SEQ ID NO: 832, a CDR2 region of SEQ ID NO: 833, and a CDR3 region of SEQ ID NO: 834;
140) a binding molecule comprising a) a light-chain variable region comprising a CDR1 region of SEQ ID NO: 835, a CDR2 region of SEQ ID NO: 836, and a CDR3 region of SEQ ID NO: 837, and b) a heavy-chain variable region comprising a CDR1 region of SEQ ID NO: 838, a CDR2 region of SEQ ID NO: 839, and a CDR3 region of SEQ ID NO: 840;
141) a binding molecule comprising a) a light-chain variable region comprising a CDR1 region of SEQ ID NO: 841, a CDR2 region of SEQ ID NO: 842, and a CDR3 region of SEQ ID NO: 843, and b) a heavy-chain variable region comprising a CDR1 region of SEQ ID NO: 844, a CDR2 region of SEQ ID NO: 845, and a CDR3 region of SEQ ID NO: 846;
142) a binding molecule comprising a) a light-chain variable region comprising a CDR1 region of SEQ ID NO: 847, a CDR2 region of SEQ ID NO: 848, and a CDR3 region of SEQ ID NO: 849, and b) a heavy-chain variable region comprising a CDR1 region of SEQ ID NO: 850, a CDR2 region of SEQ ID NO: 851, and a CDR3 region of SEQ ID NO: 852;
143) a binding molecule comprising a) a light-chain variable region comprising a CDR1 region of SEQ ID NO: 853, a CDR2 region of SEQ ID NO: 854, and a CDR3 region of SEQ ID NO: 855, and b) a heavy-chain variable region comprising a CDR1 region of SEQ ID NO: 856, a CDR2 region of SEQ ID NO: 857, and a CDR3 region of SEQ ID NO: 858;
144) a binding molecule comprising a) a light-chain variable region comprising a CDR1 region of SEQ ID NO: 859, a CDR2 region of SEQ ID NO: 860, and a CDR3 region of SEQ ID NO: 861, and b) a heavy-chain variable region comprising a CDR1 region of SEQ ID NO: 862, a CDR2 region of SEQ ID NO: 863, and a CDR3 region of SEQ ID NO: 864;
145) a binding molecule comprising a) a light-chain variable region comprising a CDR1 region of SEQ ID NO: 865, a CDR2 region of SEQ ID NO: 866, and a CDR3 region of SEQ ID NO: 867, and b) a heavy-chain variable region comprising a CDR1 region of SEQ ID NO: 868, a CDR2 region of SEQ ID NO: 869, and a CDR3 region of SEQ ID NO: 870;
146) a binding molecule comprising a) a light-chain variable region comprising a CDR1 region of SEQ ID NO: 871, a CDR2 region of SEQ ID NO: 872, and a CDR3 region of SEQ ID NO: 873, and b) a heavy-chain variable region comprising a CDR1 region of SEQ ID NO: 874, a CDR2 region of SEQ ID NO: 875, and a CDR3 region of SEQ ID NO: 876;
147) a binding molecule comprising a) a light-chain variable region comprising a CDR1 region of SEQ ID NO: 877, a CDR2 region of SEQ ID NO: 878, and a CDR3 region of SEQ ID NO: 879, and b) a heavy-chain variable region comprising a CDR1 region of SEQ ID NO: 880, a CDR2 region of SEQ ID NO: 881, and a CDR3 region of SEQ ID NO: 882;
148) a binding molecule comprising a) a light-chain variable region comprising a CDR1 region of SEQ ID NO: 883, a CDR2 region of SEQ ID NO: 884, and a CDR3 region of SEQ ID NO: 885, and b) a heavy-chain variable region comprising a CDR1 region of SEQ ID NO: 886, a CDR2 region of SEQ ID NO: 887, and a CDR3 region of SEQ ID NO: 888;
149) a binding molecule comprising a) a light-chain variable region comprising a CDR1 region of SEQ ID NO: 889, a CDR2 region of SEQ ID NO: 890, and a CDR3 region of SEQ ID NO: 891, and b) a heavy-chain variable region comprising a CDR1 region of SEQ ID NO: 892, a CDR2 region of SEQ ID NO: 893, and a CDR3 region of SEQ ID NO: 894;
150) a binding molecule comprising a) a light-chain variable region comprising a CDR1 region of SEQ ID NO: 895, a CDR2 region of SEQ ID NO: 896, and a CDR3 region of SEQ ID NO: 897, and b) a heavy-chain variable region comprising a CDR1 region of SEQ ID NO: 898, a CDR2 region of SEQ ID NO: 899, and a CDR3 region of SEQ ID NO: 900;
151) a binding molecule comprising a) a light-chain variable region comprising a CDR1 region of SEQ ID NO: 901, a CDR2 region of SEQ ID NO: 902, and a CDR3 region of SEQ ID NO: 903, and b) a heavy-chain variable region comprising a CDR1 region of SEQ ID NO: 904, a CDR2 region of SEQ ID NO: 905, and a CDR3 region of SEQ ID NO: 906;
152) a binding molecule comprising a) a light-chain variable region comprising a CDR1 region of SEQ ID NO: 907, a CDR2 region of SEQ ID NO: 908, and a CDR3 region of SEQ ID NO: 909, and b) a heavy-chain variable region comprising a CDR1 region of SEQ ID NO: 910, a CDR2 region of SEQ ID NO: 911, and a CDR3 region of SEQ ID NO: 912;
153) a binding molecule comprising a) a light-chain variable region comprising a CDR1 region of SEQ ID NO: 913, a CDR2 region of SEQ ID NO: 914, and a CDR3 region of SEQ ID NO: 915, and b) a heavy-chain variable region comprising a CDR1 region of SEQ ID NO: 916, a CDR2 region of SEQ ID NO: 917, and a CDR3 region of SEQ ID NO: 918;
154) a binding molecule comprising a) a light-chain variable region comprising a CDR1 region of SEQ ID NO: 919, a CDR2 region of SEQ ID NO: 920, and a CDR3 region of SEQ ID NO: 921, and b) a heavy-chain variable region comprising a CDR1 region of SEQ ID NO: 922, a CDR2 region of SEQ ID NO: 923, and a CDR3 region of SEQ ID NO: 924;
155) a binding molecule comprising a) a light-chain variable region comprising a CDR1 region of SEQ ID NO: 925, a CDR2 region of SEQ ID NO: 926, and a CDR3 region of SEQ ID NO: 927, and b) a heavy-chain variable region comprising a CDR1 region of SEQ ID NO: 928, a CDR2 region of SEQ ID NO: 929, and a CDR3 region of SEQ ID NO: 930;
156) a binding molecule comprising a) a light-chain variable region comprising a CDR1 region of SEQ ID NO: 931, a CDR2 region of SEQ ID NO: 932, and a CDR3 region of SEQ ID NO: 933, and b) a heavy-chain variable region comprising a CDR1 region of SEQ ID NO: 934, a CDR2 region of SEQ ID NO: 935, and a CDR3 region of SEQ ID NO: 936;
157) a binding molecule comprising a) a light-chain variable region comprising a CDR1 region of SEQ ID NO: 937, a CDR2 region of SEQ ID NO: 938, and a CDR3 region of SEQ ID NO: 939, and b) a heavy-chain variable region comprising a CDR1 region of SEQ ID NO: 940, a CDR2 region of SEQ ID NO: 941, and a CDR3 region of SEQ ID NO: 942;
158) a binding molecule comprising a) a light-chain variable region comprising a CDR1 region of SEQ ID NO: 943, a CDR2 region of SEQ ID NO: 944, and a CDR3 region of SEQ ID NO: 945, and b) a heavy-chain variable region comprising a CDR1 region of SEQ ID NO: 946, a CDR2 region of SEQ ID NO: 947, and a CDR3 region of SEQ ID NO: 948;
159) a binding molecule comprising a) a light-chain variable region comprising a CDR1 region of SEQ ID NO: 949, a CDR2 region of SEQ ID NO: 950, and a CDR3 region of SEQ ID NO: 951, and b) a heavy-chain variable region comprising a CDR1 region of SEQ ID NO: 952, a CDR2 region of SEQ ID NO: 953, and a CDR3 region of SEQ ID NO: 954;
160) a binding molecule comprising a) a light-chain variable region comprising a CDR1 region of SEQ ID NO: 955, a CDR2 region of SEQ ID NO: 956, and a CDR3 region of SEQ ID NO: 957, and b) a heavy-chain variable region comprising a CDR1 region of SEQ ID NO: 958, a CDR2 region of SEQ ID NO: 959, and a CDR3 region of SEQ ID NO: 960;
161) a binding molecule comprising a) a light-chain variable region comprising a CDR1 region of SEQ ID NO: 961, a CDR2 region of SEQ ID NO: 962, and a CDR3 region of SEQ ID NO: 963, and b) a heavy-chain variable region comprising a CDR1 region of SEQ ID NO: 964, a CDR2 region of SEQ ID NO: 965, and a CDR3 region of SEQ ID NO: 966;
162) a binding molecule comprising a) a light-chain variable region comprising a CDR1 region of SEQ ID NO: 967, a CDR2 region of SEQ ID NO: 968, and a CDR3 region of SEQ ID NO: 969, and b) a heavy-chain variable region comprising a CDR1 region of SEQ ID NO: 970, a CDR2 region of SEQ ID NO: 971, and a CDR3 region of SEQ ID NO: 972;
163) a binding molecule comprising a) a light-chain variable region comprising a CDR1 region of SEQ ID NO: 973, a CDR2 region of SEQ ID NO: 974, and a CDR3 region of SEQ ID NO: 975, and b) a heavy-chain variable region comprising a CDR1 region of SEQ ID NO: 976, a CDR2 region of SEQ ID NO: 977, and a CDR3 region of SEQ ID NO: 978;
164) a binding molecule comprising a) a light-chain variable region comprising a CDR1 region of SEQ ID NO: 979, a CDR2 region of SEQ ID NO: 980, and a CDR3 region of SEQ ID NO: 981, and b) a heavy-chain variable region comprising a CDR1 region of SEQ ID NO: 982, a CDR2 region of SEQ ID NO: 983, and a CDR3 region of SEQ ID NO: 984;
165) a binding molecule comprising a) a light-chain variable region comprising a CDR1 region of SEQ ID NO: 985, a CDR2 region of SEQ ID NO: 986, and a CDR3 region of SEQ ID NO: 987, and b) a heavy-chain variable region comprising a CDR1 region of SEQ ID NO: 988, a CDR2 region of SEQ ID NO: 989, and a CDR3 region of SEQ ID NO: 990;
166) a binding molecule comprising a) a light-chain variable region comprising a CDR1 region of SEQ ID NO: 991, a CDR2 region of SEQ ID NO: 992, and a CDR3 region of SEQ ID NO: 993, and b) a heavy-chain variable region comprising a CDR1 region of SEQ ID NO: 994, a CDR2 region of SEQ ID NO: 995, and a CDR3 region of SEQ ID NO: 996;
167) a binding molecule comprising a) a light-chain variable region comprising a CDR1 region of SEQ ID NO: 997, a CDR2 region of SEQ ID NO: 998, and a CDR3 region of SEQ ID NO: 999, and b) a heavy-chain variable region comprising a CDR1 region of SEQ ID NO: 1,000, a CDR2 region of SEQ ID NO: 1,001, and a CDR3 region of SEQ ID NO: 1,002;
168) a binding molecule comprising a) a light-chain variable region comprising a CDR1 region of SEQ ID NO: 1,003, a CDR2 region of SEQ ID NO: 1,004, and a CDR3 region of SEQ ID NO: 1,005, and b) a heavy-chain variable region comprising a CDR1 region of SEQ ID NO: 1,006, a CDR2 region of SEQ ID NO: 1,007, and a CDR3 region of SEQ ID NO: 1,008;
169) a binding molecule comprising a) a light-chain variable region comprising a CDR1 region of SEQ ID NO: 1,009, a CDR2 region of SEQ ID NO: 1,010, and a CDR3 region of SEQ ID NO: 1,011, and b) a heavy-chain variable region comprising a CDR1 region of SEQ ID NO: 1,012, a CDR2 region of SEQ ID NO: 1,013, and a CDR3 region of SEQ ID NO: 1,014;
170) a binding molecule comprising a) a light-chain variable region comprising a CDR1 region of SEQ ID NO: 1,015, a CDR2 region of SEQ ID NO: 1,016, and a CDR3 region of SEQ ID NO: 1,017, and b) a heavy-chain variable region comprising a CDR1 region of SEQ ID NO: 1,018, a CDR2 region of SEQ ID NO: 1,019, and a CDR3 region of SEQ ID NO: 1,020;
171) a binding molecule comprising a) a light-chain variable region comprising a CDR1 region of SEQ ID NO: 1,021, a CDR2 region of SEQ ID NO: 1,022, and a CDR3 region of SEQ ID NO: 1,023, and b) a heavy-chain variable region comprising a CDR1 region of SEQ ID NO: 1,024, a CDR2 region of SEQ ID NO: 1,025, and a CDR3 region of SEQ ID NO: 1,026;
172) a binding molecule comprising a) a light-chain variable region comprising a CDR1 region of SEQ ID NO: 1,027, a CDR2 region of SEQ ID NO: 1,028, and a CDR3 region of SEQ ID NO: 1,029, and b) a heavy-chain variable region comprising a CDR1 region of SEQ ID NO: 1,030, a CDR2 region of SEQ ID NO: 1,031, and a CDR3 region of SEQ ID NO: 1,032;
173) a binding molecule comprising a) a light-chain variable region comprising a CDR1 region of SEQ ID NO: 1,033, a CDR2 region of SEQ ID NO: 1,034, and a CDR3 region of SEQ ID NO: 1,035, and b) a heavy-chain variable region comprising a CDR1 region of SEQ ID NO: 1,036, a CDR2 region of SEQ ID NO: 1,037, and a CDR3 region of SEQ ID NO: 1,038;
174) a binding molecule comprising a) a light-chain variable region comprising a CDR1 region of SEQ ID NO: 1,039, a CDR2 region of SEQ ID NO: 1,040, and a CDR3 region of SEQ ID NO: 1,041, and b) a heavy-chain variable region comprising a CDR1 region of SEQ ID NO: 1,042, a CDR2 region of SEQ ID NO: 1,043, and a CDR3 region of SEQ ID NO: 1,044;
175) a binding molecule comprising a) a light-chain variable region comprising a CDR1 region of SEQ ID NO: 1,045, a CDR2 region of SEQ ID NO: 1,046, and a CDR3 region of SEQ ID NO: 1,047, and b) a heavy-chain variable region comprising a CDR1 region of SEQ ID NO: 1,048, a CDR2 region of SEQ ID NO: 1,049, and a CDR3 region of SEQ ID NO: 1,050;
176) a binding molecule comprising a) a light-chain variable region comprising a CDR1 region of SEQ ID NO: 1,051, a CDR2 region of SEQ ID NO: 1,052, and a CDR3 region of SEQ ID NO: 1,053, and b) a heavy-chain variable region comprising a CDR1 region of SEQ ID NO: 1,054, a CDR2 region of SEQ ID NO: 1,055, and a CDR3 region of SEQ ID NO: 1,056;
177) a binding molecule comprising a) a light-chain variable region comprising a CDR1 region of SEQ ID NO: 1,057, a CDR2 region of SEQ ID NO: 1,058, and a CDR3 region of SEQ ID NO: 1,059, and b) a heavy-chain variable region comprising a CDR1 region of SEQ ID NO: 1,060, a CDR2 region of SEQ ID NO: 1,061, and a CDR3 region of SEQ ID NO: 1,062;
178) a binding molecule comprising a) a light-chain variable region comprising a CDR1 region of SEQ ID NO: 1,063, a CDR2 region of SEQ ID NO: 1,064, and a CDR3 region of SEQ ID NO: 1,065, and b) a heavy-chain variable region comprising a CDR1 region of SEQ ID NO: 1,066, a CDR2 region of SEQ ID NO: 1,067, and a CDR3 region of SEQ ID NO: 1,068;
179) a binding molecule comprising a) a light-chain variable region comprising a CDR1 region of SEQ ID NO: 1,069, a CDR2 region of SEQ ID NO: 1,070, and a CDR3 region of SEQ ID NO: 1,071, and b) a heavy-chain variable region comprising a CDR1 region of SEQ ID NO: 1,072, a CDR2 region of SEQ ID NO: 1,073, and a CDR3 region of SEQ ID NO: 1,074;
180) a binding molecule comprising a) a light-chain variable region comprising a CDR1 region of SEQ ID NO: 1,075, a CDR2 region of SEQ ID NO: 1,076, and a CDR3 region of SEQ ID NO: 1,077, and b) a heavy-chain variable region comprising a CDR1 region of SEQ ID NO: 1,078, a CDR2 region of SEQ ID NO: 1,079, and a CDR3 region of SEQ ID NO: 1,080;
181) a binding molecule comprising a) a light-chain variable region comprising a CDR1 region of SEQ ID NO: 1,081, a CDR2 region of SEQ ID NO: 1,082, and a CDR3 region of SEQ ID NO: 1,083, and b) a heavy-chain variable region comprising a CDR1 region of SEQ ID NO: 1,084, a CDR2 region of SEQ ID NO: 1,085, and a CDR3 region of SEQ ID NO: 1,086;
182) a binding molecule comprising a) a light-chain variable region comprising a CDR1 region of SEQ ID NO: 1,087, a CDR2 region of SEQ ID NO: 1,088, and a CDR3 region of SEQ ID NO: 1,089, and b) a heavy-chain variable region comprising a CDR1 region of SEQ ID NO: 1,090, a CDR2 region of SEQ ID NO: 1,091, and a CDR3 region of SEQ ID NO: 1,092;
183) a binding molecule comprising a) a light-chain variable region comprising a CDR1 region of SEQ ID NO: 1,093, a CDR2 region of SEQ ID NO: 1,094, and a CDR3 region of SEQ ID NO: 1,095, and b) a heavy-chain variable region comprising a CDR1 region of SEQ ID NO: 1,096, a CDR2 region of SEQ ID NO: 1,097, and a CDR3 region of SEQ ID NO: 1,098;
184) a binding molecule comprising a) a light-chain variable region comprising a CDR1 region of SEQ ID NO: 1,099, a CDR2 region of SEQ ID NO: 1,100, and a CDR3 region of SEQ ID NO: 1,101, and b) a heavy-chain variable region comprising a CDR1 region of SEQ ID NO: 1,102, a CDR2 region of SEQ ID NO: 1,103, and a CDR3 region of SEQ ID NO: 1,104;
185) a binding molecule comprising a) a light-chain variable region comprising a CDR1 region of SEQ ID NO: 1,105, a CDR2 region of SEQ ID NO: 1,106, and a CDR3 region of SEQ ID NO: 1,107, and b) a heavy-chain variable region comprising a CDR1 region of SEQ ID NO: 1,108, a CDR2 region of SEQ ID NO: 1,109, and a CDR3 region of SEQ ID NO: 1,110;
186) a binding molecule comprising a) a light-chain variable region comprising a CDR1 region of SEQ ID NO: 1,111, a CDR2 region of SEQ ID NO: 1,112, and a CDR3 region of SEQ ID NO: 1,113, and b) a heavy-chain variable region comprising a CDR1 region of SEQ ID NO: 1,114, a CDR2 region of SEQ ID NO: 1,115, and a CDR3 region of SEQ ID NO: 1,116;
187) a binding molecule comprising a) a light-chain variable region comprising a CDR1 region of SEQ ID NO: 1,117, a CDR2 region of SEQ ID NO: 1,118, and a CDR3 region of SEQ ID NO: 1,119, and b) a heavy-chain variable region comprising a CDR1 region of SEQ ID NO: 1,120, a CDR2 region of SEQ ID NO: 1,121, and a CDR3 region of SEQ ID NO: 1,122;
188) a binding molecule comprising a) a light-chain variable region comprising a CDR1 region of SEQ ID NO: 1,123, a CDR2 region of SEQ ID NO: 1,124, and a CDR3 region of SEQ ID NO: 1,125, and b) a heavy-chain variable region comprising a CDR1 region of SEQ ID NO: 1,126, a CDR2 region of SEQ ID NO: 1,127, and a CDR3 region of SEQ ID NO: 1,128;
189) a binding molecule comprising a) a light-chain variable region comprising a CDR1 region of SEQ ID NO: 1,129, a CDR2 region of SEQ ID NO: 1,130, and a CDR3 region of SEQ ID NO: 1,131, and b) a heavy-chain variable region comprising a CDR1 region of SEQ ID NO: 1,132, a CDR2 region of SEQ ID NO: 1,133, and a CDR3 region of SEQ ID NO: 1,134; and
190) a binding molecule comprising a) a light-chain variable region comprising a CDR1 region of SEQ ID NO: 1,135, a CDR2 region of SEQ ID NO: 1,136, and a CDR3 region of SEQ ID NO: 1,137, and b) a heavy-chain variable region comprising a CDR1 region of SEQ ID NO: 1,138, a CDR2 region of SEQ ID NO: 1,139, and a CDR3 region of SEQ ID NO: 1,140.

In one embodiment of the present invention, the binding molecule is a binding molecule that binds to a spike protein (S protein) on the surface of SARS-coronavirus-2 (SARS-CoV-2), and comprises a binding molecule that competes with any one binding molecule selected from the group consisting of the following binding molecules 1) to 49), and this binding molecule is included within the scope of the present invention as long as it achieves the purposes and effects of the present invention:
1) a binding molecule comprising a) a light-chain variable region comprising a CDR1 region of SEQ ID NO: 7, a CDR2 region of SEQ ID NO: 8, and a CDR3 region of SEQ ID NO: 9, and b) a heavy-chain variable region comprising a CDR1 region of SEQ ID NO: 10, a CDR2 region of SEQ ID NO: 11, and a CDR3 region of SEQ ID NO: 12;
2) a binding molecule comprising a) a light-chain variable region comprising a CDR1 region of SEQ ID NO: 13, a CDR2 region of SEQ ID NO: 14, and a CDR3 region of SEQ ID NO: 15, and b) a heavy-chain variable region comprising a CDR1 region of SEQ ID NO: 16, a CDR2 region of SEQ ID NO: 17, and a CDR3 region of SEQ ID NO: 18;
3) a binding molecule comprising a) a light-chain variable region comprising a CDR1 region of SEQ ID NO: 67, a CDR2 region of SEQ ID NO: 68, and a CDR3 region of SEQ ID NO: 69, and b) a heavy-chain variable region comprising a CDR1 region of SEQ ID NO: 70, a CDR2 region of SEQ ID NO: 71, and a CDR3 region of SEQ ID NO: 72;
4) a binding molecule comprising a) a light-chain variable region comprising a CDR1 region of SEQ ID NO: 85, a CDR2 region of SEQ ID NO: 86, and a CDR3 region of SEQ ID NO: 87, and b) a heavy-chain variable region comprising a CDR1 region of SEQ ID NO: 88, a CDR2 region of SEQ ID NO: 89, and a CDR3 region of SEQ ID NO: 90;
5) a binding molecule comprising a) a light-chain variable region comprising a CDR1 region of SEQ ID NO: 91, a CDR2 region of SEQ ID NO: 92, and a CDR3 region of SEQ ID NO: 93, and b) a heavy-chain variable region comprising a CDR1 region of SEQ ID NO: 94, a CDR2 region of SEQ ID NO: 95, and a CDR3 region of SEQ ID NO: 96;
6) a binding molecule comprising a) a light-chain variable region comprising a CDR1 region of SEQ ID NO: 103, a CDR2 region of SEQ ID NO: 104, and a CDR3 region of SEQ ID NO: 105, and b) a heavy-chain variable region comprising a CDR1 region of SEQ ID NO: 106, a CDR2 region of SEQ ID NO: 107, and a CDR3 region of SEQ ID NO: 108;
7) a binding molecule comprising a) a light-chain variable region comprising a CDR1 region of SEQ ID NO: 109, CDR2 region of SEQ ID NO: 110, and CDR3 region of SEQ ID NO: 111, and b) a heavy-chain variable region comprising a CDR1 region of SEQ ID NO: 112, a CDR2 region of SEQ ID NO: 113, and a CDR3 region of SEQ ID NO: 114;
8) a binding molecule comprising a) a light-chain variable region comprising a CDR1 region of SEQ ID NO: 115, a CDR2 region of SEQ ID NO: 116, and a CDR3 region of SEQ ID NO: 117, and b) a heavy-chain variable region comprising a CDR1 region of SEQ ID NO: 118, a CDR2 region of SEQ ID NO: 119, and a CDR3 region of SEQ ID NO: 120;
9) a binding molecule comprising a) a light-chain variable region comprising a CDR1 region of SEQ ID NO: 127, a CDR2 region of SEQ ID NO: 128, and a CDR3 region of SEQ ID NO: 129, and b) a heavy-chain variable region comprising a CDR1 region of SEQ ID NO: 130, a CDR2 region of SEQ ID NO: 131, and a CDR3 region of SEQ ID NO: 132;
10) a binding molecule comprising a) a light-chain variable region comprising a CDR1 region of SEQ ID NO: 133, a CDR2 region of SEQ ID NO: 134, and a CDR3 region of SEQ ID NO: 135, and b) a heavy-chain variable region comprising a CDR1 region of SEQ ID NO: 136, a CDR2 region of SEQ ID NO: 137, and a CDR3 region of SEQ ID NO: 138;
11) a binding molecule comprising a) a light-chain variable region comprising a CDR1 region of SEQ ID NO: 139, a CDR2 region of SEQ ID NO: 140, and a CDR3 region of SEQ ID NO: 141, and b) a heavy-chain variable region comprising a CDR1 region of SEQ ID NO: 142, a CDR2 region of SEQ ID NO: 143, and a CDR3 region of SEQ ID NO: 144;
12) a binding molecule comprising a) a light-chain variable region comprising a CDR1 region of SEQ ID NO: 151, a CDR2 region of SEQ ID NO: 152, and a CDR3 region of SEQ ID NO: 153, and b) a heavy-chain variable region comprising a CDR1 region of SEQ ID NO: 154, a CDR2 region of SEQ ID NO: 155, and a CDR3 region of SEQ ID NO: 156;
13) a binding molecule comprising a) a light-chain variable region comprising a CDR1 region of SEQ ID NO: 181, a CDR2 region of SEQ ID NO: 182, and a CDR3 region of SEQ ID NO: 183, and b) a heavy-chain variable region comprising a CDR1 region of SEQ ID NO: 184, a CDR2 region of SEQ ID NO: 185, and a CDR3 region of SEQ ID NO: 186;
14) a binding molecule comprising a) a light-chain variable region comprising a CDR1 region of SEQ ID NO: 187, a CDR2 region of SEQ ID NO: 188, and a CDR3 region of SEQ ID NO: 189, and b) a heavy-chain variable region comprising a CDR1 region of SEQ ID NO: 190, a CDR2 region of SEQ ID NO: 191, and a CDR3 region of SEQ ID NO: 192;
15) a binding molecule comprising a) a light-chain variable region comprising a CDR1 region of SEQ ID NO: 193, a CDR2 region of SEQ ID NO: 194, and a CDR3 region of SEQ ID NO: 195, and b) a heavy-chain variable region comprising a CDR1 region of SEQ ID NO: 196, a CDR2 region of SEQ ID NO: 197, and a CDR3 region of SEQ ID NO: 198;
16) a binding molecule comprising a) a light-chain variable region comprising a CDR1 region of SEQ ID NO: 199, a CDR2 region of SEQ ID NO: 200, and a CDR3 region of SEQ ID NO: 201, and b) a heavy-chain variable region comprising a CDR1 region of SEQ ID NO: 202, a CDR2 region of SEQ ID NO: 203, and a CDR3 region of SEQ ID NO: 204;
17) a binding molecule comprising a) a light-chain variable region comprising a CDR1 region of SEQ ID NO: 217, a CDR2 region of SEQ ID NO: 218, and a CDR3 region of SEQ ID NO: 219, and b) a heavy-chain variable region comprising a CDR1 region of SEQ ID NO: 220, a CDR2 region of SEQ ID NO: 221, and a CDR3 region of SEQ ID NO: 222;
18) a binding molecule comprising a) a light-chain variable region comprising a CDR1 region of SEQ ID NO: 253, a CDR2 region of SEQ ID NO: 254, and a CDR3 region of SEQ ID NO: 255, and b) a heavy-chain variable region comprising a CDR1 region of SEQ ID NO: 256, a CDR2 region of SEQ ID NO: 257, and a CDR3 region of SEQ ID NO: 258;
19) a binding molecule comprising a) a light-chain variable region comprising a CDR1 region of SEQ ID NO: 265, a CDR2 region of SEQ ID NO: 266, and a CDR3 region of SEQ ID NO: 267, and b) a heavy-chain variable region comprising a CDR1 region of SEQ ID NO: 268, a CDR2 region of SEQ ID NO: 269, and a CDR3 region of SEQ ID NO: 270;
20) a binding molecule comprising a) a light-chain variable region comprising a CDR1 region of SEQ ID NO: 283, a CDR2 region of SEQ ID NO: 284, and a CDR3 region of SEQ ID NO: 285, and b) a heavy-chain variable region comprising a CDR1 region of SEQ ID NO: 286, a CDR2 region of SEQ ID NO: 287, and a CDR3 region of SEQ ID NO: 288;
21) a binding molecule comprising a) a light-chain variable region comprising a CDR1 region of SEQ ID NO: 295, a CDR2 region of SEQ ID NO: 296, and a CDR3 region of SEQ ID NO: 297, and b) a heavy-chain variable region comprising a CDR1 region of SEQ ID NO: 298, a CDR2 region of SEQ ID NO: 299, and a CDR3 region of SEQ ID NO: 300;
22) a binding molecule comprising a) a light-chain variable region comprising a CDR1 region of SEQ ID NO: 301, a CDR2 region of SEQ ID NO: 302, and a CDR3 region of SEQ ID NO: 303, and b) a heavy-chain variable region comprising a CDR1 region of SEQ ID NO: 304, a CDR2 region of SEQ ID NO: 305, and a CDR3 region of SEQ ID NO: 306;
23) a binding molecule comprising a) a light-chain variable region comprising a CDR1 region of SEQ ID NO: 313, a CDR2 region of SEQ ID NO: 314, and a CDR3 region of SEQ ID NO: 315, and b) a heavy-chain variable region comprising a CDR1 region of SEQ ID NO: 316, a CDR2 region of SEQ ID NO: 317, and a CDR3 region of SEQ ID NO: 318;
24) a binding molecule comprising a) a light-chain variable region comprising a CDR1 region of SEQ ID NO: 319, a CDR2 region of SEQ ID NO: 320, and a CDR3 region of SEQ ID NO: 321, and b) a heavy-chain variable region comprising a CDR1 region of SEQ ID NO: 322, a CDR2 region of SEQ ID NO: 323, and a CDR3 region of SEQ ID NO: 324;
25) a binding molecule comprising a) a light-chain variable region comprising a CDR1 region of SEQ ID NO: 325, a CDR2 region of SEQ ID NO: 326, and a CDR3 region of SEQ ID NO: 327, and b) a heavy-chain variable region comprising a CDR1 region of SEQ ID NO: 328, a CDR2 region of SEQ ID NO: 329, and a CDR3 region of SEQ ID NO: 330;
26) a binding molecule comprising a) a light-chain variable region comprising a CDR1 region of SEQ ID NO: 331, a CDR2 region of SEQ ID NO: 332, and a CDR3 region of SEQ ID NO: 333, and b) a heavy-chain variable region comprising a CDR1 region of SEQ ID NO: 334, a CDR2 region of SEQ ID NO: 335, and a CDR3 region of SEQ ID NO: 336;
27) a binding molecule comprising a) a light-chain variable region comprising a CDR1 region of SEQ ID NO: 349, a CDR2 region of SEQ ID NO: 350, and a CDR3 region of SEQ ID NO: 351, and b) a heavy-chain variable region comprising a CDR1 region of SEQ ID NO: 352, a CDR2 region of SEQ ID NO: 353, and a CDR3 region of SEQ ID NO: 354;
28) a binding molecule comprising a) a light-chain variable region comprising a CDR1 region of SEQ ID NO: 355, a CDR2 region of SEQ ID NO: 356, and a CDR3 region of SEQ ID NO: 357, and b) a heavy-chain variable region comprising a CDR1 region of SEQ ID NO: 358, a CDR2 region of SEQ ID NO: 359, and a CDR3 region of SEQ ID NO: 360;
29) a binding molecule comprising a) a light-chain variable region comprising a CDR1 region of SEQ ID NO: 361, a CDR2 region of SEQ ID NO: 362, and a CDR3 region of SEQ ID NO: 363, and b) a heavy-chain variable region comprising a CDR1 region of SEQ ID NO: 364, a CDR2 region of SEQ ID NO: 365, and a CDR3 region of SEQ ID NO: 366;
30) a binding molecule comprising a) a light-chain variable region comprising a CDR1 region of SEQ ID NO: 391, a CDR2 region of SEQ ID NO: 392, and a CDR3 region of SEQ ID NO: 393, and b) a heavy-chain variable region comprising a CDR1 region of SEQ ID NO: 394, a CDR2 region of SEQ ID NO: 395, and a CDR3 region of SEQ ID NO: 396;
31) a binding molecule comprising a) a light-chain variable region comprising a CDR1 region of SEQ ID NO: 409, a CDR2 region of SEQ ID NO: 410, and a CDR3 region of SEQ ID NO: 411, and b) a heavy-chain variable region comprising a CDR1 region of SEQ ID NO: 412, a CDR2 region of SEQ ID NO: 413, and a CDR3 region of SEQ ID NO: 414;
32) a binding molecule comprising a) a light-chain variable region comprising a CDR1 region of SEQ ID NO: 439, a CDR2 region of SEQ ID NO: 440, and a CDR3 region of SEQ ID NO: 441, and b) a heavy-chain variable region comprising a CDR1 region of SEQ ID NO: 442, a CDR2 region of SEQ ID NO: 443, and a CDR3 region of SEQ ID NO: 444;
33) a binding molecule comprising a) a light-chain variable region comprising a CDR1 region of SEQ ID NO: 445, a CDR2 region of SEQ ID NO: 446, and a CDR3 region of SEQ ID NO: 447, and b) a heavy-chain variable region comprising a CDR1 region of SEQ ID NO: 448, a CDR2 region of SEQ ID NO: 449, and a CDR3 region of SEQ ID NO: 450;
34) a binding molecule comprising a) a light-chain variable region comprising a CDR1 region of SEQ ID NO: 451, a CDR2 region of SEQ ID NO: 452, and a CDR3 region of SEQ ID NO: 453, and b) a heavy-chain variable region comprising a CDR1 region of SEQ ID NO: 454, a CDR2 region of SEQ ID NO: 455, and a CDR3 region of SEQ ID NO: 456;
35) a binding molecule comprising a) a light-chain variable region comprising a CDR1 region of SEQ ID NO: 481, a CDR2 region of SEQ ID NO: 482, and a CDR3 region of SEQ ID NO: 483, and b) a heavy-chain variable region comprising a CDR1 region of SEQ ID NO: 484, a CDR2 region of SEQ ID NO: 485, and a CDR3 region of SEQ ID NO: 486;
36) a binding molecule comprising a) a light-chain variable region comprising a CDR1 region of SEQ ID NO: 487, a CDR2 region of SEQ ID NO: 488, and a CDR3 region of SEQ ID NO: 489, and b) a heavy-chain variable region comprising a CDR1 region of SEQ ID NO: 490, a CDR2 region of SEQ ID NO: 491, and a CDR3 region of SEQ ID NO: 492;
37) a binding molecule comprising a) a light-chain variable region comprising a CDR1 region of SEQ ID NO: 493, a CDR2 region of SEQ ID NO: 494, and a CDR3 region of SEQ ID NO: 495, and b) a heavy-chain variable region comprising a CDR1 region of SEQ ID NO: 496, a CDR2 region of SEQ ID NO: 497, and a CDR3 region of SEQ ID NO: 498;
38) a binding molecule comprising a) a light-chain variable region comprising a CDR1 region of SEQ ID NO: 505, a CDR2 region of SEQ ID NO: 506, and a CDR3 region of SEQ ID NO: 507, and b) a heavy-chain variable region comprising a CDR1 region of SEQ ID NO: 508, a CDR2 region of SEQ ID NO: 509, and a CDR3 region of SEQ ID NO: 510;
39) a binding molecule comprising a) a light-chain variable region comprising a CDR1 region of SEQ ID NO: 511, a CDR2 region of SEQ ID NO: 512, and a CDR3 region of SEQ ID NO: 513, and b) a heavy-chain variable region comprising a CDR1 region of SEQ ID NO: 514, a CDR2 region of SEQ ID NO: 515, and a CDR3 region of SEQ ID NO: 516;
40) a binding molecule comprising a) a light-chain variable region comprising a CDR1 region of SEQ ID NO: 517, a CDR2 region of SEQ ID NO: 518, and a CDR3 region of SEQ ID NO: 519, and b) a heavy-chain variable region comprising a CDR1 region of SEQ ID NO: 520, a CDR2 region of SEQ ID NO: 521, and a CDR3 region of SEQ ID NO: 522;
41) a binding molecule comprising a) a light-chain variable region comprising a CDR1 region of SEQ ID NO: 553, a CDR2 region of SEQ ID NO: 554, and a CDR3 region of SEQ ID NO: 555, and b) a heavy-chain variable region comprising a CDR1 region of SEQ ID NO: 556, a CDR2 region of SEQ ID NO: 557, and a CDR3 region of SEQ ID NO: 558;
42) a binding molecule comprising a) a light-chain variable region comprising a CDR1 region of SEQ ID NO: 559, a CDR2 region of SEQ ID NO: 560, and a CDR3 region of SEQ ID NO: 561, and b) a heavy-chain variable region comprising a CDR1 region of SEQ ID NO: 562, a CDR2 region of SEQ ID NO: 563, and a CDR3 region of SEQ ID NO: 564;
43) a binding molecule comprising a) a light-chain variable region comprising a CDR1 region of SEQ ID NO: 565, a CDR2 region of SEQ ID NO: 566, and a CDR3 region of SEQ ID NO: 567, and b) a heavy-chain variable region comprising a CDR1 region of SEQ ID NO: 568, a CDR2 region of SEQ ID NO: 569, and a CDR3 region of SEQ ID NO: 570;
44) a binding molecule comprising a) a light-chain variable region comprising a CDR1 region of SEQ ID NO: 571, a CDR2 region of SEQ ID NO: 572, and a CDR3 region of SEQ ID NO: 573, and b) a heavy-chain variable region comprising a CDR1 region of SEQ ID NO: 574, a CDR2 region of SEQ ID NO: 575, and a CDR3 region of SEQ ID NO: 576;
45) a binding molecule comprising a) a light-chain variable region comprising a CDR1 region of SEQ ID NO: 583, a CDR2 region of SEQ ID NO: 584, and a CDR3 region of SEQ ID NO: 585, and b) a heavy-chain variable region comprising a CDR1 region of SEQ ID NO: 586, a CDR2 region of SEQ ID NO: 587, and a CDR3 region of SEQ ID NO: 588;
46) a binding molecule comprising a) a light-chain variable region comprising a CDR1 region of SEQ ID NO: 607, a CDR2 region of SEQ ID NO: 608, and a CDR3 region of SEQ ID NO: 609, and b) a heavy-chain variable region comprising a CDR1 region of SEQ ID NO: 610, a CDR2 region of SEQ ID NO: 611, and a CDR3 region of SEQ ID NO: 612;
47) a binding molecule comprising a) a light-chain variable region comprising a CDR1 region of SEQ ID NO: 613, a CDR2 region of SEQ ID NO: 614, and a CDR3 region of SEQ ID NO: 615, and b) a heavy-chain variable region comprising a CDR1 region of SEQ ID NO: 616, a CDR2 region of SEQ ID NO: 617, and a CDR3 region of SEQ ID NO: 618;
48) a binding molecule comprising a) a light-chain variable region comprising a CDR1 region of SEQ ID NO: 619, a CDR2 region of SEQ ID NO: 620, and a CDR3 region of SEQ ID NO: 621, and b) a heavy-chain variable region comprising a CDR1 region of SEQ ID NO: 622, a CDR2 region of SEQ ID NO: 623, and a CDR3 region of SEQ ID NO: 624; and
49) a binding molecule comprising a) a light-chain variable region comprising a CDR1 region of SEQ ID NO: 643, a CDR2 region of SEQ ID NO: 644, and a CDR3 region of SEQ ID NO: 645, and b) a heavy-chain variable region comprising a CDR1 region of SEQ ID NO: 646, a CDR2 region of SEQ ID NO: 647, and a CDR3 region of SEQ ID NO: 648.

In one embodiment of the present invention, the binding molecule may be a binding molecule comprising a light-chain variable region comprising the following LC CDR1, LC CDR2 and LC CDR3, and a heavy-chain variable region comprising the following HC CDR1, HC CDR2 and HC CDR3.

The LC CDR1 is:
the sequence SGSX₁SNIGX₂NTVN, where X₁ is S or T, and X₂ is S or T; or
the sequence X₁₃GSX₁₄SX₁₅IX₁₆X₁₇X₁₈X₁₉VS, where X₁₃ is S or T, X₁₄ is S or N, X₁₅ is N or S, X₁₆ is G or E, X₁₇ is Nor S, X₁₈ is N or S, and X₁₉ is Y or F; or
the sequence RASQSVX₃₂X₃₃X₃₄X₃₅A, where X₃₂ is S, N or R, X₃₃ is S or N, X₃₄ is Y or N, and X₃₅ is L or S; or
the sequence RASQSVX₄₈X₄₉X₅₀YLA, where X₄₈ is S or G, X₄₉ is S or R, and X₅₀ is S or N; or
the sequence TGTSSX₆₂VGX₆₃YX₆₄X₆₅VS, where X₆₂ is N or D, X₆₃ is D or G, X₆₄ is N or S, and X₆₅ is Y or H; or
the sequence SGX₆₉X₇₀SNIGSNX₇₁VX₇₂, where X₆₉ is S or L, X₇₀ is S or T, X₇₁ is Y, T or F, and X₇₂ is Y, H or N; or
the sequence TGTSSDVGGYNYVS; or
the sequence RASQSVSSSYLA; or
the sequence TGX₈₉X₉₀SDX₉₁GGYNX₉₂X₉₃S, where X₈₉ is N or T, X₉₀ is R or S, X₉₁ is I or V, X₉₂ is S or Y, and X₉₃ is L or V; or
the sequence SGSSSNIGNNYVS; or
the sequence GGNNIGSKSVH; or
the sequence X₁₀₆GTX₁₀₇SDVGGYNYVS, where X₁₀₆ is T or S, and X₁₀₇ is S or N; or
the sequence SGSSSNIGSNX₁₁₁VN, where X₁₁₁ is A or T; or
the sequence TGSSSNIGAGYDVH; or
the sequence RASQSVSSYLA; or
the sequence X₁₂₁GSX₁₂₂SNIGX₁₂₃NX₁₂₄VX₁₂₅, where X₁₂₁ is S or T, X₁₂₂ is S or R, X₁₂₃ is S or I, X₁₂₄ is T or S, and X₁₂₅ is S or N; or
the sequence SGSSSNIGSNTVN; or
the sequence TGTSSDIGGYNYVS; or
the sequence RASQSVSX₁₃₄X₁₃₅LA, where X₁₃₄ is S or T, and X₁₃₅ is Y or F; or
the sequence TGTSX₁₄₂DX₁₄₃GX₁₄₄YNYVS, where X₁₄₂ is T or S, X₁₄₃ is I or V, and X₁₄₄ is R or A; or
the sequence RASQX₁₆₁X₁₆₂X₁₆₃X₁₆₄X₁₆₅X₁₆₆A, where X₁₆₁ is S or G, X₁₆₂ is V or I, X₁₆₃ is S or R, X₁₆₄ is S or N, X₁₆₅ is Y, S or D, and X₁₆₆ is L or I; or
the sequence X₁₇₃ASQX₁₇₄ISSYLN, where X₁₇₃ is R or Q, and X₁₇₄ is S or D; or
the sequence SGSX₁₇₇SNIGX₁₇₈NYVX₁₇₉, where X₁₇₇ is S or G, X₁₇₈ is N or S, and X₁₇₉ is S or H; or
the sequence TGTRGDIGAYDGVS; or
the sequence TGTX₁₈₇X₁₈₈DX₁₈₉GX₁₉₀YX₁₉₁X₁₉₂VS, where X₁₈₇ is S or R, X₁₈₈ is S or G, X₁₈₉ is V or I, X₁₉₀ is G or A, X₁₉₁ is N or D, and X₁₉₂ is Y or G; or
the sequence SGSSSNIGX₂₀₅NX₂₀₆VX₂₀₇, where X₂₀₅ is S or N, X₂₀₆ is T or Y, and X₂₀₇ is N or S; or
the sequence TGTSX₂₁₃DVGX₂₁₄YNX₂₁₅VS, where X₂₁₃ is T or S, X₂₁₄ is G or S, and X₂₁₅ is Y or L; or
the sequence TGTSSDVGX₂₃₂YNYVS, where X₂₃₂ is N or G; or
the sequence RASQSVSSNLA; or
the sequence RASQGISNSL; or
the sequence RASQSVSSX₂₅₈LA, where X₂₅₈ is Y or F; or
the sequence SSSTGAVTRGHFPN; or
the sequence CRASQSVSSSYLA; or
the sequence RASQSISSYLN; or
the sequence TGTSSDVGGYNX₂₉₅VS, where X₂₉₅ is Y or N; or
the sequence SGSSSNIGX₃₀₈NYVS, where X₃₀₈ is R or N; or
the sequence SGSSSX₃₃₇X₃₃₈GSNX₃₃₉VN, where X₃₃₇ is N or D, X₃₃₈ is V or I, and X₃₃₉ is I or T; or
the sequence SGSSSNIGX₃₅₃NYVX₃₅₄, where X₃₅₃ is N or S, and X₃₅₄ is S or Y; or
the sequence SGSSSNIGX₃₇₃NYVX₃₇₄, where X₃₇₃ is N or S, and X₃₇₄ is S or N.

The LC CDR2 is:
the sequence X₃X₄NX₅RPS, where X₃ is S or T, X₄ is N or D, and X₅ Q is R; or
the sequence X₂₀NX₂₁X₂₂RPS, where X₂₀ is D or R, X₂₁ is N or S, and X₂₂ is K, R, Q or V; or
the sequence X₃₆ASX₃₇RAT, where X₃₆ is D or G, and X₃₇ is N, T or S; or
the sequence X₅₁ASX₅₂RAT, where X₅₁ is G or D, and X₅₂ is S or T or
the sequence X₆₆VX₆₇X₆₈RPS, where X₆₆ is D or E, X₆₇ is T or S, and X₆₈ is N or D; or
the sequence X₇₃NX₇₄X₇₅RPS, where X₇₃ is R or S, X₇₄ is N or D, and X₇₅ is Q or E; or
the sequence X₈₃VSNRPS, where X₈₃ is D or E; or
the sequence GASSRAT; or
the sequence DVX₉₄NRPS, where X₉₄ is N or S; or
the sequence DX₉₈X₉₉X₁₀₀RPS, where X₉₈ is N or D, X₉₉ is N or S, and X₁₀₀ is K or D; or
the sequence DVX₁₀₈KRPS, where X₁₀₈ is S or T; or
the sequence X₁₁₂NSX₁₁₃RPS, where X₁₁₂ is G or S, and X₁₁₃ is N or Y; or
the sequence DASNRAT; or
the sequence SNNQRPS; or
the sequence X₁₂₇NX₁₂₈X₁₂₉RPS, where X₁₂₇ is G or S, X₁₂₈ is S or N, and X₁₂₉ is N or Q; or
the sequence X₁₃₂X₁₃₃SKRPS, where X₁₃₂ is A or Q, and X₁₃₃ is V or D; or
the sequence DASNRA; or
the sequence DVSKRPS; or
the sequence DNNKRPS; or
the sequence X₁₅₂NNX₁₅₃RPS, where X₁₅₂ is S, T or N, and X₁₅₃ is Q or R; or
the sequence DX₁₆₇SX₁₆₈X₁₆₉X₁₇₀T, where X₁₆₇ is A or T, X₁₆₈ is N or T, X₁₆₉ is R or L, and X₁₇₀ is A or E; or
the sequence AASRLES; or
the sequence X₁₇₅ASSLX₁₇₆S, where X₁₇₅ is K or A, and X₁₇₆ is E or Q; or
the sequence X₁₈₀NX₁₈₁X₁₈₂RPS, where X₁₈₀ is D or R, X₁₈₁ is N or S, and X₁₈₂ is K or G; or
the sequence AVTQRPS; or
the sequence X₁₈₄NX₁₈₅X₁₈₆RPS, where X₁₈₄ is G or D, X₁₈₅ is S or N, and X₁₈₆ is N or K; or
the sequence X₁₉₃VX₁₉₄X₁₉₅RPS, where X₁₉₃ is D or A, X₁₉₄ is S or T, and X₁₉₅ is K or Q; or
the sequence X₂₀₈NNX₂₀₉RPS, where X₂₀₈ is S or D, and X₂₀₉ is Q or K; or
the sequence DVSX₂₁₆RPS, where X₂₁₆ is K or N; or
the sequence DVX₂₁₇KRPS, where X₂₁₇ is G or S; or
the sequence GNSNRPS; or
the sequence DVSX₂₃₃RPS, where X₂₃₃ is N or K; or
the sequence X₂₅₀AX₂₅₁X₂₅₂RX₂₅₃X₂₅₄, where X₂₅₀ is W or A, X₂₅₁ is S or A, X₂₅₂ is T or S, X₂₅₃ is E or L, and X₂₅₄ is S or E; or
the sequence YDSDRPS; or
the sequence STSNKHS; or
the sequence AASSLQS; or
the sequence DVSX₂₉₃RPS, where X₂₉₃ is K or N; or
the sequence DVSX₂₉₆RPS, where X₂₉₆ is N or K; or
the sequence DNNX₃₀₉RPS, where X₃₀₉ is R or K; or
the sequence X₃₄₀NNQRPS, where X₃₄₀ is N or S; or
the sequence X₃₅₅NNX₃₅₆RPS, where X₃₅₅ is D or R, and X₃₅₆ is K or Q; or
the sequence X₃₇₅NNX₃₇₆RPS, where X₃₇₅ is D or S, and X₃₇₆ is K or Q.

The LC CDR3 is:
the sequence AX₆X₇DDX₈LX₉X₁₀X₁₁X₁₂, where X₆ is A or S, X₇ is W or R, X₈ is S or R, X₉ is Nor S, X₁₀ is A or G, X₁₁ is S, L, V, Y, P, A or T, X₁₂ is L or V; or
the sequence X₂₃X₂₄WDX₂₅SLSX₂₆X₂₇V, where X₂₃ is G or A, X₂₄ is T or A, X₂₅ is S, N, D or T, X₂₆ is A, G or S, and X₂₇ is W or Y; or
the sequence GTWDSSLSAYWV; or
the sequence X₃₈QX₃₉X₄₀X₄₁X₄₂PX₄₃T, where X₃₈ is Q or H, X₃₉ is Y or H, X₄₀ is G, N, Y or H, X₄₁ is S, N, T, Q or R, X₄₂ is S, W or V, and X₄₃ is L, F, I or Q; or
the sequence QQYGSSPSIT; or
the sequence QQYGX₅₃SPX₅₄X₅₅, where X₅₃ is S or T, X₅₄ is L or I, and X₅₅ is T or A; or
the sequence SSYTSGSTPVV; or
the sequence NSYTSSSTWV; or
the sequence X₇₆X₇₇WDDX₇₈LX₇₉GX₈₀V, where X₇₆ is A or S, X₇₇ is A or T, X₇₈ is S or T, X₇₉ is S, N or V, and X₈₀ is R or K; or
the sequence SSYAGSNYVV; or
the sequence SSYTSSSTX₈₄X₈₅V, where X₈₄ is L or R, and Xss is G or Y; or
the sequence QQYGSSPX₈₇T, where X₈₇ is Y or F; or
the sequence QQYGSSPALT; or
the sequence X₉₅SYTSX₉₆X₉₇TWV, where X₉₅ is N or S, X₉₆ is K or S, and X₉₇ is N or S; or
the sequence GTWDSSLSAGWV; or
the sequence X₁₀₁X₁₀₂WDSSX₁₀₃X₁₀₄X₁₀₅VV, where X₁₀₁ is G or Q, X₁₀₂ is T or V, X₁₀₃ is L or S, X₁₀₄ is S or D, and X₁₀₅ is A or H; or
the sequence SSYTSSX₁₀₉TWV, where X₁₀₉ is S or R; or
the sequence SSYTSSSTPWV; or
the sequence QSYDSX₁₁₄LX₁₁₅X₁₁₆X₁₁₇X₁₁₈, where X₁₁₄ is S or A, X₁₁₅ is S or R, X₁₁₆ is D or G, X₁₁₇ is V or P, and X₁₁₈ is V or L; or
the sequence QQRRNWPPX₁₁₉X₁₂₀T, where X₁₁₉ is V or R, and X₁₂₀ is L or I; or
the sequence AAWDDSLNGX₁₂₆V, where X₁₂₆ is V or Y; or
the sequence AAWDDSLX₁₃₀GX₁₃₁V, where X₁₃₀ is G or N, and X₁₃₁ is P or W; or
the sequence SSYTSSSGTLNV;
the sequence QAWDSSTV;
the sequence QQRSNWPPX₁₃₆X₁₃₇T, where X₁₃₆ is A, R, K or M, and X₁₃₇ is L, I or Y; or
the sequence SYTSSSTYA; or
the sequence X₁₄₅X₁₄₆WDX₁₄₇SLSX₁₄₈X₁₄₉X₁₅₀, where X₁₄₅ is G or E, X₁₄₆ is T or A, X₁₄₇ is S or T, X₁₄₈ is A or D, X₁₄₉ is V or G, and X₁₅₀ is V or L; or
the sequence AAWDDSLX₁₅₄GX₁₅₅V, where X₁₅₄ is N or S, and X₁₅₅ is L, P, W or V; or
the sequence or AAWDDSLNGX₁₅₆VV, where X₁₅₆ is S or H; or
the sequence QQYYX₁₇₁TPX₁₇₂T, where X₁₇₁ is S or T, and X₁₇₂ is F or I; or
the sequence QQSYSTWT; or
the sequence AAWDDSLX₁₈₃GWV, where X₁₈₃ is N or S; or
the sequence AAWDDSLNGVV; or
the sequence SSYTSSSSWV; or
the sequence QSYDSSLSGLWV; or
the sequence QSYDSSLSAWV; or
the sequence SSYTX₁₉₆SSX₁₉₇WV, where X₁₉₆ is T or S, and X₁₉₇ is T or S; or
the sequence AAWDDSLX₂₁₀GPV, where X₂₁₀ is N or S; or
the sequence SSYTSSSTLV; or
the sequence SSYTSSSTX₂₁₈V, where X₂₁₈ is W or L; or
the sequence QSYDSSLSGX₂₂₅WV, where X₂₂₅ is P or S; or
the sequence SSYTSSX₂₃₄TX₂₃₅V, where X₂₃₄ is G or S, and X₂₃₅ is L or Y; or
the sequence QSYDSSLSGWV; or
the sequence LQYFTIPWT; or
the sequence SQQYYSTPYT; or
the sequence QQYGSSPPIT; or
the sequence QQRSNWPPSIT; or
the sequence QVWDSSSDHWV; or
the sequence LLYDAGAPGWV; or
the sequence GAWDSSLSTPNW;
the sequence AAWDDSLNGWV;
the sequence QQYGSSPX₂₆₉T, where X₂₆₉ is Y or P; or
the sequence QQSYSTPLT; or
the sequence QQSYSTPX₂₇₅T, where X₂₇₅ is F or L; or
the sequence QSYDSSLSGPNWV; or
the sequence QSYDSRLRAVV; or
the sequence QSYDSSLSGX₂₉₄V, where X₂₉₄ is V or P; or
the sequence SSYTSSSPWV; or
the sequence GTWDSSLSAVX₃₁₀, where X₃₁₀ is A or V; or
the sequence GTWDSSLSAX₃₂₁V, where X₃₂₁ is G or V; or
the sequence AAWDDSLNX₃₄₁WV, where X₃₄₁ is A or G; or
the sequence X₃₅₇X₃₅₈WDX₃₅₉SLSX₃₆₀X₃₆₁X₃₆₂, where X₃₅₇ is G or A, X₃₅₈ is T or A, X₃₅₉ is S or D, X₃₆₀ is A or G, X₃₆₁ is G or W, and X₃₆₂ is P or V; or
the sequence X₃₇₇TWDX₃₇₈SLX₃₇₉X₃₈₀X₃₈₁V, where X₃₇₇ is G or A, X₃₇₈ is S or D, X₃₇₉ is S or N, X₃₈₀ is A or G, and X₃₈₁ is G or Q.

The HC CDR1 is:
the sequence GYYWS; or
the sequence X₂₈YYMH, where X₂₈ is S or G; or
the sequence X₄₄SSYYWG, where X₄₄ is S or G; or
the sequence SX₅₆X₅₇YYWX₅₈, where X₅₆ is S or G, X₅₇ is S or G, and X₅₈ is G or S; or
the sequence SSSYYWG; or
the sequence SYAIS; or
the sequence SYAMX₈₆, where X₈₆ is S or N; or
the sequence SSPMH; or
the sequence DYGMH; or
the sequence X₁₁₀YAMH, where X₁₁₀ is S or G; or
the sequence SYGIS; or
the sequence TYGMH; or
the sequence SYAIX₁₃₈, where X₁₃₈ is S or I; or
the sequence EVAIH; or
the sequence RYAMS; or
the sequence SX₁₅₇WIX₁₅₈, where X₁₅₇ is H or Y, and X₁₅₈ is A, V or G; or
the sequence DYYIQ; or
the sequence SYAMH; or
the sequence SNYMS; or
the sequence ELSX₁₉₈H, where X₁₉₈ is I or M; or
the sequence X₂₁₁YAMS, where X₂₁₁ is S or R; or
the sequence NYGMH; or
the sequence X₂₁₉YAIS, where X₂₁₉ is F or R; or
the sequence RYAIX₂₂₆, where X₂₂₆ is N or S; or
the sequence ELSIH; or
the sequence NYGIS; or
the sequence NAWMX₂₅₅, where X₂₅₅ is S or T; or
the sequence DYAMS; or
the sequence SYYIH; or
the sequence SYPMH; or
the sequence X₂₆₀X₂₆₁AMX₂₆₂, where X₂₆₀ is S or N, X₂₆₁ is F or Y, and X₂₆₂ is H or N; or
the sequence NYVIN; or
the sequence SNYMX₂₇₀, where X₂₇₀ is T or S, or
the sequence SX₂₇₆AX₂₇₇X₂₇₈, where X₂₇₆ is Y or S, X₂₇₇ is M or I, and X₂₇₈ is H or N, or
the sequence TQYLH; or
the sequence RGDYWG; or
the sequence THALS; or
the sequence DYAMH; or
the sequence SYDIS; or
the sequence SYAX₂₉₇S, where X₂₉₇ is M or I; or
the sequence SYX₃₁₁X₃₁₂X₃₁₃, where X₃₁₁ is Y or A, X₃₁₂ is M or I, and X₃₁₃ is H or I; or
the sequence SX₃₂₂X₃₂₃IX₃₂₄, where X₃₂₂ is N or Y, X₃₂₃ is W or V, and X₃₂₄ is A or S; or
the sequence NHWIA; or
the sequence NYAIN; or
the sequence TSGVGVG; or
the sequence ELPIH; or
the sequence DYX₃₈₂MX₃₈₃, where X₃₈₂ is G or W, and X₃₈₃ is H or S.

The HC CDR2 is:
the sequence EINHSGSTNYNPSLKS; or
the sequence X₂₉INRSGGSTIYAQTFQX₃₀, where X₂₉ is I or V, X₃₀ is G or S; or
the sequence X₄₅IX₄₆YSGSTYYNPSLKS, where X₄₅ is S or N, and X₄₆ is Y or F; or
the sequence X₅₉IX₆₀YSGSTYYNPSLKS, where X₅₉ is S, Y or N, and X₆₀ is Y or F; or
the sequence NIFYSGSTYYNPSLKS;
the sequence GIIPIFGTANYAQX₈₁FQX₈₂, where Xsi is K or R, and X₈₂ is G or D; or
the sequence AISGSGGTTYYADSVKG; or
the sequence VISYX₈₈GSNKYYADSVKG, where X₈₈ is D or G; or
the sequence VISYDGSNKYYADSVKG; or
the sequence AISYDGSNKYYADSVKG; or
the sequence VISYDGSHKNYADSVKG; or
the sequence GIIPILATTKFAQKFQG; or
the sequence GIIPIFGTANYAQKFQG; or
the sequence VISYDGFNKYYADSVKG;
the sequence GFDPEDGETSYAQKFQG; or
the sequence AISGSGGX₁₅₁TYYADSVKG, where X₁₅₁ is S or T; or
the sequence X₁₅₉IYPGDSDSRYSPSFQG, where X₁₅₉ is I or T; or
the sequence WINPNSGDTNYAHKFQG; or
the sequence GIIPMFGTTNYAQKFQG; or
the sequence AISGSGGSTYYADSVKG; or
the sequence VIYPGGSTFFADSVQG; or
the sequence GFDPEDX₁₉₉ETIYAQKFQG, where X₁₉₉ is G or A; or
the sequence AISGSGGX₂₁₂TYYADSVKG, where X₂₁₂ is D or S; or
the sequence VMSYDGSNKYYADSVKG; or
the sequence GIIPX₂₂₀FGX₂₂₁X₂₂₂X₂₂₃YAQX₂₂₄FQD, where X₂₂₀ is L or I, X₂₂₁ is T or K, X₂₂₂ is A or V, X₂₂₃ is K or N, and X₂₂₄ is R or K; or
the sequence GIIPX₂₂₇X₂₂₈GTX₂₂₉X₂₃₀YX₂₃₁QKFQG, where X₂₂₇ is L or I, X₂₂₈ is L or F, X₂₂₉ is A or G, X₂₃₀ is D or N, and X₂₃₁ is P or A; or
the sequence GFDPEX₂₃₆X₂₃₇ETIX₂₃₈AQX₂₃₉FQG, where X₂₃₆ is N or D, X₂₃₇ is G or A, X₂₃₈ is H or Y, and X₂₃₉ is R or K; or
the sequence X₂₄₆ISX₂₄₇X₂₄₈NGNTX₂₄₉YAQKLQG, where X₂₄₆ is G or W, X₂₄₇ is S or A, X₂₄₈ is H or Y, and X₂₄₉ is K or N; or
the sequence RIKTKTDGGTTDYAAPVKG; or
the sequence GIIPIFGTX₂₅₉NYAQKFQG, where X₂₅₉ is T or A; or
the sequence VISGSGGSTSNADSVKG; or
the sequence FINPSDVTTYYAQKFQG; or
the sequence VISYDGSLKYYVDSVKG; or
the sequence X₂₆₃ISX₂₆₄X₂₆₅GX₂₆₆X₂₆₇X₂₆₈YYADSVKG, where X₂₆₃ is V or T, X₂₆₄ is F or G, X₂₆₅ is D or S, X₂₆₆ is S or G, X₂₆₇ is N or S, and X₂₆₈ is K or T; or
the sequence GFIPVFGIADYAQKFQG; or
the sequence X₂₇₁IYPGGSTX₂₇₂X₂₇₃ADSVX₂₇₄G, where X₂₇₁ is I or V, X₂₇₂ is Y or F, X₂₇₃ is Y or F, and X₂₇₄ is K or Q; or
the sequence VISYDGINKYYADSVKG; or
the sequence GIIPIFGTVNYAQKFQG; or
the sequence X₂₈₆IX₂₈₇PX₂₈₈X₂₈₉GX₂₉₀X₂₉₁X₂₉₂YAQKFQG, where X₂₈₆ is G or I, X₂₈₇ is I or N, X₂₈₈ is F or Y, X₂₈₉ is F or G, X₂₉₀ is T or S, X₂₉₁ is S or T, and X₂₉₂ is N or T; or
the sequence SIYHSGSTYYNPSLKS; or
the sequence GIIPIFGPADYAQKFQG; or
the sequence GISWNSGTIGYADSVKG; or
the sequence GIIPILGIPNYAQKFQG; or
the sequence GIX₂₉₈X₂₉₉X₃₀₀X₃₀₁GX₃₀₂TX₃₀₃YAX₃₀₄X₃₀₅X₃₀₆X₃₀₇G, where X₂₉₅ is S or I, X₂₉₉ is G or P, X₃₀₀ is S or M, X₃₀₁ is G or F, X₃₀₂ is S or T, X₃₀₃ is Y or N, X₃₀₄ is D or Q, X₃₀₅ is S or K, X₃₀₆ is V or F, and X₃₀₇ is K or Q; or
the sequence X₃₁₄IX₃₁₅PX₃₁₆X₃₁₇GX₃₁₈X₃₁₉X₃₂₀YAQKFQG, where X₃₁₄ is I or G, X₃₁₅ is N or I, X₃₁₆ is S or I, X₃₁₇ is G or F, X₃₁₈ is S or T, X₃₁₉ is T or A, and X₃₂₀ is S or N; or
the sequence X₃₂₅IX₃₂₆PX₃₂₇X₃₂₈X₃₂₉X₃₃₀X₃₃₁X₃₃₂X₃₃₃X₃₃₄X₃₃₅X₃₃₆FQG, where X₃₂₅ is I or R, X₃₂₆ is Y or I, X₃₂₇ is G or I, X₃₂₈ is D or F, X₃₂₉ is S or G, X₃₃₀ is D or T, X₃₃₁ is T or V, X₃₃₂ is R or K, X₃₃₃ is N or Y, X₃₃₄ is S or A, X₃₃₅ is P or Q, and X₃₃₆ is S or K; or
the sequence X₃₄₂IX₃₄₃PX₃₄₄X₃₄₅X₃₄₆X₃₄₇X₃₄₈X₃₄₉YX₃₅₀X₃₅₁X₃₅₂FQG, where X₃₄₂ is I or G, X₃₄₃ is Y or I, X₃₄₄ is Y or I, X₃₄₅ is D or F, X₃₄₆ is S or G, X₃₄₇ is D or T, X₃₄₈ is T or A, X₃₄₉ is K or N, X₃₅₀ is S or A, X₃₅₁ is P or Q, and X₃₅₂ is S or K; or
the sequence LIDWDDNKYYTTSLKT; or
the sequence RFDPEDGETIYAQNFQG; or
the sequence X₃₈₄IX₃₈₅X₃₈₆DGSX₃₈₇KYYX₃₈₈DSVKG, where X₃₈₄ is A or N, X₃₈₅ is S or K, X₃₈₆ is Y or E, X₃₈₇ is N or E, and X₃₈₈ is A or V.

The HC CDR3 is:
the sequence GRYSSNWYEAWTPRGIGMDV; or
the sequence GGRHSLDX₃₁, where X₃₁ is V or A; or
the sequence GSRGYYDX₄₇LTGYSTGGFDY, where X₄₇ is F or I; or
the sequence GSRGYYDX₆₁LTGYSTGGFDY, where X₆₁ is F or I; or
the sequence GSRGYYDII,TGYSTGGFDY; or
the sequence DGVVVPAVMYDTTDPYYYGMDV; or
the sequence APSDLSFIWTGYYSEYYFDY;
the sequence EIALNNYYGMDV; or
the sequence DSGLYGSGWSTYQYYAMDV; or
the sequence DNCGGDCGGGMDV; or
the sequence GWGLRLFGEFSVWFDP; or
the sequence VEGYDSSGYYLDY; or
the sequence RYSSNWYEAWTPRGIGMDV; or
the sequence SLGGNYYYGMDV; or
the sequence VX₁₃₉GYDX₁₄₀SGYYQX₁₄₁Y, where X₁₃₉ is T or S, X₁₄₀ is S or G, and X₁₄₁ is D or E; or
the sequence GPVLGLSKWLEFDP; or
the sequence GPRGQDYGDYGFLDY; or
the sequence GPNX₁₆₀YNWFDS, where X₁₆₀ is L or I; or
the sequence GGSYYNVMYWFDP; or
the sequence ARGGSYLYGMDV; or
the sequence DPTSRSTYYYYSGSYYP; or
the sequence SYDFLTDYTDAFDI;
the sequence SPAX₂₀₀X₂₀₁X₂₀₂X₂₀₃X₂₀₄WFDP, where X₂₀₀ is V or I, X₂₀₁ is T or I, X₂₀₂ is T or R, X₂₀₃ is A or V, and X₂₀₄ is G or D; or
the sequence VRYYDFWSGWDVMDV; or
the sequence PDDSSGYYPDY; or
the sequence GGWIYRGNWFDP; or
the sequence GGSTWYGGNWFDP; or
the sequence TLYYYDRSGNARTDDYFDH; or
the sequence SLYYYDRSGYPISEDYFDY; or
the sequence SX₂₄₀X₂₄₁X₂₄₂X₂₄₃X₂₄₄AX₂₄₅WFDP, where X₂₄₀ is T or P, X₂₄₁ is P or A, X₂₄₂ is M or V, X₂₄₃ is I or T, X₂₄₄ is R or T, and X₂₄₅ is S or G; or
the sequence EGGYYYGSGSYYNPRFAFDI; or
the sequence PRGYSGYGSNWYF; or
the sequence X₂₅₆SX₂₅₇PDY, where X₂₅₅ is F or H, and X₂₅₇ is T or R; or
the sequence DPTGWYSGYFDY; or
the sequence VGTYDSSGYSFDY; or
the sequence VYCGDDCYPVVGTPGDAFDI; or
the sequence SRIAPTEDFFDY; or
the sequence DSSCSGGSCFDY; or
the sequence GDYYGSGSYYNPSPFFDY; or
the sequence GYALNP; or
the sequence SGLFDWLLPRSRHRDYFDY; or
the sequence DLPLTGTTLDY; or
the sequence X₂₇₉X₂₈₀X₂₈₁X₂₈₂X₂₈₃X₂₈₄X₂₈₅YGMDV, where X₂₇₉ is A or D, X₂₈₀ is L or H, X₂₈₁ is G or I, X₂₈₂ is G or V, X₂₈₃ is N or S, X₂₈₄ is Y or P, and X₂₈₅ is Y or L; or
the sequence GQFSDSSGYQHPYYDYGMD; or
the sequence SPSGYYGDYEGDAFDI; or
the sequence HGTSGYYYPNWFDP; or
the sequence GLSLGFCSAGSCYDYLDY; or
the sequence EELGPYSNRWYSSSDGMDV; or
the sequence SRGYSGYGANWYFDL; or
the sequence GVVTADWYFDL; or
the sequence GGDHGMDV; or
the sequence VTGYDSSGYYQDY; or
the sequence LAYFHPQRNGGYEYYFDY; or
the sequence DLPGDSRDGYNYDAFDI; or
the sequence NPTVTNWFDS; or
the sequence DRYPGYYDILTGQIGTGERNAMDV; or
the sequence X₃₆₃X₃₆₄X₃₆₅X₃₆₆X₃₆₇X₃₆₈X₃₆₉X₃₇₀X₃₇₁X₃₇₂YYYYGMDV, where X₃₆₃ is I or D, X₃₆₄ is P or L, X₃₆₅ is G or T, X₃₆₆ is F or T, X₃₆₇ is L or V, X₃₆₈ is R or T, X₃₆₉ is Y or N, X₃₇₀ is R or P, X₃₇₁ is N or L, and X₃₇₂ is R or N; or
the sequence GYSGNF.

In another embodiment of the present invention, the binding molecule may be a binding molecule that binds to a spike protein (S protein) on the surface of SARS-coronavirus-2 (SARS-CoV-2), wherein the binding molecule comprises a light-chain variable region comprising LC CDR1, LC CDR2 and LC CDR3, and a heavy-chain variable region comprising HC CDR1, HC CDR2 and HC CDR3, and is any one selected from the group consisting of the following binding molecules 1) to 49):
1) a binding molecule in which the LC CDR1 comprises the sequence SGSX₁SNIGX₂NTVN, where X₁ is S or T, and X₂ is S or T,
   the LC CDR2 comprises the sequence X₃X₄NX₅RPS, where X₃ is S or T, X₄ is N or D, and X₅ is Q or R,
   the LC CDR3 comprises the sequence AX₆X₇DDX₈LX₉X₁₀X₁₁X₁₂, where X₆ is A or S, X₇ is W or R, X₈ is S or R, X₉ is N or S, X₁₀ is A or G, X₁₁ is S, L, V, Y, P, A or T, X₁₂ is L or V,
   the HC CDR1 comprises the sequence GYYWS,
   the HC CDR2 comprises the sequence EINHSGSTNYNPSLKS, and
   the HC CDR3 comprises the sequence GRYS SNWYEAWTPRGIGMD V;
2) a binding molecule in which the LC CDR1 comprises the sequence X₁₃GSX₁₄SX₁₅IX₁₆X₁₇X₁₈X₁₉VS, where X₁₃ is S or T, X₁₄ is S or N, X₁₅ is N or S, X₁₆ is G or E, X₁₇ is N or S, X₁₈ is N or S, and X₁₉ is Y or F,
   the LC CDR2 comprises the sequence X₂₀NX₂₁X₂₂RPS, where X₂₀ is D or R, X₂₁ is N or S, and X₂₂ is K, R, Q or V,
   the LC CDR3 comprises the sequence X₂₃X₂₄WDX₂₅SLSX₂₆X₂₇V or GTWDSSLSAYWV, where X₂₃ is G or A, X₂₄ is T or A, X₂₅ is S, N, D or T, X₂₆ is A, G or S, and X₂₇ is W or Y,
   the HC CDR1 comprises the sequence X₂₈YYMH, where X₂₈ is S or G,
   the HC CDR2 comprises the sequence X₂₉INRSGGSTIYAQTFQX₃₀, where X₂₉ is I or V, X₃₀ is G or S, and
   the HC CDR3 comprises the sequence GGRHSLDX₃₁, where X₃₁ is V or A;
3) a binding molecule in which the LC CDR1 comprises the sequence RASQSVX₃₂X₃₃X₃₄X₃₅A, where X₃₂ is S, N or R, X₃₃ is S or N, X₃₄ is Y or N, and X₃₅ is L or S,
   the LC CDR2 comprises the sequence X₃₆ASX₃₇RAT, where X₃₆ is D or G, and X₃₇ is N, T or S,
   the LC CDR3 comprises the sequence X₃₈QX₃₉X₄₀X₄₁X₄₂PX₄₃T or QQYGSSPSIT, where X₃₈ is Q or H, X₃₉ is Y or H, X₄₀ is G, N, Y or H, X₄₁ is S, N, T, Q or R, X₄₂ is S, W or V, and X₄₃ is L, F, I or Q,
   the HC CDR1 comprises the sequence X₄₄SSYYWG, where X₄₄ is S or G,
   the HC CDR2 comprises the sequence X₄₅IX₄₆YSGSTYYNPSLKS, where X₄₅ is S or N, and X₄₆ is Y or F, and
   the HC CDR3 comprises the sequence GSRGYYDX₄₇LTGYSTGGFDY, where X₄₇ is F or I;
4) a binding molecule in which the LC CDR1 comprises the sequence RASQSVX₄₈X₄₉X₅₀YLA, where X₄₈ is S or G, X₄₉ is S or R, and X₅₀ is S or N,
   the LC CDR2 comprises the sequence X₅₁ASX₅₂RAT, where X₅₁ is G or D, and X₅₂ is S or T,
   the LC CDR3 comprises the sequence QQYGX₅₃SPX₅₄X₅₅, where X₅₃ is S or T, X₅₄ is L or I, and X₅₅ is T or A,
   the HC CDR1 comprises the sequence SX₅₆X₅₇YYWX₅₈, where X₅₆ is S or G, X₅₇ is S or G, and X₅₈ is G or S,
   the HC CDR2 comprises the sequence X₅₉IX₆₀YSGSTYYNPSLKS, where X₅₉ is S, Y or N, and X₆₀ is Y or F, and
   the HC CDR3 comprises the sequence GSRGYYDX₆₁LTGYSTGGFDY, where X₆₁ is F or I;
5) a binding molecule in which the LC CDR1 comprises the sequence TGTSSX₆₂VGX₆₃YX₆₄X₆₅VS, where X₆₂ is N or D, X₆₃ is D or G, X₆₄ is N or S, and X₆₅ is Y or H,
   the LC CDR2 comprises the sequence X₆₆VX₆₇X₆₈RPS, where X₆₆ is D or E, X₆₇ is T or S, and X₆₈ is N or D,
   the LC CDR3 comprises the sequence SSYTSGSTPVV or NSYTSSSTWV,
   the HC CDR1 comprises the sequence SSSYYWG,
   the HC CDR2 comprises the sequence NIFYSGSTYYNPSLKS, and
   the HC CDR3 comprises the sequence GSRGYYDILTGYSTGGFDY;
6) a binding molecule in which the LC CDR1 comprises the sequence SGX₆₉X₇₀SNIGSNX₇₁VX₇₂, where X₆₉ is S or L, X₇₀ is S or T, X₇₁ is Y, T or F, and X₇₂ is Y, HorN,
   the LC CDR2 comprises the sequence X₇₃NX₇₄X₇₅RPS, where X₇₃ is R or S, X₇₄ is N or D, and X₇₅ is Q or E,
   the LC CDR3 comprises the sequence X₇₆X₇₇WDDX₇₈LX₇₉GX₈₀V, where X₇₆ is A or S, X₇₇ is A or T, X₇₈ is S or T, X₇₉ is S, N or V, and X₈₀ is R or K,
   the HC CDR1 comprises the sequence SYAIS,
   the HC CDR2 comprises the sequence GIIPIFGTANYAQX₈₁FQX₈₂, where X₈₁ is K or R, and X₈₂ is G or D, and
   the HC CDR3 comprises the sequence DGVVVPAVMYDTTDPYYYGMDV;
7) a binding molecule in which the LC CDR1 comprises the sequence TGTSSDVGGYNYVS,
   the LC CDR2 comprises the sequence X₈₃VSNRPS, where X₈₃ is D or E,
   the LC CDR3 comprises the sequence SSYAGSNYVV or SSYTSSSTX₈₄X₈₅V, where X₈₄ is L or R, and X₈₅ is G or Y,
   the HC CDR1 comprises the sequence SYAMX₈₆, where X₈₆ is S or N,
   the HC CDR2 comprises the sequence AISGSGGTTYYADSVKG, and
   the HC CDR3 comprises the sequence APSDLSFIWTGYYSEYYFDY;
8) a binding molecule in which the LC CDR1 comprises the sequence RASQSVSSSYLA,
   the LC CDR2 comprises the sequence GASSRAT,
   the LC CDR3 comprises the sequence QQYGSSPX₈₇T or QQYGSSPALT, where X₈₇ is Y or F,
   the HC CDR1 comprises the sequence SSPMH,
   the HC CDR2 comprises the sequence VISYX₈₈GSNKYYADSVKG, where X₈₈ is D or G, and
   the HC CDR3 comprises the sequence EIALNNYYGMDV;
9) a binding molecule in which the LC CDR1 comprises the sequence TGX₈₉X₉₀SDX₉₁GGYNX₉₂X₉₃S, where X₈₉ is N or T, X₉₀ is R or S, X₉₁ is I or V, X₉₂ is S or Y, and X₉₃ is L or V,
   the LC CDR2 comprises the sequence DVX₉₄NRPS, where X₉₄ is N or S,
   the LC CDR3 comprises the sequence X₉₅SYTSX₉₆X₉₇TWV, where X₉₅ is N or S, X₉₆ is K or S, and X₉₇ is N or S,
   the HC CDR1 comprises the sequence SSPMH,
   the HC CDR2 comprises the sequence VISYDGSNKYYADSVKG, and
   the HC CDR3 comprises the sequence EIALNNYYGMDV;
10) a binding molecule in which the LC CDR1 comprises the sequence SGSSSNIGNNYVS or GGNNIGSKSVH,
   the LC CDR2 comprises the sequence DX₉₈X₉₉X₁₀₀RPS, where X₉₈ is N or D, X₉₉ is N or S, and X₁₀₀ is K or D,
   the LC CDR3 comprises the sequence GTWDSSLSAGWV or X₁₀₁X₁₀₂WDSSX₁₀₃X₁₀₄X₁₀₅VV, where X₁₀₁ is G or Q, X₁₀₂ is T or V, X₁₀₃ is L or S, X₁₀₄ is S or D, and X₁₀₅ is A or H,
   the HC CDR1 comprises the sequence DYGMH,
   the sequence HC CDR2 comprises the sequence AISYDGSNKYYADSVKG, and
   the HC CDR3 comprises the sequence DSGLYGSGWSTYQYYAMDV;
11) a binding molecule in which the LC CDR1 comprises the sequence X₁₀₆GTX₁₀₇SDVGGYNYVS, where X₁₀₆ is T or S, and X₁₀₇ is S or N,
   the LC CDR2 comprises the sequence DVX₁₀₈KRPS, where X₁₀₈ is S or T,
   the LC CDR3 comprises the sequence SSYTSSX₁₀₉TWV or SSYTSSSTPWV, where X₁₀₉ is S or R,
   the HC CDR1 comprises the sequence X₁₁₀YAMH, where X₁₁₀ is S or G,
   the HC CDR2 comprises the sequence VISYDGSHKNYADSVKG, and
   the HC CDR3 comprises the sequence DNCGGDCGGGMDV;
12) a binding molecule in which the LC CDR1 comprises the sequence SGSSSNIGSNX₁₁₁VN or TGSSSNIGAGYDVH, where X₁₁₁ is A or T,
   the LC CDR2 comprises the sequence X₁₁₂NSX₁₁₃RPS, where X₁₁₂ is G or S, and X₁₁₃ is N or Y,
   the LC CDR3 comprises the sequence QSYDSX₁₁₄LX₁₁₅X₁₁₆X₁₁₇X₁₁₈, where X₁₁₄ is S or A, X₁₁₅ is S or R, X₁₁₆ is D or G, X₁₁₇ is V or P, and X₁₁₈ is V or L,
   the HC CDR1 comprises the sequence SYGIS,
   the HC CDR2 comprises the sequence GIIPII,ATTKFAQKFQG, and
   the HC CDR3 comprises the sequence GWGLRLFGEFSVWFDP;
13) a binding molecule in which the LC CDR1 comprises the sequence RASQSVSSYLA,
   the LC CDR2 comprises the sequence DASNRAT,
   the LC CDR3 comprises the sequence QQRRNWPPX₁₁₉X₁₂₀T, where X₁₁₉ is V or R, and X₁₂₀ is L or I,
   the HC CDR1 comprises the sequence SYAIS,
   the HC CDR2 comprises the sequence GIIPIFGTANYAQKFQG, and
   the HC CDR3 comprises the sequence VEGYDSSGYYLDY;
14) a binding molecule in which the LC CDR1 comprises the sequence X₁₂₁GSX₁₂₂SNIGX₁₂₃NX₁₂₄VX₁₂₅, where X₁₂₁ is S or T, X₁₂₂ is S or R, X₁₂₃ is S or I, X₁₂₄ is T or S, and X₁₂₅ is S or N,
   the LC CDR2 comprises the sequence SNNQRPS,
   the LC CDR3 comprises the sequence AAWDDSLNGX₁₂₆V, where X₁₂₆ is V or Y,
   the HC CDR1 comprises the sequence GYYWS,
   the HC CDR2 comprises the sequence EINHSGSTNYNPSLKSG, and
   the HC CDR3 comprises the sequence RYSSNWYEAWTPRGIGMDV;
15) a binding molecule in which the LC CDR1 comprises the sequence TGSSSNIGAGYDVH or SGSSSNIGSNTVN,
   the LC CDR2 comprises the sequence X₁₂₇NX₁₂₈X₁₂₉RPS, where X₁₂₇ is G or S, X₁₂₈ is S or N, and X₁₂₉ is N or Q,
   the LC CDR3 comprises the sequence AAWDDSLX₁₃₀GX₁₃₁V, where X₁₃₀ is G or N, and X₁₃₁ is P or W,
   the HC CDR1 comprises the sequence TYGMH,
   the HC CDR2 comprises the sequence VISYDGFNKYYADSVKG, and
   the HC CDR3 comprises the sequence SLGGNYYYGMDV;
16) a binding molecule in which the LC CDR1 comprises the sequence TGTSSDIGGYNYVS or GGNNIGSKSVH,
   the LC CDR2 comprises the sequence X₁₃₂X₁₃₃SKRPS, where X₁₃₂ is A or Q, and X₁₃₃ is V or D,
   the LC CDR3 comprises the sequence SSYTSSSGTLNV or QAWDSSTV,
   the HC CDR1 comprises the sequence TYGMH,
   the HC CDR2 comprises the sequence VISYDGFNKYYADSVKG, and
   the HC CDR3 comprises the sequence SLGGNYYYGMDV;
17) a binding molecule in which the LC CDR1 comprises the sequence RASQSVSX₁₃₄X₁₃₅LA, where X₁₃₄ is S or T, and X₁₃₅is Y or F,
   the LC CDR2 comprises the sequence DASNRAT,
   the LC CDR3 comprises the sequence QQRSNWPPX₁₃₆X₁₃₇T, where X₁₃₆ is A, R, K or M, and X₁₃₇ is L, I or Y,
   the HC CDR1 comprises the sequence SYAIX₁₃₈, where X₁₃₈ is S or I,
   the HC CDR2 comprises the sequence GIIPIFGTANYAQKFQG, and
   the HC CDR3 comprises the sequence VX₁₃₉GYDX₁₄₀SGYYQX₁₄₁Y, where X₁₃₉ is T or S, X₁₄₀ is S or G, and X₁₄₁ is D or E;
18) a binding molecule in which the LC CDR1 comprises the sequence TGTSX₁₄₂DX₁₄₃GX₁₄₄YNYVS, where X₁₄₂ is T or S, X₁₄₃is I or V, and X₁₄₄ is R or A,
   the LC CDR2 comprises the sequence DVSKRPS,
   the LC CDR3 comprises the sequence SYTSSSTYA or NSYTSSSTWV,
   the HC CDR1 comprises the sequence EVAIH,
   the HC CDR2 comprises the sequence GFDPEDGETSYAQKFQG, and
   the HC CDR3 comprises the sequence GPVLGLSKWLEFDP;
19) a binding molecule in which the LC CDR1 comprises the sequence SGSSSNIGNNYVS,
   the LC CDR2 comprises the sequence DNNKRPS,
   the LC CDR3 comprises the sequence X₁₄₅X₁₄₆WDX₁₄₇SLSX₁₄₈X₁₄₉X₁₅₀, where X₁₄₅ is G or E, X₁₄₆ is T or A, X₁₄₇ is S or T, X₁₄₈ is A or D, X₁₄₉ is V or G, and X₁₅₀ is V or L,
   the HC CDR1 comprises the sequence RYAMS,
   the HC CDR2 comprises the sequence AISGSGGX₁₅₁TYYADSVKG, where X₁₅₁ is S or T, and
   the HC CDR3 comprises the sequence GPRGQDYGDYGFLDY;
20) a binding molecule in which the LC CDR1 comprises the sequence SGSSSNIGSNTVN,
   the LC CDR2 comprises the sequence X₁₅₂NNX₁₅₃RPS, where X₁₅₂ is S, T or N, and X₁₅₃ is Q or R,
   the LC CDR3 comprises the sequence AAWDDSLX₁₅₄GX₁₅₅V or AAWDDSLNGX₁₅₆VV, where X₁₅₄ is N or S, X₁₅₅ is L, P, W or V, and X₁₅₆ is S or H,
   the HC CDR1 comprises the sequence SX₁₅₇WIX₁₅₈, where X₁₅₇ is H or Y, and X₁₅₈ is A, V or G,
   the HC CDR2 comprises the sequence X₁₅₉IYPGDSDSRYSPSFQG, where X₁₅₉ is I or T, and
   the HC CDR3 comprises the sequence GPNX₁₆₀YNWFDS, where X₁₆₀ is L or I;
21) a binding molecule in which the LC CDR1 comprises the sequence RASQX₁₆₁X₁₆₂X₁₆₃X₁₆₄X₁₆₅X₁₆₆A, where X₁₆₁ is S or G, X₁₆₂ is V or I, X₁₆₃ is S or R, X₁₆₄ is S or N, X₁₆₅ is Y, S or D, and X₁₆₆ is L or I,
   the LC CDR2 comprises the sequence DX₁₆₇SX₁₆₈X₁₆₉X₁₇₀T or AASRLES, where X₁₆₇ is A or T, X₁₆₈ is N or T, X₁₆₉ is R or L, and X₁₇₀ is A or E,
   the LC CDR3 comprises the sequence QQYYX₁₇₁TPX₁₇₂T, where X₁₇₁ is S or T, and X₁₇₂ is F or I,
   the HC CDR1 comprises the sequence DYYIQ,
   the HC CDR2 comprises the sequence WINPNSGDTNYAHKFQG, and
   the HC CDR3 comprises the sequence GGSYYNVMYWFDP;
22) a binding molecule in which the LC CDR1 comprises the sequence X₁₇₃ASQX₁₇₄ISSYLN, where X₁₇₃ is R or Q, and X₁₇₄ is S or D,
   the LC CDR2 comprises the sequence X₁₇₅ASSLX₁₇₆S, where X₁₇₅ is K or A, and X₁₇₆ is E or Q,
   the LC CDR3 comprises the sequence QQSYSTWT,
   the HC CDR1 comprises the sequence SYAMH,
   the HC CDR2 comprises the sequence VISYDGSNKYYADSVKG, and
   the HC CDR3 comprises the sequence ARGGSYI,YGMDV;
23) a binding molecule in which the LC CDR1 comprises the sequence SGSX₁₇₇SNIGX₁₇₈NYVX₁₇₉, where X₁₇₇ is S or G, X₁₇₈ is N or S, and X₁₇₉ is S or H,
   the LC CDR2 comprises the sequence X₁₈₀NX₁₈₁X₁₈₂RPS, where X₁₈₀ is D or R, X₁₈₁ is N or S, and X₁₈₂ is K or G,
   the LC CDR3 comprises the sequence AAWDDSLX₁₈₃GWV, where X₁₈₃ is N or S,
   the HC CDR1 comprises the sequence SYAIS,
   the HC CDR2 comprises the sequence GIIPMFGTTNYAQKFQG, and
   the HC CDR3 comprises the sequence DPTSRSTYYYYSGSYYP;
24) a binding molecule in which the LC CDR1 comprises the sequence SGSSSNIGNNYVS or TGTRGDIGAYDGVS,
   the LC CDR2 comprises the sequence DNNKRPS or AVTQRPS,
   the LC CDR3 comprises the sequence AAWDDSLNGVV or SSYTSSSSWV,
   the HC CDR1 comprises the sequence RYAMS,
   the HC CDR2 comprises the sequence AISGSGGSTYYADSVKG, and
   the HC CDR3 comprises the sequence GPRGQDYGDYGFLDY;
25) a binding molecule in which the LC CDR1 comprises the sequence TGSSSNIGAGYDVH or SGSSSNIGNNYVS,
   the LC CDR2 comprises the sequence X₁₈₄NX₁₈₅X₁₈₆RPS, where X₁₈₄ is G or D, X₁₈₅ is S or N, and X₁₈₆ is N or K,
   the LC CDR3 comprises the sequence QSYDSSLSGLWV or QSYDSSLSAWV,
   the HC CDR1 comprises the sequence SNYMS,
   the HC CDR2 comprises the sequence VIYPGGSTFFADSVQG, and
   the HC CDR3 comprises the sequence SYDFLTDYTDAFDI;
26) a binding molecule in which the LC CDR1 comprises the sequence TGTX₁₈₇X₁₈₈DX₁₈₉GX₁₉₀YX₁₉₁X₁₉₂VS, where X₁₈₇ is S or R, X₁₈₈ is S or G, X₁₈₉ is V or I, X₁₉₀ is G or A, X₁₉₁ is N or D, and X₁₉₂ is Y or G,
   the LC CDR2 comprises the sequence X₁₉₃VX₁₉₄X₁₉₅RPS, where X₁₉₃ is D or A, X₁₉₄ is SorT, and X₁₉₅ is K or Q,
   the LC CDR3 comprises the sequence SSYTX₁₉₆SSX₁₉₇WV, where X₁₉₆ is T or S, and X₁₉₇ is TorS,
   the HC CDR1 comprises the sequence ELSX₁₉₈H, where X₁₉₈ is I or M,
   the HC CDR2 comprises the sequence GFDPEDX₁₉₉ETIYAQKFQG, where X₁₉₉ is G or A, and
   the HC CDR3 comprises the sequence SPAX₂₀₀X₂₀₁X₂₀₂X₂₀₃X₂₀₄WFDP, where X₂₀₀ is V or I, X₂₀₁ is T or I, X₂₀₂ is T or R, X₂₀₃ is A or V, and X₂₀₄ is G or D;
27) a binding molecule in which the LC CDR1 comprises the sequence SGSSSNIGX₂₀₅NX₂₀₆VX₂₀₇, where X₂₀₅ is S or N, X₂₀₆ is T or Y, and X₂₀₇ is N or S,
   the LC CDR2 comprises the sequence X₂₀₈NNX₂₀₉RPS, where X₂₀₈ is S or D, and X₂₀₉ is Q or K,
   the LC CDR3 comprises the sequence AAWDDSLX₂₁₀GPV, where X₂₁₀ is N or S,
   the HC CDR1 comprises the sequence X₂₁₁ YAMS, where X₂₁₁ is S or R,
   the HC CDR2 comprises the sequence AISGSGGX₂₁₂TYYADSVKG, where X₂₁₂ is D or S, and
   the HC CDR3 comprises the sequence VRYYDFWSGWDVMDV;
28) a binding molecule in which the LC CDR1 comprises the sequence TGTSX₂₁₃DVGX₂₁₄YNX₂₁₅VS, where X₂₁₃ is T or S, X₂₁₄ is G or S, and X₂₁₅ is Y or L,
   the LC CDR2 comprises the sequence DVSX₂₁₆RPS, where X₂₁₆ is K or N,
   the LC CDR3 comprises the sequence SSYTSSSTLV,
   the HC CDR1 comprises the sequence NYGMH,
   the HC CDR2 comprises the sequence VMSYDGSNKYYADSVKG, and
   the HC CDR3 comprises the sequence PDDSSGYYPDY;
29) a binding molecule in which the LC CDR1 comprises the sequence TGTSSDVGGYNYVS,
   the LC CDR2 comprises the sequence DVX₂₁₇KRPS, where X₂₁₇ is G or S,
   the LC CDR3 comprises the sequence SSYTSSSTX₂₁₈V, where X₂₁₈ is W OR L,
   the HC CDR1 comprises the sequence X₂₁₉YAIS, where X₂₁₉ is F or R,
   the HC CDR2 comprises the sequence GIIPX₂₂₀FGX₂₂₁X₂₂₂X₂₂₃YAQX₂₂₄FQD, where X₂₂₀ is L or I, X₂₂₁ is T or K, X₂₂₂ is A or V, X₂₂₃ is K or N, and X₂₂₄ is R or K, and
   the HC CDR3 comprises the sequence GGWIYRGNWFDP or GGSTWYGGNWFDP;
30) a binding molecule in which the LC CDR1 comprises the sequence TGSSSNIGAGYDVH,
   the LC CDR2 comprises the sequence GNSNRPS,
   the LC CDR3 comprises the sequence QSYDSSLSGX₂₂₅WV, where X₂₂₅ is P or S,
   the HC CDR1 comprises the sequence RYAIX₂₂₆, where X₂₂₆ is N or S,
   the HC CDR2 comprises the sequence GIIPX₂₂₇X₂₂₈GTX₂₂₉X₂₃₀YX₂₃₁QKFQG, where X₂₂₇ is L or I, X₂₂₈ is L or F, X₂₂₉ is A or G, X₂₃₀ is D or N, and X₂₃₁ is P or A, and
   the HC CDR3 comprises the sequence TLYYYDRSGNARTDDYFDH or SLYYYDRSGYPISEDYFDY;
31) a binding molecule in which the LC CDR1 comprises the sequence TGTSSDVGX₂₃₂YNYVS, where X₂₃₂ is N or G,
   the LC CDR2 comprises the sequence DVSX₂₃₃RPS, where X₂₃₃ is N or K,
   the LC CDR3 comprises the sequence SSYTSSX₂₃₄TX₂₃₅V, where X₂₃₄ is G or S, and X₂₃₅ is L or Y,
   the HC CDR1 comprises the sequence ELSIH,
   the HC CDR2 comprises the sequence GFDPEX₂₃₆X₂₃₇ETIX₂₃₈AQX₂₃₉FQG, where X₂₃₆ is N or D, X₂₃₇ is G or A, X₂₃₈ is H or Y, and X₂₃₉ is R or K, and
   the HC CDR3 comprises the sequence SX₂₄₀X₂₄₁X₂₄₂X₂₄₃X₂₄₄AX₂₄₅WFDP, where X₂₄₀ is T or P, X₂₄₁ is P or A, X₂₄₂ is M or V, X₂₄₃ is I or T, X₂₄₄ is R or T, and X₂₄₅ is S or G;
32) a binding molecule in which the LC CDR1 comprises the sequence TGSSSNIGAGYDVH,
   the LC CDR2 comprises the sequence GNSNRPS,
   the LC CDR3 comprises the sequence QSYDSSLSGWV,
   the HC CDR1 comprises the sequence NYGIS,
   the HC CDR2 comprises the sequence X₂₄₆ISX₂₄₇X₂₄₈NGNTX₂₄₉YAQKLQG, where X₂₄₆ is G or W, X₂₄₇ is S or A, X₂₄₈ is H or Y, and X₂₄₉ is K or N, and
   the HC CDR3 comprises the sequence EGGYYYGSGSYYNPRFAFDI or PRGYSGYGSNWYF;
33) a binding molecule in which the LC CDR1 comprises the sequence RASQSVSSNLA or RASQGISNSL,
   the LC CDR2 comprises the sequence X₂₅₀AX₂₅₁X₂₅₂RX₂₅₃X₂₅₄, where X₂₅₀ is W or A, X₂₅₁ is S or A, X₂₅₂ is T or S, X₂₅₃ is E or L, and X₂₅₄ is S or E,
   the LC CDR3 comprises the sequence LQYFTIPWT or SQQYYSTPYT,
   the HC CDR1 comprises the sequence NAWMX₂₅₅, where X₂₅₅ is S or T,
   the HC CDR2 comprises the sequence RIKTKTDGGTTDYAAPVKG, and
   the HC CDR3 comprises the sequence X₂₅₆SX₂₅₇PDY, where X₂₅₅ is For H, and X₂₅₇ is T or R;
34) a binding molecule in which the LC CDR1 comprises the sequence RASQSVSSX₂₅₈LA, where X₂₅₈ is Y or F,
   the LC CDR2 comprises the sequence DASNRAT,
   the LC CDR3 comprises the sequence QQYGSSPPIT or QQRSNWPPSIT,
   the HC CDR1 comprises the sequence SYAIS,
   the HC CDR2 comprises the sequence GIIPIFGTX₂₅₉NYAQKFQG, where X₂₅₉ is T or A, and
   the HC CDR3 comprises the sequence DPTGWYSGYFDY or VGTYDSSGYSFDY;
35) a binding molecule in which the LC CDR1 comprises the sequence GGNNIGSKSVH,
   the LC CDR2 comprises the sequence YDSDRPS,
   the LC CDR3 comprises the sequence QVWDSSSDHWV,
   the HC CDR1 comprises the sequence DYAMS or SYYIH,
   the HC CDR2 comprises the sequence VISGSGGSTSNADSVKG or FINPSDVTTYYAQKFQG, and
   the HC CDR3 comprises the sequence VYCGDDCYPVVGTPGDAFDI or SRIAPTEDFFDY;
36) a binding molecule in which the LC CDR1 comprises the sequence SSSTGAVTRGHFPN or SGSSSNIGNNYVS,
   the LC CDR2 comprises the sequence STSNKHS or DNNKRPS,
   the LC CDR3 comprises the sequence LLYDAGAPGWV or GAWDSSLSTPNW,
   the HC CDR1 comprises the sequence SYPMH,
   the HC CDR2 comprises the sequence VISYDGSLKYYVDSVKG, and
   the HC CDR3 comprises the sequence DSSCSGGSCFDY;
37) a binding molecule in which the LC CDR1 comprises the sequence SGSSSNIGSNTVN,
   the LC CDR2 comprises the sequence SNNQRPS,
   the LC CDR3 comprises the sequence AAWDDSLNGWV,
   the HC CDR1 comprises the sequence X₂₆₀X₂₆₁AMX₂₆₂, where X₂₆₀ is S or N, X₂₆₁ is F or Y, and X₂₆₂ is H or N,
   the HC CDR2 comprises the sequence X₂₆₃ISX₂₆₄X₂₆₅GX₂₆₆X₂₆₇X₂₆₈YYADSVKG, where X₂₆₃ is V or T, X₂₆₄ is F or G, X₂₆₅ is D or S, X₂₆₆ is S or G, X₂₆₇ is N or S, and X₂₆₈ is K or T, and
   the HC CDR3 comprises the sequence GDYYGSGSYYNPSPFFDY or GYALNP;
38) a binding molecule in which the LC CDR1 comprises the sequence CRASQSVSSSYLA,
   the LC CDR2 comprises the sequence GASSRAT,
   the LC CDR3 comprises the sequence QQYGSSPX₂₆₉T, where X₂₆₉ is Y or P,
   the HC CDR1 comprises the sequence SSPMH or NYVIN,
   the HC CDR2 comprises the sequence VISYDGSNKYYADSVKG or GFIPVFGIADYAQKFQG, and
   the HC CDR3 comprises the sequence EIALNNYYGMDV or SGLFDWLLPRSRHRDYFDY;
39) a binding molecule in which the LC CDR1 comprises the sequence RASQSISSYLN,
   the LC CDR2 comprises the sequence AASSLQS,
   the LC CDR3 comprises the sequence QQSYSTPLT,
   the HC CDR1 comprises the sequence SNYMX₂₇₀, where X₂₇₀ is T or S,
   the HC CDR2 comprises the sequence X₂₇₁IYPGGSTX₂₇₂X₂₇₃ADSVX₂₇₄G, where X₂₇₁ is I or V, X₂₇₂ is Y or F, X₂₇₃ is Y or F, and X₂₇₄ is K or Q, and
   the HC CDR3 comprises the sequence DLPLTGTTLDY or SYDFLTDYTDAFDI;
40) a binding molecule in which the LC CDR1 comprises the sequence RASQSISSYLN,
   the LC CDR2 comprises the sequence AASSLQS,
   the LC CDR3 comprises the sequence QQSYSTPX₂₇₅T, where X₂₇₅ is F or L,
   the HC CDR1 comprises the sequence SX₂₇₆AX₂₇₇X₂₇₈, where X₂₇₆ is Y or S, X₂₇₇ is M or I, and X₂₇₈ is H or N,
   the HC CDR2 comprises the sequence VISYDGINKYYADSVKG or GIIPIFGTVNYAQKFQG, and
   the HC CDR3 comprises the sequence X₂₇₉X₂₈₀X₂₈₁X₂₈₂X₂₈₃X₂₈₄X₂₈₅YGMDV, where X₂₇₉ is A or D, X₂₈₀ is L or H, X₂₈₁ is G or I, X₂₈₂ is G or V, X₂₈₃ is N or S, X₂₈₄ is Y or P, and X₂₈₅ is Y or L;
41) a binding molecule in which the LC CDR1 comprises the sequence TGSSSNIGAGYDVH,
   the LC CDR2 comprises the sequence GNSNRPS,
   the LC CDR3 comprises the sequence QSYDSSLSGPNWV or QSYDSRLRAVV,
   the HC CDR1 comprises the sequence SYAIS or TQYLH,
   the HC CDR2 comprises the sequence X₂₈₆IX₂₈₇PX₂₈₈X₂₈₉GX₂₉₀X₂₉₁X₂₉₂YAQKFQG, where X₂₈₆ is G or I, X₂₈₇ is I or N, X₂₈₈ is F or Y, X₂₈₉ is F or G, X₂₉₀ is T or S, X₂₉₁ is S or T, and X₂₉₂ is N or T, and
   the HC CDR3 comprises the sequence GQFSDSSGYQHPYYDYGMD or SPSGYYGDYEGDAFDI;
42) a binding molecule in which the LC CDR1 comprises the sequence TGTSSDVGGYNYVS,
   the LC CDR2 comprises the sequence DVSX₂₉₃RPS, where X₂₉₃ is K or N,
   the LC CDR3 comprises the sequence SSYTSSSTLV,
   the HC CDR1 comprises the sequence RGDYWG or THALS,
   the HC CDR2 comprises the sequence SIYHSGSTYYNPSLKS or GIIPIFGPADYAQKFQG, and
   the HC CDR3 comprises the sequence HGTSGYYYPNWFDP or GLSLGFCSAGSCYDYLDY;
43) a binding molecule in which the LC CDR1 comprises the sequence TGSSSNIGAGYDVH,
   the LC CDR2 comprises the sequence GNSNRPS,
   the LC CDR3 comprises the sequence QSYDSSLSGX₂₉₄V, where X₂₉₄ is V or P,
   the HC CDR1 comprises the sequence DYAMH or SYDIS,
   the HC CDR2 comprises the sequence GISWNSGTIGYADSVKG or GIIPII,GIPNYAQKFQG, and
   the HC CDR3 comprises the sequence EELGPYSNRWYSSSDGMDV or SRGYSGYGANWYFDL;
44) a binding molecule in which the LC CDR1 comprises the sequence TGTSSDVGGYNX₂₉₅VS, where X₂₉₅ is Y or N,
   the LC CDR2 comprises the sequence DVSX₂₉₆RPS, where X₂₉₆ is N or K,
   the LC CDR3 comprises the sequence SSYTSSSPWV,
   the HC CDR1 comprises the sequence SYAX₂₉₇S, where X₂₉₇ is M or I,
   the HC CDR2 comprises the sequence GIX₂₉₈X₂₉₉X₃₀₀X₃₀₁GX₃₀₂TX₃₀₃YAX₃₀₄X₃₀₅X₃₀₆X₃₀₇G, where X₂₉₅ is S or I, X₂₉₉ is G or P, X₃₀₀ is S or M, X₃₀₁ is G or F, X₃₀₂ is S or T, X₃₀₃ is Y or N, X₃₀₄ is D or Q, X₃₀₅ is S or K, X₃₀₆ is V or F, and X₃₀₇ is K or Q, and
   the HC CDR3 comprises the sequence GVVTADWYFDL or DPTSRSTYYYYSGSYYP;
45) a binding molecule in which the LC CDR1 comprises the sequence SGSSSNIGX₃₀₈NYVS, where X₃₀₈ is R or N,
   the LC CDR2 comprises the sequence DNNX₃₀₉RPS, where X₃₀₉ is R or K,
   the LC CDR3 comprises the sequence GTWDSSLSAVX₃₁₀, where X₃₁₀ is A or V,
   the HC CDR1 comprises the sequence SYX₃₁₁X₃₁₂X₃₁₃, where X₃₁₁ is Y or A, X₃₁₂ is M or I, and X₃₁₃ is H or I,
   the HC CDR2 comprises the sequence X₃₁₄IX₃₁₅PX₃₁₆X₃₁₇GX₃₁₈X₃₁₉X₃₂₀YAQKFQG, where X₃₁₄ is I or G, X₃₁₅ is N or I, X₃₁₆ is S or I, X₃₁₇ is G or F, X₃₁₈ is S or T, X₃₁₉ is T or A, and X₃₂₀ is S or N, and
   the HC CDR3 comprises the sequence GGDHGMDV or VTGYDSSGYYQDY;
46) a binding molecule in which the LC CDR1 comprises the sequence SGSSSNIGNNYVS,
   the LC CDR2 comprises the sequence DNNKRPS,
   the LC CDR3 comprises the sequence GTWDSSLSAX₃₂₁V, where X₃₂₁ is G or V,
   the HC CDR1 comprises the sequence SX₃₂₂X₃₂₃IX₃₂₄, where X₃₂₂ is N or Y, X₃₂₃ is W or V, and X₃₂₄ is A or S,
   the HC CDR2 comprises the sequence X₃₂₅IX₃₂₆PX₃₂₇X₃₂₈X₃₂₉X₃₃₀X₃₃₁X₃₃₂X₃₃₃X₃₃₄X₃₃₅X₃₃₆FQG, where X₃₂₅ is I or R, X₃₂₆ is Y or I, X₃₂₇ is G or I, X₃₂₈ is D or F, X₃₂₉ is S or G, X₃₃₀ is D or T, X₃₃₁ is T or V, X₃₃₂ is R or K, X₃₃₃ is N or Y, X₃₃₄ is S or A, X₃₃₅ is P or Q, and X₃₃₆ is S or K, and
   the HC CDR3 comprises the sequence LAYFHPQRNGGYEYYFDY or DLPGDSRDGYNYDAFDI;
47) a binding molecule in which the LC CDR1 comprises the sequence SGSSSX₃₃₇X₃₃₈GSNX₃₃₉VN, where X₃₃₇ is N or D, X₃₃₈ is V or I, and X₃₃₉ is I or T,
   the LC CDR2 comprises the sequence X₃₄₀NNQRPS, where X₃₄₀ is N or S,
   the LC CDR3 comprises the sequence AAWDDSLNX₃₄₁WV, where X₃₄₁ is A or G,
   the HC CDR1 comprises the sequence NHWIA or NYAIN,
   the HC CDR2 comprises the sequence X₃₄₂IX₃₄₃PX₃₄₄X₃₄₅X₃₄₆X₃₄₇X₃₄₈X₃₄₉YX₃₅₀X₃₅₁X₃₅₂FQG, where X₃₄₂ is I or G, X₃₄₃ is Y or I, X₃₄₄ is Y or I, X₃₄₅ is D or F, X₃₄₆ is S or G, X₃₄₇ is D or T, X₃₄₈ is T or A, X₃₄₉ is K or N, X₃₅₀ is S or A, X₃₅₁ is P or Q, and X₃₅₂ is S or K, and
   the HC CDR3 comprises the sequence NPTVTNWFDS or DRYPGYYDILTGQIGTGERNAMDV;
48) a binding molecule in which the LC CDR1 comprises the sequence SGSSSNIGX₃₅₃NYVX₃₅₄, where X₃₅₃ is N or S, and X₃₅₄ is S or Y,
   the LC CDR2 comprises the sequence X₃₅₅NNX₃₅₆RPS, where X₃₅₅ is D or R, and X₃₅₆ is K or Q,
   the LC CDR3 comprises the sequence X₃₅₇X₃₅₈WDX₃₅₉SLSX₃₆₀X₃₆₁X₃₆₂, where X₃₅₇ is G or A, X₃₅₈ is T or A, X₃₅₉ is S or D, X₃₆₀ is A or G, X₃₆₁ is G or W, and X₃₆₂ is P or V,
   the HC CDR1 comprises the sequence TSGVGVG or ELPIH,
   the HC CDR2 comprises the sequence LIDWDDNKYYTTSLKT or RFDPEDGETIYAQNFQG, and
   the HC CDR3 comprises the sequence X₃₆₃X₃₆₄X₃₆₅X₃₆₆X₃₆₇X₃₆₈X₃₆₉X₃₇₀X₃₇₁X₃₇₂YYYYGMDV, where X₃₆₃ is I or D, X₃₆₄ is P or L, X₃₆₅ is G or T, X₃₆₆ is F or T, X₃₆₇ is L or V, X₃₆₈ is R or T, X₃₆₉ is Y or N, X₃₇₀ is R or P, X₃₇₁ is N or L, and X₃₇₂ is R or N; and
49) a binding molecule in which the LC CDR1 comprises the sequence SGSSSNIGX₃₇₃NYVX₃₇₄, where X₃₇₃ is N or S, and X₃₇₄ is S or N,
   the LC CDR2 comprises the sequence X₃₇₅NNX₃₇₆RPS, where X₃₇₅ is D or S, and X₃₇₆ is K or Q,
   the LC CDR3 comprises the sequence X₃₇₇TWDX₃₇₈SLX₃₇₉X₃₈₀X₃₈₁V, where X₃₇₇ is G or A, X₃₇₈ is S or D, X₃₇₉ is S or N, X₃₈₀ is A or G, and X₃₈₁ is G or Q,
   the HC CDR1 comprises the sequence DYX₃₈₂MX₃₈₃, where X₃₈₂ is G or W, and X₃₈₃ is H or S,
   the HC CDR2 comprises the sequence X₃₈₄IX₃₈₅X₃₈₆DGSX₃₈₇KYYX₃₈₈DSVKG, where X₃₈₄ is A or N, X₃₈₅ is S or K, X₃₈₆ is Y or E, X₃₈₇ is N or E, and X₃₈₈ is A or V, and
   the HC CDR3 comprises the sequence DSGLYGSGWSTYQYYAMDV or GYSGNF.

In one embodiment of the present invention, the binding molecule according to the present invention may have an IC₅₀ value of preferably 200 ng/ml or less, more preferably 100 ng/ml or less, even more preferably 50 ng/ml or less, even more preferably 25 ng/ml or less, even more preferably 10 ng/ml or less, as evaluated according to a plaque reduction neutralization test (PRNT) method for SARS-coronavirus-2 (SARS-CoV-2), without being limited thereto. Here, the IC₅₀ value refers to the antibody concentration at which the antibody exhibits 50% of neutralizing activity against the virus.

In one embodiment of the present invention, the minimum concentration value of the binding molecule according to the present invention that neutralizes 100 TCID50 of virus for SARS-coronavirus-2 (SARS-CoV-2) may be preferably 2 µg/ml or less, more preferably 1 µg/ml or less, even more preferably 0.5 µg/ml or less, even more preferably 0.25 µg/ml or less, even more preferably 0.125 µg/ml or less, without being limited thereto.

In one embodiment of the present invention, the binding molecule according to the present invention is able to bind to a receptor-binding domain (RBD) of the spike protein of SARS-coronavirus-2 with an equilibrium dissociation constant (K_{D}) of preferably 1.0×10⁻⁸ M or less, more preferably 1.0 × 10⁻⁹ M or less, even more preferably 1.0 x 10⁻¹⁰ M or less, without being limited thereto.

In one embodiment of the present invention, the binding molecule may be a polypeptide, particularly an antibody or an antigen-binding fragment thereof. The antibody may be a monoclonal antibody, preferably a chimeric antibody, a humanized antibody, or a human antibody, without being limited thereto. The antigen-binding fragment may be Fab, F(ab'), F(ab')2, Fv, dAb, Fd, a single-chain antibody fragment (scFv), scFv-Fc, a complementarity-determining region (CDR) fragment, a bivalent single-chain antibody fragment, a single-chain phage antibody fragment, a diabody, a triabody, or a tetrabody, without being limited thereto.

An embodiment of the present invention provides scFv-Fc that binds to the S protein of SARS-CoV-2. Another embodiment of the present invention provides a fully human antibody (full IgG) that binds to the S protein of SARS-CoV-2.

An embodiment of the present invention provides scFv-Fc that binds to the S protein of SARS-CoV-1. Another embodiment of the present invention provides a fully human antibody (full IgG) that binds to the S protein of SARS-CoV-1.

An embodiment of the present invention provides scFv-Fc that binds to the S protein of MERS-CoV. Another embodiment of the present invention provides a fully human antibody (full IgG) that binds to the S protein of MERS-CoV.

In the present invention, the binding molecules include those that exist in the human body and those that are not present in the human body but are wholly or partially artificially produced. In particular, the binding molecule of the present invention may be one that is wholly or partially artificially produced. Wholly or partially artificially produced examples include, but are not limited to, those produced *in vitro* or isolated, those produced by mammalian cell culture, those artificially produced by transformed cells, or those artificially produced through shuffling recombination of genetic information of light-chain and/or heavy-chain variable regions of the antibody.

As used herein, the term "antibody" is used in the broadest sense, and particularly includes an intact monoclonal antibody, a polyclonal antibody, a multispecific antibody (e.g., a bispecific antibody) formed from two or more intact antibodies, and an antibody fragment that shows a desired biological activity. The antibody is a protein that is produced by an immune system capable of recognizing and binding to a specific antigen. Structurally, the antibody generally has a Y-shaped protein comprising four amino acid chains (two heavy chains and two light chains). Each antibody generally has two regions (a variable region and a constant region). The variable region, which is located at the ends of the arms of the Y, binds to and interacts with a target antigen. The variable region includes a complementarity-determining region (CDR) that recognizes and binds to a specific binding site on a specific antigen. The constant region, which is located at the tail of the Y, is recognized by the immune system and interacts therewith. The target antigen generally has a plurality of binding sites called epitopes, which are recognized by CDRs on antibodies. Respective antibodies that specifically bind to different epitopes have different structures. Therefore, a single antigen may have at least one antibody corresponding thereto.

Moreover, the present invention includes functional variants of the binding molecule. Binding molecules are considered to be functional variants of the binding molecule of the present invention so long as the variants are capable of competing with the binding molecule of the present invention in order to specifically bind to coronavirus or to an S protein thereof and also have neutralizing activity against coronavirus. Such functional variants include, but are not limited to, derivatives that are substantially similar in primary structural sequence, and examples thereof include *in-vitro* or *in-vivo* modifications, chemicals and/or biochemicals, that are not found in the parental monoclonal antibody of the present invention. Such modifications include, for example, acetylation, acylation, covalent attachment of a nucleotide or nucleotide derivative, covalent attachment of a lipid or lipid derivative, cross-linking, disulfide bond formation, glycosylation, hydroxylation, methylation, oxidation, pegylation, proteolytic processing, phosphorylation, and the like. Alternatively, the functional variants may be antibodies comprising an amino acid sequence containing one or more substitutions, insertions, deletions or combinations thereof of one or more amino acids compared to the amino acid sequence of the parental antibody. Furthermore, the functional variants may comprise truncated forms of the amino acid sequence at either or both the amino or carboxyl termini. The functional variants of the present invention may have the same or different, either higher or lower, binding affinities compared to the parental antibody of the present invention but are still capable of binding to coronavirus or an S protein thereof. For instance, the amino acid sequences of the variable regions, including, but not limited to, framework regions, hypervariable regions, in particular, the light-chain or heavy-chain complementarity-determining regions (CDRs), may be modified. Generally, the light chain or heavy chain variable regions comprise three hypervariable regions, comprising three CDRs, and more conserved regions, the so-called framework regions (FRs). The hypervariable regions comprise amino acid residues from CDRs and amino acid residues from hypervariable loops. Functional variants falling within the scope of the present invention may have about 50% to 99%, about 60% to 99%, about 80% to 99%, about 90% to 99%, about 95% to 99%, or about 97% to 99% amino acid sequence identity with the parental antibody defined herein. Computer algorithms such as Gap or Bestfit known to a person skilled in the art may be used to optimally align amino acid sequences to be compared and to define similar or identical amino acid residues. The functional variants may be obtained by altering the parental antibodies or parts thereof by general molecular biology methods known in the art, including PCR, oligonucleotide-directed mutagenesis, and site-directed mutagenesis, or by organic synthesis processes, without being limited thereto.

The present invention also provides an immunoconjugate in which at least one tag is additionally conjugated to the binding molecule. In one embodiment, a drug may be additionally conjugated to the binding molecule. Specifically, the binding molecule according to the present invention may be used in the form of antibody-drug conjugates comprising a drug conjugated thereto. The use of antibody-drug conjugates (ADCs), i.e. immunoconjugates, for the local delivery of drugs, allows targeted delivery of the drug moiety to infected cells, because administration of unconjugated drug agents may result in unacceptable levels of toxicity to normal cells. The maximal efficacy and minimal toxicity of ADC can be achieved by increasing the selectivity of polyclonal and monoclonal antibodies (mAbs) as well as drug-linking and drug-releasing properties.

Conventional means of attaching, i.e., linking through covalent bonds, a drug moiety to an antibody generally leads to a heterogeneous mixture of molecules where the drug moieties are attached at a number of sites on the antibody. For example, cytotoxic drugs have typically been conjugated to antibodies through the often-numerous lysine residues of an antibody, thereby generating a heterogeneous antibody-drug conjugate mixture. Depending on reaction conditions, the heterogeneous mixture typically contains a distribution of antibodies with from 0 to about 8 or more, attached to drug moieties. In addition, each subgroup of conjugates with a particular integer ratio of drug moieties to antibody is a potentially heterogeneous mixture where the drug moiety is attached at various sites on the antibody. Antibodies are large, complex and structurally diverse biomolecules, often with many reactive functional groups. Their reactivities with linker reagents and drug-linker intermediates are dependent on factors such as pH, concentration, salt concentration, and co-solvents.

Another embodiment of the present invention provides a nucleic acid molecule encoding the binding molecule.

The nucleic acid molecule of the present invention includes any nucleic acid molecule in which the amino acid sequence of the antibody provided in the present invention is translated into a polynucleotide sequence as known to those skilled in the art. Thus, various polynucleotide sequences may be produced using an ORF (open reading frame), and may also be included within the range of the nucleic acid molecule of the present invention.

Another embodiment of the present invention provides an expression vector into which the nucleic acid molecule has been inserted.

The expression vector may be any one expression vector selected from the group consisting of a MarEx vector (see Korean Patent No. 10-1076602), which is an expression vector available from Celltrion, and a commercially widely useful pCDNA vector, F, R1, RP1, Co1, pBR322, ToL, and Ti vector; a cosmid; phages, such as lambda, lambdoid, M13, Mu, p1 P22, Qµ, T-even, T2, T3, T7, etc.; and plant viruses, without being limited thereto. Any expression vector known to those skilled in the art may be used in the present invention, and the expression vector may be selected depending on the properties of the host cell of interest. The introduction of the vector into the host cell may be performed by calcium phosphate transfection, viral infection, DEAE-dextran-mediated transfection, lipofectamine transfection, or electroporation, without being limited thereto, and those skilled in the art may adopt an introduction process suitable for the expression vector used and the host cell. For example, the expression vector may contain at least one selection marker, without being limited thereto, and even when a vector not containing a selection marker is used, selection is possible depending on whether or not the product is capable of being obtained. Choosing the selection marker depends on the host cell of interest, and is performed using any method known to those skilled in the art, and thus is not critical to the present invention.

In order to facilitate the purification of the binding molecule of the present invention, a tag sequence may be inserted into the expression vector and thus fused therewith. Examples of the tag include, but are not limited to, a hexa-histidine tag, a hemagglutinin tag, a myc tag or a flag tag, and any tag known to those in the art, which facilitates purification, may be used in the present invention.

The present invention also provides a cell line transformed with the expression vector. In one embodiment of the present invention, there is provided a cell line in which the expression vector is transformed into a host cell and which produces the binding molecule having ability to bind to and neutralize coronavirus.

In the present invention, the cell line may include cells of mammalian, plant, insect, fungal or cellular origin, without being limited thereto. As the mammalian cells, any one cell type selected from the group consisting of CHO cells, F2N cells, COS cells, BHK cells, Bowes melanoma cells, HeLa cells, 911 cells, HT1080 cells, A549 cells, HEK 293 cells and HEK293T cells may be used, without being limited thereto, and any cells may be used, as long as they are useful as mammalian host cells known to those skilled in the art.

The present invention also provides a composition for the diagnosis, amelioration, prevention and/or treatment of a disease caused by coronavirus, particularly SARS-coronavirus-2 (SARS-CoV-2), SARS-coronavirus-1 (SARS-CoV-1) or Middle East respiratory syndrome coronavirus (MERS-CoV), the composition comprising at least one of the binding molecule, the immunoconjugate, the nucleic acid molecule, the expression vector, and the cell line. Here, the disease caused by coronavirus may be coronavirus infection 2019 (COVID-19), severe acute respiratory syndrome, or Middle East respiratory syndrome, without being limited thereto. The above description of the binding molecule, the immunoconjugate, the nucleic acid molecule, the expression vector, and the cell line according to the present invention applies equally to the composition of the present invention. The composition of the present invention may comprise a pharmaceutically acceptable excipient, in addition to the binding molecule. As the pharmaceutically acceptable excipient, those already well known to those skilled in the art may be equally applied.

The composition of the present invention may further comprise one or more other therapeutic agents or diagnostic agents. For example, the composition of the present invention may further comprise, as an antiviral drug, interferon, an anti-S protein monoclonal antibody, an anti-S protein polyclonal antibody, a nucleoside analogue, a DNA polymerase inhibitor, a siRNA preparation, or a therapeutic vaccine, in addition to the binding molecule.

The composition comprising the binding molecule according to the present invention may be provided in the form of a formulation, such as a sterile injectable solution, a lyophilized formulation, a pre-filled syringe solution, an oral formulation, a formulation for external use, or a suppository, according to respective conventional methods, without being limited thereto.

Also, the composition comprising the binding molecule according to may be administered orally or parenterally. For example, the administration route may be intravenous administration, without being limited thereto.

The composition of the present invention may be administered to mammals including humans, thereby preventing and/or treating coronavirus infection, particularly SARS-coronavirus-2 (SARS-CoV-2), SARS-coronavirus-1 (SARS-CoV-1) or Middle East respiratory syndrome coronavirus (MERS-CoV) infection, and a disease caused by the infection. Here, the administration dose of the binding molecule (e.g. antibody) depends on a subject to be treated, the severity of disease or condition, administration rate, and the judgment of the prescribing physician, and may be, for example, 0.1 to 300 mg/kg.

The present invention also provides a kit for the diagnosis, amelioration, prevention and/or treatment of a disease caused by coronavirus, particularly SARS-coronavirus-2 (SARS-CoV-2), SARS-coronavirus-1 (SARS-CoV-1) or Middle East respiratory syndrome coronavirus (MERS-CoV), the kit comprising at least one of the binding molecule, the immunoconjugate, the nucleic acid molecule, the expression vector, and the cell line. The above description of the binding molecule, the immunoconjugate, the nucleic acid molecule, the expression vector, and the cell line according to the present invention applies equally to the kit of the present invention.

In one embodiment of the present invention, the binding molecule or the like of the present invention, which is used in the diagnostic kit, may be detectably labeled. Various methods that may be used to label biomolecules are well known to those skilled in the art and are considered to fall within the scope of the present invention. Examples of labels that may be used in the present invention include enzymes, radioisotopes, colloidal metals, fluorescent compounds, chemiluminescent compounds and bioluminescent compounds. Commonly used labels include fluorescent substances (e.g., fluorescein, rhodamine, Texas red, etc.), enzymes (e.g., horseradish peroxidase, β-galactosidase, and alkaline phosphatase), radioisotopes (e.g., 32P or 125I), biotin, digoxigenin, colloidal metals, and chemiluminescent or bioluminescent compounds (e.g., dioxetane, luminol or acridinium). Labeling methods such as covalent bonding, iodination, phosphorylation, biotinylation, etc. of enzymes or biotinyl groups are well known in the art. Detection methods include, but are not limited to, autoradiography, fluorescence microscopy, direct and indirect enzyme reactions, and the like. As commonly used detection assays, radioisotopic or non-radioisotopic assays may be applied to the present invention. These assays include, inter alia, Western blotting, overlay analysis, RIA (radioimmunoassay), IRMA (immunoradioimmunometric assay), EIA (enzyme immunoassay), ELISA (enzyme-linked immunosorbent assay), FIA (fluorescent immunoassay) and CLIA (chemiluminescent immunoassay), without being limited thereto.

The diagnostic kit of the present invention may be used to detect the presence or absence of coronaviruses, including SARS-CoV-2, SARS-CoV-1, and MERS-CoV, by bringing the binding molecule into contact with a sample and then observing the reaction. The sample may be, but is not limited to, any one selected from the group consisting of sputum, saliva, blood, sweat, lung cells, lung tissue mucus, respiratory tissue, and spit, isolated from a subject, and the sample may be prepared using a conventional method known to those skilled in the art.

Another embodiment of the present invention also provides
a kit for diagnosis, prevention or treatment of a disease caused by coronavirus, the kit comprising:
a) the binding molecule; and
b) a container.

Here, the disease caused by the coronavirus may be coronavirus infection 2019 (COVID-19), severe acute respiratory syndrome, or Middle East respiratory syndrome, without being limited thereto.

In the kit for diagnosis, amelioration, prevention and/or treatment according to the present invention, a solid carrier may be contained in the container of the kit. The antibody of the present invention may be attached to the solid carrier, and the solid carrier may be porous or non-porous, or may be planar or non-planar.

The present invention also provides a method of diagnosing, ameliorating, preventing and/or treating a disease caused by coronavirus, particularly, SARS-coronavirus-2 (SARS-CoV-2), SARS-coronavirus-1 (SARS-CoV-1) or Middle East respiratory syndrome coronavirus (MERS-CoV), the method comprising a step of administering a therapeutically effective amount of the binding molecule, immunoconjugate or composition of the present invention to either a subject having a disease caused by coronavirus, particularly, SARS-coronavirus-2 (SARS-CoV-2), SARS-coronavirus-1 (SARS-CoV-1) or Middle East respiratory syndrome coronavirus (MERS-CoV), or a subject suspected of or concerned about having the disease. Here, the disease caused by the coronavirus may be coronavirus infection 2019 (COVID-19), severe acute respiratory syndrome, or Middle East respiratory syndrome, without being limited thereto. The above description of the binding molecule, the immunoconjugate and the composition according to the present invention also applies equally to the method of the present invention. In one embodiment of the present invention, the method for diagnosis, amelioration, prevention and/or treatment may further comprise a step of administering an antiviral drug, a virus entry inhibitor or a virus adhesion inhibitor.

The present invention also provides a composition for diagnosing a disease caused by coronavirus, particularly, SARS-coronavirus-2 (SARS-CoV-2), SARS-coronavirus-1 (SARS-CoV-1) or Middle East respiratory syndrome coronavirus (MERS-CoV), the composition comprising at least one of the binding molecule, the immunoconjugate, the nucleic acid molecule, the expression vector and the cell line. Here, the disease caused by the coronavirus may be coronavirus infection 2019 (COVID-19), severe acute respiratory syndrome, or Middle East respiratory syndrome, without being limited thereto. The above description of the binding molecule, the immunoconjugate, the nucleic acid molecule, the expression vector and the cell line according to the present invention also applies equally to the composition of the present invention. The diagnostic composition of the present invention may comprise a pharmaceutically acceptable excipient, in addition to the binding molecule. As the pharmaceutically acceptable excipient, those already well known to those skilled in the art may be equally used.

The diagnostic composition of the present invention may further comprise one or more other diagnostic agents. For example, it may further comprise a binding molecule that binds to a nucleocapsid protein (N protein) on the surface of SARS-coronavirus-2 (SARS-CoV-2), in addition to the binding molecule described above.

In another embodiment of the present invention, the present invention also provides a strip for immunochromatographic analysis, the strip comprising the binding molecule that binds to a spike protein (S protein) on the surface of coronavirus. The strip for immunochromatographic analysis may further comprise a binding molecule that binds to a nucleocapsid protein (N protein) of coronavirus. The coronavirus may be any one selected from the group consisting of SARS-coronavirus-2 (SARS-CoV-2), human coronavirus 229E (HCoV-229E), human coronavirus OC43 (HCoV-OC43), severe acute respiratory syndrome coronavirus-1 (SARS-CoV-1), human coronavirus NL63 (HCoV-NL63), human coronavirus HKU1, and Middle East respiratory syndrome coronavirus (MERS-CoV), without being limited thereto.

The strip for immunochromatographic analysis may comprise:
i) a support;
ii) a sample pad configured to accommodate a sample to be analyzed and comprising a buffer inlet and a sample inlet;
iii) a conjugate pad containing a binding molecule that specifically binds to coronavirus contained in the sample introduced from the sample pad;
iv) a signal detection pad comprising a signal detection part to configured to detect whether coronavirus is present in the sample and a control part configured to check whether the sample has moved to an absorbent pad regardless of the presence or absence of an analyte (coronavirus); and
v) an absorbent pad configured to absorb the sample after completion of the signal detection reaction.

The strip may be configured such that a binding molecule that binds to a spike protein (S protein) on the surface of coronavirus is contained in each of the conjugate pad and the signal detection pad. The coronavirus S protein-binding molecule contained in the conjugate pad may be the same as or different from that contained in the signal detection pad. Also, the coronavirus S protein-binding molecule contained in each of the conjugate pad and the signal detection pad may be a binding molecule comprising the above-described sequence.

In the strip for immunochromatographic analysis, the binding molecule contained in the conjugate pad may be labeled with metal particles, latex particles, fluorescent materials, or enzymes. For example, the metal particles may be gold particles. The gold particles may be colloidal gold particles, without being limited thereto.

More specifically, the binding molecule of the present invention in the conjugate pad of the strip for immunochromatographic analysis may be detectably labeled. Various methods that may be used to label biomolecules are well known to those skilled in the art and are considered to fall within the scope of the present invention. Examples of labels that may be used in the present invention include enzymes, radioisotopes, colloidal metals, fluorescent compounds, chemiluminescent compounds and bioluminescent compounds. Commonly used labels include fluorescent substances (e.g., fluorescein, rhodamine, Texas red, etc.), enzymes (e.g., horseradish peroxidase, β-galactosidase, and alkaline phosphatase), radioisotopes (e.g., 32P or 125I), biotin, digoxigenin, colloidal metals, and chemiluminescent or bioluminescent compounds (e.g., dioxetane, luminol or acridinium). Labeling methods such as covalent bonding, iodination, phosphorylation, biotinylation, etc. of enzymes or biotinyl groups are well known in the art. Detection methods include, but are not limited to, autoradiography, fluorescence microscopy, direct and indirect enzyme reactions, and the like. As commonly used detection assays, radioisotopic or non-radioisotopic assays may be applied to the present invention. These assays include, inter alia, Western blotting, overlay analysis, RIA (radioimmunoassay), IRMA (immunoradioimmunometric assay), EIA (enzyme immunoassay), ELISA (enzyme-linked immunosorbent assay), FIA (fluorescent immunoassay) and CLIA (chemiluminescent immunoassay).

In the strip for immunochromatographic analysis, detection may be performed through visual observation, optical means, electrochemical means, or electrically conductive means, without being limited thereto.

In another embodiment of the present invention, the present invention provides a kit for diagnosing a disease caused by coronavirus, particularly SARS-coronavirus-2 (SARS-CoV-2), SARS-coronavirus-1 (SARS-CoV-1) or Middle East respiratory syndrome coronavirus (MERS-CoV), the kit comprising the strip for immunochromatographic analysis.

The diagnostic kit of the present invention may be used to detect the presence or absence of coronavirus by bringing the binding molecule into contact with a sample and then observing the reaction. The sample may be, but is not limited to, any one selected from the group consisting of sputum, saliva, blood, sweat, lung cells, lung tissue mucus, respiratory tissue, and spit, isolated from a subject, and the sample may be prepared using a conventional method known to those skilled in the art.

In another embodiment of the present invention, the present invention provides a method of detecting coronavirus, particularly SARS-coronavirus-2 (SARS-CoV-2), SARS-coronavirus-1 (SARS-CoV-1) or Middle East respiratory syndrome coronavirus (MERS-CoV), by using the diagnostic kit.

In another embodiment of the present invention, the present invention provides a method of detecting a disease caused by coronavirus, particularly SARS-coronavirus-2 (SARS-CoV-2), SARS-coronavirus-1 (SARS-CoV-1) or Middle East respiratory syndrome coronavirus (MERS-CoV), by using the diagnostic kit.

In another embodiment of the present invention, the present invention provides the use of the binding molecule for preparation of a composition for diagnosis, amelioration, prevention and/or treatment of a disease caused by coronavirus, particularly SARS-coronavirus-2 (SARS-CoV-2), SARS-coronavirus-1 (SARS-CoV-1) or Middle East respiratory syndrome coronavirus (MERS-CoV). Here, the disease caused by coronavirus may be coronavirus infection 2019 (COVID-19), severe acute respiratory syndrome, or Middle East respiratory syndrome, without being limited thereto. The above description of the binding molecule according to the present invention also applies equally to the use of the present invention.

In another embodiment of the present invention, the present invention provides the use of the composition for diagnosis, amelioration, prevention and/or treatment of a disease caused by coronavirus, particularly SARS-coronavirus-2 (SARS-CoV-2), SARS-coronavirus-1 (SARS-CoV-1) or Middle East respiratory syndrome coronavirus (MERS-CoV), the use comprising a step of administering a therapeutically effective amount of the binding molecule, immunoconjugate or composition of the present invention to either a subject having a disease caused by coronavirus, particularly, SARS-coronavirus-2 (SARS-CoV-2), SARS-coronavirus-1 (SARS-CoV-1) or Middle East respiratory syndrome coronavirus (MERS-CoV), or a subject suspected of or concerned about having the disease. Here, the disease caused by coronavirus may be coronavirus infection 2019 (COVID-19), severe acute respiratory syndrome, or Middle East respiratory syndrome, without being limited thereto. The above description of the binding molecule, the immunoconjugate or the composition according to the present invention also applies equally to the use of the present invention.

In another embodiment of the present invention, the present invention provides a method of producing a binding molecule, the method comprising steps of: introducing a nucleic acid molecule encoding the binding molecule into a host cell; culturing the host cell under conditions that allow expression of the nucleic acid molecule; and selecting the binding molecule from the cultured host cell and/or a culture of the host cell. The above description of the binding molecule according to the present invention mentioned above applies equally to the production method of the present invention.

In another embodiment of the present invention, the present invention provides a binding molecule produced according to the method for producing the binding molecule.

Hereinafter, the terms used in the present invention will be defined as follows.

As used herein, the term "binding molecule" refers to intact immunoglobulins, including monoclonal antibodies, such as chimeric, humanized or human monoclonal antibodies, or antigen-binding fragments, which are immunoglobulins that bind to antigens. For example, the term indicates a variable domain, enzyme, receptor or protein comprising an immunoglobulin fragment that competes with the intact immunoglobulin in order to bind to a spike protein on SARS-CoV-2. Regardless of the structure, an antigen-binding fragment binds to the same antigen that is recognized by the intact immunoglobulin. The antigen-binding fragment may comprise a peptide or polypeptide comprising an antibody amino acid sequence comprising 2 or more contiguous amino acid residues, 20 or more contiguous amino acid residues, 25 or more contiguous amino acid residues, 30 or more contiguous amino acid residues, 35 or more contiguous amino acid residues, 40 or more contiguous amino acid residues, 50 or more contiguous amino acid residues, 60 or more contiguous amino acid residues, 70 or more contiguous amino acid residues, 80 or more contiguous amino acid residues, 90 or more contiguous amino acid residues, 100 or more contiguous amino acid residues, 125 or more contiguous amino acid residues, 150 or more contiguous amino acid residues, 175 or more contiguous amino acid residues, 200 or more contiguous amino acid residues, or 250 or more contiguous amino acid residues.

As used herein, the term "antigen-binding fragment" includes Fab, F(ab'), F(ab')2, Fv, dAb, Fd, a complementarity-determining region (CDR) fragment, a single-chain antibody fragment (scFv), a bivalent single-chain antibody fragment, a single-chain phage antibody fragment, a diabody, a triabody, a tetrabody, a polypeptide that contains at least one fragment of an immunoglobulin that is sufficient to confer specific antigen binding to the polypeptide, etc. The above fragment may be produced synthetically or by enzymatic or chemical cleavage of intact immunoglobulins, or may be produced using a genetic engineering method by a recombinant DNA technique. Such production methods are well known in the art.

As used herein, the term "pharmaceutically acceptable excipient" refers to any inert substance that is combined with an active molecule such as a drug, agent, or antibody for preparing an agreeable or convenient dosage form. The pharmaceutically acceptable excipient is an excipient that is non-toxic, or at least of which the toxicity is acceptable for its intended use, to recipients at the dosages and concentrations employed and is compatible with other ingredients of the formulation comprising the drug, agent or binding molecule.

As used herein, the term "therapeutically effective amount" refers to an amount of the binding molecule of the present invention that is effective for prevention or treatment before or after exposure to coronavirus. In one embodiment of the present invention, the therapeutically effective amount may be, for example, 0.1 to 300 mg/kg.

As used herein, the term "culturing" means the *in vitro* maintenance, differentiation, growth, proliferation and/or reproduction of cells in a medium under suitable conditions.

As used herein, the term "comprises", "comprising", "includes" or "including" means that it may further comprise other components in addition to the corresponding component, and is different from the term "consisting of" that excludes further comprising other additional components.

The features described herein may be used in combination, and the fact that the features are set forth in different dependent claims of the claims does not indicate that they cannot be used in combination.

### Mode for Invention

Hereinafter, the present invention will be described in detail with reference to examples. However, the following examples are merely illustrative of the content of the present invention, and the scope of the invention is not limited by the examples. Documents cited herein are incorporated herein by reference.

### Example 1: Isolation of PBMCs from blood of patients who recovered from SARS-CoV-2

Blood donors were people (Korean adults) who were confirmed to have been infected with SARS-CoV-2 in 2020 and no longer exhibited viruses as a result of treatment, and the donor selection and blood collection processes were performed under the approval of the Institutional Review Board (IRB). After donor selection, about 30 ml of whole blood was collected, and PBMCs (peripheral blood mononuclear cells) were isolated using a Ficoll-Paque^{™} PLUS (GE Healthcare) method. The isolated PBMCs were washed twice with a phosphate buffer solution and then adjusted to a concentration of 1×10⁷ cells/ml in a freezing medium (RPMI:FBS:DMSO = 5:4:1) and stored in a liquid nitrogen tank.

### Example 2: Production of antibody-displayed phage library

Total RNA was extracted from the PBMCs (isolated in Example 1) using a TRIzol reagent (Invitrogen), and then cDNA was synthesized therefrom using a SuperScript^{™} III First-Strand cDNA synthesis system (Invitrogen, USA).

Production of the antibody library from the synthesized cDNA was performed with reference to the related literature (Barbas C. et. al. Phage Display: A Laboratory Manual. 2001. CSHL Press). Briefly, light-chain and heavy-chain variable regions of the antibody were amplified from the synthesized cDNA by a PCR (polymerase chain reaction) method using high-fidelity Taq polymerase (Roche) and a degenerative primer set (IDT). The isolated light-chain and heavy-chain variable-region fragments were made into an scFv gene by an overlap PCR method so as to be connected as one sequence in random combination, and the scFv gene was amplified, cleaved with restriction enzymes, and then isolated using 1% agarose gel electrophoresis and a gel extraction kit (Qiagen). A phage vector was cleaved with the same restriction enzymes, isolated, and mixed with the scFv gene, and T4 DNA ligase (New England Biolabs) was added thereto. The resulting mixture was allowed to react at 16°C for 12 hours or more. The resulting reaction solution was mixed with ER2738 competent cells, and then transformed into the cells by an electroporation process. The transformed ER2738 cells were subjected to shaking culture, and then a VCSM13 helper phage (Agilent Technologies) was added thereto, followed by culturing for 12 hours or more.

### Example 3: Selection using phage enzyme immunoassay

The phage library culture prepared in Example 2 was centrifuged to remove the host cells, and then 4% PEG and 0.5 M NaCl were added thereto. The phage was precipitated by centrifugation and the supernatant was removed. The precipitated phage was diluted with 1% BSA/TBS to afford a phage library, and then panning was independently performed through association to and dissociation from various SARS-CoV-2 spike proteins (S1, S2, S1+S2), SARS-CoV-1 spike proteins or MERS-CoV spike proteins, thereby isolating an scFv-phage having ability to bind to SARS-CoV-2, SARS-CoV-1 or MERS-CoV spike proteins. For example, the phage library was added to an ELISA plate to which an S2 domain (residues S686 to P1213 on S protein), which is a portion of the SARS-CoV-2 S protein, was attached, followed by reaction at room temperature for 2 hours. The reaction solution was removed, and then the ELISA plate was washed with PBS containing 0.05% Tween 20. Next, the scFv-phage bound to the antigen was detached by adding 60 µl of 0.1 M glycine-HCl (pH 2.2), and neutralized using 2M Tris (pH 9.1). The neutralized scFv-phage was infected into ER2738 cells, and then incubated with helper phage, and used for subsequent panning. A portion of the infected ER2738 was spread on an LB plate before the addition of the helper phage, and a colony was obtained the next day.

The colonies, formed in each panning round, were shake-cultured in a culture medium in a 96-well deep well plate (Axygen), and when the OD₆₀₀ reached 0.7 or more, helper phage was added thereto, followed by shaking culture at 37°C for 12 hours or more. The culture was centrifuged, the host cells were removed, and the supernatant containing the scFv-phage was collected.

The collected scFv-phage supernatant was diluted at 1:1 with 6% BSA/PBS, and then added to each well of a 96-well microtiter plate, which has been adsorbed with SARS-CoV-2 S proteins and then blocked, and each well was incubated at 37°C for 2 hours. Each well was washed three times with PBS containing 0.05% Tween 20, and then incubated with an anti-M13 antibody labeled with HRP (horseradish peroxidase) 37°C for 1 hour. Each well was washed three times with PBS containing 0.05% Tween 20, and then ABTS (2,2'-azinobis[3-ethylbenzothiazoline-6-sulfonic acid]-diammonium salt) was added thereto, and the absorbance at 405 nm was measured, thereby selecting scFv-phages having ability to bind to SARS-CoV-2 S antigen proteins.

### Example 4: Evaluation of neutralizing activity of scFv-Fc antibody fragment against SARS-CoV-2

For the scFv-phages selected in Example 3, DNA was obtained through shaking culture of colonies, and then sequences for antibody variable regions were analyzed. Among them, scFv-phages, selected by excluding clones with overlapping amino acid sequences, were cloned into vectors in the form of an scFv antibody fragment (scFv-Fc) in order to evaluate the expression levels of candidate antibodies in animal cell lines. The scFv-phages were transfected into CHO cells using a transfection reagent and expressed therein, and using the culture of the cells, the 190 scFv-Fc antibody fragments were evaluated against the Korean isolate SARS-CoV-2 virus S type (betaCoV/Korea/KCDC03/2020; NCCP43326) by CPE (cytopathic effect) assay. The 190 selected antibody fractions are shown in Tables 1 and 2.

For CPE assay, each antibody sample was diluted, mixed with an equal volume of 100TCID50 virus, incubated at 37°C for 30 minutes, and infected into the VERO.E6 cell line, and viable cells were analyzed. After 4 days of incubation in an incubator at 37°C under 5% CO₂, the neutralizing activity of each antibody fragment was evaluated by analyzing viable cells. Two wells at one concentration were independently analyzed, and the neutralizing activity (%) of the antibody was expressed as 100% when the cells in the two wells were variable. 50% indicates a case in which the cells in only one well were viable, and 0% indicates a case in which the cells in the two wells were not viable.

As a result of the analysis, as shown in Table 3 below, antibody fragments having neutralizing activity were identified. No. in Table 3 below refers to the same binding molecule as the No. of each binding molecule shown in Tables 1 and 2 above. The positive control antibody in Table 3 below is Celltrion's CT-P59 COVID-19 neutralizing antibody (antibody No. 139 disclosed in Korean Patent No. 10-2205028).

**[Table 3]**

| **No.** | **scFv-Fc dilution concentration (µg/ml)** | | | | |
|---|---|---|---|---|---|
| | **2** | **1** | **0.5** | **0.25** | **0.125** |
| **2** | 0 | 0 | 50 | 0 | 0 |
| **3** | 50 | 0 | 0 | 0 | 0 |
| **12** | 50 | 0 | 0 | 0 | 0 |
| **15** | 0 | 0 | 0 | 50 | 0 |
| **16** | 50 | 0 | 0 | 0 | 0 |
| **18** | 50 | 0 | 0 | 0 | 0 |
| **19** | 50 | 0 | 0 | 0 | 0 |
| **20** | 100 | 100 | 100 | 100 | 100 |
| **22** | 0 | 50 | 0 | 0 | 0 |
| **23** | 50 | 0 | 0 | 0 | 0 |
| **24** | 100 | 100 | 100 | 0 | 0 |
| **26** | 0 | 0 | 50 | 50 | 0 |
| **31** | 50 | 0 | 0 | 0 | 0 |
| **32** | 100 | 100 | 100 | 100 | 100 |
| **33** | 50 | 50 | 0 | 0 | 0 |
| **34** | 100 | 100 | 100 | 50 | 0 |
| **37** | 50 | 50 | 50 | 50 | 0 |
| **43** | 100 | 0 | 0 | 0 | 0 |
| **45** | 100 | 50 | 50 | 50 | 0 |
| **48** | 100 | 100 | 100 | 100 | 100 |
| **50** | 0 | 50 | 0 | 0 | 0 |
| **51** | 50 | 0 | 0 | 0 | 0 |
| **53** | 50 | 50 | 50 | 0 | 0 |
| **54** | 100 | 100 | 100 | 100 | 100 |
| **55** | 0 | 0 | 50 | 0 | 0 |
| **56** | 0 | 0 | 50 | 50 | 0 |
| **59** | 50 | 50 | 0 | 0 | 0 |
| **60** | 50 | 50 | 0 | 0 | 0 |
| **61** | 100 | 100 | 100 | 50 | 0 |
| **66** | 50 | 50 | 50 | 0 | 0 |
| **69** | 50 | 0 | 0 | 0 | 0 |
| **74** | 50 | 0 | 0 | 0 | 0 |
| **75** | 50 | 50 | 0 | 0 | 0 |
| **76** | 100 | 50 | 0 | 0 | 0 |
| **81** | 100 | 100 | 100 | 100 | 100 |
| **82** | 50 | 0 | 0 | 0 | 0 |
| **83** | 50 | 0 | 0 | 0 | 0 |
| **85** | 50 | 0 | 0 | 0 | 0 |
| **86** | 50 | 0 | 0 | 0 | 0 |
| **87** | 50 | 50 | 50 | 0 | 0 |
| **93** | 50 | 50 | 50 | 0 | 0 |
| **94** | 100 | 0 | 0 | 0 | 0 |
| **95** | 100 | 0 | 0 | 0 | 0 |
| **96** | 100 | 0 | 0 | 0 | 0 |
| **98** | 50 | 50 | 0 | 0 | 0 |
| **102** | 100 | 0 | 0 | 0 | 0 |
| **103** | 0 | 0 | 100 | 100 | 50 |
| **104** | 100 | 100 | 50 | 0 | 0 |
| **108** | 0 | 50 | 0 | 0 | 0 |
| **Positive control antibody** | 100 | 100 | 100 | 100 | 100 |

### Example 5: Evaluation of binding affinity of antibody after conversion into fully human antibody (full IgG)

The selected antibody fragments (scFv-Fc) confirmed to have neutralizing activity in Example 4 above were converted into fully human antibodies (full IgG), and antibody-containing cultures were prepared according to the method of Example 4. The binding affinity (equilibrium dissociation constant, K_{D}) of each fully human antibody to SARS-CoV-2 RBD was evaluated by biolayer interference (BLI) assay using Octet. As a result of analysis, it was confirmed that 11 fully human antibodies had excellent binding affinity to the SARS-CoV-2 virus surface protein (RBD) as shown in Table 4 below. No. in Table 4 below refers to the same binding molecule as the No. of each binding molecule shown in Tables 1 and 2 above.

**[Table 4]**

| **No.** | K_{D} (M) |
|---|---|
| **32** | 1.47E-10 |
| **33** | 4.31E-11 |
| **34** | 1.85E-10 |
| **37** | 5.70E-11 |
| **45** | 7.08E-11 |
| **48** | 1.84E-10 |
| **53** | 3.39E-09 |
| **54** | 3.13E-11 |
| **59** | 4.96E-11 |
| **61** | 2.26E-11 |
| **81** | 4.28E-11 |

### Example 6: Evaluation of neutralizing activity of fully human antibody (full IgG)

### 6-1. Evaluation (1) of neutralizing activity of fully human antibody (full IgG)

The 11 fully human antibodies (full IgGs) selected based on their binding affinity to the SARS-CoV-2 S (RBD) protein and their expression level in Example 5 were evaluated for neutralizing activity against the Korean isolate SARS-CoV-2 virus GR type (hCoV-19/South Korea/KUMC17/2020) using a PRNT (plaque reduction neutralization test) method.

For PRNT, each antibody sample was diluted (10 concentrations, obtained through 1/4 serial dilution from 1 ng/µl, and 11 concentrations, obtained through 1/2 serial dilution from 0.5 ng/ µl), mixed with an equal volume of a virus (0.05 MOI), incubated at 37°C for 2 hours, and then infected into VERO.E6 cells, followed by plaque assay. After incubation in a 5% CO₂ incubator at 37°C for 60 hours, the neutralizing activity of each antibody sample was evaluated by staining with crystal violet and comparatively analyzing the number of formed plaques. The neutralizing activity (ng/ml) of each antibody is an average value obtained from the results of the neutralizing activity evaluation performed in duplicate, and was expressed as the IC₅₀ value of the antibody-treated group. Here, the IC₅₀ value is the antibody concentration at which the antibody exhibits 50% of the neutralizing activity against the virus. In Table 5 below, a lower value indicates the better neutralizing activity.

As a result of analysis, as shown in Table 5 below, fully human antibodies (full IgG) having excellent neutralizing activity were confirmed. In Table 5 below, No. refers to the same binding molecule as the No. of each binding molecule shown in Tables 1 and 2.

**[Table 5]**

| **No.** | **IC₅₀ (ng/ml)** |
|---|---|
| **32** | 92.17 |
| **33** | >1000 |
| **34** | 448.9 |
| **37** | 542.8 |
| **45** | 434.5 |
| **48** | 155.8 |
| **53** | 381.4 |
| **54** | 63.8 |
| **59** | >1000 |
| **61** | 798.6 |
| **81** | 669.9 |

### 6-2. Evaluation (2) of neutralizing activity of fully human antibody (full IgG)

6 fully human antibodies (full IgGs), selected based on antibody characteristics such as antibody expression levels, were additionally evaluated for neutralizing activity against the SARS-CoV-2 GR type virus using a PRNT (plaque reduction neutralization test) method.

As a result of analysis, it was confirmed that the antibodies had neutralizing activity as shown in Table 6 below. In Table 6 below, No. refers to the same binding molecule as the No. of each binding molecule shown in Tables 1 and 2. The positive control antibody in Table 6 below is Celltrion's CT-P59 COVID-19 neutralizing antibody.

**[Table 6]**

| **No.** | **IC₅₀ (ng/ml)** |
|---|---|
| **32** | 21.1 |
| **34** | 499.4 |
| **37** | 1082.4 |
| **45** | 521.9 |
| **54** | 16.0 |
| **32+54** | 11.1 |
| **Positive control** | 2.0 |

### 6-3. Evaluation (3) of neutralizing activity of fully human antibody (full IgG)

Antibody No. 32, selected based on antibody characteristics such as antibody expression levels, was additionally evaluated against the Korean isolate GR type (hCoV-19/South Korea/KUMC17/2020), GH type (hCoV-19/Korea/KCDC10847/2020), the mutant virus strain from the UK (hCoV-19/South Korea/KDCA0838/2020) and the mutant virus strain from South African (hCoV-19/South Korea/KDCA0463/2020) by a PRNT (plaque reduction neutralization test) method according to the analysis method described in Example 6-1..

As a result of analysis, it was confirmed that the antibody had neutralizing activity against each virus strain as shown in Table 7 below.

**[Table 7]**

| **Type** | **IC₅₀ (ng/ml)** |
|---|---|
| GR | 71.1 |
| GH | 176.2 |
| Mutant virus strain from the UK | 31.73 |
| Mutant virus strain from South African | 27.62 |

### 6-4. Evaluation (4) of neutralizing activity of fully human antibody (full IgG)

The antibodies, selected based on antibody characteristics such as antibody expression levels, were additionally evaluated for neutralizing activity against SARS-CoV-1 and MERS-CoV viruses.

As a result of analysis, it was confirmed that the antibodies had neutralizing activity as shown in Table 8 below. In Table 8 below, No. refers to the same binding molecule as the No. of each binding molecule shown in Tables 1 and 2 above. In Table 8 below, the positive control antibody against SARS-CoV-1 is CR3022 (Xiaolong Tian et al., Emerg Microbes Infect. 2020 Feb 17;9(1):382-385), and the positive control antibody against MERS-CoV is Celltrion's CT-P38 MERS neutralizing antibody (antibody 5 disclosed in Table 7 of Korean Patent Application Publication No. 10-2019-0093114).

**[Table 8]**

| **No.** | MERS-CoV | SARS-CoV-1 |
|---|---|---|
| | IC₅₀ (ng/ml) | IC₅₀ (ng/ml) |
| **2** | 6682.3 | 4001.4 |
| **59** | >10000 | 6729.5 |
| **95** | 9690.8 | >10000 |
| **102** | >10000 | 2314.7 |
| **103** | 8132.8 | >10000 |
| **32** | 8377.7 | >10000 |
| **CT-P59** | 8125.1 | >10000 |
| **CR3022** | >10000 | 6843.6 |
| **CT-P38** | 51.8 | >10000 |

### Example 6-5. Evaluation (1) of neutralizing activity of fully human antibody (full IgG) against SARS-CoV-2 mutant pseudoviruses

A test was conducted to evaluate the neutralizing activity of a cocktail of antibody No. 32 + antibody No. 54 against SARS-CoV-2 spike D614 and D614G and 20 prepared SARS-CoV-2 spike mutant pseudoviruses. Here, antibody No. 32 and antibody No. 54 respectively refer to binding molecule No. 32 and binding molecule No. 54 shown in Tables 1 and 2. The SARS-CoV-2 spike mutant pseudoviruses were prepared with reference to a portion of the epitope of the CT-P59 (Regdanvimab) antibody and the mutant viruses published in papers (A. Baum et al., Science, 2020 Aug 21;369(6506): 1014-1018, and Korber et al., 2020, Cell 182, 812-827). The amount of each mutant pseudovirus was fixed at 1.73 × 10⁷ copies, and the antibody was diluted in 10 steps of 3-fold dilutions to the highest concentration of 1,000 ng/mL. As a result of conducting a neutralization activity test using each mutant pseudovirus and each antibody dilution, it was confirmed that the antibody had neutralizing activity as shown in Table 9 below.

In Table 9 below, each mutation position was numbered from the N-terminus of the coronavirus spike protein (NCBI accession number: YP_009724390.1, SEQ ID NO: 1,521).

**[Table 9]**

| **No.** | **SARS-CoV-2 mutant pseudovirus** | **Backbone vector** | **IC₅₀ (ng/mL)** |
|---|---|---|---|
| 1 | D614 | - | 5.229 |
| 2 | S494P | D614 | 13.42 |
| 3 | R685H | D614 | 5.046 |
| 4 | S494P+R685H | D614 | 2.676 |
| 5 | E484K | D614 | 13.63 |
| 6 | Q493K | D614 | 10.15 |
| 7 | F490S | D614 | 13.25 |
| 8 | Y449N | D614 | 12.55 |
| 9 | L455F | D614 | 6.989 |
| 10 | F456L | D614 | 3.056 |
| 11 | L452R | D614 | 25.73 |
| 12 | E406Q | D614 | 15.11 |
| 13 | K444Q | D614 | 8.646 |
| 14 | V445A | D614 | 7.124 |
| 15 | N234Q | D614 | 70.01 |
| 16 | A475V | D614 | 7.35 |
| 17 | D614G | - | 6.723 |
| 18 | S477N | D614G | 4.659 |
| 19 | A222V | D614G | 6.404 |
| 20 | V1176F | D614G | 9.035 |
| 21 | N439K | D614G | 6.225 |
| 22 | Y453F | D614G | 8.515 |

### Example 6-6. Evaluation (2) of neutralizing activity of fully human antibody (full IgG) against SARS-CoV-2 mutant pseudoviruses

A test was conducted to evaluate the neutralizing activities of antibody No. 32 and antibody No. 54 against 15 SARS-CoV-2 mutant pseudoviruses. Here, antibody No. 32 and antibody No. 54 respectively refer to binding molecule No. 32 and binding number 54 shown in Tables 1 and 2. The SARS-CoV-2 spike mutant pseudoviruses were prepared with reference to a portion of the epitope of the CT-P59 (Regdanvimab) antibody and the mutant viruses published in papers (A. Baum et al., Science, 2020 Aug 21;369(6506): 1014-1018, and Korber et al., 2020, Cell 182, 812-827). The amount of each mutant pseudovirus was fixed at 1.73 × 10⁷ copies, and the antibody was diluted in 10 steps of 3-fold dilutions to the highest concentration of 1,000 ng/mL. As a result of conducting a neutralization activity test using each mutant pseudovirus and each antibody dilution, as shown in Table 10 below, it was confirmed that the antibody had neutralizing activity.

In Table 10 below, each mutation position was numbered from the N-terminus of the coronavirus spike protein (NCBI accession number: YP_009724390.1, SEQ ID NO: 1521).

**[Table 10]**

| **No.** | **SARS-CoV-2 mutant pseudovirus** | **IC₅₀ (ng/ml)** | |
|---|---|---|---|
| | | **No.32** | **No.54** |
| 1 | D614G | 7 | 100 |
| 2 | K417N+E484K+N501Y | 10 | 2870 |
| 3 | 501Y.V2 | 60 | 2180 |
| 4 | D614G | 3.25 | 12.84 |
| 5 | K417N | 1 | 4.817 |
| 6 | E484K | 5.9 | >1000 |
| 7 | L452R | 3.39 | >1000 |
| 8 | Y449N | 2.14 | 21.9 |
| 9 | L455F | 7.52 | 8.87 |
| 10 | F456L | 2.4 | 2.15 |
| 11 | Q493K | 5.62 | 9.92 |
| 12 | S494P | 6.29 | 5.85 |
| 13 | E406Q | 3.26 | 1.71 |
| 14 | F490S | 6.08 | 12 |
| 15 | K417T | 0.91 | 7.08 |

### 6-7. Evaluation of neutralizing activity of antibody No. 32

Antibody No. 32 (corresponding to No. 32 in Tables 1 and 2), which is a fully human antibody (full IgG) selected based on its binding affinity to the SARS-CoV-2 S (RBD) protein and expression level and its neutralizing activity against the wild-type SARS-CoV-2 virus, was evaluated for neutralizing activity against the mutant virus strain from South Africa (hCoV-19/South Korea/KDCA0463/2020) by a PRNT (plaque reduction neutralization test) method.

For PRNT, the antibody sample was diluted (11 concentrations obtained through 1/2 serial dilution from 1 ng/µl), mixed with an equal volume of a virus (0.05 MOI), incubated at 37°C for 2 hours, and then infected into VERO.E6 cells, followed by plaque assay. After incubation in a 5% CO₂ incubator at 37°C for 60 hours, the neutralizing activity of the antibody sample was evaluated by staining with crystal violet and comparatively analyzing the number of formed plaques. The neutralizing activity (ng/ml) of the antibody is an average value obtained from the results of the neutralizing activity evaluation performed in duplicate, and was expressed as the IC₅₀ value and IC₉₀ value of the antibody-treated group. Here, the IC₅₀ value is the antibody concentration at which the antibody exhibits 50% of the neutralizing activity against the virus, and the IC₉₀ value is the antibody concentration at which the antibody exhibits 90% of the neutralizing activity against the virus. In Table 11 below, a lower value indicates the better neutralizing activity.

As a result of analysis, it was confirmed that antibody No. 32 had neutralizing activity against the mutant virus strain from South Africa as shown in Table 11 below.

**[Table 11]**

| **Antibody** | **IC₅₀ (ng/ml)** | **IC₉₀ (ng/ml)** |
|---|---|---|
| **No. 32** | **24.45** | **59.78** |

### 6-8. Evaluation of neutralizing activity of antibody No. 32 against SARS-CoV-2 mutant pseudoviruses

A test was conducted to evaluate the neutralizing activities of antibody No. 32 against SARS-CoV-2 spike D614 and 50 prepared SARS-CoV-2 spike mutant pseudoviruses. The SARS-CoV-2 spike mutant pseudoviruses were prepared with reference to a portion of the epitope of the CT-P59 (Regdanvimab) antibody and the mutant viruses published in papers (A. Baum et al., Science, 2020 Aug 21;369(6506):1014-1018., Korber et al., 2020, Cell 182, 812-827., and Wang et al.,2021, doi: 10.1038/s41586-021-03398-2.). The amount of each mutant pseudovirus was fixed at 1.73 × 10⁷ copies, and the antibody was diluted in 10 steps of 3-fold dilutions to the highest concentration of 100 ng/mL. As a result of conducting a neutralization activity test using each mutant pseudovirus and each antibody dilution, it was confirmed that the antibody had neutralizing activity as shown in Table 12 below.

In Table 12 below, each mutation position was numbered from the N-terminus of the coronavirus spike protein (NCBI accession number: YP_009724390.1, SEQ ID NO: 1,521). In addition, the position of each mutation in UK mutant virus strain 501Y.V1 (B.1.1.7), South Africa mutant virus strain 501Y.V2 (B.1.351), Brazil mutant virus strain 501Y.V3 (P.1), California mutant virus strain (B.1.429), New York mutant virus strain (B.1.525) and New York mutant virus strain (B.1.526) is shown in Reference Table A below.

**[Table 12]**

| **No.** | **SARS-CoV-2 mutant pseudovirus** | **Backbone vector** | **IC₅₀ (ng/ml)** |
|---|---|---|---|
| **1** | D614 | - | 4.342 |
| **2** | D614G | - | 4.113 |
| **3** | Q493R | D614G | 4.941 |
| **4** | K417E | D614G | 1.133 |
| **5** | G446V | D614G | 5.019 |
| **6** | G476S | D614G | 6.036 |
| **7** | F486V | D614G | 4.222 |
| **8** | E406W | D614G | 7.836 |
| **9** | N440D | D614G | 4.238 |
| **10** | P681H | D614G | 3.011 |
| **11** | K417N | D614G | 1.344 |
| **12** | A701V | D614G | 4.139 |
| **13** | D80A | D614G | 2.657 |
| **14** | K417N+E484K+N501Y | D614G | 2.71 |
| **15** | Q493M | D614G | 4.785 |
| **16** | F486I | D614G | 3.18 |
| **17** | Y489H | D614G | 8.358 |
| **18** | N501Y | D614G | 4.838 |
| **19** | HV69-70 del | D614G | 4.156 |
| **20** | HV69-70del + N501Y | D614G | 4.633 |
| **21** | N501T | D614G | 5.959 |
| **22** | N501F | D614G | 5.952 |
| **23** | Q677H | D614G | 4.84 |
| **24** | Q498H | D614G | 2.558 |
| **25** | N460T | D614G | 16.71 |
| **26** | F486S | D614G | 2.275 |
| **27** | F486L | D614G | 2.366 |
| **28** | T478K | D614G | 2.575 |
| **29** | K417T | D614G | 0.977 |
| **30** | Q493Y | D614G | 1.124 |
| **31** | Q493A | D614G | 5.745 |
| **32** | A372V | D614G | 2.441 |
| **33** | P384L | D614G | 4.404 |
| **34** | S494L | D614G | 4.638 |
| **35** | 501Y.V3 (P.1) | D614G | 1.71 |
| **36** | 501Y.V2 (B.1.351) | D614G | 3.272 |
| **37** | B.1.526 | D614G | 3.53 |
| **38** | E484Q+L452R+P681R | D614G | 8.264 |
| **39** | UK (B.1.1.7) | D614G | 5.413 |
| **40** | California (B.1.429) | D614G | 6.723 |
| **41** | New York (B.1.525) | D614G | 5.569 |
| **42** | S477R | D614G | 8.772 |
| **43** | D420N | D614G | - |
| **44** | Y473F | D614G | 5.248 |
| **45** | S494Q | D614G | 2.756 |
| **46** | E484G | D614G | 3.556 |
| **47** | S477N | D614G | 4.561 |
| **48** | S494P | D614G | 6.49 |
| **49** | Y453F | D614G | 4.422 |
| **50** | N439K | D614G | 5.352 |
| **51** | A222V | D614G | 4.721 |
| **52** | N234Q | D614G | 14.41 |

### (Reference Table A)

| | | | | | | | | | | |
|---|---|---|---|---|---|---|---|---|---|---|
| UK 501Y.V1 (B.1.1.7) | HV 69-70del | Y144del | | | N501Y | D614G | T716I | | S982A | D1118H |
| | | | | | A570D | P681H | | | | |
| South Africa 501Y.V2 (B.1.351) | L18F | | D215G | K417N | N501Y | D614G | A701V | | | |
| | D80A | LAL 242-244del | | E484K | | | | | | |
| Brazil 501Y.V3 (P.1) | L18F | D138Y | | K417T | N501Y | D614G | | | | T1027I |
| | T20N | R190S | | E484K | | H655Y | | | | V1176F |
| | P26S | | | | | | | | | |
| California (B.1.429) | S13I | W152C | | L452R | | D614G | | | | |
| | A67V | Y144del | | E484K | | D614G | | F888L | | |
| New York (B.1.525) | HV 69-70del | | | | | Q677H | | | | |
| New York (B.1.526) | L5F | D253G | | E484K | | D614G | A701V | | | |
| | T951 | | | | | | | | | |

### 6-9. Evaluation of neutralizing activity of antibody No. 32 against SARS-CoV-2 mutant viruses from US and South Africa

Antibody No. 32, selected based on antibody characteristics such as antibody expression level, was additionally evaluated against the mutant virus strains from California, USA ((hCoV-19/Korea/KDCA49671/2021 (B. 1.427) and hCoV-19/Korea/KDCA59777/2021 (B. 1.429) and the mutant virus strain from South Africa (hCoV-19/South Korea/KDCA0463/2020, B.1.351) by a PRNT (plaque reduction neutralization test) method as described above. As a result, it was confirmed that the antibody had neutralizing activity as shown in Table 13 below.

**[Table 13]**

| **No.** | **Mutant virus** | **IC₅₀ (ng/ml)** |
|---|---|---|
| 1 | (B.1.427) from California, USA | 58.48 |
| 2 | (B.1.429) from California, USA | 34.28 |
| 3 | Mutant strain (B.1.351) from South Africa | 20.58 |

### 6-10. Evaluation of neutralizing activity of antibody No. 32 against SARS-CoV-2 mutant viruses from New York and Nigeria

Antibody No. 32, selected based on antibody characteristics such as antibody expression level, was additionally evaluated against the Korean isolate GR type (B.1.1.119) as a control, the mutant virus strain from New York, USA (hCoV-19/Korea/KDCA82438/2021, B.1.526), and the mutant virus strain (B.1.525) from UK/Nigeria by a PRNT (plaque reduction neutralization test) method as described above. As a result, it was confirmed that the antibody had neutralizing activity as shown in Table 14 below.

**[Table 14]**

| **No.** | **Mutant virus** | **IC₅₀ (ng/ml)** |
|---|---|---|
| 1 | (B.1.526) from New York, USA | 44.44 |
| 2 | (B.1.525) from UK/Nigeria | 14.53 |
| 3 | GR (B.1.1.119) | 50.5 |

### 6-11. Evaluation of neutralizing activity of antibody No. 32 against SARS-CoV-2 mutant virus (P.1) from Brazil

Antibody No. 32, selected based on antibody characteristics such as antibody expression level, was additionally evaluated against the mutant virus strain (P.1) from Brazil by a PRNT (plaque reduction neutralization test) method as described above. As a result of analysis, it was confirmed that the antibody had neutralizing activity as shown in Table 15 below.

**[Table 15]**

| **No.** | **Mutant virus** | **IC₅₀ (ng/ml)** |
|---|---|---|
| 1 | (P.1) from Brazil | 7.21 |

### 6-12. Evaluation of neutralizing activity of antibody No. 32 against SARS-CoV-2 mutant viruses (B.1.617.1, and B.1.617.2) from India

Antibody No. 32, selected based on antibody characteristics such as antibody expression level, was additionally evaluated against the mutant virus strains (hCoV-19/Korea/KDCA2950/2021 (B.1.617.1), and hCoV-19/Korea119861/KDCA/2021(B.1.617.2)) from India by a PRNT (plaque reduction neutralization test) method as described above. As a result of analysis, it was confirmed that the antibody had neutralizing activity as shown in Table 16 below.

**[Table 16]**

| **No.** | **Mutant virus** | **IC₅₀ (ng/ml)** |
|---|---|---|
| 1 | (B.1.617.1) from India | 25.84 |
| 2 | (B.1.617.2) from India | 18.88 |

### Example 7. Evaluation of binding affinity and mechanism of action of fully human antibody (full IgG)

The binding affinity (antibody No. 32 and No. 54) and mechanism of action (antibody No. 32) of the antibodies (full IgG), selected in Examples 5 and 6, to SARS-CoV-2 RBD mutant proteins, were evaluated by performing Octet analysis.

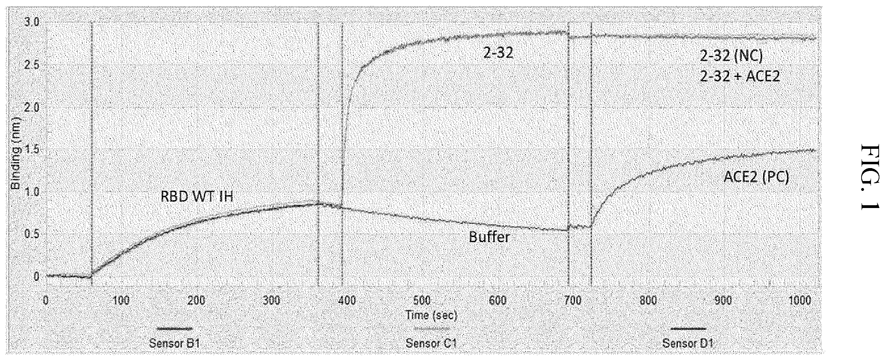

SARS-CoV-2 virus can initiate infection of human cells by binding of the surface protein (RBD) thereof to the human receptor (ACE2). Thus, the mechanism of action of antibody No. 32 (full IgG) was evaluated by performing biolayer interference (BLI) assay using Octet. As a result of analysis, it was confirmed that the antibodies had excellent binding affinity even to mutant proteins of the SARS-CoV-2 virus surface protein (RBD) as shown in Table 17 below, and that the binding of the SARS-CoV-2 virus surface protein (RBD) to the human receptor (ACE2) was completely inhibited by antibody No. 32 (full IgG) as shown in FIG. 1.

**[Table 17]**

| **No.** | **RBD type** | **K_{D} (M)** | **kon (1/Ms)** | **kdis (1/s)** |
|---|---|---|---|---|
| **32** | WT | 3.05E-10 | 6.33E+05 | 1.93E-04 |
| **54** | | 1.33E-10 | 8.15E+05 | 1.08E-04 |
| **32** | S494P | 1.61E-10 | 5.48E+05 | 8.82E-05 |
| **54** | | 1.84E-11 | 8.22E+05 | 1.51E-05 |
| **32** | K417N | 3.13E-10 | 4.74E+05 | 1.48E-04 |
| **54** | | 6.63E-11 | 1.06E+06 | 7.04E-05 |
| **32** | E484K | 2.90E-10 | 6.63E+05 | 1.92E-04 |
| **54** | | 1.73E-09 | 2.32E+05 | 4.01E-04 |
| **32** | N501Y | 3.79E-10 | 4.72E+05 | 1.79E-04 |
| **54** | | 1.18E-10 | 8.13E+05 | 9.56E-05 |
| **32+54** | WT | 2.74E-10 | 3.72E+05 | 1.02E-04 |
| **32+54** | S494P | 1.99E-10 | 4.14E+05 | 8.22E-05 |
| **32+54** | K417N | 1.60E-10 | 4.62E+05 | 7.39E-05 |
| **32+54** | E484K | 6.90E-10 | 2.48E+05 | 1.71E-04 |
| **32+54** | N501Y | 2.35E-10 | 4.13E+05 | 9.68E-05 |

### Example 8. Evaluation of neutralizing activity of fully human antibody (full IgG) against SARS-CoV-2 virus through animal experiment

### 8-1. Experiment for evaluation of preventive efficacy in mice

Using TG mice (B6.Cg-Tg(K18-ACE2)2Prlmn/J [Stock No: 034860 | K18-hACE2] from The Jackson Laboratory) as an animal model that is naturally infected with SARS-CoV-2 virus and shows clinical symptoms and lesions similar to those in humans, an experiment was conducted as follows in order to evaluate the *in-vivo* preventive efficacy of antibody No. 32 and No. 54.

Mice were divided into a total of 10 groups, including a non-infected group, an infected group, and administered groups (1 mg/kg or 10 mg/kg CT-P59, 1 mg/kg or 10 mg/kg antibody No. 32, 1 mg/kg or 10 mg/kg antibody No. 54, and 1 mg/kg or 10 mg/kg antibody No. 32 + antibody No. 54 cocktail). The non-infected group consisted of 3 mice, and the infected group and the administered groups consisted of 6 mice per group. 24 hours after administration of DPBS or 1 mg/kg or 10 mg/kg of each antibody to each group, 1×10⁵ PFU/50 µL of SARS-CoV-2 virus (NMC-nCoV02) was inoculated into the nasal cavity, and observation was performed for a maximum of 6 days. Additionally, the body weight of each mouse in each group was evaluated before viral inoculation and daily for 6 days after viral inoculation. In order to measure the virus titer in the tissue, the mice were sacrificed on days 3 and 6 after viral inoculation, and the lung tissue and nasal lavage fluid were obtained therefrom. The virus titer of each tissue was measured through plaque assay using Vero cells.

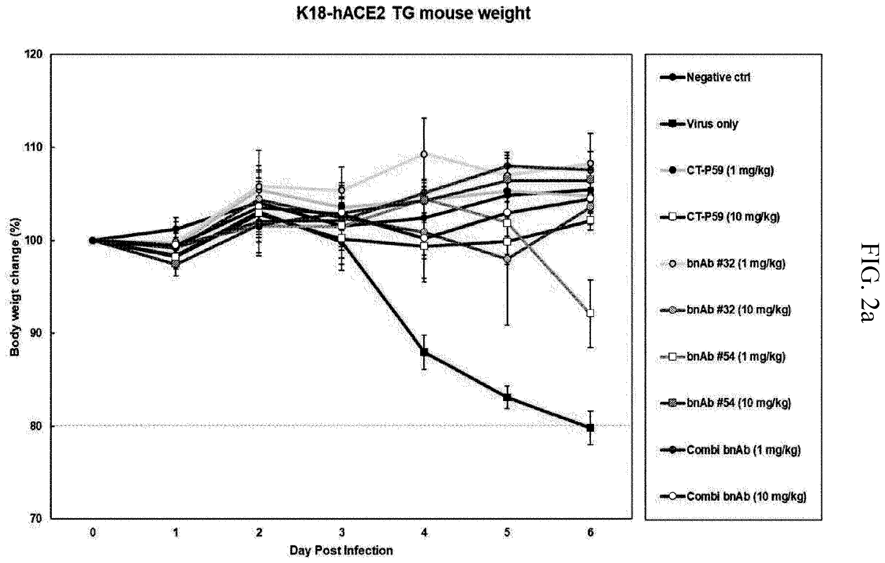

As a result, antibody No. 32 (1 and 10 mg/kg) and antibody No. 54 (10 mg/kg) showed non-inferiority in terms of body weight change compared to CT-P59. A low dose (1 mg/kg) of antibody No. 54 showed significantly lower results in terms of body weight change compared to CT-P59 and antibody No. 32 (FIG. 2a).

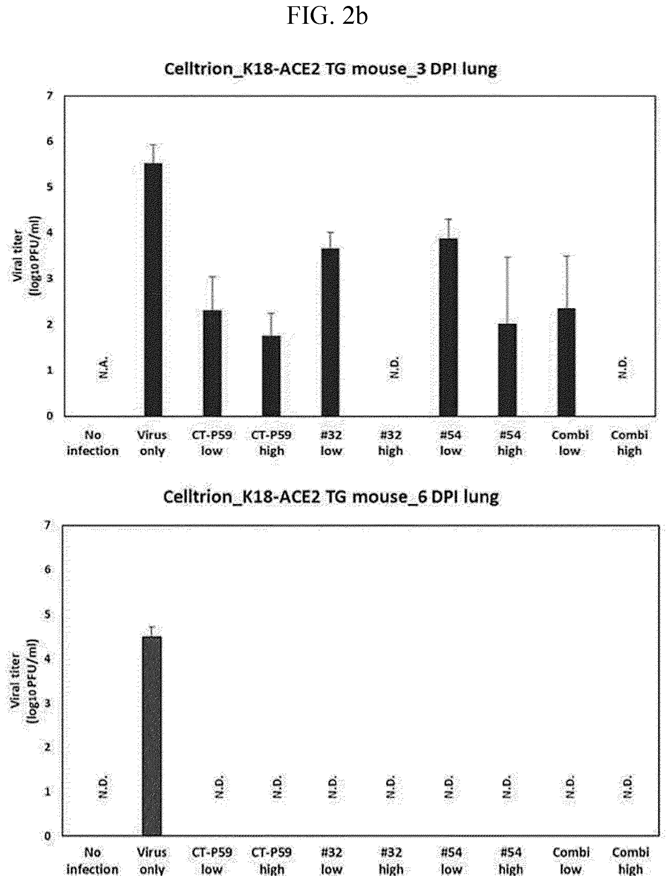

The results of measuring the virus titer in the lungs by plaque assay are as follows. A high dose (10 mg/kg) of antibody No. 32 antibody and a high dose (10 mg/kg) of the cocktail of antibody No. 32 and antibody No. 54 showed the best preventive efficacy because the virus was not detected from day 3. On day 6, all of the antibody-administered groups showed preventive efficacy (FIG. 2b).

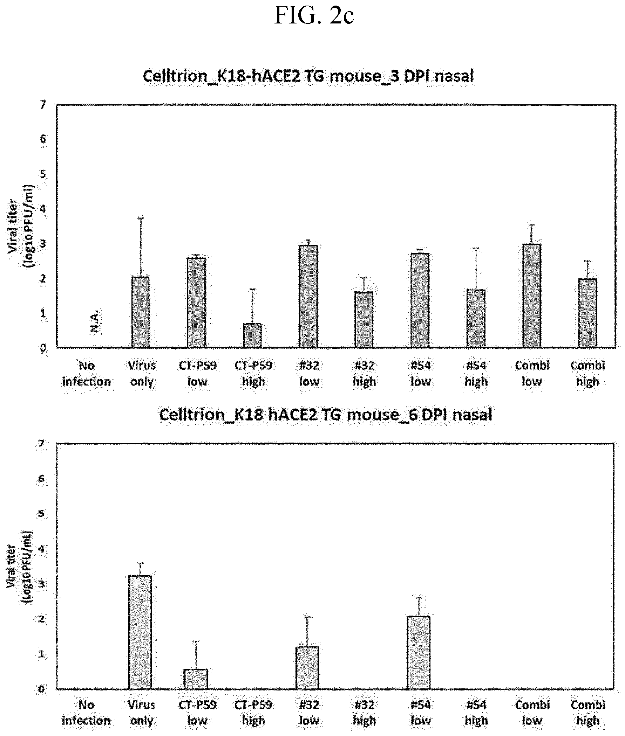

In addition, the results of measuring the viral titer in the nasal lavage fluid by plaque assay are as follows. On day 3, there was no difference in preventive efficacy between the low-dose (1 mg/kg) groups. Only the antibody No. 32 + antibody No. 54 cocktail group showed the best preventive efficacy in comparison between the low-dose (1 mg/kg) groups because the virus was not detected on day 6. The high dose (10 mg/kg) showed preventive efficacy on day 6 in all of the administered groups (FIG. 2c).

## Claims

1. A binding molecule that binds to a spike protein (S protein) on a surface of coronavirus.

2. The binding molecule according to claim 1, wherein the coronavirus is at least one selected from the group consisting of SARS-coronavirus-2 (SARS-CoV-2), SARS-coronavirus-1 (SARS-CoV-1), and Middle East respiratory syndrome coronavirus (MERS-CoV).

3. The binding molecule according to claim 1, wherein the coronavirus is:
i) SARS-coronavirus-2 (SARS-CoV-2) and SARS-coronavirus-1 (SARS-CoV-1);
ii) SARS-coronavirus-2 (SARS-CoV-2) and Middle East respiratory syndrome coronavirus (MERS-CoV); or
iii) SARS-coronavirus-2 (SARS-CoV-2), SARS-coronavirus-1 (SARS-CoV-1) and Middle East respiratory syndrome coronavirus (MERS-CoV).

4. The binding molecule according to claim 1, wherein the binding molecule comprises three light-chain CDR regions (LC CDR1, LC CDR2, and LC CDR3) contained in a light-chain (LC) variable region set forth in any one selected from the group consisting of SEQ ID NOS: 1141, 1143, 1145, 1147, 1149, 1151, 1153, 1155, 1157, 1159, 1161, 1163, 1165, 1167, 1169, 1171, 1173, 1175, 1177, 1179, 1181, 1183, 1185, 1187, 1189, 1191, 1193, 1195, 1197, 1199, 1201, 1203, 1205, 1207, 1209, 1211, 1213, 1215, 1217, 1219, 1221, 1223, 1225, 1227, 1229, 1231, 1233, 1235, 1237, 1239, 1241, 1243, 1245, 1247, 1249, 1251, 1253, 1255, 1257, 1259, 1261, 1263, 1265, 1267, 1269, 1271, 1273, 1275, 1277, 1279, 1281, 1283, 1285, 1287, 1289, 1291, 1293, 1295, 1297, 1299, 1301, 1303, 1305, 1307, 1309, 1311, 1313, 1315, 1317, 1319, 1321, 1323, 1325, 1327, 1329, 1331, 1333, 1335, 1337, 1339, 1341, 1343, 1345, 1347, 1349, 1351, 1353, 1355, 1357, 1359, 1361, 1363, 1365, 1367, 1369, 1371, 1373, 1375, 1377, 1379, 1381, 1383, 1385, 1387, 1389, 1391, 1393, 1395, 1397, 1399, 1401, 1403, 1405, 1407, 1409, 1411, 1413, 1415, 1417, 1419, 1421, 1423, 1425, 1427, 1429, 1431, 1433, 1435, 1437, 1439, 1441, 1443, 1445, 1447, 1449, 1451, 1453, 1455, 1457, 1459, 1461, 1463, 1465, 1467, 1469, 1471, 1473, 1475, 1477, 1479, 1481, 1483, 1485, 1487, 1489, 1491, 1493, 1495, 1497, 1499, 1501, 1503, 1505, 1507, 1509, 1511, 1513, 1515, 1517, and 1519, and three heavy-chain CDR regions (HC CDR1, HC CDR2, and HC CDR3) contained in a heavy-chain (HC) variable region set forth in any one selected from the group consisting of SEQ ID NOS: 1142, 1144, 1146, 1148, 1150, 1152, 1154, 1156, 1158, 1160, 1162, 1164, 1166, 1168, 1170, 1172, 1174, 1176, 1178, 1180, 1182, 1184, 1186, 1188, 1190, 1192, 1194, 1196, 1198, 1200, 1202, 1204, 1206, 1208, 1210, 1212, 1214, 1216, 1218, 1220, 1222, 1224, 1226, 1228, 1230, 1232, 1234, 1236, 1238, 1240, 1242, 1244, 1246, 1248, 1250, 1252, 1254, 1256, 1258, 1260, 1262, 1264, 1266, 1268, 1270, 1272, 1274, 1276, 1278, 1280, 1282, 1284, 1286, 1288, 1290, 1292, 1294, 1296, 1298, 1300, 1302, 1304, 1306, 1308, 1310, 1312, 1314, 1316, 1318, 1320, 1322, 1324, 1326, 1328, 1330, 1332, 1334, 1336, 1338, 1340, 1342, 1344, 1346, 1348, 1350, 1352, 1354, 1356, 1358, 1360, 1362, 1364, 1366, 1368, 1370, 1372, 1374, 1376, 1378, 1380, 1382, 1384, 1386, 1388, 1390, 1392, 1394, 1396, 1398, 1400, 1402, 1404, 1406, 1408, 1410, 1412, 1414, 1416, 1418, 1420, 1422, 1424, 1426, 1428, 1430, 1432, 1434, 1436, 1438, 1440, 1442, 1444, 1446, 1448, 1450, 1452, 1454, 1456, 1458, 1460, 1462, 1464, 1466, 1468, 1470, 1472, 1474, 1476, 1478, 1480, 1482, 1484, 1486, 1488, 1490, 1492, 1494, 1496, 1498, 1500, 1502, 1504, 1506, 1508, 1510, 1512, 1514, 1516, 1518 and 1520.

5. The binding molecule according to claim 4, wherein the LC CDR1 is any one selected from the group consisting of SEQ ID NOS: 1, 7, 13, 19, 25, 31, 37, 43, 49, 55, 61, 67, 73, 79, 85, 91, 97, 103, 109, 115, 121, 127, 133, 139, 145, 151, 157, 163, 169, 175, 181, 187, 193, 199, 205, 211, 217, 223, 229, 235, 241, 247, 253, 259, 265, 271, 277, 283, 289, 295, 301, 307, 313, 319, 325, 331, 337, 343, 349, 355, 361, 367, 373, 379, 385, 391, 397, 403, 409, 415, 421, 427, 433, 439, 445, 451, 457, 463, 469, 475, 481, 487, 493, 499, 505, 511, 517, 523, 529, 535, 541, 547, 553, 559, 565, 571, 577, 583, 589, 595, 601, 607, 613, 619, 625, 631, 637, 643, 649, 655, 661, 667, 673, 679, 685, 691, 697, 703, 709, 715, 721, 727, 733, 739, 745, 751, 757, 763, 769, 775, 781, 787, 793, 799, 805, 811, 817, 823, 829, 835, 841, 847, 853, 859, 865, 871, 877, 883, 889, 895, 901, 907, 913, 919, 925, 931, 937, 943, 949, 955, 961, 967, 973, 979, 985, 991, 997, 1003, 1009, 1015, 1021, 1027, 1033, 1039, 1045, 1051, 1057, 1063, 1069, 1075, 1081, 1087, 1093, 1099, 1105, 1111, 1117, 1123, 1129 and 1135,
the LC CDR2 is any one selected from the group consisting of SEQ ID NOS: 2, 8, 14, 20, 26, 32, 38, 44, 50, 56, 62, 68, 74, 80, 86, 92, 98, 104, 110, 116, 122, 128, 134, 140, 146, 152, 158, 164, 170, 176, 182, 188, 194, 200, 206, 212, 218, 224, 230, 236, 242, 248, 254, 260, 266, 272, 278, 284, 290, 296, 302, 308, 314, 320, 326, 332, 338, 344, 350, 356, 362, 368, 374, 380, 386, 392, 398, 404, 410, 416, 422, 428, 434, 440, 446, 452, 458, 464, 470, 476, 482, 488, 494, 500, 506, 512, 518, 524, 530, 536, 542, 548, 554, 560, 566, 572, 578, 584, 590, 596, 602, 608, 614, 620, 626, 632, 638, 644, 650, 656, 662, 668, 674, 680, 686, 692, 698, 704, 710, 716, 722, 728, 734, 740, 746, 752, 758, 764, 770, 776, 782, 788, 794, 800, 806, 812, 818, 824, 830, 836, 842, 848, 854, 860, 866, 872, 878, 884, 890, 896, 902, 908, 914, 920, 926, 932, 938, 944, 950, 956, 962, 968, 974, 980, 986, 992, 998, 1004, 1010, 1016, 1022, 1028, 1034, 1040, 1046, 1052, 1058, 1064, 1070, 1076, 1082, 1088, 1094, 1100, 1106, 1112, 1118, 1124, 1130 and 1136,
the LC CDR3 is any one selected from the group consisting of SEQ ID NOS: 3, 9, 15, 21, 27, 33, 39, 45, 51, 57, 63, 69, 75, 81, 87, 93, 99, 105, 111, 117, 123, 129, 135, 141, 147, 153, 159, 165, 171, 177, 183, 189, 195, 201, 207, 213, 219, 225, 231, 237, 243, 249, 255, 261, 267, 273, 279, 285, 291, 297, 303, 309, 315, 321, 327, 333, 339, 345, 351, 357, 363, 369, 375, 381, 387, 393, 399, 405, 411, 417, 423, 429, 435, 441, 447, 453, 459, 465, 471, 477, 483, 489, 495, 501, 507, 513, 519, 525, 531, 537, 543, 549, 555, 561, 567, 573, 579, 585, 591, 597, 603, 609, 615, 621, 627, 633, 639, 645, 651, 657, 663, 669, 675, 681, 687, 693, 699, 705, 711, 717, 723, 729, 735, 741, 747, 753, 759, 765, 771, 777, 783, 789, 795, 801, 807, 813, 819, 825, 831, 837, 843, 849, 855, 861, 867, 873, 879, 885, 891, 897, 903, 909, 915, 921, 927, 933, 939, 945, 951, 957, 963, 969, 975, 981, 987, 993, 999, 1005, 1011, 1017, 1023, 1029, 1035, 1041, 1047, 1053, 1059, 1065, 1071, 1077, 1083, 1089, 1095, 1101, 1107, 1113, 1119, 1125, 1131 and 1137,
the HC CDR1 is any one selected from the group consisting of SEQ ID NOS: 4, 10, 16, 22, 28, 34, 40, 46, 52, 58, 64, 70, 76, 82, 88, 94, 100, 106, 112, 118, 124, 130, 136, 142, 148, 154, 160, 166, 172, 178, 184, 190, 196, 202, 208, 214, 220, 226, 232, 238, 244, 250, 256, 262, 268, 274, 280, 286, 292, 298, 304, 310, 316, 322, 328, 334, 340, 346, 352, 358, 364, 370, 376, 382, 388, 394, 400, 406, 412, 418, 424, 430, 436, 442, 448, 454, 460, 466, 472, 478, 484, 490, 496, 502, 508, 514, 520, 526, 532, 538, 544, 550, 556, 562, 568, 574, 580, 586, 592, 598, 604, 610, 616, 622, 628, 634, 640, 646, 652, 658, 664, 670, 676, 682, 688, 694, 700, 706, 712, 718, 724, 730, 736, 742, 748, 754, 760, 766, 772, 778, 784, 790, 796, 802, 808, 814, 820, 826, 832, 838, 844, 850, 856, 862, 868, 874, 880, 886, 892, 898, 904, 910, 916, 922, 928, 934, 940, 946, 952, 958, 964, 970, 976, 982, 988, 994, 1000, 1006, 1012, 1018, 1024, 1030, 1036, 1042, 1048, 1054, 1060, 1066, 1072, 1078, 1084, 1090, 1096, 1102, 1108, 1114, 1120, 1126, 1132 and 1138,
the HC CDR2 is any one selected from the group consisting of SEQ ID NOS: 5, 11, 17, 23, 29, 35, 41, 47, 53, 59, 65, 71, 77, 83, 89, 95, 101, 107, 113, 119, 125, 131, 137, 143, 149, 155, 161, 167, 173, 179, 185, 191, 197, 203, 209, 215, 221, 227, 233, 239, 245, 251, 257, 263, 269, 275, 281, 287, 293, 299, 305, 311, 317, 323, 329, 335, 341, 347, 353, 359, 365, 371, 377, 383, 389, 395, 401, 407, 413, 419, 425, 431, 437, 443, 449, 455, 461, 467, 473, 479, 485, 491, 497, 503, 509, 515, 521, 527, 533, 539, 545, 551, 557, 563, 569, 575, 581, 587, 593, 599, 605, 611, 617, 623, 629, 635, 641, 647, 653, 659, 665, 671, 677, 683, 689, 695, 701, 707, 713, 719, 725, 731, 737, 743, 749, 755, 761, 767, 773, 779, 785, 791, 797, 803, 809, 815, 821, 827, 833, 839, 845, 851, 857, 863, 869, 875, 881, 887, 893, 899, 905, 911, 917, 923, 929, 935, 941, 947, 953, 959, 965, 971, 977, 983, 989, 995, 1001, 1007, 1013, 1019, 1025, 1031, 1037, 1043, 1049, 1055, 1061, 1067, 1073, 1079, 1085, 1091, 1097, 1103, 1109, 1115, 1121, 1127, 1133 and 1139, and
the HC CDR3 is any one selected from the group consisting of SEQ ID NOS: 6, 12, 18, 24, 30, 36, 42, 48, 54, 60, 66, 72, 78, 84, 90, 96, 102, 108, 114, 120, 126, 132, 138, 144, 150, 156, 162, 168, 174, 180, 186, 192, 198, 204, 210, 216, 222, 228, 234, 240, 246, 252, 258, 264, 270, 276, 282, 288, 294, 300, 306, 312, 318, 324, 330, 336, 342, 348, 354, 360, 366, 372, 378, 384, 390, 396, 402, 408, 414, 420, 426, 432, 438, 444, 450, 456, 462, 468, 474, 480, 486, 492, 498, 504, 510, 516, 522, 528, 534, 540, 546, 552, 558, 564, 570, 576, 582, 588, 594, 600, 606, 612, 618, 624, 630, 636, 642, 648, 654, 660, 666, 672, 678, 684, 690, 696, 702, 708, 714, 720, 726, 732, 738, 744, 750, 756, 762, 768, 774, 780, 786, 792, 798, 804, 810, 816, 822, 828, 834, 840, 846, 852, 858, 864, 870, 876, 882, 888, 894, 900, 906, 912, 918, 924, 930, 936, 942, 948, 954, 960, 966, 972, 978, 984, 990, 996, 1002, 1008, 1014, 1020, 1026, 1032, 1038, 1044, 1050, 1056, 1062, 1068, 1074, 1080, 1086, 1092, 1098, 1104, 1110, 1116, 1122, 1128, 1134 and 1140.

6. The binding molecule according to claim 4, wherein the binding molecule comprises three light-chain CDR regions (LC CDR1, LC CDR2, and LC CDR3) contained in a light-chain (LC) variable region set forth in any one selected from the group consisting of SEQ ID NOS: 1143, 1145, 1163, 1169, 1171, 1175, 1177, 1179, 1183, 1185, 1187, 1191, 1201, 1203, 1205, 1207, 1213, 1225, 1229, 1235, 1239, 1241, 1245, 1247, 1249, 1251, 1257, 1259, 1261, 1271, 1277, 1287, 1289, 1291, 1301, 1303, 1305, 1309, 1311, 1313, 1325, 1327, 1329, 1331, 1335, 1343, 1345, 1347 and 1355, and three heavy-chain CDR regions (HC CDR1, HC CDR2, and HC CDR3) contained in a heavy-chain (HC) variable region set forth in any one selected from the group consisting of SEQ ID NOS: 1144, 1146, 1164, 1170, 1172, 1176, 1178, 1180, 1184, 1186, 1188, 1192, 1202, 1204, 1206, 1208, 1214, 1226, 1230, 1236, 1240, 1242, 1246, 1248, 1250, 1252, 1258, 1260, 1262, 1272, 1278, 1288, 1290, 1292, 1302, 1304, 1306, 1310, 1312, 1314, 1326, 1328, 1330, 1332, 1336, 1344, 1346, 1348 and 1356.

7. The binding molecule according to claim 6, wherein the LC CDR1 is any one selected from the group consisting of SEQ ID NOS: 7, 13, 67, 85, 91, 103, 109, 115, 127, 133, 139, 151, 181, 187, 193, 199, 217, 253, 265, 283, 295, 301, 313, 319, 325, 331, 349, 355, 361, 391, 409, 439, 445, 451, 481, 487, 493, 505, 511, 517, 553, 559, 565, 571, 583, 607, 613, 619 and 643,
the LC CDR2 is any one selected from the group consisting of SEQ ID NOS: 8, 14, 68, 86, 92, 104, 110, 116, 128, 134, 140, 152, 182, 188, 194, 200, 218, 254, 266, 284, 296, 302, 314, 320, 326, 332, 350, 356, 362, 392, 410, 440, 446, 452, 482, 488, 494, 506, 512, 518, 554, 560, 566, 572, 584, 608, 614, 620 and 644,
the LC CDR3 is any one selected from the group consisting of SEQ ID NOS: 9, 15, 69, 87, 93, 105, 111, 117, 129, 135, 141, 153, 183, 189, 195, 201, 219, 255, 267, 285, 297, 303, 315, 321, 327, 333, 351, 357, 363, 393, 411, 441, 447, 453, 483, 489, 495, 507, 513, 519, 555, 561, 567, 573, 585, 609, 615, 621 and 645,
the HC CDR1 is any one selected from the group consisting of SEQ ID NOS: 10, 16, 70, 88, 94, 106, 112, 118, 130, 136, 142, 154, 184, 190, 196, 202, 220, 256, 268, 286, 298, 304, 316, 322, 328, 334, 352, 358, 364, 394, 412, 442, 448, 454, 484, 490, 496, 508, 514, 520, 556, 562, 568, 574, 586, 610, 616, 622 and 646,
the HC CDR2 is any one selected from the group consisting of SEQ ID NOS: 11, 17, 71, 89, 95, 107, 113, 119, 131, 137, 143, 155, 185, 191, 197, 203, 221, 257, 269, 287, 299, 305, 317, 323, 329, 335, 353, 359, 365, 395, 413, 443, 449, 455, 485, 491, 497, 509, 515, 521, 557, 563, 569, 575, 587, 611, 617, 623 and 647, and
the HC CDR3 is any one selected from the group consisting of SEQ ID NOS: 12, 18, 72, 90, 96, 108, 114, 120, 132, 138, 144, 156, 186, 192, 198, 204, 222, 258, 270, 288, 300, 306, 318, 324, 330, 336, 354, 360, 366, 396, 414, 444, 450, 456, 486, 492, 498, 510, 516, 522, 558, 564, 570, 576, 588, 612, 618, 624 and 648.

8. The binding molecule according to claim 1, wherein the binding molecule is any one selected from the group consisting of the following binding molecules 1) to 190):
1) a binding molecule comprising a) a light-chain variable region comprising a CDR1 region of SEQ ID NO: 1, a CDR2 region of SEQ ID NO: 2, and a CDR3 region of SEQ ID NO: 3, and b) a heavy-chain variable region comprising a CDR1 region of SEQ ID NO: 4, a CDR2 region of SEQ ID NO: 5, and a CDR3 region of SEQ ID NO: 6;
2) a binding molecule comprising a) a light-chain variable region comprising a CDR1 region of SEQ ID NO: 7, a CDR2 region of SEQ ID NO: 8, and a CDR3 region of SEQ ID NO: 9, and b) a heavy-chain variable region comprising a CDR1 region of SEQ ID NO: 10, a CDR2 region of SEQ ID NO: 11, and a CDR3 region of SEQ ID NO: 12;
3) a binding molecule comprising a) a light-chain variable region comprising a CDR1 region of SEQ ID NO: 13, a CDR2 region of SEQ ID NO: 14, and a CDR3 region of SEQ ID NO: 15, and b) a heavy-chain variable region comprising a CDR1 region of SEQ ID NO: 16, a CDR2 region of SEQ ID NO: 17, and a CDR3 region of SEQ ID NO: 18;
4) a binding molecule comprising a) a light-chain variable region comprising a CDR1 region of SEQ ID NO: 19, a CDR2 region of SEQ ID NO: 20, and a CDR3 region of SEQ ID NO: 21, and b) a heavy-chain variable region comprising a CDR1 region of SEQ ID NO: 22, a CDR2 region of SEQ ID NO: 23, and a CDR3 region of SEQ ID NO: 24;
5) a binding molecule comprising a) a light-chain variable region comprising a CDR1 region of SEQ ID NO: 25, a CDR2 region of SEQ ID NO: 26, and a CDR3 region of SEQ ID NO: 27, and b) a heavy-chain variable region comprising a CDR1 region of SEQ ID NO: 28, a CDR2 region of SEQ ID NO: 29, and a CDR3 region of SEQ ID NO: 30;
6) a binding molecule comprising a) a light-chain variable region comprising a CDR1 region of SEQ ID NO: 31, a CDR2 region of SEQ ID NO: 32, and a CDR3 region of SEQ ID NO: 33, and b) a heavy-chain variable region comprising a CDR1 region of SEQ ID NO: 34, a CDR2 region of SEQ ID NO: 35, and a CDR3 region of SEQ ID NO: 36;
7) a binding molecule comprising a) a light-chain variable region comprising a CDR1 region of SEQ ID NO: 37, a CDR2 region of SEQ ID NO: 38, and a CDR3 region of SEQ ID NO: 39, and b) a heavy-chain variable region comprising a CDR1 region of SEQ ID NO: 40, a CDR2 region of SEQ ID NO: 41, and a CDR3 region of SEQ ID NO: 42;
8) a binding molecule comprising a) a light-chain variable region comprising a CDR1 region of SEQ ID NO: 43, a CDR2 region of SEQ ID NO: 44, and a CDR3 region of SEQ ID NO: 45, and b) a heavy-chain variable region comprising a CDR1 region of SEQ ID NO: 46, a CDR2 region of SEQ ID NO: 47, and a CDR3 region of SEQ ID NO: 48;
9) a binding molecule comprising a) a light-chain variable region comprising a CDR1 region of SEQ ID NO: 49, a CDR2 region of SEQ ID NO: 50, and a CDR3 region of SEQ ID NO: 51, and b) a heavy-chain variable region comprising a CDR1 region of SEQ ID NO: 52, a CDR2 region of SEQ ID NO: 53, and a CDR3 region of SEQ ID NO: 54;
10) a binding molecule comprising a) a light-chain variable region comprising a CDR1 region of SEQ ID NO: 55, a CDR2 region of SEQ ID NO: 56, and a CDR3 region of SEQ ID NO: 57, and b) a heavy-chain variable region comprising a CDR1 region of SEQ ID NO: 58, a CDR2 region of SEQ ID NO: 59, and a CDR3 region of SEQ ID NO: 60;
11) a binding molecule comprising a) a light-chain variable region comprising a CDR1 region of SEQ ID NO: 61, a CDR2 region of SEQ ID NO: 62, and a CDR3 region of SEQ ID NO: 63, and b) a heavy-chain variable region comprising a CDR1 region of SEQ ID NO: 64, a CDR2 region of SEQ ID NO: 65, and a CDR3 region of SEQ ID NO: 66;
12) a binding molecule comprising a) a light-chain variable region comprising a CDR1 region of SEQ ID NO: 67, a CDR2 region of SEQ ID NO: 68, and a CDR3 region of SEQ ID NO: 69, and b) a heavy-chain variable region comprising a CDR1 region of SEQ ID NO: 70, a CDR2 region of SEQ ID NO: 71, and a CDR3 region of SEQ ID NO: 72;
13) a binding molecule comprising a) a light-chain variable region comprising a CDR1 region of SEQ ID NO: 73, a CDR2 region of SEQ ID NO: 74, and a CDR3 region of SEQ ID NO: 75, and b) a heavy-chain variable region comprising a CDR1 region of SEQ ID NO: 76, a CDR2 region of SEQ ID NO: 77, and a CDR3 region of SEQ ID NO: 78;
14) a binding molecule comprising a) a light-chain variable region comprising a CDR1 region of SEQ ID NO: 79, a CDR2 region of SEQ ID NO: 80, and a CDR3 region of SEQ ID NO: 81, and b) a heavy-chain variable region comprising a CDR1 region of SEQ ID NO: 82, a CDR2 region of SEQ ID NO: 83, and a CDR3 region of SEQ ID NO: 84;
15) a binding molecule comprising a) a light-chain variable region comprising a CDR1 region of SEQ ID NO: 85, a CDR2 region of SEQ ID NO: 86, and a CDR3 region of SEQ ID NO: 87, and b) a heavy-chain variable region comprising a CDR1 region of SEQ ID NO: 88, a CDR2 region of SEQ ID NO: 89, and a CDR3 region of SEQ ID NO: 90;
16) a binding molecule comprising a) a light-chain variable region comprising a CDR1 region of SEQ ID NO: 91, a CDR2 region of SEQ ID NO: 92, and a CDR3 region of SEQ ID NO: 93, and b) a heavy-chain variable region comprising a CDR1 region of SEQ ID NO: 94, a CDR2 region of SEQ ID NO: 95, and a CDR3 region of SEQ ID NO: 96;
17) a binding molecule comprising a) a light-chain variable region comprising a CDR1 region of SEQ ID NO: 97, a CDR2 region of SEQ ID NO: 98, and a CDR3 region of SEQ ID NO: 99, and b) a heavy-chain variable region comprising a CDR1 region of SEQ ID NO: 100, a CDR2 region of SEQ ID NO: 101, and a CDR3 region of SEQ ID NO: 102;
18) a binding molecule comprising a) a light-chain variable region comprising a CDR1 region of SEQ ID NO: 103, a CDR2 region of SEQ ID NO: 104, and a CDR3 region of SEQ ID NO: 105, and b) a heavy-chain variable region comprising a CDR1 region of SEQ ID NO: 106, a CDR2 region of SEQ ID NO: 107, and a CDR3 region of SEQ ID NO: 108;
19) a binding molecule comprising a) a light-chain variable region comprising a CDR1 region of SEQ ID NO: 109, CDR2 region of SEQ ID NO: 110, and CDR3 region of SEQ ID NO: 111, and b) a heavy-chain variable region comprising a CDR1 region of SEQ ID NO: 112, a CDR2 region of SEQ ID NO: 113, and a CDR3 region of SEQ ID NO: 114;
20) a binding molecule comprising a) a light-chain variable region comprising a CDR1 region of SEQ ID NO: 115, a CDR2 region of SEQ ID NO: 116, and a CDR3 region of SEQ ID NO: 117, and b) a heavy-chain variable region comprising a CDR1 region of SEQ ID NO: 118, a CDR2 region of SEQ ID NO: 119, and a CDR3 region of SEQ ID NO: 120;
21) a binding molecule comprising a) a light-chain variable region comprising a CDR1 region of SEQ ID NO: 121, a CDR2 region of SEQ ID NO: 122, and a CDR3 region of SEQ ID NO: 123, and b) a heavy-chain variable region comprising a CDR1 region of SEQ ID NO: 124, a CDR2 region of SEQ ID NO: 125, and a CDR3 region of SEQ ID NO: 126;
22) a binding molecule comprising a) a light-chain variable region comprising a CDR1 region of SEQ ID NO: 127, a CDR2 region of SEQ ID NO: 128, and a CDR3 region of SEQ ID NO: 129, and b) a heavy-chain variable region comprising a CDR1 region of SEQ ID NO: 130, a CDR2 region of SEQ ID NO: 131, and a CDR3 region of SEQ ID NO: 132;
23) a binding molecule comprising a) a light-chain variable region comprising a CDR1 region of SEQ ID NO: 133, a CDR2 region of SEQ ID NO: 134, and a CDR3 region of SEQ ID NO: 135, and b) a heavy-chain variable region comprising a CDR1 region of SEQ ID NO: 136, a CDR2 region of SEQ ID NO: 137, and a CDR3 region of SEQ ID NO: 138;
24) a binding molecule comprising a) a light-chain variable region comprising a CDR1 region of SEQ ID NO: 139, a CDR2 region of SEQ ID NO: 140, and a CDR3 region of SEQ ID NO: 141, and b) a heavy-chain variable region comprising a CDR1 region of SEQ ID NO: 142, a CDR2 region of SEQ ID NO: 143, and a CDR3 region of SEQ ID NO: 144;
25) a binding molecule comprising a) a light-chain variable region comprising a CDR1 region of SEQ ID NO: 145, a CDR2 region of SEQ ID NO: 146, and a CDR3 region of SEQ ID NO: 147, and b) a heavy-chain variable region comprising a CDR1 region of SEQ ID NO: 148, a CDR2 region of SEQ ID NO: 149, and a CDR3 region of SEQ ID NO: 150;
26) a binding molecule comprising a) a light-chain variable region comprising a CDR1 region of SEQ ID NO: 151, a CDR2 region of SEQ ID NO: 152, and a CDR3 region of SEQ ID NO: 153, and b) a heavy-chain variable region comprising a CDR1 region of SEQ ID NO: 154, a CDR2 region of SEQ ID NO: 155, and a CDR3 region of SEQ ID NO: 156;
27) a binding molecule comprising a) a light-chain variable region comprising a CDR1 region of SEQ ID NO: 157, a CDR2 region of SEQ ID NO: 158, and a CDR3 region of SEQ ID NO: 159, and b) a heavy-chain variable region comprising a CDR1 region of SEQ ID NO: 160, a CDR2 region of SEQ ID NO: 161, and a CDR3 region of SEQ ID NO: 162;
28) a binding molecule comprising a) a light-chain variable region comprising a CDR1 region of SEQ ID NO: 163, a CDR2 region of SEQ ID NO: 164, and a CDR3 region of SEQ ID NO: 165, and b) a heavy-chain variable region comprising a CDR1 region of SEQ ID NO: 166, a CDR2 region of SEQ ID NO: 167, and a CDR3 region of SEQ ID NO: 168;
29) a binding molecule comprising a) a light-chain variable region comprising a CDR1 region of SEQ ID NO: 169, a CDR2 region of SEQ ID NO: 170, and a CDR3 region of SEQ ID NO: 171, and b) a heavy-chain variable region comprising a CDR1 region of SEQ ID NO: 172, a CDR2 region of SEQ ID NO: 173, and a CDR3 region of SEQ ID NO: 174;
30) a binding molecule comprising a) a light-chain variable region comprising a CDR1 region of SEQ ID NO: 175, a CDR2 region of SEQ ID NO: 176, and a CDR3 region of SEQ ID NO: 177, and b) a heavy-chain variable region comprising a CDR1 region of SEQ ID NO: 178, a CDR2 region of SEQ ID NO: 179, and a CDR3 region of SEQ ID NO: 180;
31) a binding molecule comprising a) a light-chain variable region comprising a CDR1 region of SEQ ID NO: 181, a CDR2 region of SEQ ID NO: 182, and a CDR3 region of SEQ ID NO: 183, and b) a heavy-chain variable region comprising a CDR1 region of SEQ ID NO: 184, a CDR2 region of SEQ ID NO: 185, and a CDR3 region of SEQ ID NO: 186;
32) a binding molecule comprising a) a light-chain variable region comprising a CDR1 region of SEQ ID NO: 187, a CDR2 region of SEQ ID NO: 188, and a CDR3 region of SEQ ID NO: 189, and b) a heavy-chain variable region comprising a CDR1 region of SEQ ID NO: 190, a CDR2 region of SEQ ID NO: 191, and a CDR3 region of SEQ ID NO: 192;
33) a binding molecule comprising a) a light-chain variable region comprising a CDR1 region of SEQ ID NO: 193, a CDR2 region of SEQ ID NO: 194, and a CDR3 region of SEQ ID NO: 195, and b) a heavy-chain variable region comprising a CDR1 region of SEQ ID NO: 196, a CDR2 region of SEQ ID NO: 197, and a CDR3 region of SEQ ID NO: 198;
34) a binding molecule comprising a) a light-chain variable region comprising a CDR1 region of SEQ ID NO: 199, a CDR2 region of SEQ ID NO: 200, and a CDR3 region of SEQ ID NO: 201, and b) a heavy-chain variable region comprising a CDR1 region of SEQ ID NO: 202, a CDR2 region of SEQ ID NO: 203, and a CDR3 region of SEQ ID NO: 204;
35) a binding molecule comprising a) a light-chain variable region comprising a CDR1 region of SEQ ID NO: 205, a CDR2 region of SEQ ID NO: 206, and a CDR3 region of SEQ ID NO: 207, and b) a heavy-chain variable region comprising a CDR1 region of SEQ ID NO: 208, a CDR2 region of SEQ ID NO: 209, and a CDR3 region of SEQ ID NO: 210;
36) a binding molecule comprising a) a light-chain variable region comprising a CDR1 region of SEQ ID NO: 211, a CDR2 region of SEQ ID NO: 212, and a CDR3 region of SEQ ID NO: 213, and b) a heavy-chain variable region comprising a CDR1 region of SEQ ID NO: 214, a CDR2 region of SEQ ID NO: 215, and a CDR3 region of SEQ ID NO: 216;
37) a binding molecule comprising a) a light-chain variable region comprising a CDR1 region of SEQ ID NO: 217, a CDR2 region of SEQ ID NO: 218, and a CDR3 region of SEQ ID NO: 219, and b) a heavy-chain variable region comprising a CDR1 region of SEQ ID NO: 220, a CDR2 region of SEQ ID NO: 221, and a CDR3 region of SEQ ID NO: 222;
38) a binding molecule comprising a) a light-chain variable region comprising a CDR1 region of SEQ ID NO: 223, a CDR2 region of SEQ ID NO: 224, and a CDR3 region of SEQ ID NO: 225, and b) a heavy-chain variable region comprising a CDR1 region of SEQ ID NO: 226, a CDR2 region of SEQ ID NO: 227, and a CDR3 region of SEQ ID NO: 228;
39) a binding molecule comprising a) a light-chain variable region comprising a CDR1 region of SEQ ID NO: 229, a CDR2 region of SEQ ID NO: 230, and a CDR3 region of SEQ ID NO: 231, and b) a heavy-chain variable region comprising a CDR1 region of SEQ ID NO: 232, a CDR2 region of SEQ ID NO: 233, and a CDR3 region of SEQ ID NO: 234;
40) a binding molecule comprising a) a light-chain variable region comprising a CDR1 region of SEQ ID NO: 235, a CDR2 region of SEQ ID NO: 236, and a CDR3 region of SEQ ID NO: 237, and b) a heavy-chain variable region comprising a CDR1 region of SEQ ID NO: 238, a CDR2 region of SEQ ID NO: 239, and a CDR3 region of SEQ ID NO: 240;
41) a binding molecule comprising a) a light-chain variable region comprising a CDR1 region of SEQ ID NO: 241, a CDR2 region of SEQ ID NO: 242, and a CDR3 region of SEQ ID NO: 243, and b) a heavy-chain variable region comprising a CDR1 region of SEQ ID NO: 244, a CDR2 region of SEQ ID NO: 245, and a CDR3 region of SEQ ID NO: 246;
42) a binding molecule comprising a) a light-chain variable region comprising a CDR1 region of SEQ ID NO: 247, a CDR2 region of SEQ ID NO: 248, and a CDR3 region of SEQ ID NO: 249, and b) a heavy-chain variable region comprising a CDR1 region of SEQ ID NO: 250, a CDR2 region of SEQ ID NO: 251, and a CDR3 region of SEQ ID NO: 252;
43) a binding molecule comprising a) a light-chain variable region comprising a CDR1 region of SEQ ID NO: 253, a CDR2 region of SEQ ID NO: 254, and a CDR3 region of SEQ ID NO: 255, and b) a heavy-chain variable region comprising a CDR1 region of SEQ ID NO: 256, a CDR2 region of SEQ ID NO: 257, and a CDR3 region of SEQ ID NO: 258;
44) a binding molecule comprising a) a light-chain variable region comprising a CDR1 region of SEQ ID NO: 259, a CDR2 region of SEQ ID NO: 260, and a CDR3 region of SEQ ID NO: 261, and b) a heavy-chain variable region comprising a CDR1 region of SEQ ID NO: 262, a CDR2 region of SEQ ID NO: 263, and a CDR3 region of SEQ ID NO: 264;
45) a binding molecule comprising a) a light-chain variable region comprising a CDR1 region of SEQ ID NO: 265, a CDR2 region of SEQ ID NO: 266, and a CDR3 region of SEQ ID NO: 267, and b) a heavy-chain variable region comprising a CDR1 region of SEQ ID NO: 268, a CDR2 region of SEQ ID NO: 269, and a CDR3 region of SEQ ID NO: 270;
46) a binding molecule comprising a) a light-chain variable region comprising a CDR1 region of SEQ ID NO: 271, a CDR2 region of SEQ ID NO: 272, and a CDR3 region of SEQ ID NO: 273, and b) a heavy-chain variable region comprising a CDR1 region of SEQ ID NO: 274, a CDR2 region of SEQ ID NO: 275, and a CDR3 region of SEQ ID NO: 276;
47) a binding molecule comprising a) a light-chain variable region comprising a CDR1 region of SEQ ID NO: 277, a CDR2 region of SEQ ID NO: 278, and a CDR3 region of SEQ ID NO: 279, and b) a heavy-chain variable region comprising a CDR1 region of SEQ ID NO: 280, a CDR2 region of SEQ ID NO: 281, and a CDR3 region of SEQ ID NO: 282;
48) a binding molecule comprising a) a light-chain variable region comprising a CDR1 region of SEQ ID NO: 283, a CDR2 region of SEQ ID NO: 284, and a CDR3 region of SEQ ID NO: 285, and b) a heavy-chain variable region comprising a CDR1 region of SEQ ID NO: 286, a CDR2 region of SEQ ID NO: 287, and a CDR3 region of SEQ ID NO: 288;
49) a binding molecule comprising a) a light-chain variable region comprising a CDR1 region of SEQ ID NO: 289, a CDR2 region of SEQ ID NO: 290, and a CDR3 region of SEQ ID NO: 291, and b) a heavy-chain variable region comprising a CDR1 region of SEQ ID NO: 292, a CDR2 region of SEQ ID NO: 293, and a CDR3 region of SEQ ID NO: 294;
50) a binding molecule comprising a) a light-chain variable region comprising a CDR1 region of SEQ ID NO: 295, a CDR2 region of SEQ ID NO: 296, and a CDR3 region of SEQ ID NO: 297, and b) a heavy-chain variable region comprising a CDR1 region of SEQ ID NO: 298, a CDR2 region of SEQ ID NO: 299, and a CDR3 region of SEQ ID NO: 300;
51) a binding molecule comprising a) a light-chain variable region comprising a CDR1 region of SEQ ID NO: 301, a CDR2 region of SEQ ID NO: 302, and a CDR3 region of SEQ ID NO: 303, and b) a heavy-chain variable region comprising a CDR1 region of SEQ ID NO: 304, a CDR2 region of SEQ ID NO: 305, and a CDR3 region of SEQ ID NO: 306;
52) a binding molecule comprising a) a light-chain variable region comprising a CDR1 region of SEQ ID NO: 307, a CDR2 region of SEQ ID NO: 308, and a CDR3 region of SEQ ID NO: 309, and b) a heavy-chain variable region comprising a CDR1 region of SEQ ID NO: 310, a CDR2 region of SEQ ID NO: 311, and a CDR3 region of SEQ ID NO: 312;
53) a binding molecule comprising a) a light-chain variable region comprising a CDR1 region of SEQ ID NO: 313, a CDR2 region of SEQ ID NO: 314, and a CDR3 region of SEQ ID NO: 315, and b) a heavy-chain variable region comprising a CDR1 region of SEQ ID NO: 316, a CDR2 region of SEQ ID NO: 317, and a CDR3 region of SEQ ID NO: 318;
54) a binding molecule comprising a) a light-chain variable region comprising a CDR1 region of SEQ ID NO: 319, a CDR2 region of SEQ ID NO: 320, and a CDR3 region of SEQ ID NO: 321, and b) a heavy-chain variable region comprising a CDR1 region of SEQ ID NO: 322, a CDR2 region of SEQ ID NO: 323, and a CDR3 region of SEQ ID NO: 324;
55) a binding molecule comprising a) a light-chain variable region comprising a CDR1 region of SEQ ID NO: 325, a CDR2 region of SEQ ID NO: 326, and a CDR3 region of SEQ ID NO: 327, and b) a heavy-chain variable region comprising a CDR1 region of SEQ ID NO: 328, a CDR2 region of SEQ ID NO: 329, and a CDR3 region of SEQ ID NO: 330;
56) a binding molecule comprising a) a light-chain variable region comprising a CDR1 region of SEQ ID NO: 331, a CDR2 region of SEQ ID NO: 332, and a CDR3 region of SEQ ID NO: 333, and b) a heavy-chain variable region comprising a CDR1 region of SEQ ID NO: 334, a CDR2 region of SEQ ID NO: 335, and a CDR3 region of SEQ ID NO: 336;
57) a binding molecule comprising a) a light-chain variable region comprising a CDR1 region of SEQ ID NO: 337, a CDR2 region of SEQ ID NO: 338, and a CDR3 region of SEQ ID NO: 339, and b) a heavy-chain variable region comprising a CDR1 region of SEQ ID NO: 340, a CDR2 region of SEQ ID NO: 341, and a CDR3 region of SEQ ID NO: 342;
58) a binding molecule comprising a) a light-chain variable region comprising a CDR1 region of SEQ ID NO: 343, a CDR2 region of SEQ ID NO: 344, and a CDR3 region of SEQ ID NO: 345, and b) a heavy-chain variable region comprising a CDR1 region of SEQ ID NO: 346, a CDR2 region of SEQ ID NO: 347, and a CDR3 region of SEQ ID NO: 348;
59) a binding molecule comprising a) a light-chain variable region comprising a CDR1 region of SEQ ID NO: 349, a CDR2 region of SEQ ID NO: 350, and a CDR3 region of SEQ ID NO: 351, and b) a heavy-chain variable region comprising a CDR1 region of SEQ ID NO: 352, a CDR2 region of SEQ ID NO: 353, and a CDR3 region of SEQ ID NO: 354;
60) a binding molecule comprising a) a light-chain variable region comprising a CDR1 region of SEQ ID NO: 355, a CDR2 region of SEQ ID NO: 356, and a CDR3 region of SEQ ID NO: 357, and b) a heavy-chain variable region comprising a CDR1 region of SEQ ID NO: 358, a CDR2 region of SEQ ID NO: 359, and a CDR3 region of SEQ ID NO: 360;
61) a binding molecule comprising a) a light-chain variable region comprising a CDR1 region of SEQ ID NO: 361, a CDR2 region of SEQ ID NO: 362, and a CDR3 region of SEQ ID NO: 363, and b) a heavy-chain variable region comprising a CDR1 region of SEQ ID NO: 364, a CDR2 region of SEQ ID NO: 365, and a CDR3 region of SEQ ID NO: 366;
62) a binding molecule comprising a) a light-chain variable region comprising a CDR1 region of SEQ ID NO: 367, a CDR2 region of SEQ ID NO: 368, and a CDR3 region of SEQ ID NO: 369, and b) a heavy-chain variable region comprising a CDR1 region of SEQ ID NO: 370, a CDR2 region of SEQ ID NO: 371, and a CDR3 region of SEQ ID NO: 372;
63) a binding molecule comprising a) a light-chain variable region comprising a CDR1 region of SEQ ID NO: 373, a CDR2 region of SEQ ID NO: 374, and a CDR3 region of SEQ ID NO: 375, and b) a heavy-chain variable region comprising a CDR1 region of SEQ ID NO: 376, a CDR2 region of SEQ ID NO: 377, and a CDR3 region of SEQ ID NO: 378;
64) a binding molecule comprising a) a light-chain variable region comprising a CDR1 region of SEQ ID NO: 379, a CDR2 region of SEQ ID NO: 380, and a CDR3 region of SEQ ID NO: 381, and b) a heavy-chain variable region comprising a CDR1 region of SEQ ID NO: 382, a CDR2 region of SEQ ID NO: 383, and a CDR3 region of SEQ ID NO: 384;
65) a binding molecule comprising a) a light-chain variable region comprising a CDR1 region of SEQ ID NO: 385, a CDR2 region of SEQ ID NO: 386, and a CDR3 region of SEQ ID NO: 387, and b) a heavy-chain variable region comprising a CDR1 region of SEQ ID NO: 388, a CDR2 region of SEQ ID NO: 389, and a CDR3 region of SEQ ID NO: 390;
66) a binding molecule comprising a) a light-chain variable region comprising a CDR1 region of SEQ ID NO: 391, a CDR2 region of SEQ ID NO: 392, and a CDR3 region of SEQ ID NO: 393, and b) a heavy-chain variable region comprising a CDR1 region of SEQ ID NO: 394, a CDR2 region of SEQ ID NO: 395, and a CDR3 region of SEQ ID NO: 396;
67) a binding molecule comprising a) a light-chain variable region comprising a CDR1 region of SEQ ID NO: 397, a CDR2 region of SEQ ID NO: 398, and a CDR3 region of SEQ ID NO: 399, and b) a heavy-chain variable region comprising a CDR1 region of SEQ ID NO: 400, a CDR2 region of SEQ ID NO: 401, and a CDR3 region of SEQ ID NO: 402;
68) a binding molecule comprising a) a light-chain variable region comprising a CDR1 region of SEQ ID NO: 403, a CDR2 region of SEQ ID NO: 404, and a CDR3 region of SEQ ID NO: 405, and b) a heavy-chain variable region comprising a CDR1 region of SEQ ID NO: 406, a CDR2 region of SEQ ID NO: 407, and a CDR3 region of SEQ ID NO: 408;
69) a binding molecule comprising a) a light-chain variable region comprising a CDR1 region of SEQ ID NO: 409, a CDR2 region of SEQ ID NO: 410, and a CDR3 region of SEQ ID NO: 411, and b) a heavy-chain variable region comprising a CDR1 region of SEQ ID NO: 412, a CDR2 region of SEQ ID NO: 413, and a CDR3 region of SEQ ID NO: 414;
70) a binding molecule comprising a) a light-chain variable region comprising a CDR1 region of SEQ ID NO: 415, a CDR2 region of SEQ ID NO: 416, and a CDR3 region of SEQ ID NO: 417, and b) a heavy-chain variable region comprising a CDR1 region of SEQ ID NO: 418, a CDR2 region of SEQ ID NO: 419, and a CDR3 region of SEQ ID NO: 420;
71) a binding molecule comprising a) a light-chain variable region comprising a CDR1 region of SEQ ID NO: 421, a CDR2 region of SEQ ID NO: 422, and a CDR3 region of SEQ ID NO: 423, and b) a heavy-chain variable region comprising a CDR1 region of SEQ ID NO: 424, a CDR2 region of SEQ ID NO: 425, and a CDR3 region of SEQ ID NO: 426;
72) a binding molecule comprising a) a light-chain variable region comprising a CDR1 region of SEQ ID NO: 427, a CDR2 region of SEQ ID NO: 428, and a CDR3 region of SEQ ID NO: 429, and b) a heavy-chain variable region comprising a CDR1 region of SEQ ID NO: 430, a CDR2 region of SEQ ID NO: 431, and a CDR3 region of SEQ ID NO: 432;
73) a binding molecule comprising a) a light-chain variable region comprising a CDR1 region of SEQ ID NO: 433, a CDR2 region of SEQ ID NO: 434, and a CDR3 region of SEQ ID NO: 435, and b) a heavy-chain variable region comprising a CDR1 region of SEQ ID NO: 436, a CDR2 region of SEQ ID NO: 437, and a CDR3 region of SEQ ID NO: 438;
74) a binding molecule comprising a) a light-chain variable region comprising a CDR1 region of SEQ ID NO: 439, a CDR2 region of SEQ ID NO: 440, and a CDR3 region of SEQ ID NO: 441, and b) a heavy-chain variable region comprising a CDR1 region of SEQ ID NO: 442, a CDR2 region of SEQ ID NO: 443, and a CDR3 region of SEQ ID NO: 444;
75) a binding molecule comprising a) a light-chain variable region comprising a CDR1 region of SEQ ID NO: 445, a CDR2 region of SEQ ID NO: 446, and a CDR3 region of SEQ ID NO: 447, and b) a heavy-chain variable region comprising a CDR1 region of SEQ ID NO: 448, a CDR2 region of SEQ ID NO: 449, and a CDR3 region of SEQ ID NO: 450;
76) a binding molecule comprising a) a light-chain variable region comprising a CDR1 region of SEQ ID NO: 451, a CDR2 region of SEQ ID NO: 452, and a CDR3 region of SEQ ID NO: 453, and b) a heavy-chain variable region comprising a CDR1 region of SEQ ID NO: 454, a CDR2 region of SEQ ID NO: 455, and a CDR3 region of SEQ ID NO: 456;
77) a binding molecule comprising a) a light-chain variable region comprising a CDR1 region of SEQ ID NO: 457, a CDR2 region of SEQ ID NO: 458, and a CDR3 region of SEQ ID NO: 459, and b) a heavy-chain variable region comprising a CDR1 region of SEQ ID NO: 460, a CDR2 region of SEQ ID NO: 461, and a CDR3 region of SEQ ID NO: 462;
78) a binding molecule comprising a) a light-chain variable region comprising a CDR1 region of SEQ ID NO: 463, a CDR2 region of SEQ ID NO: 464, and a CDR3 region of SEQ ID NO: 465, and b) a heavy-chain variable region comprising a CDR1 region of SEQ ID NO: 466, a CDR2 region of SEQ ID NO: 467, and a CDR3 region of SEQ ID NO: 468;
79) a binding molecule comprising a) a light-chain variable region comprising a CDR1 region of SEQ ID NO: 469, a CDR2 region of SEQ ID NO: 470, and a CDR3 region of SEQ ID NO: 471, and b) a heavy-chain variable region comprising a CDR1 region of SEQ ID NO: 472, a CDR2 region of SEQ ID NO: 473, and a CDR3 region of SEQ ID NO: 474;
80) a binding molecule comprising a) a light-chain variable region comprising a CDR1 region of SEQ ID NO: 475, a CDR2 region of SEQ ID NO: 476, and a CDR3 region of SEQ ID NO: 477, and b) a heavy-chain variable region comprising a CDR1 region of SEQ ID NO: 478, a CDR2 region of SEQ ID NO: 479, and a CDR3 region of SEQ ID NO: 480;
81) a binding molecule comprising a) a light-chain variable region comprising a CDR1 region of SEQ ID NO: 481, a CDR2 region of SEQ ID NO: 482, and a CDR3 region of SEQ ID NO: 483, and b) a heavy-chain variable region comprising a CDR1 region of SEQ ID NO: 484, a CDR2 region of SEQ ID NO: 485, and a CDR3 region of SEQ ID NO: 486;
82) a binding molecule comprising a) a light-chain variable region comprising a CDR1 region of SEQ ID NO: 487, a CDR2 region of SEQ ID NO: 488, and a CDR3 region of SEQ ID NO: 489, and b) a heavy-chain variable region comprising a CDR1 region of SEQ ID NO: 490, a CDR2 region of SEQ ID NO: 491, and a CDR3 region of SEQ ID NO: 492;
83) a binding molecule comprising a) a light-chain variable region comprising a CDR1 region of SEQ ID NO: 493, a CDR2 region of SEQ ID NO: 494, and a CDR3 region of SEQ ID NO: 495, and b) a heavy-chain variable region comprising a CDR1 region of SEQ ID NO: 496, a CDR2 region of SEQ ID NO: 497, and a CDR3 region of SEQ ID NO: 498;
84) a binding molecule comprising a) a light-chain variable region comprising a CDR1 region of SEQ ID NO: 499, a CDR2 region of SEQ ID NO: 500, and a CDR3 region of SEQ ID NO: 501, and b) a heavy-chain variable region comprising a CDR1 region of SEQ ID NO: 502, a CDR2 region of SEQ ID NO: 503, and a CDR3 region of SEQ ID NO: 504;
85) a binding molecule comprising a) a light-chain variable region comprising a CDR1 region of SEQ ID NO: 505, a CDR2 region of SEQ ID NO: 506, and a CDR3 region of SEQ ID NO: 507, and b) a heavy-chain variable region comprising a CDR1 region of SEQ ID NO: 508, a CDR2 region of SEQ ID NO: 509, and a CDR3 region of SEQ ID NO: 510;
86) a binding molecule comprising a) a light-chain variable region comprising a CDR1 region of SEQ ID NO: 511, a CDR2 region of SEQ ID NO: 512, and a CDR3 region of SEQ ID NO: 513, and b) a heavy-chain variable region comprising a CDR1 region of SEQ ID NO: 514, a CDR2 region of SEQ ID NO: 515, and a CDR3 region of SEQ ID NO: 516;
87) a binding molecule comprising a) a light-chain variable region comprising a CDR1 region of SEQ ID NO: 517, a CDR2 region of SEQ ID NO: 518, and a CDR3 region of SEQ ID NO: 519, and b) a heavy-chain variable region comprising a CDR1 region of SEQ ID NO: 520, a CDR2 region of SEQ ID NO: 521, and a CDR3 region of SEQ ID NO: 522;
88) a binding molecule comprising a) a light-chain variable region comprising a CDR1 region of SEQ ID NO: 523, a CDR2 region of SEQ ID NO: 524, and a CDR3 region of SEQ ID NO: 525, and b) a heavy-chain variable region comprising a CDR1 region of SEQ ID NO: 526, a CDR2 region of SEQ ID NO: 527, and a CDR3 region of SEQ ID NO: 528;
89) a binding molecule comprising a) a light-chain variable region comprising a CDR1 region of SEQ ID NO: 529, a CDR2 region of SEQ ID NO: 530, and a CDR3 region of SEQ ID NO: 531, and b) a heavy-chain variable region comprising a CDR1 region of SEQ ID NO: 532, a CDR2 region of SEQ ID NO: 533, and a CDR3 region of SEQ ID NO: 534;
90) a binding molecule comprising a) a light-chain variable region comprising a CDR1 region of SEQ ID NO: 535, a CDR2 region of SEQ ID NO: 536, and a CDR3 region of SEQ ID NO: 537, and b) a heavy-chain variable region comprising a CDR1 region of SEQ ID NO: 538, a CDR2 region of SEQ ID NO: 539, and a CDR3 region of SEQ ID NO: 540;
91) a binding molecule comprising a) a light-chain variable region comprising a CDR1 region of SEQ ID NO: 541, a CDR2 region of SEQ ID NO: 542, and a CDR3 region of SEQ ID NO: 543, and b) a heavy-chain variable region comprising a CDR1 region of SEQ ID NO: 544, a CDR2 region of SEQ ID NO: 545, and a CDR3 region of SEQ ID NO: 546;
92) a binding molecule comprising a) a light-chain variable region comprising a CDR1 region of SEQ ID NO: 547, a CDR2 region of SEQ ID NO: 548, and a CDR3 region of SEQ ID NO: 549, and b) a heavy-chain variable region comprising a CDR1 region of SEQ ID NO: 550, a CDR2 region of SEQ ID NO: 551, and a CDR3 region of SEQ ID NO: 552;
93) a binding molecule comprising a) a light-chain variable region comprising a CDR1 region of SEQ ID NO: 553, a CDR2 region of SEQ ID NO: 554, and a CDR3 region of SEQ ID NO: 555, and b) a heavy-chain variable region comprising a CDR1 region of SEQ ID NO: 556, a CDR2 region of SEQ ID NO: 557, and a CDR3 region of SEQ ID NO: 558;
94) a binding molecule comprising a) a light-chain variable region comprising a CDR1 region of SEQ ID NO: 559, a CDR2 region of SEQ ID NO: 560, and a CDR3 region of SEQ ID NO: 561, and b) a heavy-chain variable region comprising a CDR1 region of SEQ ID NO: 562, a CDR2 region of SEQ ID NO: 563, and a CDR3 region of SEQ ID NO: 564;
95) a binding molecule comprising a) a light-chain variable region comprising a CDR1 region of SEQ ID NO: 565, a CDR2 region of SEQ ID NO: 566, and a CDR3 region of SEQ ID NO: 567, and b) a heavy-chain variable region comprising a CDR1 region of SEQ ID NO: 568, a CDR2 region of SEQ ID NO: 569, and a CDR3 region of SEQ ID NO: 570;
96) a binding molecule comprising a) a light-chain variable region comprising a CDR1 region of SEQ ID NO: 571, a CDR2 region of SEQ ID NO: 572, and a CDR3 region of SEQ ID NO: 573, and b) a heavy-chain variable region comprising a CDR1 region of SEQ ID NO: 574, a CDR2 region of SEQ ID NO: 575, and a CDR3 region of SEQ ID NO: 576;
97) a binding molecule comprising a) a light-chain variable region comprising a CDR1 region of SEQ ID NO: 577, a CDR2 region of SEQ ID NO: 578, and a CDR3 region of SEQ ID NO: 579, and b) a heavy-chain variable region comprising a CDR1 region of SEQ ID NO: 580, a CDR2 region of SEQ ID NO: 581, and a CDR3 region of SEQ ID NO: 582;
98) a binding molecule comprising a) a light-chain variable region comprising a CDR1 region of SEQ ID NO: 583, a CDR2 region of SEQ ID NO: 584, and a CDR3 region of SEQ ID NO: 585, and b) a heavy-chain variable region comprising a CDR1 region of SEQ ID NO: 586, a CDR2 region of SEQ ID NO: 587, and a CDR3 region of SEQ ID NO: 588;
99) a binding molecule comprising a) a light-chain variable region comprising a CDR1 region of SEQ ID NO: 589, a CDR2 region of SEQ ID NO: 590, and a CDR3 region of SEQ ID NO: 591, and b) a heavy-chain variable region comprising a CDR1 region of SEQ ID NO: 592, a CDR2 region of SEQ ID NO: 593, and a CDR3 region of SEQ ID NO: 594;
100) a binding molecule comprising a) a light-chain variable region comprising a CDR1 region of SEQ ID NO: 595, a CDR2 region of SEQ ID NO: 596, and a CDR3 region of SEQ ID NO: 597, and b) a heavy-chain variable region comprising a CDR1 region of SEQ ID NO: 598, a CDR2 region of SEQ ID NO: 599, and a CDR3 region of SEQ ID NO: 600;
101) a binding molecule comprising a) a light-chain variable region comprising a CDR1 region of SEQ ID NO: 601, a CDR2 region of SEQ ID NO: 602, and a CDR3 region of SEQ ID NO: 603, and b) a heavy-chain variable region comprising a CDR1 region of SEQ ID NO: 604, a CDR2 region of SEQ ID NO: 605, and a CDR3 region of SEQ ID NO: 606;
102) a binding molecule comprising a) a light-chain variable region comprising a CDR1 region of SEQ ID NO: 607, a CDR2 region of SEQ ID NO: 608, and a CDR3 region of SEQ ID NO: 609, and b) a heavy-chain variable region comprising a CDR1 region of SEQ ID NO: 610, a CDR2 region of SEQ ID NO: 611, and a CDR3 region of SEQ ID NO: 612;
103) a binding molecule comprising a) a light-chain variable region comprising a CDR1 region of SEQ ID NO: 613, a CDR2 region of SEQ ID NO: 614, and a CDR3 region of SEQ ID NO: 615, and b) a heavy-chain variable region comprising a CDR1 region of SEQ ID NO: 616, a CDR2 region of SEQ ID NO: 617, and a CDR3 region of SEQ ID NO: 618;
104) a binding molecule comprising a) a light-chain variable region comprising a CDR1 region of SEQ ID NO: 619, a CDR2 region of SEQ ID NO: 620, and a CDR3 region of SEQ ID NO: 621, and b) a heavy-chain variable region comprising a CDR1 region of SEQ ID NO: 622, a CDR2 region of SEQ ID NO: 623, and a CDR3 region of SEQ ID NO: 624;
105) a binding molecule comprising a) a light-chain variable region comprising a CDR1 region of SEQ ID NO: 625, a CDR2 region of SEQ ID NO: 626, and a CDR3 region of SEQ ID NO: 627, and b) a heavy-chain variable region comprising a CDR1 region of SEQ ID NO: 628, a CDR2 region of SEQ ID NO: 629, and a CDR3 region of SEQ ID NO: 630;
106) a binding molecule comprising a) a light-chain variable region comprising a CDR1 region of SEQ ID NO: 631, a CDR2 region of SEQ ID NO: 632, and a CDR3 region of SEQ ID NO: 633, and b) a heavy-chain variable region comprising a CDR1 region of SEQ ID NO: 634, a CDR2 region of SEQ ID NO: 635, and a CDR3 region of SEQ ID NO: 636;
107) a binding molecule comprising a) a light-chain variable region comprising a CDR1 region of SEQ ID NO: 637, a CDR2 region of SEQ ID NO: 638, and a CDR3 region of SEQ ID NO: 639, and b) a heavy-chain variable region comprising a CDR1 region of SEQ ID NO: 640, a CDR2 region of SEQ ID NO: 641, and a CDR3 region of SEQ ID NO: 642;
108) a binding molecule comprising a) a light-chain variable region comprising a CDR1 region of SEQ ID NO: 643, a CDR2 region of SEQ ID NO: 644, and a CDR3 region of SEQ ID NO: 645, and b) a heavy-chain variable region comprising a CDR1 region of SEQ ID NO: 646, a CDR2 region of SEQ ID NO: 647, and a CDR3 region of SEQ ID NO: 648;
109) a binding molecule comprising a) a light-chain variable region comprising a CDR1 region of SEQ ID NO: 649, a CDR2 region of SEQ ID NO: 650, and a CDR3 region of SEQ ID NO: 651, and b) a heavy-chain variable region comprising a CDR1 region of SEQ ID NO: 652, a CDR2 region of SEQ ID NO: 653, and a CDR3 region of SEQ ID NO: 654;
110) a binding molecule comprising a) a light-chain variable region comprising a CDR1 region of SEQ ID NO: 655, a CDR2 region of SEQ ID NO: 656, and a CDR3 region of SEQ ID NO: 657, and b) a heavy-chain variable region comprising a CDR1 region of SEQ ID NO: 658, a CDR2 region of SEQ ID NO: 659, and a CDR3 region of SEQ ID NO: 660;
111) a binding molecule comprising a) a light-chain variable region comprising a CDR1 region of SEQ ID NO: 661, a CDR2 region of SEQ ID NO: 662, and a CDR3 region of SEQ ID NO: 663, and b) a heavy-chain variable region comprising a CDR1 region of SEQ ID NO: 664, a CDR2 region of SEQ ID NO: 665, and a CDR3 region of SEQ ID NO: 666;
112) a binding molecule comprising a) a light-chain variable region comprising a CDR1 region of SEQ ID NO: 667, a CDR2 region of SEQ ID NO: 668, and a CDR3 region of SEQ ID NO: 669, and b) a heavy-chain variable region comprising a CDR1 region of SEQ ID NO: 670, a CDR2 region of SEQ ID NO: 671, and a CDR3 region of SEQ ID NO: 672;
113) a binding molecule comprising a) a light-chain variable region comprising a CDR1 region of SEQ ID NO: 673, a CDR2 region of SEQ ID NO: 674, and a CDR3 region of SEQ ID NO: 675, and b) a heavy-chain variable region comprising a CDR1 region of SEQ ID NO: 676, a CDR2 region of SEQ ID NO: 677, and a CDR3 region of SEQ ID NO: 678;
114) a binding molecule comprising a) a light-chain variable region comprising a CDR1 region of SEQ ID NO: 679, a CDR2 region of SEQ ID NO: 680, and a CDR3 region of SEQ ID NO: 681, and b) a heavy-chain variable region comprising a CDR1 region of SEQ ID NO: 682, a CDR2 region of SEQ ID NO: 683, and a CDR3 region of SEQ ID NO: 684;
115) a binding molecule comprising a) a light-chain variable region comprising a CDR1 region of SEQ ID NO: 685, a CDR2 region of SEQ ID NO: 686, and a CDR3 region of SEQ ID NO: 687, and b) a heavy-chain variable region comprising a CDR1 region of SEQ ID NO: 688, a CDR2 region of SEQ ID NO: 689, and a CDR3 region of SEQ ID NO: 690;
116) a binding molecule comprising a) a light-chain variable region comprising a CDR1 region of SEQ ID NO: 691, a CDR2 region of SEQ ID NO: 692, and a CDR3 region of SEQ ID NO: 693, and b) a heavy-chain variable region comprising a CDR1 region of SEQ ID NO: 694, a CDR2 region of SEQ ID NO: 695, and a CDR3 region of SEQ ID NO: 696;
117) a binding molecule comprising a) a light-chain variable region comprising a CDR1 region of SEQ ID NO: 697, a CDR2 region of SEQ ID NO: 698, and a CDR3 region of SEQ ID NO: 699, and b) a heavy-chain variable region comprising a CDR1 region of SEQ ID NO: 700, a CDR2 region of SEQ ID NO: 701, and a CDR3 region of SEQ ID NO: 702;
118) a binding molecule comprising a) a light-chain variable region comprising a CDR1 region of SEQ ID NO: 703, a CDR2 region of SEQ ID NO: 704, and a CDR3 region of SEQ ID NO: 705, and b) a heavy-chain variable region comprising a CDR1 region of SEQ ID NO: 706, a CDR2 region of SEQ ID NO: 707, and a CDR3 region of SEQ ID NO: 708;
119) a binding molecule comprising a) a light-chain variable region comprising a CDR1 region of SEQ ID NO: 709, a CDR2 region of SEQ ID NO: 710, and a CDR3 region of SEQ ID NO: 711, and b) a heavy-chain variable region comprising a CDR1 region of SEQ ID NO: 712, a CDR2 region of SEQ ID NO: 713, and a CDR3 region of SEQ ID NO: 714;
120) a binding molecule comprising a) a light-chain variable region comprising a CDR1 region of SEQ ID NO: 715, a CDR2 region of SEQ ID NO: 716, and a CDR3 region of SEQ ID NO: 717, and b) a heavy-chain variable region comprising a CDR1 region of SEQ ID NO: 718, a CDR2 region of SEQ ID NO: 719, and a CDR3 region of SEQ ID NO: 720;
121) a binding molecule comprising a) a light-chain variable region comprising a CDR1 region of SEQ ID NO: 721, a CDR2 region of SEQ ID NO: 722, and a CDR3 region of SEQ ID NO: 723, and b) a heavy-chain variable region comprising a CDR1 region of SEQ ID NO: 724, a CDR2 region of SEQ ID NO: 725, and a CDR3 region of SEQ ID NO: 726;
122) a binding molecule comprising a) a light-chain variable region comprising a CDR1 region of SEQ ID NO: 727, a CDR2 region of SEQ ID NO: 728, and a CDR3 region of SEQ ID NO: 729, and b) a heavy-chain variable region comprising a CDR1 region of SEQ ID NO: 730, a CDR2 region of SEQ ID NO: 731, and a CDR3 region of SEQ ID NO: 732;
123) a binding molecule comprising a) a light-chain variable region comprising a CDR1 region of SEQ ID NO: 733, a CDR2 region of SEQ ID NO: 734, and a CDR3 region of SEQ ID NO: 735, and b) a heavy-chain variable region comprising a CDR1 region of SEQ ID NO: 736, a CDR2 region of SEQ ID NO: 737, and a CDR3 region of SEQ ID NO: 738;
124) a binding molecule comprising a) a light-chain variable region comprising a CDR1 region of SEQ ID NO: 739, a CDR2 region of SEQ ID NO: 740, and a CDR3 region of SEQ ID NO: 741, and b) a heavy-chain variable region comprising a CDR1 region of SEQ ID NO: 742, a CDR2 region of SEQ ID NO: 743, and a CDR3 region of SEQ ID NO: 744;
125) a binding molecule comprising a) a light-chain variable region comprising a CDR1 region of SEQ ID NO: 745, a CDR2 region of SEQ ID NO: 746, and a CDR3 region of SEQ ID NO: 747, and b) a heavy-chain variable region comprising a CDR1 region of SEQ ID NO: 748, a CDR2 region of SEQ ID NO: 749, and a CDR3 region of SEQ ID NO: 750;
126) a binding molecule comprising a) a light-chain variable region comprising a CDR1 region of SEQ ID NO: 751, a CDR2 region of SEQ ID NO: 752, and a CDR3 region of SEQ ID NO: 753, and b) a heavy-chain variable region comprising a CDR1 region of SEQ ID NO: 754, a CDR2 region of SEQ ID NO: 755, and a CDR3 region of SEQ ID NO: 756;
127) a binding molecule comprising a) a light-chain variable region comprising a CDR1 region of SEQ ID NO: 757, a CDR2 region of SEQ ID NO: 758, and a CDR3 region of SEQ ID NO: 759, and b) a heavy-chain variable region comprising a CDR1 region of SEQ ID NO: 760, a CDR2 region of SEQ ID NO: 761, and a CDR3 region of SEQ ID NO: 762;
128) a binding molecule comprising a) a light-chain variable region comprising a CDR1 region of SEQ ID NO: 763, a CDR2 region of SEQ ID NO: 764, and a CDR3 region of SEQ ID NO: 765, and b) a heavy-chain variable region comprising a CDR1 region of SEQ ID NO: 766, a CDR2 region of SEQ ID NO: 767, and a CDR3 region of SEQ ID NO: 768;
129) a binding molecule comprising a) a light-chain variable region comprising a CDR1 region of SEQ ID NO: 769, a CDR2 region of SEQ ID NO: 770, and a CDR3 region of SEQ ID NO: 771, and b) a heavy-chain variable region comprising a CDR1 region of SEQ ID NO: 772, a CDR2 region of SEQ ID NO: 773, and a CDR3 region of SEQ ID NO: 774;
130) a binding molecule comprising a) a light-chain variable region comprising a CDR1 region of SEQ ID NO: 775, a CDR2 region of SEQ ID NO: 776, and a CDR3 region of SEQ ID NO: 777, and b) a heavy-chain variable region comprising a CDR1 region of SEQ ID NO: 778, a CDR2 region of SEQ ID NO: 779, and a CDR3 region of SEQ ID NO: 780;
131) a binding molecule comprising a) a light-chain variable region comprising a CDR1 region of SEQ ID NO: 781, a CDR2 region of SEQ ID NO: 782, and a CDR3 region of SEQ ID NO: 783, and b) a heavy-chain variable region comprising a CDR1 region of SEQ ID NO: 784, a CDR2 region of SEQ ID NO: 785, and a CDR3 region of SEQ ID NO: 786;
132) a binding molecule comprising a) a light-chain variable region comprising a CDR1 region of SEQ ID NO: 787, a CDR2 region of SEQ ID NO: 788, and a CDR3 region of SEQ ID NO: 789, and b) a heavy-chain variable region comprising a CDR1 region of SEQ ID NO: 790, a CDR2 region of SEQ ID NO: 791, and a CDR3 region of SEQ ID NO: 792;
133) a binding molecule comprising a) a light-chain variable region comprising a CDR1 region of SEQ ID NO: 793, a CDR2 region of SEQ ID NO: 794, and a CDR3 region of SEQ ID NO: 795, and b) a heavy-chain variable region comprising a CDR1 region of SEQ ID NO: 796, a CDR2 region of SEQ ID NO: 797, and a CDR3 region of SEQ ID NO: 798;
134) a binding molecule comprising a) a light-chain variable region comprising a CDR1 region of SEQ ID NO: 799, a CDR2 region of SEQ ID NO: 800, and a CDR3 region of SEQ ID NO: 801, and b) a heavy-chain variable region comprising a CDR1 region of SEQ ID NO: 802, a CDR2 region of SEQ ID NO: 803, and a CDR3 region of SEQ ID NO: 804;
135) a binding molecule comprising a) a light-chain variable region comprising a CDR1 region of SEQ ID NO: 805, a CDR2 region of SEQ ID NO: 806, and a CDR3 region of SEQ ID NO: 807, and b) a heavy-chain variable region comprising a CDR1 region of SEQ ID NO: 808, a CDR2 region of SEQ ID NO: 809, and a CDR3 region of SEQ ID NO: 810;
136) a binding molecule comprising a) a light-chain variable region comprising a CDR1 region of SEQ ID NO: 811, a CDR2 region of SEQ ID NO: 812, and a CDR3 region of SEQ ID NO: 813, and b) a heavy-chain variable region comprising a CDR1 region of SEQ ID NO: 814, a CDR2 region of SEQ ID NO: 815, and a CDR3 region of SEQ ID NO: 816;
137) a binding molecule comprising a) a light-chain variable region comprising a CDR1 region of SEQ ID NO: 817, a CDR2 region of SEQ ID NO: 818, and a CDR3 region of SEQ ID NO: 819, and b) a heavy-chain variable region comprising a CDR1 region of SEQ ID NO: 820, a CDR2 region of SEQ ID NO: 821, and a CDR3 region of SEQ ID NO: 822;
138) a binding molecule comprising a) a light-chain variable region comprising a CDR1 region of SEQ ID NO: 823, a CDR2 region of SEQ ID NO: 824, and a CDR3 region of SEQ ID NO: 825, and b) a heavy-chain variable region comprising a CDR1 region of SEQ ID NO: 826, a CDR2 region of SEQ ID NO: 827, and a CDR3 region of SEQ ID NO: 828;
139) a binding molecule comprising a) a light-chain variable region comprising a CDR1 region of SEQ ID NO: 829, a CDR2 region of SEQ ID NO: 830, and a CDR3 region of SEQ ID NO: 831, and b) a heavy-chain variable region comprising a CDR1 region of SEQ ID NO: 832, a CDR2 region of SEQ ID NO: 833, and a CDR3 region of SEQ ID NO: 834;
140) a binding molecule comprising a) a light-chain variable region comprising a CDR1 region of SEQ ID NO: 835, a CDR2 region of SEQ ID NO: 836, and a CDR3 region of SEQ ID NO: 837, and b) a heavy-chain variable region comprising a CDR1 region of SEQ ID NO: 838, a CDR2 region of SEQ ID NO: 839, and a CDR3 region of SEQ ID NO: 840;
141) a binding molecule comprising a) a light-chain variable region comprising a CDR1 region of SEQ ID NO: 841, a CDR2 region of SEQ ID NO: 842, and a CDR3 region of SEQ ID NO: 843, and b) a heavy-chain variable region comprising a CDR1 region of SEQ ID NO: 844, a CDR2 region of SEQ ID NO: 845, and a CDR3 region of SEQ ID NO: 846;
142) a binding molecule comprising a) a light-chain variable region comprising a CDR1 region of SEQ ID NO: 847, a CDR2 region of SEQ ID NO: 848, and a CDR3 region of SEQ ID NO: 849, and b) a heavy-chain variable region comprising a CDR1 region of SEQ ID NO: 850, a CDR2 region of SEQ ID NO: 851, and a CDR3 region of SEQ ID NO: 852;
143) a binding molecule comprising a) a light-chain variable region comprising a CDR1 region of SEQ ID NO: 853, a CDR2 region of SEQ ID NO: 854, and a CDR3 region of SEQ ID NO: 855, and b) a heavy-chain variable region comprising a CDR1 region of SEQ ID NO: 856, a CDR2 region of SEQ ID NO: 857, and a CDR3 region of SEQ ID NO: 858;
144) a binding molecule comprising a) a light-chain variable region comprising a CDR1 region of SEQ ID NO: 859, a CDR2 region of SEQ ID NO: 860, and a CDR3 region of SEQ ID NO: 861, and b) a heavy-chain variable region comprising a CDR1 region of SEQ ID NO: 862, a CDR2 region of SEQ ID NO: 863, and a CDR3 region of SEQ ID NO: 864;
145) a binding molecule comprising a) a light-chain variable region comprising a CDR1 region of SEQ ID NO: 865, a CDR2 region of SEQ ID NO: 866, and a CDR3 region of SEQ ID NO: 867, and b) a heavy-chain variable region comprising a CDR1 region of SEQ ID NO: 868, a CDR2 region of SEQ ID NO: 869, and a CDR3 region of SEQ ID NO: 870;
146) a binding molecule comprising a) a light-chain variable region comprising a CDR1 region of SEQ ID NO: 871, a CDR2 region of SEQ ID NO: 872, and a CDR3 region of SEQ ID NO: 873, and b) a heavy-chain variable region comprising a CDR1 region of SEQ ID NO: 874, a CDR2 region of SEQ ID NO: 875, and a CDR3 region of SEQ ID NO: 876;
147) a binding molecule comprising a) a light-chain variable region comprising a CDR1 region of SEQ ID NO: 877, a CDR2 region of SEQ ID NO: 878, and a CDR3 region of SEQ ID NO: 879, and b) a heavy-chain variable region comprising a CDR1 region of SEQ ID NO: 880, a CDR2 region of SEQ ID NO: 881, and a CDR3 region of SEQ ID NO: 882;
148) a binding molecule comprising a) a light-chain variable region comprising a CDR1 region of SEQ ID NO: 883, a CDR2 region of SEQ ID NO: 884, and a CDR3 region of SEQ ID NO: 885, and b) a heavy-chain variable region comprising a CDR1 region of SEQ ID NO: 886, a CDR2 region of SEQ ID NO: 887, and a CDR3 region of SEQ ID NO: 888;
149) a binding molecule comprising a) a light-chain variable region comprising a CDR1 region of SEQ ID NO: 889, a CDR2 region of SEQ ID NO: 890, and a CDR3 region of SEQ ID NO: 891, and b) a heavy-chain variable region comprising a CDR1 region of SEQ ID NO: 892, a CDR2 region of SEQ ID NO: 893, and a CDR3 region of SEQ ID NO: 894;
150) a binding molecule comprising a) a light-chain variable region comprising a CDR1 region of SEQ ID NO: 895, a CDR2 region of SEQ ID NO: 896, and a CDR3 region of SEQ ID NO: 897, and b) a heavy-chain variable region comprising a CDR1 region of SEQ ID NO: 898, a CDR2 region of SEQ ID NO: 899, and a CDR3 region of SEQ ID NO: 900;
151) a binding molecule comprising a) a light-chain variable region comprising a CDR1 region of SEQ ID NO: 901, a CDR2 region of SEQ ID NO: 902, and a CDR3 region of SEQ ID NO: 903, and b) a heavy-chain variable region comprising a CDR1 region of SEQ ID NO: 904, a CDR2 region of SEQ ID NO: 905, and a CDR3 region of SEQ ID NO: 906;
152) a binding molecule comprising a) a light-chain variable region comprising a CDR1 region of SEQ ID NO: 907, a CDR2 region of SEQ ID NO: 908, and a CDR3 region of SEQ ID NO: 909, and b) a heavy-chain variable region comprising a CDR1 region of SEQ ID NO: 910, a CDR2 region of SEQ ID NO: 911, and a CDR3 region of SEQ ID NO: 912;
153) a binding molecule comprising a) a light-chain variable region comprising a CDR1 region of SEQ ID NO: 913, a CDR2 region of SEQ ID NO: 914, and a CDR3 region of SEQ ID NO: 915, and b) a heavy-chain variable region comprising a CDR1 region of SEQ ID NO: 916, a CDR2 region of SEQ ID NO: 917, and a CDR3 region of SEQ ID NO: 918;
154) a binding molecule comprising a) a light-chain variable region comprising a CDR1 region of SEQ ID NO: 919, a CDR2 region of SEQ ID NO: 920, and a CDR3 region of SEQ ID NO: 921, and b) a heavy-chain variable region comprising a CDR1 region of SEQ ID NO: 922, a CDR2 region of SEQ ID NO: 923, and a CDR3 region of SEQ ID NO: 924;
155) a binding molecule comprising a) a light-chain variable region comprising a CDR1 region of SEQ ID NO: 925, a CDR2 region of SEQ ID NO: 926, and a CDR3 region of SEQ ID NO: 927, and b) a heavy-chain variable region comprising a CDR1 region of SEQ ID NO: 928, a CDR2 region of SEQ ID NO: 929, and a CDR3 region of SEQ ID NO: 930;
156) a binding molecule comprising a) a light-chain variable region comprising a CDR1 region of SEQ ID NO: 931, a CDR2 region of SEQ ID NO: 932, and a CDR3 region of SEQ ID NO: 933, and b) a heavy-chain variable region comprising a CDR1 region of SEQ ID NO: 934, a CDR2 region of SEQ ID NO: 935, and a CDR3 region of SEQ ID NO: 936;
157) a binding molecule comprising a) a light-chain variable region comprising a CDR1 region of SEQ ID NO: 937, a CDR2 region of SEQ ID NO: 938, and a CDR3 region of SEQ ID NO: 939, and b) a heavy-chain variable region comprising a CDR1 region of SEQ ID NO: 940, a CDR2 region of SEQ ID NO: 941, and a CDR3 region of SEQ ID NO: 942;
158) a binding molecule comprising a) a light-chain variable region comprising a CDR1 region of SEQ ID NO: 943, a CDR2 region of SEQ ID NO: 944, and a CDR3 region of SEQ ID NO: 945, and b) a heavy-chain variable region comprising a CDR1 region of SEQ ID NO: 946, a CDR2 region of SEQ ID NO: 947, and a CDR3 region of SEQ ID NO: 948;
159) a binding molecule comprising a) a light-chain variable region comprising a CDR1 region of SEQ ID NO: 949, a CDR2 region of SEQ ID NO: 950, and a CDR3 region of SEQ ID NO: 951, and b) a heavy-chain variable region comprising a CDR1 region of SEQ ID NO: 952, a CDR2 region of SEQ ID NO: 953, and a CDR3 region of SEQ ID NO: 954;
160) a binding molecule comprising a) a light-chain variable region comprising a CDR1 region of SEQ ID NO: 955, a CDR2 region of SEQ ID NO: 956, and a CDR3 region of SEQ ID NO: 957, and b) a heavy-chain variable region comprising a CDR1 region of SEQ ID NO: 958, a CDR2 region of SEQ ID NO: 959, and a CDR3 region of SEQ ID NO: 960;
161) a binding molecule comprising a) a light-chain variable region comprising a CDR1 region of SEQ ID NO: 961, a CDR2 region of SEQ ID NO: 962, and a CDR3 region of SEQ ID NO: 963, and b) a heavy-chain variable region comprising a CDR1 region of SEQ ID NO: 964, a CDR2 region of SEQ ID NO: 965, and a CDR3 region of SEQ ID NO: 966;
162) a binding molecule comprising a) a light-chain variable region comprising a CDR1 region of SEQ ID NO: 967, a CDR2 region of SEQ ID NO: 968, and a CDR3 region of SEQ ID NO: 969, and b) a heavy-chain variable region comprising a CDR1 region of SEQ ID NO: 970, a CDR2 region of SEQ ID NO: 971, and a CDR3 region of SEQ ID NO: 972;
163) a binding molecule comprising a) a light-chain variable region comprising a CDR1 region of SEQ ID NO: 973, a CDR2 region of SEQ ID NO: 974, and a CDR3 region of SEQ ID NO: 975, and b) a heavy-chain variable region comprising a CDR1 region of SEQ ID NO: 976, a CDR2 region of SEQ ID NO: 977, and a CDR3 region of SEQ ID NO: 978;
164) a binding molecule comprising a) a light-chain variable region comprising a CDR1 region of SEQ ID NO: 979, a CDR2 region of SEQ ID NO: 980, and a CDR3 region of SEQ ID NO: 981, and b) a heavy-chain variable region comprising a CDR1 region of SEQ ID NO: 982, a CDR2 region of SEQ ID NO: 983, and a CDR3 region of SEQ ID NO: 984;
165) a binding molecule comprising a) a light-chain variable region comprising a CDR1 region of SEQ ID NO: 985, a CDR2 region of SEQ ID NO: 986, and a CDR3 region of SEQ ID NO: 987, and b) a heavy-chain variable region comprising a CDR1 region of SEQ ID NO: 988, a CDR2 region of SEQ ID NO: 989, and a CDR3 region of SEQ ID NO: 990;
166) a binding molecule comprising a) a light-chain variable region comprising a CDR1 region of SEQ ID NO: 991, a CDR2 region of SEQ ID NO: 992, and a CDR3 region of SEQ ID NO: 993, and b) a heavy-chain variable region comprising a CDR1 region of SEQ ID NO: 994, a CDR2 region of SEQ ID NO: 995, and a CDR3 region of SEQ ID NO: 996;
167) a binding molecule comprising a) a light-chain variable region comprising a CDR1 region of SEQ ID NO: 997, a CDR2 region of SEQ ID NO: 998, and a CDR3 region of SEQ ID NO: 999, and b) a heavy-chain variable region comprising a CDR1 region of SEQ ID NO: 1,000, a CDR2 region of SEQ ID NO: 1,001, and a CDR3 region of SEQ ID NO: 1,002;
168) a binding molecule comprising a) a light-chain variable region comprising a CDR1 region of SEQ ID NO: 1,003, a CDR2 region of SEQ ID NO: 1,004, and a CDR3 region of SEQ ID NO: 1,005, and b) a heavy-chain variable region comprising a CDR1 region of SEQ ID NO: 1,006, a CDR2 region of SEQ ID NO: 1,007, and a CDR3 region of SEQ ID NO: 1,008;
169) a binding molecule comprising a) a light-chain variable region comprising a CDR1 region of SEQ ID NO: 1,009, a CDR2 region of SEQ ID NO: 1,010, and a CDR3 region of SEQ ID NO: 1,011, and b) a heavy-chain variable region comprising a CDR1 region of SEQ ID NO: 1,012, a CDR2 region of SEQ ID NO: 1,013, and a CDR3 region of SEQ ID NO: 1,014;
170) a binding molecule comprising a) a light-chain variable region comprising a CDR1 region of SEQ ID NO: 1,015, a CDR2 region of SEQ ID NO: 1,016, and a CDR3 region of SEQ ID NO: 1,017, and b) a heavy-chain variable region comprising a CDR1 region of SEQ ID NO: 1,018, a CDR2 region of SEQ ID NO: 1,019, and a CDR3 region of SEQ ID NO: 1,020;
171) a binding molecule comprising a) a light-chain variable region comprising a CDR1 region of SEQ ID NO: 1,021, a CDR2 region of SEQ ID NO: 1,022, and a CDR3 region of SEQ ID NO: 1,023, and b) a heavy-chain variable region comprising a CDR1 region of SEQ ID NO: 1,024, a CDR2 region of SEQ ID NO: 1,025, and a CDR3 region of SEQ ID NO: 1,026;
172) a binding molecule comprising a) a light-chain variable region comprising a CDR1 region of SEQ ID NO: 1,027, a CDR2 region of SEQ ID NO: 1,028, and a CDR3 region of SEQ ID NO: 1,029, and b) a heavy-chain variable region comprising a CDR1 region of SEQ ID NO: 1,030, a CDR2 region of SEQ ID NO: 1,031, and a CDR3 region of SEQ ID NO: 1,032;
173) a binding molecule comprising a) a light-chain variable region comprising a CDR1 region of SEQ ID NO: 1,033, a CDR2 region of SEQ ID NO: 1,034, and a CDR3 region of SEQ ID NO: 1,035, and b) a heavy-chain variable region comprising a CDR1 region of SEQ ID NO: 1,036, a CDR2 region of SEQ ID NO: 1,037, and a CDR3 region of SEQ ID NO: 1,038;
174) a binding molecule comprising a) a light-chain variable region comprising a CDR1 region of SEQ ID NO: 1,039, a CDR2 region of SEQ ID NO: 1,040, and a CDR3 region of SEQ ID NO: 1,041, and b) a heavy-chain variable region comprising a CDR1 region of SEQ ID NO: 1,042, a CDR2 region of SEQ ID NO: 1,043, and a CDR3 region of SEQ ID NO: 1,044;
175) a binding molecule comprising a) a light-chain variable region comprising a CDR1 region of SEQ ID NO: 1,045, a CDR2 region of SEQ ID NO: 1,046, and a CDR3 region of SEQ ID NO: 1,047, and b) a heavy-chain variable region comprising a CDR1 region of SEQ ID NO: 1,048, a CDR2 region of SEQ ID NO: 1,049, and a CDR3 region of SEQ ID NO: 1,050;
176) a binding molecule comprising a) a light-chain variable region comprising a CDR1 region of SEQ ID NO: 1,051, a CDR2 region of SEQ ID NO: 1,052, and a CDR3 region of SEQ ID NO: 1,053, and b) a heavy-chain variable region comprising a CDR1 region of SEQ ID NO: 1,054, a CDR2 region of SEQ ID NO: 1,055, and a CDR3 region of SEQ ID NO: 1,056;
177) a binding molecule comprising a) a light-chain variable region comprising a CDR1 region of SEQ ID NO: 1,057, a CDR2 region of SEQ ID NO: 1,058, and a CDR3 region of SEQ ID NO: 1,059, and b) a heavy-chain variable region comprising a CDR1 region of SEQ ID NO: 1,060, a CDR2 region of SEQ ID NO: 1,061, and a CDR3 region of SEQ ID NO: 1,062;
178) a binding molecule comprising a) a light-chain variable region comprising a CDR1 region of SEQ ID NO: 1,063, a CDR2 region of SEQ ID NO: 1,064, and a CDR3 region of SEQ ID NO: 1,065, and b) a heavy-chain variable region comprising a CDR1 region of SEQ ID NO: 1,066, a CDR2 region of SEQ ID NO: 1,067, and a CDR3 region of SEQ ID NO: 1,068;
179) a binding molecule comprising a) a light-chain variable region comprising a CDR1 region of SEQ ID NO: 1,069, a CDR2 region of SEQ ID NO: 1,070, and a CDR3 region of SEQ ID NO: 1,071, and b) a heavy-chain variable region comprising a CDR1 region of SEQ ID NO: 1,072, a CDR2 region of SEQ ID NO: 1,073, and a CDR3 region of SEQ ID NO: 1,074;
180) a binding molecule comprising a) a light-chain variable region comprising a CDR1 region of SEQ ID NO: 1,075, a CDR2 region of SEQ ID NO: 1,076, and a CDR3 region of SEQ ID NO: 1,077, and b) a heavy-chain variable region comprising a CDR1 region of SEQ ID NO: 1,078, a CDR2 region of SEQ ID NO: 1,079, and a CDR3 region of SEQ ID NO: 1,080;
181) a binding molecule comprising a) a light-chain variable region comprising a CDR1 region of SEQ ID NO: 1,081, a CDR2 region of SEQ ID NO: 1,082, and a CDR3 region of SEQ ID NO: 1,083, and b) a heavy-chain variable region comprising a CDR1 region of SEQ ID NO: 1,084, a CDR2 region of SEQ ID NO: 1,085, and a CDR3 region of SEQ ID NO: 1,086;
182) a binding molecule comprising a) a light-chain variable region comprising a CDR1 region of SEQ ID NO: 1,087, a CDR2 region of SEQ ID NO: 1,088, and a CDR3 region of SEQ ID NO: 1,089, and b) a heavy-chain variable region comprising a CDR1 region of SEQ ID NO: 1,090, a CDR2 region of SEQ ID NO: 1,091, and a CDR3 region of SEQ ID NO: 1,092;
183) a binding molecule comprising a) a light-chain variable region comprising a CDR1 region of SEQ ID NO: 1,093, a CDR2 region of SEQ ID NO: 1,094, and a CDR3 region of SEQ ID NO: 1,095, and b) a heavy-chain variable region comprising a CDR1 region of SEQ ID NO: 1,096, a CDR2 region of SEQ ID NO: 1,097, and a CDR3 region of SEQ ID NO: 1,098;
184) a binding molecule comprising a) a light-chain variable region comprising a CDR1 region of SEQ ID NO: 1,099, a CDR2 region of SEQ ID NO: 1,100, and a CDR3 region of SEQ ID NO: 1,101, and b) a heavy-chain variable region comprising a CDR1 region of SEQ ID NO: 1,102, a CDR2 region of SEQ ID NO: 1,103, and a CDR3 region of SEQ ID NO: 1,104;
185) a binding molecule comprising a) a light-chain variable region comprising a CDR1 region of SEQ ID NO: 1,105, a CDR2 region of SEQ ID NO: 1,106, and a CDR3 region of SEQ ID NO: 1,107, and b) a heavy-chain variable region comprising a CDR1 region of SEQ ID NO: 1,108, a CDR2 region of SEQ ID NO: 1,109, and a CDR3 region of SEQ ID NO: 1,110;
186) a binding molecule comprising a) a light-chain variable region comprising a CDR1 region of SEQ ID NO: 1,111, a CDR2 region of SEQ ID NO: 1,112, and a CDR3 region of SEQ ID NO: 1,113, and b) a heavy-chain variable region comprising a CDR1 region of SEQ ID NO: 1,114, a CDR2 region of SEQ ID NO: 1,115, and a CDR3 region of SEQ ID NO: 1,116;
187) a binding molecule comprising a) a light-chain variable region comprising a CDR1 region of SEQ ID NO: 1,117, a CDR2 region of SEQ ID NO: 1,118, and a CDR3 region of SEQ ID NO: 1,119, and b) a heavy-chain variable region comprising a CDR1 region of SEQ ID NO: 1,120, a CDR2 region of SEQ ID NO: 1,121, and a CDR3 region of SEQ ID NO: 1,122;
188) a binding molecule comprising a) a light-chain variable region comprising a CDR1 region of SEQ ID NO: 1,123, a CDR2 region of SEQ ID NO: 1,124, and a CDR3 region of SEQ ID NO: 1,125, and b) a heavy-chain variable region comprising a CDR1 region of SEQ ID NO: 1,126, a CDR2 region of SEQ ID NO: 1,127, and a CDR3 region of SEQ ID NO: 1,128;
189) a binding molecule comprising a) a light-chain variable region comprising a CDR1 region of SEQ ID NO: 1,129, a CDR2 region of SEQ ID NO: 1,130, and a CDR3 region of SEQ ID NO: 1,131, and b) a heavy-chain variable region comprising a CDR1 region of SEQ ID NO: 1,132, a CDR2 region of SEQ ID NO: 1,133, and a CDR3 region of SEQ ID NO: 1,134; and
190) a binding molecule comprising a) a light-chain variable region comprising a CDR1 region of SEQ ID NO: 1,135, a CDR2 region of SEQ ID NO: 1,136, and a CDR3 region of SEQ ID NO: 1,137, and b) a heavy-chain variable region comprising a CDR1 region of SEQ ID NO: 1,138, a CDR2 region of SEQ ID NO: 1,139, and a CDR3 region of SEQ ID NO: 1,140.

9. The binding molecule according to claim 1, wherein the binding molecule is any one selected from the group consisting of the following binding molecules 1 to 190:
1) a binding molecule comprising a) a light-chain variable region of SEQ ID NO: 1,141, and b) a heavy-chain variable region of SEQ ID NO: 1,142;
2) a binding molecule comprising a) a light-chain variable region of SEQ ID NO: 1,143, and b) a heavy-chain variable region of SEQ ID NO: 1,144;
3) a binding molecule comprising a) a light-chain variable region of SEQ ID NO: 1,145, and b) a heavy-chain variable region of SEQ ID NO: 1,146;
4) a binding molecule comprising a) a light-chain variable region of SEQ ID NO: 1,147, and b) a heavy-chain variable region of SEQ ID NO: 1,148;
5) a binding molecule comprising a) a light-chain variable region of SEQ ID NO: 1,149, and b) a heavy-chain variable region of SEQ ID NO: 1,150;
6) a binding molecule comprising a) a light-chain variable region of SEQ ID NO: 1,151, and b) a heavy-chain variable region of SEQ ID NO: 1,152;
7) a binding molecule comprising a) a light-chain variable region of SEQ ID NO: 1,153, and b) a heavy-chain variable region of SEQ ID NO: 1,154;
8) a binding molecule comprising a) a light-chain variable region of SEQ ID NO: 1,155, and b) a heavy-chain variable region of SEQ ID NO: 1,156;
9) a binding molecule comprising a) a light-chain variable region of SEQ ID NO: 1,157, and b) a heavy-chain variable region of SEQ ID NO: 1,158;
10) a binding molecule comprising a) a light-chain variable region of SEQ ID NO: 1,159, and b) a heavy-chain variable region of SEQ ID NO: 1,160;
11) a binding molecule comprising a) a light-chain variable region of SEQ ID NO: 1,161, and b) a heavy-chain variable region of SEQ ID NO: 1,162;
12) a binding molecule comprising a) a light-chain variable region of SEQ ID NO: 1,163, and b) a heavy-chain variable region of SEQ ID NO: 1,164;
13) a binding molecule comprising a) a light-chain variable region of SEQ ID NO: 1,165, and b) a heavy-chain variable region of SEQ ID NO: 1,166;
14) a binding molecule comprising a) a light-chain variable region of SEQ ID NO: 1,167, and b) a heavy-chain variable region of SEQ ID NO: 1,168;
15) a binding molecule comprising a) a light-chain variable region of SEQ ID NO: 1,169, and b) a heavy-chain variable region of SEQ ID NO: 1,170;
16) a binding molecule comprising a) a light-chain variable region of SEQ ID NO: 1,171, and b) a heavy-chain variable region of SEQ ID NO: 1,172;
17) a binding molecule comprising a) a light-chain variable region of SEQ ID NO: 1,173, and b) a heavy-chain variable region of SEQ ID NO: 1,174;
18) a binding molecule comprising a) a light-chain variable region of SEQ ID NO: 1,175, and b) a heavy-chain variable region of SEQ ID NO: 1,176;
19) a binding molecule comprising a) a light-chain variable region of SEQ ID NO: 1,177, and b) a heavy-chain variable region of SEQ ID NO: 1,178;
20) a binding molecule comprising a) a light-chain variable region of SEQ ID NO: 1,179, and b) a heavy-chain variable region of SEQ ID NO: 1,180;
21) a binding molecule comprising a) a light-chain variable region of SEQ ID NO: 1,181, and b) a heavy-chain variable region of SEQ ID NO: 1,182;
22) a binding molecule comprising a) a light-chain variable region of SEQ ID NO: 1,183, and b) a heavy-chain variable region of SEQ ID NO: 1,184;
23) a binding molecule comprising a) a light-chain variable region of SEQ ID NO: 1,185, and b) a heavy-chain variable region of SEQ ID NO: 1,186;
24) a binding molecule comprising a) a light-chain variable region of SEQ ID NO: 1,187, and b) a heavy-chain variable region of SEQ ID NO: 1,188;
25) a binding molecule comprising a) a light-chain variable region of SEQ ID NO: 1,189, and b) a heavy-chain variable region of SEQ ID NO: 1,190;
26) a binding molecule comprising a) a light-chain variable region of SEQ ID NO: 1,191, and b) a heavy-chain variable region of SEQ ID NO: 1,192;
27) a binding molecule comprising a) a light-chain variable region of SEQ ID NO: 1,193, and b) a heavy-chain variable region of SEQ ID NO: 1,194;
28) a binding molecule comprising a) a light-chain variable region of SEQ ID NO: 1,195, and b) a heavy-chain variable region of SEQ ID NO: 1,196;
29) a binding molecule comprising a) a light-chain variable region of SEQ ID NO: 1,197, and b) a heavy-chain variable region of SEQ ID NO: 1,198;
30) a binding molecule comprising a) a light-chain variable region of SEQ ID NO: 1,199, and b) a heavy-chain variable region of SEQ ID NO: 1,200;
31) a binding molecule comprising a) a light-chain variable region of SEQ ID NO: 1,201, and b) a heavy-chain variable region of SEQ ID NO: 1,202;
32) a binding molecule comprising a) a light-chain variable region of SEQ ID NO: 1,203, and b) a heavy-chain variable region of SEQ ID NO: 1,204;
33) a binding molecule comprising a) a light-chain variable region of SEQ ID NO: 1,205, and b) a heavy-chain variable region of SEQ ID NO: 1,206;
34) a binding molecule comprising a) a light-chain variable region of SEQ ID NO: 1,207, and b) a heavy-chain variable region of SEQ ID NO: 1,208;
35) a binding molecule comprising a) a light-chain variable region of SEQ ID NO: 1,209, and b) a heavy-chain variable region of SEQ ID NO: 1,210;
36) a binding molecule comprising a) a light-chain variable region of SEQ ID NO: 1,211, and b) a heavy-chain variable region of SEQ ID NO: 1,212;
37) a binding molecule comprising a) a light-chain variable region of SEQ ID NO: 1,213, and b) a heavy-chain variable region of SEQ ID NO: 1,214;
38) a binding molecule comprising a) a light-chain variable region of SEQ ID NO: 1,215, and b) a heavy-chain variable region of SEQ ID NO: 1,216;
39) a binding molecule comprising a) a light-chain variable region of SEQ ID NO: 1,217, and b) a heavy-chain variable region of SEQ ID NO: 1,218;
40) a binding molecule comprising a) a light-chain variable region of SEQ ID NO: 1,219, and b) a heavy-chain variable region of SEQ ID NO: 1,220;
41) a binding molecule comprising a) a light-chain variable region of SEQ ID NO: 1,221, and b) a heavy-chain variable region of SEQ ID NO: 1,222;
42) a binding molecule comprising a) a light-chain variable region of SEQ ID NO: 1,223, and b) a heavy-chain variable region of SEQ ID NO: 1,224;
43) a binding molecule comprising a) a light-chain variable region of SEQ ID NO: 1,225, and b) a heavy-chain variable region of SEQ ID NO: 1,226;
44) a binding molecule comprising a) a light-chain variable region of SEQ ID NO: 1,227, and b) a heavy-chain variable region of SEQ ID NO: 1,228;
45) a binding molecule comprising a) a light-chain variable region of SEQ ID NO: 1,229, and b) a heavy-chain variable region of SEQ ID NO: 1,230;
46) a binding molecule comprising a) a light-chain variable region of SEQ ID NO: 1,231, and b) a heavy-chain variable region of SEQ ID NO: 1,232;
47) a binding molecule comprising a) a light-chain variable region of SEQ ID NO: 1,233, and b) a heavy-chain variable region of SEQ ID NO: 1,234;
48) a binding molecule comprising a) a light-chain variable region of SEQ ID NO: 1,235, and b) a heavy-chain variable region of SEQ ID NO: 1,236;
49) a binding molecule comprising a) a light-chain variable region of SEQ ID NO: 1,237, and b) a heavy-chain variable region of SEQ ID NO: 1,238;
50) a binding molecule comprising a) a light-chain variable region of SEQ ID NO: 1,239, and b) a heavy-chain variable region of SEQ ID NO: 1,240;
51) a binding molecule comprising a) a light-chain variable region of SEQ ID NO: 1,241, and b) a heavy-chain variable region of SEQ ID NO: 1,242;
52) a binding molecule comprising a) a light-chain variable region of SEQ ID NO: 1,243, and b) a heavy-chain variable region of SEQ ID NO: 1,244;
53) a binding molecule comprising a) a light-chain variable region of SEQ ID NO: 1,245, and b) a heavy-chain variable region of SEQ ID NO: 1,246;
54) a binding molecule comprising a) a light-chain variable region of SEQ ID NO: 1,247, and b) a heavy-chain variable region of SEQ ID NO: 1,248;
55) a binding molecule comprising a) a light-chain variable region of SEQ ID NO: 1,249, and b) a heavy-chain variable region of SEQ ID NO: 1,250;
56) a binding molecule comprising a) a light-chain variable region of SEQ ID NO: 1,251, and b) a heavy-chain variable region of SEQ ID NO: 1,252;
57) a binding molecule comprising a) a light-chain variable region of SEQ ID NO: 1,253, and b) a heavy-chain variable region of SEQ ID NO: 1,254;
58) a binding molecule comprising a) a light-chain variable region of SEQ ID NO: 1,255, and b) a heavy-chain variable region of SEQ ID NO: 1,256;
59) a binding molecule comprising a) a light-chain variable region of SEQ ID NO: 1,257, and b) a heavy-chain variable region of SEQ ID NO: 1,258;
60) a binding molecule comprising a) a light-chain variable region of SEQ ID NO: 1,259, and b) a heavy-chain variable region of SEQ ID NO: 1,260;
61) a binding molecule comprising a) a light-chain variable region of SEQ ID NO: 1,261, and b) a heavy-chain variable region of SEQ ID NO: 1,262;
62) a binding molecule comprising a) a light-chain variable region of SEQ ID NO: 1,263, and b) a heavy-chain variable region of SEQ ID NO: 1,264;
63) a binding molecule comprising a) a light-chain variable region of SEQ ID NO: 1,265, and b) a heavy-chain variable region of SEQ ID NO: 1,266;
64) a binding molecule comprising a) a light-chain variable region of SEQ ID NO: 1,267, and b) a heavy-chain variable region of SEQ ID NO: 1,268;
65) a binding molecule comprising a) a light-chain variable region of SEQ ID NO: 1,269, and b) a heavy-chain variable region of SEQ ID NO: 1,270;
66) a binding molecule comprising a) a light-chain variable region of SEQ ID NO: 1,271, and b) a heavy-chain variable region of SEQ ID NO: 1,272;
67) a binding molecule comprising a) a light-chain variable region of SEQ ID NO: 1,273, and b) a heavy-chain variable region of SEQ ID NO: 1,274;
68) a binding molecule comprising a) a light-chain variable region of SEQ ID NO: 1,275, and b) a heavy-chain variable region of SEQ ID NO: 1,276;
69) a binding molecule comprising a) a light-chain variable region of SEQ ID NO: 1,277, and b) a heavy-chain variable region of SEQ ID NO: 1,278;
70) a binding molecule comprising a) a light-chain variable region of SEQ ID NO: 1,279, and b) a heavy-chain variable region of SEQ ID NO: 1,280;
71) a binding molecule comprising a) a light-chain variable region of SEQ ID NO: 1,281, and b) a heavy-chain variable region of SEQ ID NO: 1,282;
72) a binding molecule comprising a) a light-chain variable region of SEQ ID NO: 1,283, and b) a heavy-chain variable region of SEQ ID NO: 1,284;
73) a binding molecule comprising a) a light-chain variable region of SEQ ID NO: 1,285, and b) a heavy-chain variable region of SEQ ID NO: 1,286;
74) a binding molecule comprising a) a light-chain variable region of SEQ ID NO: 1,287, and b) a heavy-chain variable region of SEQ ID NO: 1,288;
75) a binding molecule comprising a) a light-chain variable region of SEQ ID NO: 1,289, and b) a heavy-chain variable region of SEQ ID NO: 1,290;
76) a binding molecule comprising a) a light-chain variable region of SEQ ID NO: 1,291, and b) a heavy-chain variable region of SEQ ID NO: 1,292;
77) a binding molecule comprising a) a light-chain variable region of SEQ ID NO: 1,293, and b) a heavy-chain variable region of SEQ ID NO: 1,294;
78) a binding molecule comprising a) a light-chain variable region of SEQ ID NO: 1,295, and b) a heavy-chain variable region of SEQ ID NO: 1,296;
79) a binding molecule comprising a) a light-chain variable region of SEQ ID NO: 1,297, and b) a heavy-chain variable region of SEQ ID NO: 1,298;
80) a binding molecule comprising a) a light-chain variable region of SEQ ID NO: 1,299, and b) a heavy-chain variable region of SEQ ID NO: 1,300;
81) a binding molecule comprising a) a light-chain variable region of SEQ ID NO: 1,301, and b) a heavy-chain variable region of SEQ ID NO: 1,302;
82) a binding molecule comprising a) a light-chain variable region of SEQ ID NO: 1,303, and b) a heavy-chain variable region of SEQ ID NO: 1,304;
83) a binding molecule comprising a) a light-chain variable region of SEQ ID NO: 1,305, and b) a heavy-chain variable region of SEQ ID NO: 1,306;
84) a binding molecule comprising a) a light-chain variable region of SEQ ID NO: 1,307, and b) a heavy-chain variable region of SEQ ID NO: 1,308;
85) a binding molecule comprising a) a light-chain variable region of SEQ ID NO: 1,309, and b) a heavy-chain variable region of SEQ ID NO: 1,310;
86) a binding molecule comprising a) a light-chain variable region of SEQ ID NO: 1,311, and b) a heavy-chain variable region of SEQ ID NO: 1,312;
87) a binding molecule comprising a) a light-chain variable region of SEQ ID NO: 1,313, and b) a heavy-chain variable region of SEQ ID NO: 1,314;
88) a binding molecule comprising a) a light-chain variable region of SEQ ID NO: 1,315, and b) a heavy-chain variable region of SEQ ID NO: 1,316;
89) a binding molecule comprising a) a light-chain variable region of SEQ ID NO: 1,317, and b) a heavy-chain variable region of SEQ ID NO: 1,318;
90) a binding molecule comprising a) a light-chain variable region of SEQ ID NO: 1,319, and b) a heavy-chain variable region of SEQ ID NO: 1,320;
91) a binding molecule comprising a) a light-chain variable region of SEQ ID NO: 1,321, and b) a heavy-chain variable region of SEQ ID NO: 1,322;
92) a binding molecule comprising a) a light-chain variable region of SEQ ID NO: 1,323, and b) a heavy-chain variable region of SEQ ID NO: 1,324;
93) a binding molecule comprising a) a light-chain variable region of SEQ ID NO: 1,325, and b) a heavy-chain variable region of SEQ ID NO: 1,326;
94) a binding molecule comprising a) a light-chain variable region of SEQ ID NO: 1,327, and b) a heavy-chain variable region of SEQ ID NO: 1,328;
95) a binding molecule comprising a) a light-chain variable region of SEQ ID NO: 1,329, and b) a heavy-chain variable region of SEQ ID NO: 1,330;
96) a binding molecule comprising a) a light-chain variable region of SEQ ID NO: 1,331, and b) a heavy-chain variable region of SEQ ID NO: 1,332;
97) a binding molecule comprising a) a light-chain variable region of SEQ ID NO: 1,333, and b) a heavy-chain variable region of SEQ ID NO: 1,334;
98) a binding molecule comprising a) a light-chain variable region of SEQ ID NO: 1,335, and b) a heavy-chain variable region of SEQ ID NO: 1,336;
99) a binding molecule comprising a) a light-chain variable region of SEQ ID NO: 1,337, and b) a heavy-chain variable region of SEQ ID NO: 1,338;
100) a binding molecule comprising a) a light-chain variable region of SEQ ID NO: 1,339, and b) a heavy-chain variable region of SEQ ID NO: 1,340;
101) a binding molecule comprising a) a light-chain variable region of SEQ ID NO: 1,341, and b) a heavy-chain variable region of SEQ ID NO: 1,342;
102) a binding molecule comprising a) a light-chain variable region of SEQ ID NO: 1,343, and b) a heavy-chain variable region of SEQ ID NO: 1,344;
103) a binding molecule comprising a) a light-chain variable region of SEQ ID NO: 1,345, and b) a heavy-chain variable region of SEQ ID NO: 1,346;
104) a binding molecule comprising a) a light-chain variable region of SEQ ID NO: 1,347, and b) a heavy-chain variable region of SEQ ID NO: 1,348;
105) a binding molecule comprising a) a light-chain variable region of SEQ ID NO: 1,349, and b) a heavy-chain variable region of SEQ ID NO: 1,350;
106) a binding molecule comprising a) a light-chain variable region of SEQ ID NO: 1,351, and b) a heavy-chain variable region of SEQ ID NO: 1,352;
107) a binding molecule comprising a) a light-chain variable region of SEQ ID NO: 1,353, and b) a heavy-chain variable region of SEQ ID NO: 1,354;
108) a binding molecule comprising a) a light-chain variable region of SEQ ID NO: 1,355, and b) a heavy-chain variable region of SEQ ID NO: 1,356;
109) a binding molecule comprising a) a light-chain variable region of SEQ ID NO: 1,357, and b) a heavy-chain variable region of SEQ ID NO: 1,358;
110) a binding molecule comprising a) a light-chain variable region of SEQ ID NO: 1,359, and b) a heavy-chain variable region of SEQ ID NO: 1,360;
111) a binding molecule comprising a) a light-chain variable region of SEQ ID NO: 1,361, and b) a heavy-chain variable region of SEQ ID NO: 1,362;
112) a binding molecule comprising a) a light-chain variable region of SEQ ID NO: 1,363, and b) a heavy-chain variable region of SEQ ID NO: 1,364;
113) a binding molecule comprising a) a light-chain variable region of SEQ ID NO: 1,365, and b) a heavy-chain variable region of SEQ ID NO: 1,366;
114) a binding molecule comprising a) a light-chain variable region of SEQ ID NO: 1,367, and b) a heavy-chain variable region of SEQ ID NO: 1,368;
115) a binding molecule comprising a) a light-chain variable region of SEQ ID NO: 1,369, and b) a heavy-chain variable region of SEQ ID NO: 1,370;
116) a binding molecule comprising a) a light-chain variable region of SEQ ID NO: 1,371, and b) a heavy-chain variable region of SEQ ID NO: 1,372;
117) a binding molecule comprising a) a light-chain variable region of SEQ ID NO: 1,373, and b) a heavy-chain variable region of SEQ ID NO: 1,374;
118) a binding molecule comprising a) a light-chain variable region of SEQ ID NO: 1,375, and b) a heavy-chain variable region of SEQ ID NO: 1,376;
119) a binding molecule comprising a) a light-chain variable region of SEQ ID NO: 1,377, and b) a heavy-chain variable region of SEQ ID NO: 1,378;
120) a binding molecule comprising a) a light-chain variable region of SEQ ID NO: 1,379, and b) a heavy-chain variable region of SEQ ID NO: 1,380;
121) a binding molecule comprising a) a light-chain variable region of SEQ ID NO: 1,381, and b) a heavy-chain variable region of SEQ ID NO: 1,382;
122) a binding molecule comprising a) a light-chain variable region of SEQ ID NO: 1,383, and b) a heavy-chain variable region of SEQ ID NO: 1,384;
123) a binding molecule comprising a) a light-chain variable region of SEQ ID NO: 1,385, and b) a heavy-chain variable region of SEQ ID NO: 1,386;
124) a binding molecule comprising a) a light-chain variable region of SEQ ID NO: 1,387, and b) a heavy-chain variable region of SEQ ID NO: 1,388;
125) a binding molecule comprising a) a light-chain variable region of SEQ ID NO: 1,389, and b) a heavy-chain variable region of SEQ ID NO: 1,390;
126) a binding molecule comprising a) a light-chain variable region of SEQ ID NO: 1,391, and b) a heavy-chain variable region of SEQ ID NO: 1,392;
127) a binding molecule comprising a) a light-chain variable region of SEQ ID NO: 1,393, and b) a heavy-chain variable region of SEQ ID NO: 1,394;
128) a binding molecule comprising a) a light-chain variable region of SEQ ID NO: 1,395, and b) a heavy-chain variable region of SEQ ID NO: 1,396;
129) a binding molecule comprising a) a light-chain variable region of SEQ ID NO: 1,397, and b) a heavy-chain variable region of SEQ ID NO: 1,398;
130) a binding molecule comprising a) a light-chain variable region of SEQ ID NO: 1,399, and b) a heavy-chain variable region of SEQ ID NO: 1,400;
131) a binding molecule comprising a) a light-chain variable region of SEQ ID NO: 1,401, and b) a heavy-chain variable region of SEQ ID NO: 1,402;
132) a binding molecule comprising a) a light-chain variable region of SEQ ID NO: 1,403, and b) a heavy-chain variable region of SEQ ID NO: 1,404;
133) a binding molecule comprising a) a light-chain variable region of SEQ ID NO: 1,405, and b) a heavy-chain variable region of SEQ ID NO: 1,406;
134) a binding molecule comprising a) a light-chain variable region of SEQ ID NO: 1,407, and b) a heavy-chain variable region of SEQ ID NO: 1,408;
135) a binding molecule comprising a) a light-chain variable region of SEQ ID NO: 1,409, and b) a heavy-chain variable region of SEQ ID NO: 1,410;
136) a binding molecule comprising a) a light-chain variable region of SEQ ID NO: 1,411, and b) a heavy-chain variable region of SEQ ID NO: 1,412;
137) a binding molecule comprising a) a light-chain variable region of SEQ ID NO: 1,413, and b) a heavy-chain variable region of SEQ ID NO: 1,414;
138) a binding molecule comprising a) a light-chain variable region of SEQ ID NO: 1,415, and b) a heavy-chain variable region of SEQ ID NO: 1,416;
139) a binding molecule comprising a) a light-chain variable region of SEQ ID NO: 1,417, and b) a heavy-chain variable region of SEQ ID NO: 1,418;
140) a binding molecule comprising a) a light-chain variable region of SEQ ID NO: 1,419, and b) a heavy-chain variable region of SEQ ID NO: 1,420;
141) a binding molecule comprising a) a light-chain variable region of SEQ ID NO: 1,421, and b) a heavy-chain variable region of SEQ ID NO: 1,422;
142) a binding molecule comprising a) a light-chain variable region of SEQ ID NO: 1,423, and b) a heavy-chain variable region of SEQ ID NO: 1,424;
143) a binding molecule comprising a) a light-chain variable region of SEQ ID NO: 1,425, and b) a heavy-chain variable region of SEQ ID NO: 1,426;
144) a binding molecule comprising a) a light-chain variable region of SEQ ID NO: 1,427, and b) a heavy-chain variable region of SEQ ID NO: 1,428;
145) a binding molecule comprising a) a light-chain variable region of SEQ ID NO: 1,429, and b) a heavy-chain variable region of SEQ ID NO: 1,430;
146) a binding molecule comprising a) a light-chain variable region of SEQ ID NO: 1,431, and b) a heavy-chain variable region of SEQ ID NO: 1,432;
147) a binding molecule comprising a) a light-chain variable region of SEQ ID NO: 1,433, and b) a heavy-chain variable region of SEQ ID NO: 1,434;
148) a binding molecule comprising a) a light-chain variable region of SEQ ID NO: 1,435, and b) a heavy-chain variable region of SEQ ID NO: 1,436;
149) a binding molecule comprising a) a light-chain variable region of SEQ ID NO: 1,437, and b) a heavy-chain variable region of SEQ ID NO: 1,438;
150) a binding molecule comprising a) a light-chain variable region of SEQ ID NO: 1,439, and b) a heavy-chain variable region of SEQ ID NO: 1,440;
151) a binding molecule comprising a) a light-chain variable region of SEQ ID NO: 1,441, and b) a heavy-chain variable region of SEQ ID NO: 1,442;
152) a binding molecule comprising a) a light-chain variable region of SEQ ID NO: 1,443, and b) a heavy-chain variable region of SEQ ID NO: 1,444;
153) a binding molecule comprising a) a light-chain variable region of SEQ ID NO: 1,445, and b) a heavy-chain variable region of SEQ ID NO: 1,446;
154) a binding molecule comprising a) a light-chain variable region of SEQ ID NO: 1,447, and b) a heavy-chain variable region of SEQ ID NO: 1,448;
155) a binding molecule comprising a) a light-chain variable region of SEQ ID NO: 1,449, and b) a heavy-chain variable region of SEQ ID NO: 1,450;
156) a binding molecule comprising a) a light-chain variable region of SEQ ID NO: 1,451, and b) a heavy-chain variable region of SEQ ID NO: 1,452;
157) a binding molecule comprising a) a light-chain variable region of SEQ ID NO: 1,453, and b) a heavy-chain variable region of SEQ ID NO: 1,454;
158) a binding molecule comprising a) a light-chain variable region of SEQ ID NO: 1,455, and b) a heavy-chain variable region of SEQ ID NO: 1,456;
159) a binding molecule comprising a) a light-chain variable region of SEQ ID NO: 1,457, and b) a heavy-chain variable region of SEQ ID NO: 1,458;
160) a binding molecule comprising a) a light-chain variable region of SEQ ID NO: 1,459, and b) a heavy-chain variable region of SEQ ID NO: 1,460;
161) a binding molecule comprising a) a light-chain variable region of SEQ ID NO: 1,461, and b) a heavy-chain variable region of SEQ ID NO: 1,462;
162) a binding molecule comprising a) a light-chain variable region of SEQ ID NO: 1,463, and b) a heavy-chain variable region of SEQ ID NO: 1,464;
163) a binding molecule comprising a) a light-chain variable region of SEQ ID NO: 1,465, and b) a heavy-chain variable region of SEQ ID NO: 1,466;
164) a binding molecule comprising a) a light-chain variable region of SEQ ID NO: 1,467, and b) a heavy-chain variable region of SEQ ID NO: 1,468;
165) a binding molecule comprising a) a light-chain variable region of SEQ ID NO: 1,469, and b) a heavy-chain variable region of SEQ ID NO: 1,470;
166) a binding molecule comprising a) a light-chain variable region of SEQ ID NO: 1,471, and b) a heavy-chain variable region of SEQ ID NO: 1,472;
167) a binding molecule comprising a) a light-chain variable region of SEQ ID NO: 1,473, and b) a heavy-chain variable region of SEQ ID NO: 1,474;
168) a binding molecule comprising a) a light-chain variable region of SEQ ID NO: 1,475, and b) a heavy-chain variable region of SEQ ID NO: 1,476;
169) a binding molecule comprising a) a light-chain variable region of SEQ ID NO: 1,477, and b) a heavy-chain variable region of SEQ ID NO: 1,478;
170) a binding molecule comprising a) a light-chain variable region of SEQ ID NO: 1,479, and b) a heavy-chain variable region of SEQ ID NO: 1,480;
171) a binding molecule comprising a) a light-chain variable region of SEQ ID NO: 1,481, and b) a heavy-chain variable region of SEQ ID NO: 1,482;
172) a binding molecule comprising a) a light-chain variable region of SEQ ID NO: 1,483, and b) a heavy-chain variable region of SEQ ID NO: 1,484;
173) a binding molecule comprising a) a light-chain variable region of SEQ ID NO: 1,485, and b) a heavy-chain variable region of SEQ ID NO: 1,486;
174) a binding molecule comprising a) a light-chain variable region of SEQ ID NO: 1,487, and b) a heavy-chain variable region of SEQ ID NO: 1,488;
175) a binding molecule comprising a) a light-chain variable region of SEQ ID NO: 1,489, and b) a heavy-chain variable region of SEQ ID NO: 1,490;
176) a binding molecule comprising a) a light-chain variable region of SEQ ID NO: 1,491, and b) a heavy-chain variable region of SEQ ID NO: 1,492;
177) a binding molecule comprising a) a light-chain variable region of SEQ ID NO: 1,493, and b) a heavy-chain variable region of SEQ ID NO: 1,494;
178) a binding molecule comprising a) a light-chain variable region of SEQ ID NO: 1,495, and b) a heavy-chain variable region of SEQ ID NO: 1,496;
179) a binding molecule comprising a) a light-chain variable region of SEQ ID NO: 1,497, and b) a heavy-chain variable region of SEQ ID NO: 1,498;
180) a binding molecule comprising a) a light-chain variable region of SEQ ID NO: 1,499, and b) a heavy-chain variable region of SEQ ID NO: 1,500;
181) a binding molecule comprising a) a light-chain variable region of SEQ ID NO: 1,501, and b) a heavy-chain variable region of SEQ ID NO: 1,502;
182) a binding molecule comprising a) a light-chain variable region of SEQ ID NO: 1,503, and b) a heavy-chain variable region of SEQ ID NO: 1,504;
183) a binding molecule comprising a) a light-chain variable region of SEQ ID NO: 1,505, and b) a heavy-chain variable region of SEQ ID NO: 1,506;
184) a binding molecule comprising a) a light-chain variable region of SEQ ID NO: 1,507, and b) a heavy-chain variable region of SEQ ID NO: 1,508;
185) a binding molecule comprising a) a light-chain variable region of SEQ ID NO: 1,509, and b) a heavy-chain variable region of SEQ ID NO: 1,510;
186) a binding molecule comprising a) a light-chain variable region of SEQ ID NO: 1,511, and b) a heavy-chain variable region of SEQ ID NO: 1,512;
187) a binding molecule comprising a) a light-chain variable region of SEQ ID NO: 1,513, and b) a heavy-chain variable region of SEQ ID NO: 1,514;
188) a binding molecule comprising a) a light-chain variable region of SEQ ID NO: 1,515, and b) a heavy-chain variable region of SEQ ID NO: 1,516;
189) a binding molecule comprising a) a light-chain variable region of SEQ ID NO: 1,517, and b) a heavy-chain variable region of SEQ ID NO: 1,518; and
190) a binding molecule comprising a) a light-chain variable region of SEQ ID NO: 1,519, and b) a heavy-chain variable region of SEQ ID NO: 1,520.

10. The binding molecule according to any one of claims 1 to 9, wherein the binding molecule is an antibody or an antigen-binding fragment thereof.

11. The binding molecule according to claim 10, wherein the antibody is a monoclonal antibody.

12. The binding molecule according to claim 11, wherein the monoclonal antibody is a chimeric antibody, a humanized antibody, or a human antibody.

13. The binding molecule according to claim 10, wherein the antigen-binding fragment is Fab, F(ab'), F(ab')2, Fv, dAb, Fd, a single-chain antibody fragment (scFv), scFv-Fc, a complementarity-determining region (CDR) fragment, a bivalent single-chain antibody fragment, a single-chain phage antibody fragment, diabody, triabody, or tetrabody.

14. The binding molecule according to any one of claims 1 to 13, wherein the binding molecule has neutralizing activity against a mutant virus having a mutation in the spike protein of SARS-coronavirus-2.

15. The binding molecule according to any one of claims 1 to 14, wherein the binding molecule has neutralizing activity against SARS-coronavirus-2 S-type, L-type, V-type, G-type, GH-type or GR-type.

16. The binding molecule according to any one of claims 1 to 15, wherein the binding molecule has neutralizing activity against any one or mutant viruses selected from the group consisting of the following mutant viruses 1) to 39):
1) a mutant virus having HV69-70del mutation at amino acid positions 69 and 70 of the spike protein of SARS-coronavirus-2;
2) a mutant virus having D80A mutation at amino acid position 80 of the spike protein of SARS-coronavirus-2;
3) a mutant virus having A222V mutation at amino acid position 222 of the spike protein of SARS-coronavirus-2;
4) a mutant virus having N234Q mutation at amino acid position 234 of the spike protein of SARS-coronavirus-2;
5) a mutant virus having A372V mutation at amino acid position 372 of the spike protein of SARS-coronavirus-2;
6) a mutant virus having P384L mutation at amino acid position 384 of the spike protein of SARS-coronavirus-2;
7) a mutant virus having E406Q mutation or E406W mutation at amino acid position 406 of the spike protein of SARS-coronavirus-2;
8) a mutant virus having K417N mutation, K417T mutation or K417E mutation at amino acid position 417 of the spike protein of SARS-coronavirus-2;
9) a mutant virus having D420N mutation at amino acid position 420 of the spike protein of SARS-coronavirus-2;
10) a mutant virus having N439K mutation at amino acid position 439 of the spike protein of SARS-coronavirus-2;
11) a mutant virus having N440D mutation at amino acid position 440 of the spike protein of SARS-coronavirus-2;
12) a mutant virus having K444Q mutation at amino acid position 444 of the spike protein of SARS-coronavirus-2;
13) a mutant virus having V445A mutation at amino acid position 445 of the spike protein of SARS-coronavirus-2;
14) a mutant virus having G446V mutation or G446S mutation at amino acid position 446 of the spike protein of SARS-coronavirus-2;
15) a mutant virus having Y449N mutation at amino acid position 449 of the spike protein of SARS-coronavirus-2;
16) a mutant virus having L452R mutation at amino acid position 452 of the spike protein of SARS-coronavirus-2;
17) a mutant virus having Y453F mutation at amino acid position 453 of the spike protein of SARS-coronavirus-2;
18) a mutant virus having L455F mutation at amino acid position 455 of the spike protein of SARS-coronavirus-2;
19) a mutant virus having F456L mutation at amino acid position 456 of the spike protein of SARS-coronavirus-2;
20) a mutant virus having N460T mutation at amino acid position 460 of the spike protein of SARS-coronavirus-2;
21) a mutant virus having Y473F mutation at amino acid position 473 of the spike protein of SARS-coronavirus-2;
22) a mutant virus having A475V mutation at amino acid position 475 of the spike protein of SARS-coronavirus-2;
23) a mutant virus having G476S mutation at amino acid position 476 of the spike protein of SARS-coronavirus-2;
24) a mutant virus having S477N mutation or S477R mutation at amino acid position 477 of the spike protein of SARS-coronavirus-2;
25) a mutant virus having T478K mutation at amino acid position 478 of the spike protein of SARS-coronavirus-2;
26) a mutant virus having E484K mutation, E484G mutation or E484Q mutation at amino acid position 484 of the spike protein of SARS-coronavirus-2;
27) a mutant virus having F486V mutation, F486I mutation, F486S mutation or F486L mutation at amino acid position 486 of the spike protein of SARS-coronavirus-2;
28) a mutant virus having Y489H mutation at amino acid position 489 of the spike protein of SARS-coronavirus-2;
29) a mutant virus having F490S mutation at amino acid position 490 of the spike protein of SARS-coronavirus-2;
30) a mutant virus having Q493K mutation, Q493R mutation, Q493M mutation, Q493Y mutation or Q493A mutation at amino acid position 493 of the spike protein of SARS-coronavirus-2;
31) a mutant virus having S494P mutation, S494Q mutation or S494L mutation at amino acid position 494 of the spike protein of SARS-coronavirus-2;
32) a mutant virus having Q498H mutation at amino acid position 498 of the spike protein of SARS-coronavirus-2;
33) a mutant virus having N501Y mutation, N501T mutation or N501F mutation at amino acid position 501 of the spike protein of SARS-coronavirus-2;
34) a mutant virus having D614G mutation at amino acid position 614 of the spike protein of SARS-coronavirus-2;
35) a mutant virus having Q677H mutation at amino acid position 677 of the spike protein of SARS-coronavirus-2;
36) a mutant virus having P681H mutation or P681R mutation at amino acid position 681 of the spike protein of SARS-coronavirus-2;
37) a mutant virus having R685H mutation at amino acid position 685 of the spike protein of SARS-coronavirus-2;
38) a mutant virus having A701V mutation at amino acid position 701 of the spike protein of SARS-coronavirus-2; and
39) a mutant virus having V 1176F mutation at amino acid position 1,176 of the spike protein of SARS-coronavirus-2.

17. An immunoconjugate in which at least one tag is additionally conjugated to the binding molecule according to any one of claims 1 to 16.

18. A nucleic acid molecule encoding the binding molecule according to any one of claims 1 to 16.

19. An expression vector into which the nucleic acid molecule according to claim 18 has been inserted.

20. A cell line transformed with the expression vector according to claim 19.

21. The cell line according to claim 20, wherein the cell line is any one selected from the group consisting of a CHO cell line, an F2N cell line, a COS cell line, a BHK cell line, a Bowes melanoma cell line, a HeLa cell line, a 911 cell line, a HT1080 cell line, an A549 cell line, a HEK 293 cell line, and a HEK293T cell line.

22. A composition for diagnosing, preventing or treating a disease caused by coronavirus, the composition comprising the binding molecule according to any one of claims 1 to 16.

23. The composition according to claim 22, wherein the disease caused by coronavirus is coronavirus infection 2019 (COVID-19), severe acute respiratory syndrome, or Middle East respiratory syndrome.

24. The composition according to claim 22, wherein the composition is a sterile injectable solution, a lyophilized formulation, a pre-filled syringe solution, an oral formulation, a formulation for external use, or a suppository.

25. A kit for diagnosing, preventing or treating a disease caused by coronavirus, the kit comprising the binding molecule according to any one of claims 1 to 16.

26. The kit according to claim 25, wherein the disease caused by coronavirus is coronavirus infection 2019 (COVID-19), severe acute respiratory syndrome, or Middle East respiratory syndrome.

27. A method for diagnosing, preventing or treating a disease caused by coronavirus, the method comprising a step of administering a therapeutically effective amount of the composition according to claim 22 or 23 to a subject having the disease caused by coronavirus.

28. The method according to claim 27, wherein the disease caused by coronavirus is coronavirus infection 2019 (COVID-19), severe acute respiratory syndrome, or Middle East respiratory syndrome.

29. Use of the binding molecule according to any one of claims 1 to 16 for preparation of a composition for diagnosing, preventing or treating a disease caused by coronavirus.

30. Use of the composition according to claim 22 for diagnosis, prevention or treatment of a disease caused by coronavirus, comprising a step of administering a therapeutically effective amount of the composition to a subject having the disease caused by coronavirus.

31. A method of producing a binding molecule, the method comprising steps of:
a) introducing a nucleic acid molecule encoding the binding molecule according to any one of claims 1 to 16 into a host cell;
b) culturing the host cell under conditions that allow expression of the nucleic acid molecule; and
c) selecting the binding molecule from the cultured host cell and/or a culture.

32. A binding molecule produced according to the method according to claim 31.

33. A binding molecule that binds to a spike protein (S protein) on a surface of coronavirus, wherein the binding molecule has a sequence identity of 80% to 99% to any one binding molecule selected from the group consisting of the following binding molecules 1) to 190):
1) a binding molecule comprising a) a light-chain variable region of SEQ ID NO: 1,141, and b) a heavy-chain variable region of SEQ ID NO: 1,142;
2) a binding molecule comprising a) a light-chain variable region of SEQ ID NO: 1,143, and b) a heavy-chain variable region of SEQ ID NO: 1,144;
3) a binding molecule comprising a) a light-chain variable region of SEQ ID NO: 1,145, and b) a heavy-chain variable region of SEQ ID NO: 1,146;
4) a binding molecule comprising a) a light-chain variable region of SEQ ID NO: 1,147, and b) a heavy-chain variable region of SEQ ID NO: 1,148;
5) a binding molecule comprising a) a light-chain variable region of SEQ ID NO: 1,149, and b) a heavy-chain variable region of SEQ ID NO: 1,150;
6) a binding molecule comprising a) a light-chain variable region of SEQ ID NO: 1,151, and b) a heavy-chain variable region of SEQ ID NO: 1,152;
7) a binding molecule comprising a) a light-chain variable region of SEQ ID NO: 1,153, and b) a heavy-chain variable region of SEQ ID NO: 1,154;
8) a binding molecule comprising a) a light-chain variable region of SEQ ID NO: 1,155, and b) a heavy-chain variable region of SEQ ID NO: 1,156;
9) a binding molecule comprising a) a light-chain variable region of SEQ ID NO: 1,157, and b) a heavy-chain variable region of SEQ ID NO: 1,158;
10) a binding molecule comprising a) a light-chain variable region of SEQ ID NO: 1,159, and b) a heavy-chain variable region of SEQ ID NO: 1,160;
11) a binding molecule comprising a) a light-chain variable region of SEQ ID NO: 1,161, and b) a heavy-chain variable region of SEQ ID NO: 1,162;
12) a binding molecule comprising a) a light-chain variable region of SEQ ID NO: 1,163, and b) a heavy-chain variable region of SEQ ID NO: 1,164;
13) a binding molecule comprising a) a light-chain variable region of SEQ ID NO: 1,165, and b) a heavy-chain variable region of SEQ ID NO: 1,166;
14) a binding molecule comprising a) a light-chain variable region of SEQ ID NO: 1,167, and b) a heavy-chain variable region of SEQ ID NO: 1,168;
15) a binding molecule comprising a) a light-chain variable region of SEQ ID NO: 1,169, and b) a heavy-chain variable region of SEQ ID NO: 1,170;
16) a binding molecule comprising a) a light-chain variable region of SEQ ID NO: 1,171, and b) a heavy-chain variable region of SEQ ID NO: 1,172;
17) a binding molecule comprising a) a light-chain variable region of SEQ ID NO: 1,173, and b) a heavy-chain variable region of SEQ ID NO: 1,174;
18) a binding molecule comprising a) a light-chain variable region of SEQ ID NO: 1,175, and b) a heavy-chain variable region of SEQ ID NO: 1,176;
19) a binding molecule comprising a) a light-chain variable region of SEQ ID NO: 1,177, and b) a heavy-chain variable region of SEQ ID NO: 1,178;
20) a binding molecule comprising a) a light-chain variable region of SEQ ID NO: 1,179, and b) a heavy-chain variable region of SEQ ID NO: 1,180;
21) a binding molecule comprising a) a light-chain variable region of SEQ ID NO: 1,181, and b) a heavy-chain variable region of SEQ ID NO: 1,182;
22) a binding molecule comprising a) a light-chain variable region of SEQ ID NO: 1,183, and b) a heavy-chain variable region of SEQ ID NO: 1,184;
23) a binding molecule comprising a) a light-chain variable region of SEQ ID NO: 1,185, and b) a heavy-chain variable region of SEQ ID NO: 1,186;
24) a binding molecule comprising a) a light-chain variable region of SEQ ID NO: 1,187, and b) a heavy-chain variable region of SEQ ID NO: 1,188;
25) a binding molecule comprising a) a light-chain variable region of SEQ ID NO: 1,189, and b) a heavy-chain variable region of SEQ ID NO: 1,190;
26) a binding molecule comprising a) a light-chain variable region of SEQ ID NO: 1,191, and b) a heavy-chain variable region of SEQ ID NO: 1,192;
27) a binding molecule comprising a) a light-chain variable region of SEQ ID NO: 1,193, and b) a heavy-chain variable region of SEQ ID NO: 1,194;
28) a binding molecule comprising a) a light-chain variable region of SEQ ID NO: 1,195, and b) a heavy-chain variable region of SEQ ID NO: 1,196;
29) a binding molecule comprising a) a light-chain variable region of SEQ ID NO: 1,197, and b) a heavy-chain variable region of SEQ ID NO: 1,198;
30) a binding molecule comprising a) a light-chain variable region of SEQ ID NO: 1,199, and b) a heavy-chain variable region of SEQ ID NO: 1,200;
31) a binding molecule comprising a) a light-chain variable region of SEQ ID NO: 1,201, and b) a heavy-chain variable region of SEQ ID NO: 1,202;
32) a binding molecule comprising a) a light-chain variable region of SEQ ID NO: 1,203, and b) a heavy-chain variable region of SEQ ID NO: 1,204;
33) a binding molecule comprising a) a light-chain variable region of SEQ ID NO: 1,205, and b) a heavy-chain variable region of SEQ ID NO: 1,206;
34) a binding molecule comprising a) a light-chain variable region of SEQ ID NO: 1,207, and b) a heavy-chain variable region of SEQ ID NO: 1,208;
35) a binding molecule comprising a) a light-chain variable region of SEQ ID NO: 1,209, and b) a heavy-chain variable region of SEQ ID NO: 1,210;
36) a binding molecule comprising a) a light-chain variable region of SEQ ID NO: 1,211, and b) a heavy-chain variable region of SEQ ID NO: 1,212;
37) a binding molecule comprising a) a light-chain variable region of SEQ ID NO: 1,213, and b) a heavy-chain variable region of SEQ ID NO: 1,214;
38) a binding molecule comprising a) a light-chain variable region of SEQ ID NO: 1,215, and b) a heavy-chain variable region of SEQ ID NO: 1,216;
39) a binding molecule comprising a) a light-chain variable region of SEQ ID NO: 1,217, and b) a heavy-chain variable region of SEQ ID NO: 1,218;
40) a binding molecule comprising a) a light-chain variable region of SEQ ID NO: 1,219, and b) a heavy-chain variable region of SEQ ID NO: 1,220;
41) a binding molecule comprising a) a light-chain variable region of SEQ ID NO: 1,221, and b) a heavy-chain variable region of SEQ ID NO: 1,222;
42) a binding molecule comprising a) a light-chain variable region of SEQ ID NO: 1,223, and b) a heavy-chain variable region of SEQ ID NO: 1,224;
43) a binding molecule comprising a) a light-chain variable region of SEQ ID NO: 1,225, and b) a heavy-chain variable region of SEQ ID NO: 1,226;
44) a binding molecule comprising a) a light-chain variable region of SEQ ID NO: 1,227, and b) a heavy-chain variable region of SEQ ID NO: 1,228;
45) a binding molecule comprising a) a light-chain variable region of SEQ ID NO: 1,229, and b) a heavy-chain variable region of SEQ ID NO: 1,230;
46) a binding molecule comprising a) a light-chain variable region of SEQ ID NO: 1,231, and b) a heavy-chain variable region of SEQ ID NO: 1,232;
47) a binding molecule comprising a) a light-chain variable region of SEQ ID NO: 1,233, and b) a heavy-chain variable region of SEQ ID NO: 1,234;
48) a binding molecule comprising a) a light-chain variable region of SEQ ID NO: 1,235, and b) a heavy-chain variable region of SEQ ID NO: 1,236;
49) a binding molecule comprising a) a light-chain variable region of SEQ ID NO: 1,237, and b) a heavy-chain variable region of SEQ ID NO: 1,238;
50) a binding molecule comprising a) a light-chain variable region of SEQ ID NO: 1,239, and b) a heavy-chain variable region of SEQ ID NO: 1,240;
51) a binding molecule comprising a) a light-chain variable region of SEQ ID NO: 1,241, and b) a heavy-chain variable region of SEQ ID NO: 1,242;
52) a binding molecule comprising a) a light-chain variable region of SEQ ID NO: 1,243, and b) a heavy-chain variable region of SEQ ID NO: 1,244;
53) a binding molecule comprising a) a light-chain variable region of SEQ ID NO: 1,245, and b) a heavy-chain variable region of SEQ ID NO: 1,246;
54) a binding molecule comprising a) a light-chain variable region of SEQ ID NO: 1,247, and b) a heavy-chain variable region of SEQ ID NO: 1,248;
55) a binding molecule comprising a) a light-chain variable region of SEQ ID NO: 1,249, and b) a heavy-chain variable region of SEQ ID NO: 1,250;
56) a binding molecule comprising a) a light-chain variable region of SEQ ID NO: 1,251, and b) a heavy-chain variable region of SEQ ID NO: 1,252;
57) a binding molecule comprising a) a light-chain variable region of SEQ ID NO: 1,253, and b) a heavy-chain variable region of SEQ ID NO: 1,254;
58) a binding molecule comprising a) a light-chain variable region of SEQ ID NO: 1,255, and b) a heavy-chain variable region of SEQ ID NO: 1,256;
59) a binding molecule comprising a) a light-chain variable region of SEQ ID NO: 1,257, and b) a heavy-chain variable region of SEQ ID NO: 1,258;
60) a binding molecule comprising a) a light-chain variable region of SEQ ID NO: 1,259, and b) a heavy-chain variable region of SEQ ID NO: 1,260;
61) a binding molecule comprising a) a light-chain variable region of SEQ ID NO: 1,261, and b) a heavy-chain variable region of SEQ ID NO: 1,262;
62) a binding molecule comprising a) a light-chain variable region of SEQ ID NO: 1,263, and b) a heavy-chain variable region of SEQ ID NO: 1,264;
63) a binding molecule comprising a) a light-chain variable region of SEQ ID NO: 1,265, and b) a heavy-chain variable region of SEQ ID NO: 1,266;
64) a binding molecule comprising a) a light-chain variable region of SEQ ID NO: 1,267, and b) a heavy-chain variable region of SEQ ID NO: 1,268;
65) a binding molecule comprising a) a light-chain variable region of SEQ ID NO: 1,269, and b) a heavy-chain variable region of SEQ ID NO: 1,270;
66) a binding molecule comprising a) a light-chain variable region of SEQ ID NO: 1,271, and b) a heavy-chain variable region of SEQ ID NO: 1,272;
67) a binding molecule comprising a) a light-chain variable region of SEQ ID NO: 1,273, and b) a heavy-chain variable region of SEQ ID NO: 1,274;
68) a binding molecule comprising a) a light-chain variable region of SEQ ID NO: 1,275, and b) a heavy-chain variable region of SEQ ID NO: 1,276;
69) a binding molecule comprising a) a light-chain variable region of SEQ ID NO: 1,277, and b) a heavy-chain variable region of SEQ ID NO: 1,278;
70) a binding molecule comprising a) a light-chain variable region of SEQ ID NO: 1,279, and b) a heavy-chain variable region of SEQ ID NO: 1,280;
71) a binding molecule comprising a) a light-chain variable region of SEQ ID NO: 1,281, and b) a heavy-chain variable region of SEQ ID NO: 1,282;
72) a binding molecule comprising a) a light-chain variable region of SEQ ID NO: 1,283, and b) a heavy-chain variable region of SEQ ID NO: 1,284;
73) a binding molecule comprising a) a light-chain variable region of SEQ ID NO: 1,285, and b) a heavy-chain variable region of SEQ ID NO: 1,286;
74) a binding molecule comprising a) a light-chain variable region of SEQ ID NO: 1,287, and b) a heavy-chain variable region of SEQ ID NO: 1,288;
75) a binding molecule comprising a) a light-chain variable region of SEQ ID NO: 1,289, and b) a heavy-chain variable region of SEQ ID NO: 1,290;
76) a binding molecule comprising a) a light-chain variable region of SEQ ID NO: 1,291, and b) a heavy-chain variable region of SEQ ID NO: 1,292;
77) a binding molecule comprising a) a light-chain variable region of SEQ ID NO: 1,293, and b) a heavy-chain variable region of SEQ ID NO: 1,294;
78) a binding molecule comprising a) a light-chain variable region of SEQ ID NO: 1,295, and b) a heavy-chain variable region of SEQ ID NO: 1,296;
79) a binding molecule comprising a) a light-chain variable region of SEQ ID NO: 1,297, and b) a heavy-chain variable region of SEQ ID NO: 1,298;
80) a binding molecule comprising a) a light-chain variable region of SEQ ID NO: 1,299, and b) a heavy-chain variable region of SEQ ID NO: 1,300;
81) a binding molecule comprising a) a light-chain variable region of SEQ ID NO: 1,301, and b) a heavy-chain variable region of SEQ ID NO: 1,302;
82) a binding molecule comprising a) a light-chain variable region of SEQ ID NO: 1,303, and b) a heavy-chain variable region of SEQ ID NO: 1,304;
83) a binding molecule comprising a) a light-chain variable region of SEQ ID NO: 1,305, and b) a heavy-chain variable region of SEQ ID NO: 1,306;
84) a binding molecule comprising a) a light-chain variable region of SEQ ID NO: 1,307, and b) a heavy-chain variable region of SEQ ID NO: 1,308;
85) a binding molecule comprising a) a light-chain variable region of SEQ ID NO: 1,309, and b) a heavy-chain variable region of SEQ ID NO: 1,310;
86) a binding molecule comprising a) a light-chain variable region of SEQ ID NO: 1,311, and b) a heavy-chain variable region of SEQ ID NO: 1,312;
87) a binding molecule comprising a) a light-chain variable region of SEQ ID NO: 1,313, and b) a heavy-chain variable region of SEQ ID NO: 1,314;
88) a binding molecule comprising a) a light-chain variable region of SEQ ID NO: 1,315, and b) a heavy-chain variable region of SEQ ID NO: 1,316;
89) a binding molecule comprising a) a light-chain variable region of SEQ ID NO: 1,317, and b) a heavy-chain variable region of SEQ ID NO: 1,318;
90) a binding molecule comprising a) a light-chain variable region of SEQ ID NO: 1,319, and b) a heavy-chain variable region of SEQ ID NO: 1,320;
91) a binding molecule comprising a) a light-chain variable region of SEQ ID NO: 1,321, and b) a heavy-chain variable region of SEQ ID NO: 1,322;
92) a binding molecule comprising a) a light-chain variable region of SEQ ID NO: 1,323, and b) a heavy-chain variable region of SEQ ID NO: 1,324;
93) a binding molecule comprising a) a light-chain variable region of SEQ ID NO: 1,325, and b) a heavy-chain variable region of SEQ ID NO: 1,326;
94) a binding molecule comprising a) a light-chain variable region of SEQ ID NO: 1,327, and b) a heavy-chain variable region of SEQ ID NO: 1,328;
95) a binding molecule comprising a) a light-chain variable region of SEQ ID NO: 1,329, and b) a heavy-chain variable region of SEQ ID NO: 1,330;
96) a binding molecule comprising a) a light-chain variable region of SEQ ID NO: 1,331, and b) a heavy-chain variable region of SEQ ID NO: 1,332;
97) a binding molecule comprising a) a light-chain variable region of SEQ ID NO: 1,333, and b) a heavy-chain variable region of SEQ ID NO: 1,334;
98) a binding molecule comprising a) a light-chain variable region of SEQ ID NO: 1,335, and b) a heavy-chain variable region of SEQ ID NO: 1,336;
99) a binding molecule comprising a) a light-chain variable region of SEQ ID NO: 1,337, and b) a heavy-chain variable region of SEQ ID NO: 1,338;
100) a binding molecule comprising a) a light-chain variable region of SEQ ID NO: 1,339, and b) a heavy-chain variable region of SEQ ID NO: 1,340;
101) a binding molecule comprising a) a light-chain variable region of SEQ ID NO: 1,341, and b) a heavy-chain variable region of SEQ ID NO: 1,342;
102) a binding molecule comprising a) a light-chain variable region of SEQ ID NO: 1,343, and b) a heavy-chain variable region of SEQ ID NO: 1,344;
103) a binding molecule comprising a) a light-chain variable region of SEQ ID NO: 1,345, and b) a heavy-chain variable region of SEQ ID NO: 1,346;
104) a binding molecule comprising a) a light-chain variable region of SEQ ID NO: 1,347, and b) a heavy-chain variable region of SEQ ID NO: 1,348;
105) a binding molecule comprising a) a light-chain variable region of SEQ ID NO: 1,349, and b) a heavy-chain variable region of SEQ ID NO: 1,350;
106) a binding molecule comprising a) a light-chain variable region of SEQ ID NO: 1,351, and b) a heavy-chain variable region of SEQ ID NO: 1,352;
107) a binding molecule comprising a) a light-chain variable region of SEQ ID NO: 1,353, and b) a heavy-chain variable region of SEQ ID NO: 1,354;
108) a binding molecule comprising a) a light-chain variable region of SEQ ID NO: 1,355, and b) a heavy-chain variable region of SEQ ID NO: 1,356;
109) a binding molecule comprising a) a light-chain variable region of SEQ ID NO: 1,357, and b) a heavy-chain variable region of SEQ ID NO: 1,358;
110) a binding molecule comprising a) a light-chain variable region of SEQ ID NO: 1,359, and b) a heavy-chain variable region of SEQ ID NO: 1,360;
111) a binding molecule comprising a) a light-chain variable region of SEQ ID NO: 1,361, and b) a heavy-chain variable region of SEQ ID NO: 1,362;
112) a binding molecule comprising a) a light-chain variable region of SEQ ID NO: 1,363, and b) a heavy-chain variable region of SEQ ID NO: 1,364;
113) a binding molecule comprising a) a light-chain variable region of SEQ ID NO: 1,365, and b) a heavy-chain variable region of SEQ ID NO: 1,366;
114) a binding molecule comprising a) a light-chain variable region of SEQ ID NO: 1,367, and b) a heavy-chain variable region of SEQ ID NO: 1,368;
115) a binding molecule comprising a) a light-chain variable region of SEQ ID NO: 1,369, and b) a heavy-chain variable region of SEQ ID NO: 1,370;
116) a binding molecule comprising a) a light-chain variable region of SEQ ID NO: 1,371, and b) a heavy-chain variable region of SEQ ID NO: 1,372;
117) a binding molecule comprising a) a light-chain variable region of SEQ ID NO: 1,373, and b) a heavy-chain variable region of SEQ ID NO: 1,374;
118) a binding molecule comprising a) a light-chain variable region of SEQ ID NO: 1,375, and b) a heavy-chain variable region of SEQ ID NO: 1,376;
119) a binding molecule comprising a) a light-chain variable region of SEQ ID NO: 1,377, and b) a heavy-chain variable region of SEQ ID NO: 1,378;
120) a binding molecule comprising a) a light-chain variable region of SEQ ID NO: 1,379, and b) a heavy-chain variable region of SEQ ID NO: 1,380;
121) a binding molecule comprising a) a light-chain variable region of SEQ ID NO: 1,381, and b) a heavy-chain variable region of SEQ ID NO: 1,382;
122) a binding molecule comprising a) a light-chain variable region of SEQ ID NO: 1,383, and b) a heavy-chain variable region of SEQ ID NO: 1,384;
123) a binding molecule comprising a) a light-chain variable region of SEQ ID NO: 1,385, and b) a heavy-chain variable region of SEQ ID NO: 1,386;
124) a binding molecule comprising a) a light-chain variable region of SEQ ID NO: 1,387, and b) a heavy-chain variable region of SEQ ID NO: 1,388;
125) a binding molecule comprising a) a light-chain variable region of SEQ ID NO: 1,389, and b) a heavy-chain variable region of SEQ ID NO: 1,390;
126) a binding molecule comprising a) a light-chain variable region of SEQ ID NO: 1,391, and b) a heavy-chain variable region of SEQ ID NO: 1,392;
127) a binding molecule comprising a) a light-chain variable region of SEQ ID NO: 1,393, and b) a heavy-chain variable region of SEQ ID NO: 1,394;
128) a binding molecule comprising a) a light-chain variable region of SEQ ID NO: 1,395, and b) a heavy-chain variable region of SEQ ID NO: 1,396;
129) a binding molecule comprising a) a light-chain variable region of SEQ ID NO: 1,397, and b) a heavy-chain variable region of SEQ ID NO: 1,398;
130) a binding molecule comprising a) a light-chain variable region of SEQ ID NO: 1,399, and b) a heavy-chain variable region of SEQ ID NO: 1,400;
131) a binding molecule comprising a) a light-chain variable region of SEQ ID NO: 1,401, and b) a heavy-chain variable region of SEQ ID NO: 1,402;
132) a binding molecule comprising a) a light-chain variable region of SEQ ID NO: 1,403, and b) a heavy-chain variable region of SEQ ID NO: 1,404;
133) a binding molecule comprising a) a light-chain variable region of SEQ ID NO: 1,405, and b) a heavy-chain variable region of SEQ ID NO: 1,406;
134) a binding molecule comprising a) a light-chain variable region of SEQ ID NO: 1,407, and b) a heavy-chain variable region of SEQ ID NO: 1,408;
135) a binding molecule comprising a) a light-chain variable region of SEQ ID NO: 1,409, and b) a heavy-chain variable region of SEQ ID NO: 1,410;
136) a binding molecule comprising a) a light-chain variable region of SEQ ID NO: 1,411, and b) a heavy-chain variable region of SEQ ID NO: 1,412;
137) a binding molecule comprising a) a light-chain variable region of SEQ ID NO: 1,413, and b) a heavy-chain variable region of SEQ ID NO: 1,414;
138) a binding molecule comprising a) a light-chain variable region of SEQ ID NO: 1,415, and b) a heavy-chain variable region of SEQ ID NO: 1,416;
139) a binding molecule comprising a) a light-chain variable region of SEQ ID NO: 1,417, and b) a heavy-chain variable region of SEQ ID NO: 1,418;
140) a binding molecule comprising a) a light-chain variable region of SEQ ID NO: 1,419, and b) a heavy-chain variable region of SEQ ID NO: 1,420;
141) a binding molecule comprising a) a light-chain variable region of SEQ ID NO: 1,421, and b) a heavy-chain variable region of SEQ ID NO: 1,422;
142) a binding molecule comprising a) a light-chain variable region of SEQ ID NO: 1,423, and b) a heavy-chain variable region of SEQ ID NO: 1,424;
143) a binding molecule comprising a) a light-chain variable region of SEQ ID NO: 1,425, and b) a heavy-chain variable region of SEQ ID NO: 1,426;
144) a binding molecule comprising a) a light-chain variable region of SEQ ID NO: 1,427, and b) a heavy-chain variable region of SEQ ID NO: 1,428;
145) a binding molecule comprising a) a light-chain variable region of SEQ ID NO: 1,429, and b) a heavy-chain variable region of SEQ ID NO: 1,430;
146) a binding molecule comprising a) a light-chain variable region of SEQ ID NO: 1,431, and b) a heavy-chain variable region of SEQ ID NO: 1,432;
147) a binding molecule comprising a) a light-chain variable region of SEQ ID NO: 1,433, and b) a heavy-chain variable region of SEQ ID NO: 1,434;
148) a binding molecule comprising a) a light-chain variable region of SEQ ID NO: 1,435, and b) a heavy-chain variable region of SEQ ID NO: 1,436;
149) a binding molecule comprising a) a light-chain variable region of SEQ ID NO: 1,437, and b) a heavy-chain variable region of SEQ ID NO: 1,438;
150) a binding molecule comprising a) a light-chain variable region of SEQ ID NO: 1,439, and b) a heavy-chain variable region of SEQ ID NO: 1,440;
151) a binding molecule comprising a) a light-chain variable region of SEQ ID NO: 1,441, and b) a heavy-chain variable region of SEQ ID NO: 1,442;
152) a binding molecule comprising a) a light-chain variable region of SEQ ID NO: 1,443, and b) a heavy-chain variable region of SEQ ID NO: 1,444;
153) a binding molecule comprising a) a light-chain variable region of SEQ ID NO: 1,445, and b) a heavy-chain variable region of SEQ ID NO: 1,446;
154) a binding molecule comprising a) a light-chain variable region of SEQ ID NO: 1,447, and b) a heavy-chain variable region of SEQ ID NO: 1,448;
155) a binding molecule comprising a) a light-chain variable region of SEQ ID NO: 1,449, and b) a heavy-chain variable region of SEQ ID NO: 1,450;
156) a binding molecule comprising a) a light-chain variable region of SEQ ID NO: 1,451, and b) a heavy-chain variable region of SEQ ID NO: 1,452;
157) a binding molecule comprising a) a light-chain variable region of SEQ ID NO: 1,453, and b) a heavy-chain variable region of SEQ ID NO: 1,454;
158) a binding molecule comprising a) a light-chain variable region of SEQ ID NO: 1,455, and b) a heavy-chain variable region of SEQ ID NO: 1,456;
159) a binding molecule comprising a) a light-chain variable region of SEQ ID NO: 1,457, and b) a heavy-chain variable region of SEQ ID NO: 1,458;
160) a binding molecule comprising a) a light-chain variable region of SEQ ID NO: 1,459, and b) a heavy-chain variable region of SEQ ID NO: 1,460;
161) a binding molecule comprising a) a light-chain variable region of SEQ ID NO: 1,461, and b) a heavy-chain variable region of SEQ ID NO: 1,462;
162) a binding molecule comprising a) a light-chain variable region of SEQ ID NO: 1,463, and b) a heavy-chain variable region of SEQ ID NO: 1,464;
163) a binding molecule comprising a) a light-chain variable region of SEQ ID NO: 1,465, and b) a heavy-chain variable region of SEQ ID NO: 1,466;
164) a binding molecule comprising a) a light-chain variable region of SEQ ID NO: 1,467, and b) a heavy-chain variable region of SEQ ID NO: 1,468;
165) a binding molecule comprising a) a light-chain variable region of SEQ ID NO: 1,469, and b) a heavy-chain variable region of SEQ ID NO: 1,470;
166) a binding molecule comprising a) a light-chain variable region of SEQ ID NO: 1,471, and b) a heavy-chain variable region of SEQ ID NO: 1,472;
167) a binding molecule comprising a) a light-chain variable region of SEQ ID NO: 1,473, and b) a heavy-chain variable region of SEQ ID NO: 1,474;
168) a binding molecule comprising a) a light-chain variable region of SEQ ID NO: 1,475, and b) a heavy-chain variable region of SEQ ID NO: 1,476;
169) a binding molecule comprising a) a light-chain variable region of SEQ ID NO: 1,477, and b) a heavy-chain variable region of SEQ ID NO: 1,478;
170) a binding molecule comprising a) a light-chain variable region of SEQ ID NO: 1,479, and b) a heavy-chain variable region of SEQ ID NO: 1,480;
171) a binding molecule comprising a) a light-chain variable region of SEQ ID NO: 1,481, and b) a heavy-chain variable region of SEQ ID NO: 1,482;
172) a binding molecule comprising a) a light-chain variable region of SEQ ID NO: 1,483, and b) a heavy-chain variable region of SEQ ID NO: 1,484;
173) a binding molecule comprising a) a light-chain variable region of SEQ ID NO: 1,485, and b) a heavy-chain variable region of SEQ ID NO: 1,486;
174) a binding molecule comprising a) a light-chain variable region of SEQ ID NO: 1,487, and b) a heavy-chain variable region of SEQ ID NO: 1,488;
175) a binding molecule comprising a) a light-chain variable region of SEQ ID NO: 1,489, and b) a heavy-chain variable region of SEQ ID NO: 1,490;
176) a binding molecule comprising a) a light-chain variable region of SEQ ID NO: 1,491, and b) a heavy-chain variable region of SEQ ID NO: 1,492;
177) a binding molecule comprising a) a light-chain variable region of SEQ ID NO: 1,493, and b) a heavy-chain variable region of SEQ ID NO: 1,494;
178) a binding molecule comprising a) a light-chain variable region of SEQ ID NO: 1,495, and b) a heavy-chain variable region of SEQ ID NO: 1,496;
179) a binding molecule comprising a) a light-chain variable region of SEQ ID NO: 1,497, and b) a heavy-chain variable region of SEQ ID NO: 1,498;
180) a binding molecule comprising a) a light-chain variable region of SEQ ID NO: 1,499, and b) a heavy-chain variable region of SEQ ID NO: 1,500;
181) a binding molecule comprising a) a light-chain variable region of SEQ ID NO: 1,501, and b) a heavy-chain variable region of SEQ ID NO: 1,502;
182) a binding molecule comprising a) a light-chain variable region of SEQ ID NO: 1,503, and b) a heavy-chain variable region of SEQ ID NO: 1,504;
183) a binding molecule comprising a) a light-chain variable region of SEQ ID NO: 1,505, and b) a heavy-chain variable region of SEQ ID NO: 1,506;
184) a binding molecule comprising a) a light-chain variable region of SEQ ID NO: 1,507, and b) a heavy-chain variable region of SEQ ID NO: 1,508;
185) a binding molecule comprising a) a light-chain variable region of SEQ ID NO: 1,509, and b) a heavy-chain variable region of SEQ ID NO: 1,510;
186) a binding molecule comprising a) a light-chain variable region of SEQ ID NO: 1,511, and b) a heavy-chain variable region of SEQ ID NO: 1,512;
187) a binding molecule comprising a) a light-chain variable region of SEQ ID NO: 1,513, and b) a heavy-chain variable region of SEQ ID NO: 1,514;
188) a binding molecule comprising a) a light-chain variable region of SEQ ID NO: 1,515, and b) a heavy-chain variable region of SEQ ID NO: 1,516;
189) a binding molecule comprising a) a light-chain variable region of SEQ ID NO: 1,517, and b) a heavy-chain variable region of SEQ ID NO: 1,518; and
190) a binding molecule comprising a) a light-chain variable region of SEQ ID NO: 1,519, and b) a heavy-chain variable region of SEQ ID NO: 1,520.

34. The binding molecule according to claim 33, wherein the binding molecule has an IC₅₀ value of 200 ng/ml or less, as evaluated according to a plaque reduction neutralization test (PRNT) method for SARS-coronavirus-2 (SARS-CoV-2).

35. The binding molecule according to claim 33, wherein a minimum concentration value of the binding molecule that neutralizes 100 TCID50 of virus for SARS-coronavirus-2 (SARS-CoV-2) is 2 µg/ml or less.

36. A binding molecule that binds to a spike protein (S protein) on a surface of coronavirus, wherein the binding molecule competes with any one binding molecule selected from the group consisting of the following binding molecules 1) to 190):
1) a binding molecule comprising a) a light-chain variable region comprising a CDR1 region of SEQ ID NO: 1, a CDR2 region of SEQ ID NO: 2, and a CDR3 region of SEQ ID NO: 3, and b) a heavy-chain variable region comprising a CDR1 region of SEQ ID NO: 4, a CDR2 region of SEQ ID NO: 5, and a CDR3 region of SEQ ID NO: 6;
2) a binding molecule comprising a) a light-chain variable region comprising a CDR1 region of SEQ ID NO: 7, a CDR2 region of SEQ ID NO: 8, and a CDR3 region of SEQ ID NO: 9, and b) a heavy-chain variable region comprising a CDR1 region of SEQ ID NO: 10, a CDR2 region of SEQ ID NO: 11, and a CDR3 region of SEQ ID NO: 12;
3) a binding molecule comprising a) a light-chain variable region comprising a CDR1 region of SEQ ID NO: 13, a CDR2 region of SEQ ID NO: 14, and a CDR3 region of SEQ ID NO: 15, and b) a heavy-chain variable region comprising a CDR1 region of SEQ ID NO: 16, a CDR2 region of SEQ ID NO: 17, and a CDR3 region of SEQ ID NO: 18;
4) a binding molecule comprising a) a light-chain variable region comprising a CDR1 region of SEQ ID NO: 19, a CDR2 region of SEQ ID NO: 20, and a CDR3 region of SEQ ID NO: 21, and b) a heavy-chain variable region comprising a CDR1 region of SEQ ID NO: 22, a CDR2 region of SEQ ID NO: 23, and a CDR3 region of SEQ ID NO: 24;
5) a binding molecule comprising a) a light-chain variable region comprising a CDR1 region of SEQ ID NO: 25, a CDR2 region of SEQ ID NO: 26, and a CDR3 region of SEQ ID NO: 27, and b) a heavy-chain variable region comprising a CDR1 region of SEQ ID NO: 28, a CDR2 region of SEQ ID NO: 29, and a CDR3 region of SEQ ID NO: 30;
6) a binding molecule comprising a) a light-chain variable region comprising a CDR1 region of SEQ ID NO: 31, a CDR2 region of SEQ ID NO: 32, and a CDR3 region of SEQ ID NO: 33, and b) a heavy-chain variable region comprising a CDR1 region of SEQ ID NO: 34, a CDR2 region of SEQ ID NO: 35, and a CDR3 region of SEQ ID NO: 36;
7) a binding molecule comprising a) a light-chain variable region comprising a CDR1 region of SEQ ID NO: 37, a CDR2 region of SEQ ID NO: 38, and a CDR3 region of SEQ ID NO: 39, and b) a heavy-chain variable region comprising a CDR1 region of SEQ ID NO: 40, a CDR2 region of SEQ ID NO: 41, and a CDR3 region of SEQ ID NO: 42;
8) a binding molecule comprising a) a light-chain variable region comprising a CDR1 region of SEQ ID NO: 43, a CDR2 region of SEQ ID NO: 44, and a CDR3 region of SEQ ID NO: 45, and b) a heavy-chain variable region comprising a CDR1 region of SEQ ID NO: 46, a CDR2 region of SEQ ID NO: 47, and a CDR3 region of SEQ ID NO: 48;
9) a binding molecule comprising a) a light-chain variable region comprising a CDR1 region of SEQ ID NO: 49, a CDR2 region of SEQ ID NO: 50, and a CDR3 region of SEQ ID NO: 51, and b) a heavy-chain variable region comprising a CDR1 region of SEQ ID NO: 52, a CDR2 region of SEQ ID NO: 53, and a CDR3 region of SEQ ID NO: 54;
10) a binding molecule comprising a) a light-chain variable region comprising a CDR1 region of SEQ ID NO: 55, a CDR2 region of SEQ ID NO: 56, and a CDR3 region of SEQ ID NO: 57, and b) a heavy-chain variable region comprising a CDR1 region of SEQ ID NO: 58, a CDR2 region of SEQ ID NO: 59, and a CDR3 region of SEQ ID NO: 60;
11) a binding molecule comprising a) a light-chain variable region comprising a CDR1 region of SEQ ID NO: 61, a CDR2 region of SEQ ID NO: 62, and a CDR3 region of SEQ ID NO: 63, and b) a heavy-chain variable region comprising a CDR1 region of SEQ ID NO: 64, a CDR2 region of SEQ ID NO: 65, and a CDR3 region of SEQ ID NO: 66;
12) a binding molecule comprising a) a light-chain variable region comprising a CDR1 region of SEQ ID NO: 67, a CDR2 region of SEQ ID NO: 68, and a CDR3 region of SEQ ID NO: 69, and b) a heavy-chain variable region comprising a CDR1 region of SEQ ID NO: 70, a CDR2 region of SEQ ID NO: 71, and a CDR3 region of SEQ ID NO: 72;
13) a binding molecule comprising a) a light-chain variable region comprising a CDR1 region of SEQ ID NO: 73, a CDR2 region of SEQ ID NO: 74, and a CDR3 region of SEQ ID NO: 75, and b) a heavy-chain variable region comprising a CDR1 region of SEQ ID NO: 76, a CDR2 region of SEQ ID NO: 77, and a CDR3 region of SEQ ID NO: 78;
14) a binding molecule comprising a) a light-chain variable region comprising a CDR1 region of SEQ ID NO: 79, a CDR2 region of SEQ ID NO: 80, and a CDR3 region of SEQ ID NO: 81, and b) a heavy-chain variable region comprising a CDR1 region of SEQ ID NO: 82, a CDR2 region of SEQ ID NO: 83, and a CDR3 region of SEQ ID NO: 84;
15) a binding molecule comprising a) a light-chain variable region comprising a CDR1 region of SEQ ID NO: 85, a CDR2 region of SEQ ID NO: 86, and a CDR3 region of SEQ ID NO: 87, and b) a heavy-chain variable region comprising a CDR1 region of SEQ ID NO: 88, a CDR2 region of SEQ ID NO: 89, and a CDR3 region of SEQ ID NO: 90;
16) a binding molecule comprising a) a light-chain variable region comprising a CDR1 region of SEQ ID NO: 91, a CDR2 region of SEQ ID NO: 92, and a CDR3 region of SEQ ID NO: 93, and b) a heavy-chain variable region comprising a CDR1 region of SEQ ID NO: 94, a CDR2 region of SEQ ID NO: 95, and a CDR3 region of SEQ ID NO: 96;
17) a binding molecule comprising a) a light-chain variable region comprising a CDR1 region of SEQ ID NO: 97, a CDR2 region of SEQ ID NO: 98, and a CDR3 region of SEQ ID NO: 99, and b) a heavy-chain variable region comprising a CDR1 region of SEQ ID NO: 100, a CDR2 region of SEQ ID NO: 101, and a CDR3 region of SEQ ID NO: 102;
18) a binding molecule comprising a) a light-chain variable region comprising a CDR1 region of SEQ ID NO: 103, a CDR2 region of SEQ ID NO: 104, and a CDR3 region of SEQ ID NO: 105, and b) a heavy-chain variable region comprising a CDR1 region of SEQ ID NO: 106, a CDR2 region of SEQ ID NO: 107, and a CDR3 region of SEQ ID NO: 108;
19) a binding molecule comprising a) a light-chain variable region comprising a CDR1 region of SEQ ID NO: 109, CDR2 region of SEQ ID NO: 110, and CDR3 region of SEQ ID NO: 111, and b) a heavy-chain variable region comprising a CDR1 region of SEQ ID NO: 112, a CDR2 region of SEQ ID NO: 113, and a CDR3 region of SEQ ID NO: 114;
20) a binding molecule comprising a) a light-chain variable region comprising a CDR1 region of SEQ ID NO: 115, a CDR2 region of SEQ ID NO: 116, and a CDR3 region of SEQ ID NO: 117, and b) a heavy-chain variable region comprising a CDR1 region of SEQ ID NO: 118, a CDR2 region of SEQ ID NO: 119, and a CDR3 region of SEQ ID NO: 120;
21) a binding molecule comprising a) a light-chain variable region comprising a CDR1 region of SEQ ID NO: 121, a CDR2 region of SEQ ID NO: 122, and a CDR3 region of SEQ ID NO: 123, and b) a heavy-chain variable region comprising a CDR1 region of SEQ ID NO: 124, a CDR2 region of SEQ ID NO: 125, and a CDR3 region of SEQ ID NO: 126;
22) a binding molecule comprising a) a light-chain variable region comprising a CDR1 region of SEQ ID NO: 127, a CDR2 region of SEQ ID NO: 128, and a CDR3 region of SEQ ID NO: 129, and b) a heavy-chain variable region comprising a CDR1 region of SEQ ID NO: 130, a CDR2 region of SEQ ID NO: 131, and a CDR3 region of SEQ ID NO: 132;
23) a binding molecule comprising a) a light-chain variable region comprising a CDR1 region of SEQ ID NO: 133, a CDR2 region of SEQ ID NO: 134, and a CDR3 region of SEQ ID NO: 135, and b) a heavy-chain variable region comprising a CDR1 region of SEQ ID NO: 136, a CDR2 region of SEQ ID NO: 137, and a CDR3 region of SEQ ID NO: 13 8;
24) a binding molecule comprising a) a light-chain variable region comprising a CDR1 region of SEQ ID NO: 139, a CDR2 region of SEQ ID NO: 140, and a CDR3 region of SEQ ID NO: 141, and b) a heavy-chain variable region comprising a CDR1 region of SEQ ID NO: 142, a CDR2 region of SEQ ID NO: 143, and a CDR3 region of SEQ ID NO: 144;
25) a binding molecule comprising a) a light-chain variable region comprising a CDR1 region of SEQ ID NO: 145, a CDR2 region of SEQ ID NO: 146, and a CDR3 region of SEQ ID NO: 147, and b) a heavy-chain variable region comprising a CDR1 region of SEQ ID NO: 148, a CDR2 region of SEQ ID NO: 149, and a CDR3 region of SEQ ID NO: 150;
26) a binding molecule comprising a) a light-chain variable region comprising a CDR1 region of SEQ ID NO: 151, a CDR2 region of SEQ ID NO: 152, and a CDR3 region of SEQ ID NO: 153, and b) a heavy-chain variable region comprising a CDR1 region of SEQ ID NO: 154, a CDR2 region of SEQ ID NO: 155, and a CDR3 region of SEQ ID NO: 156;
27) a binding molecule comprising a) a light-chain variable region comprising a CDR1 region of SEQ ID NO: 157, a CDR2 region of SEQ ID NO: 158, and a CDR3 region of SEQ ID NO: 159, and b) a heavy-chain variable region comprising a CDR1 region of SEQ ID NO: 160, a CDR2 region of SEQ ID NO: 161, and a CDR3 region of SEQ ID NO: 162;
28) a binding molecule comprising a) a light-chain variable region comprising a CDR1 region of SEQ ID NO: 163, a CDR2 region of SEQ ID NO: 164, and a CDR3 region of SEQ ID NO: 165, and b) a heavy-chain variable region comprising a CDR1 region of SEQ ID NO: 166, a CDR2 region of SEQ ID NO: 167, and a CDR3 region of SEQ ID NO: 168;
29) a binding molecule comprising a) a light-chain variable region comprising a CDR1 region of SEQ ID NO: 169, a CDR2 region of SEQ ID NO: 170, and a CDR3 region of SEQ ID NO: 171, and b) a heavy-chain variable region comprising a CDR1 region of SEQ ID NO: 172, a CDR2 region of SEQ ID NO: 173, and a CDR3 region of SEQ ID NO: 174;
30) a binding molecule comprising a) a light-chain variable region comprising a CDR1 region of SEQ ID NO: 175, a CDR2 region of SEQ ID NO: 176, and a CDR3 region of SEQ ID NO: 177, and b) a heavy-chain variable region comprising a CDR1 region of SEQ ID NO: 178, a CDR2 region of SEQ ID NO: 179, and a CDR3 region of SEQ ID NO: 180;
31) a binding molecule comprising a) a light-chain variable region comprising a CDR1 region of SEQ ID NO: 181, a CDR2 region of SEQ ID NO: 182, and a CDR3 region of SEQ ID NO: 183, and b) a heavy-chain variable region comprising a CDR1 region of SEQ ID NO: 184, a CDR2 region of SEQ ID NO: 185, and a CDR3 region of SEQ ID NO: 186;
32) a binding molecule comprising a) a light-chain variable region comprising a CDR1 region of SEQ ID NO: 187, a CDR2 region of SEQ ID NO: 188, and a CDR3 region of SEQ ID NO: 189, and b) a heavy-chain variable region comprising a CDR1 region of SEQ ID NO: 190, a CDR2 region of SEQ ID NO: 191, and a CDR3 region of SEQ ID NO: 192;
33) a binding molecule comprising a) a light-chain variable region comprising a CDR1 region of SEQ ID NO: 193, a CDR2 region of SEQ ID NO: 194, and a CDR3 region of SEQ ID NO: 195, and b) a heavy-chain variable region comprising a CDR1 region of SEQ ID NO: 196, a CDR2 region of SEQ ID NO: 197, and a CDR3 region of SEQ ID NO: 198;
34) a binding molecule comprising a) a light-chain variable region comprising a CDR1 region of SEQ ID NO: 199, a CDR2 region of SEQ ID NO: 200, and a CDR3 region of SEQ ID NO: 201, and b) a heavy-chain variable region comprising a CDR1 region of SEQ ID NO: 202, a CDR2 region of SEQ ID NO: 203, and a CDR3 region of SEQ ID NO: 204;
35) a binding molecule comprising a) a light-chain variable region comprising a CDR1 region of SEQ ID NO: 205, a CDR2 region of SEQ ID NO: 206, and a CDR3 region of SEQ ID NO: 207, and b) a heavy-chain variable region comprising a CDR1 region of SEQ ID NO: 208, a CDR2 region of SEQ ID NO: 209, and a CDR3 region of SEQ ID NO: 210;
36) a binding molecule comprising a) a light-chain variable region comprising a CDR1 region of SEQ ID NO: 211, a CDR2 region of SEQ ID NO: 212, and a CDR3 region of SEQ ID NO: 213, and b) a heavy-chain variable region comprising a CDR1 region of SEQ ID NO: 214, a CDR2 region of SEQ ID NO: 215, and a CDR3 region of SEQ ID NO: 216;
37) a binding molecule comprising a) a light-chain variable region comprising a CDR1 region of SEQ ID NO: 217, a CDR2 region of SEQ ID NO: 218, and a CDR3 region of SEQ ID NO: 219, and b) a heavy-chain variable region comprising a CDR1 region of SEQ ID NO: 220, a CDR2 region of SEQ ID NO: 221, and a CDR3 region of SEQ ID NO: 222;
38) a binding molecule comprising a) a light-chain variable region comprising a CDR1 region of SEQ ID NO: 223, a CDR2 region of SEQ ID NO: 224, and a CDR3 region of SEQ ID NO: 225, and b) a heavy-chain variable region comprising a CDR1 region of SEQ ID NO: 226, a CDR2 region of SEQ ID NO: 227, and a CDR3 region of SEQ ID NO: 228;
39) a binding molecule comprising a) a light-chain variable region comprising a CDR1 region of SEQ ID NO: 229, a CDR2 region of SEQ ID NO: 230, and a CDR3 region of SEQ ID NO: 231, and b) a heavy-chain variable region comprising a CDR1 region of SEQ ID NO: 232, a CDR2 region of SEQ ID NO: 233, and a CDR3 region of SEQ ID NO: 234;
40) a binding molecule comprising a) a light-chain variable region comprising a CDR1 region of SEQ ID NO: 235, a CDR2 region of SEQ ID NO: 236, and a CDR3 region of SEQ ID NO: 237, and b) a heavy-chain variable region comprising a CDR1 region of SEQ ID NO: 238, a CDR2 region of SEQ ID NO: 239, and a CDR3 region of SEQ ID NO: 240;
41) a binding molecule comprising a) a light-chain variable region comprising a CDR1 region of SEQ ID NO: 241, a CDR2 region of SEQ ID NO: 242, and a CDR3 region of SEQ ID NO: 243, and b) a heavy-chain variable region comprising a CDR1 region of SEQ ID NO: 244, a CDR2 region of SEQ ID NO: 245, and a CDR3 region of SEQ ID NO: 246;
42) a binding molecule comprising a) a light-chain variable region comprising a CDR1 region of SEQ ID NO: 247, a CDR2 region of SEQ ID NO: 248, and a CDR3 region of SEQ ID NO: 249, and b) a heavy-chain variable region comprising a CDR1 region of SEQ ID NO: 250, a CDR2 region of SEQ ID NO: 251, and a CDR3 region of SEQ ID NO: 252;
43) a binding molecule comprising a) a light-chain variable region comprising a CDR1 region of SEQ ID NO: 253, a CDR2 region of SEQ ID NO: 254, and a CDR3 region of SEQ ID NO: 255, and b) a heavy-chain variable region comprising a CDR1 region of SEQ ID NO: 256, a CDR2 region of SEQ ID NO: 257, and a CDR3 region of SEQ ID NO: 258;
44) a binding molecule comprising a) a light-chain variable region comprising a CDR1 region of SEQ ID NO: 259, a CDR2 region of SEQ ID NO: 260, and a CDR3 region of SEQ ID NO: 261, and b) a heavy-chain variable region comprising a CDR1 region of SEQ ID NO: 262, a CDR2 region of SEQ ID NO: 263, and a CDR3 region of SEQ ID NO: 264;
45) a binding molecule comprising a) a light-chain variable region comprising a CDR1 region of SEQ ID NO: 265, a CDR2 region of SEQ ID NO: 266, and a CDR3 region of SEQ ID NO: 267, and b) a heavy-chain variable region comprising a CDR1 region of SEQ ID NO: 268, a CDR2 region of SEQ ID NO: 269, and a CDR3 region of SEQ ID NO: 270;
46) a binding molecule comprising a) a light-chain variable region comprising a CDR1 region of SEQ ID NO: 271, a CDR2 region of SEQ ID NO: 272, and a CDR3 region of SEQ ID NO: 273, and b) a heavy-chain variable region comprising a CDR1 region of SEQ ID NO: 274, a CDR2 region of SEQ ID NO: 275, and a CDR3 region of SEQ ID NO: 276;
47) a binding molecule comprising a) a light-chain variable region comprising a CDR1 region of SEQ ID NO: 277, a CDR2 region of SEQ ID NO: 278, and a CDR3 region of SEQ ID NO: 279, and b) a heavy-chain variable region comprising a CDR1 region of SEQ ID NO: 280, a CDR2 region of SEQ ID NO: 281, and a CDR3 region of SEQ ID NO: 282;
48) a binding molecule comprising a) a light-chain variable region comprising a CDR1 region of SEQ ID NO: 283, a CDR2 region of SEQ ID NO: 284, and a CDR3 region of SEQ ID NO: 285, and b) a heavy-chain variable region comprising a CDR1 region of SEQ ID NO: 286, a CDR2 region of SEQ ID NO: 287, and a CDR3 region of SEQ ID NO: 288;
49) a binding molecule comprising a) a light-chain variable region comprising a CDR1 region of SEQ ID NO: 289, a CDR2 region of SEQ ID NO: 290, and a CDR3 region of SEQ ID NO: 291, and b) a heavy-chain variable region comprising a CDR1 region of SEQ ID NO: 292, a CDR2 region of SEQ ID NO: 293, and a CDR3 region of SEQ ID NO: 294;
50) a binding molecule comprising a) a light-chain variable region comprising a CDR1 region of SEQ ID NO: 295, a CDR2 region of SEQ ID NO: 296, and a CDR3 region of SEQ ID NO: 297, and b) a heavy-chain variable region comprising a CDR1 region of SEQ ID NO: 298, a CDR2 region of SEQ ID NO: 299, and a CDR3 region of SEQ ID NO: 300;
51) a binding molecule comprising a) a light-chain variable region comprising a CDR1 region of SEQ ID NO: 301, a CDR2 region of SEQ ID NO: 302, and a CDR3 region of SEQ ID NO: 303, and b) a heavy-chain variable region comprising a CDR1 region of SEQ ID NO: 304, a CDR2 region of SEQ ID NO: 305, and a CDR3 region of SEQ ID NO: 306;
52) a binding molecule comprising a) a light-chain variable region comprising a CDR1 region of SEQ ID NO: 307, a CDR2 region of SEQ ID NO: 308, and a CDR3 region of SEQ ID NO: 309, and b) a heavy-chain variable region comprising a CDR1 region of SEQ ID NO: 310, a CDR2 region of SEQ ID NO: 311, and a CDR3 region of SEQ ID NO: 312;
53) a binding molecule comprising a) a light-chain variable region comprising a CDR1 region of SEQ ID NO: 313, a CDR2 region of SEQ ID NO: 314, and a CDR3 region of SEQ ID NO: 315, and b) a heavy-chain variable region comprising a CDR1 region of SEQ ID NO: 316, a CDR2 region of SEQ ID NO: 317, and a CDR3 region of SEQ ID NO: 318;
54) a binding molecule comprising a) a light-chain variable region comprising a CDR1 region of SEQ ID NO: 319, a CDR2 region of SEQ ID NO: 320, and a CDR3 region of SEQ ID NO: 321, and b) a heavy-chain variable region comprising a CDR1 region of SEQ ID NO: 322, a CDR2 region of SEQ ID NO: 323, and a CDR3 region of SEQ ID NO: 324;
55) a binding molecule comprising a) a light-chain variable region comprising a CDR1 region of SEQ ID NO: 325, a CDR2 region of SEQ ID NO: 326, and a CDR3 region of SEQ ID NO: 327, and b) a heavy-chain variable region comprising a CDR1 region of SEQ ID NO: 328, a CDR2 region of SEQ ID NO: 329, and a CDR3 region of SEQ ID NO: 330;
56) a binding molecule comprising a) a light-chain variable region comprising a CDR1 region of SEQ ID NO: 331, a CDR2 region of SEQ ID NO: 332, and a CDR3 region of SEQ ID NO: 333, and b) a heavy-chain variable region comprising a CDR1 region of SEQ ID NO: 334, a CDR2 region of SEQ ID NO: 335, and a CDR3 region of SEQ ID NO: 336;
57) a binding molecule comprising a) a light-chain variable region comprising a CDR1 region of SEQ ID NO: 337, a CDR2 region of SEQ ID NO: 338, and a CDR3 region of SEQ ID NO: 339, and b) a heavy-chain variable region comprising a CDR1 region of SEQ ID NO: 340, a CDR2 region of SEQ ID NO: 341, and a CDR3 region of SEQ ID NO: 342;
58) a binding molecule comprising a) a light-chain variable region comprising a CDR1 region of SEQ ID NO: 343, a CDR2 region of SEQ ID NO: 344, and a CDR3 region of SEQ ID NO: 345, and b) a heavy-chain variable region comprising a CDR1 region of SEQ ID NO: 346, a CDR2 region of SEQ ID NO: 347, and a CDR3 region of SEQ ID NO: 348;
59) a binding molecule comprising a) a light-chain variable region comprising a CDR1 region of SEQ ID NO: 349, a CDR2 region of SEQ ID NO: 350, and a CDR3 region of SEQ ID NO: 351, and b) a heavy-chain variable region comprising a CDR1 region of SEQ ID NO: 352, a CDR2 region of SEQ ID NO: 353, and a CDR3 region of SEQ ID NO: 354;
60) a binding molecule comprising a) a light-chain variable region comprising a CDR1 region of SEQ ID NO: 355, a CDR2 region of SEQ ID NO: 356, and a CDR3 region of SEQ ID NO: 357, and b) a heavy-chain variable region comprising a CDR1 region of SEQ ID NO: 358, a CDR2 region of SEQ ID NO: 359, and a CDR3 region of SEQ ID NO: 360;
61) a binding molecule comprising a) a light-chain variable region comprising a CDR1 region of SEQ ID NO: 361, a CDR2 region of SEQ ID NO: 362, and a CDR3 region of SEQ ID NO: 363, and b) a heavy-chain variable region comprising a CDR1 region of SEQ ID NO: 364, a CDR2 region of SEQ ID NO: 365, and a CDR3 region of SEQ ID NO: 366;
62) a binding molecule comprising a) a light-chain variable region comprising a CDR1 region of SEQ ID NO: 367, a CDR2 region of SEQ ID NO: 368, and a CDR3 region of SEQ ID NO: 369, and b) a heavy-chain variable region comprising a CDR1 region of SEQ ID NO: 370, a CDR2 region of SEQ ID NO: 371, and a CDR3 region of SEQ ID NO: 372;
63) a binding molecule comprising a) a light-chain variable region comprising a CDR1 region of SEQ ID NO: 373, a CDR2 region of SEQ ID NO: 374, and a CDR3 region of SEQ ID NO: 375, and b) a heavy-chain variable region comprising a CDR1 region of SEQ ID NO: 376, a CDR2 region of SEQ ID NO: 377, and a CDR3 region of SEQ ID NO: 378;
64) a binding molecule comprising a) a light-chain variable region comprising a CDR1 region of SEQ ID NO: 379, a CDR2 region of SEQ ID NO: 380, and a CDR3 region of SEQ ID NO: 381, and b) a heavy-chain variable region comprising a CDR1 region of SEQ ID NO: 382, a CDR2 region of SEQ ID NO: 383, and a CDR3 region of SEQ ID NO: 384;
65) a binding molecule comprising a) a light-chain variable region comprising a CDR1 region of SEQ ID NO: 385, a CDR2 region of SEQ ID NO: 386, and a CDR3 region of SEQ ID NO: 387, and b) a heavy-chain variable region comprising a CDR1 region of SEQ ID NO: 388, a CDR2 region of SEQ ID NO: 389, and a CDR3 region of SEQ ID NO: 390;
66) a binding molecule comprising a) a light-chain variable region comprising a CDR1 region of SEQ ID NO: 391, a CDR2 region of SEQ ID NO: 392, and a CDR3 region of SEQ ID NO: 393, and b) a heavy-chain variable region comprising a CDR1 region of SEQ ID NO: 394, a CDR2 region of SEQ ID NO: 395, and a CDR3 region of SEQ ID NO: 396;
67) a binding molecule comprising a) a light-chain variable region comprising a CDR1 region of SEQ ID NO: 397, a CDR2 region of SEQ ID NO: 398, and a CDR3 region of SEQ ID NO: 399, and b) a heavy-chain variable region comprising a CDR1 region of SEQ ID NO: 400, a CDR2 region of SEQ ID NO: 401, and a CDR3 region of SEQ ID NO: 402;
68) a binding molecule comprising a) a light-chain variable region comprising a CDR1 region of SEQ ID NO: 403, a CDR2 region of SEQ ID NO: 404, and a CDR3 region of SEQ ID NO: 405, and b) a heavy-chain variable region comprising a CDR1 region of SEQ ID NO: 406, a CDR2 region of SEQ ID NO: 407, and a CDR3 region of SEQ ID NO: 408;
69) a binding molecule comprising a) a light-chain variable region comprising a CDR1 region of SEQ ID NO: 409, a CDR2 region of SEQ ID NO: 410, and a CDR3 region of SEQ ID NO: 411, and b) a heavy-chain variable region comprising a CDR1 region of SEQ ID NO: 412, a CDR2 region of SEQ ID NO: 413, and a CDR3 region of SEQ ID NO: 414;
70) a binding molecule comprising a) a light-chain variable region comprising a CDR1 region of SEQ ID NO: 415, a CDR2 region of SEQ ID NO: 416, and a CDR3 region of SEQ ID NO: 417, and b) a heavy-chain variable region comprising a CDR1 region of SEQ ID NO: 418, a CDR2 region of SEQ ID NO: 419, and a CDR3 region of SEQ ID NO: 420;
71) a binding molecule comprising a) a light-chain variable region comprising a CDR1 region of SEQ ID NO: 421, a CDR2 region of SEQ ID NO: 422, and a CDR3 region of SEQ ID NO: 423, and b) a heavy-chain variable region comprising a CDR1 region of SEQ ID NO: 424, a CDR2 region of SEQ ID NO: 425, and a CDR3 region of SEQ ID NO: 426;
72) a binding molecule comprising a) a light-chain variable region comprising a CDR1 region of SEQ ID NO: 427, a CDR2 region of SEQ ID NO: 428, and a CDR3 region of SEQ ID NO: 429, and b) a heavy-chain variable region comprising a CDR1 region of SEQ ID NO: 430, a CDR2 region of SEQ ID NO: 431, and a CDR3 region of SEQ ID NO: 432;
73) a binding molecule comprising a) a light-chain variable region comprising a CDR1 region of SEQ ID NO: 433, a CDR2 region of SEQ ID NO: 434, and a CDR3 region of SEQ ID NO: 435, and b) a heavy-chain variable region comprising a CDR1 region of SEQ ID NO: 436, a CDR2 region of SEQ ID NO: 437, and a CDR3 region of SEQ ID NO: 438;
74) a binding molecule comprising a) a light-chain variable region comprising a CDR1 region of SEQ ID NO: 439, a CDR2 region of SEQ ID NO: 440, and a CDR3 region of SEQ ID NO: 441, and b) a heavy-chain variable region comprising a CDR1 region of SEQ ID NO: 442, a CDR2 region of SEQ ID NO: 443, and a CDR3 region of SEQ ID NO: 444;
75) a binding molecule comprising a) a light-chain variable region comprising a CDR1 region of SEQ ID NO: 445, a CDR2 region of SEQ ID NO: 446, and a CDR3 region of SEQ ID NO: 447, and b) a heavy-chain variable region comprising a CDR1 region of SEQ ID NO: 448, a CDR2 region of SEQ ID NO: 449, and a CDR3 region of SEQ ID NO: 450;
76) a binding molecule comprising a) a light-chain variable region comprising a CDR1 region of SEQ ID NO: 451, a CDR2 region of SEQ ID NO: 452, and a CDR3 region of SEQ ID NO: 453, and b) a heavy-chain variable region comprising a CDR1 region of SEQ ID NO: 454, a CDR2 region of SEQ ID NO: 455, and a CDR3 region of SEQ ID NO: 456;
77) a binding molecule comprising a) a light-chain variable region comprising a CDR1 region of SEQ ID NO: 457, a CDR2 region of SEQ ID NO: 458, and a CDR3 region of SEQ ID NO: 459, and b) a heavy-chain variable region comprising a CDR1 region of SEQ ID NO: 460, a CDR2 region of SEQ ID NO: 461, and a CDR3 region of SEQ ID NO: 462;
78) a binding molecule comprising a) a light-chain variable region comprising a CDR1 region of SEQ ID NO: 463, a CDR2 region of SEQ ID NO: 464, and a CDR3 region of SEQ ID NO: 465, and b) a heavy-chain variable region comprising a CDR1 region of SEQ ID NO: 466, a CDR2 region of SEQ ID NO: 467, and a CDR3 region of SEQ ID NO: 468;
79) a binding molecule comprising a) a light-chain variable region comprising a CDR1 region of SEQ ID NO: 469, a CDR2 region of SEQ ID NO: 470, and a CDR3 region of SEQ ID NO: 471, and b) a heavy-chain variable region comprising a CDR1 region of SEQ ID NO: 472, a CDR2 region of SEQ ID NO: 473, and a CDR3 region of SEQ ID NO: 474;
80) a binding molecule comprising a) a light-chain variable region comprising a CDR1 region of SEQ ID NO: 475, a CDR2 region of SEQ ID NO: 476, and a CDR3 region of SEQ ID NO: 477, and b) a heavy-chain variable region comprising a CDR1 region of SEQ ID NO: 478, a CDR2 region of SEQ ID NO: 479, and a CDR3 region of SEQ ID NO: 480;
81) a binding molecule comprising a) a light-chain variable region comprising a CDR1 region of SEQ ID NO: 481, a CDR2 region of SEQ ID NO: 482, and a CDR3 region of SEQ ID NO: 483, and b) a heavy-chain variable region comprising a CDR1 region of SEQ ID NO: 484, a CDR2 region of SEQ ID NO: 485, and a CDR3 region of SEQ ID NO: 486;
82) a binding molecule comprising a) a light-chain variable region comprising a CDR1 region of SEQ ID NO: 487, a CDR2 region of SEQ ID NO: 488, and a CDR3 region of SEQ ID NO: 489, and b) a heavy-chain variable region comprising a CDR1 region of SEQ ID NO: 490, a CDR2 region of SEQ ID NO: 491, and a CDR3 region of SEQ ID NO: 492;
83) a binding molecule comprising a) a light-chain variable region comprising a CDR1 region of SEQ ID NO: 493, a CDR2 region of SEQ ID NO: 494, and a CDR3 region of SEQ ID NO: 495, and b) a heavy-chain variable region comprising a CDR1 region of SEQ ID NO: 496, a CDR2 region of SEQ ID NO: 497, and a CDR3 region of SEQ ID NO: 498;
84) a binding molecule comprising a) a light-chain variable region comprising a CDR1 region of SEQ ID NO: 499, a CDR2 region of SEQ ID NO: 500, and a CDR3 region of SEQ ID NO: 501, and b) a heavy-chain variable region comprising a CDR1 region of SEQ ID NO: 502, a CDR2 region of SEQ ID NO: 503, and a CDR3 region of SEQ ID NO: 504;
85) a binding molecule comprising a) a light-chain variable region comprising a CDR1 region of SEQ ID NO: 505, a CDR2 region of SEQ ID NO: 506, and a CDR3 region of SEQ ID NO: 507, and b) a heavy-chain variable region comprising a CDR1 region of SEQ ID NO: 508, a CDR2 region of SEQ ID NO: 509, and a CDR3 region of SEQ ID NO: 510;
86) a binding molecule comprising a) a light-chain variable region comprising a CDR1 region of SEQ ID NO: 511, a CDR2 region of SEQ ID NO: 512, and a CDR3 region of SEQ ID NO: 513, and b) a heavy-chain variable region comprising a CDR1 region of SEQ ID NO: 514, a CDR2 region of SEQ ID NO: 515, and a CDR3 region of SEQ ID NO: 516;
87) a binding molecule comprising a) a light-chain variable region comprising a CDR1 region of SEQ ID NO: 517, a CDR2 region of SEQ ID NO: 518, and a CDR3 region of SEQ ID NO: 519, and b) a heavy-chain variable region comprising a CDR1 region of SEQ ID NO: 520, a CDR2 region of SEQ ID NO: 521, and a CDR3 region of SEQ ID NO: 522;
88) a binding molecule comprising a) a light-chain variable region comprising a CDR1 region of SEQ ID NO: 523, a CDR2 region of SEQ ID NO: 524, and a CDR3 region of SEQ ID NO: 525, and b) a heavy-chain variable region comprising a CDR1 region of SEQ ID NO: 526, a CDR2 region of SEQ ID NO: 527, and a CDR3 region of SEQ ID NO: 528;
89) a binding molecule comprising a) a light-chain variable region comprising a CDR1 region of SEQ ID NO: 529, a CDR2 region of SEQ ID NO: 530, and a CDR3 region of SEQ ID NO: 531, and b) a heavy-chain variable region comprising a CDR1 region of SEQ ID NO: 532, a CDR2 region of SEQ ID NO: 533, and a CDR3 region of SEQ ID NO: 534;
90) a binding molecule comprising a) a light-chain variable region comprising a CDR1 region of SEQ ID NO: 535, a CDR2 region of SEQ ID NO: 536, and a CDR3 region of SEQ ID NO: 537, and b) a heavy-chain variable region comprising a CDR1 region of SEQ ID NO: 538, a CDR2 region of SEQ ID NO: 539, and a CDR3 region of SEQ ID NO: 540;
91) a binding molecule comprising a) a light-chain variable region comprising a CDR1 region of SEQ ID NO: 541, a CDR2 region of SEQ ID NO: 542, and a CDR3 region of SEQ ID NO: 543, and b) a heavy-chain variable region comprising a CDR1 region of SEQ ID NO: 544, a CDR2 region of SEQ ID NO: 545, and a CDR3 region of SEQ ID NO: 546;
92) a binding molecule comprising a) a light-chain variable region comprising a CDR1 region of SEQ ID NO: 547, a CDR2 region of SEQ ID NO: 548, and a CDR3 region of SEQ ID NO: 549, and b) a heavy-chain variable region comprising a CDR1 region of SEQ ID NO: 550, a CDR2 region of SEQ ID NO: 551, and a CDR3 region of SEQ ID NO: 552;
93) a binding molecule comprising a) a light-chain variable region comprising a CDR1 region of SEQ ID NO: 553, a CDR2 region of SEQ ID NO: 554, and a CDR3 region of SEQ ID NO: 555, and b) a heavy-chain variable region comprising a CDR1 region of SEQ ID NO: 556, a CDR2 region of SEQ ID NO: 557, and a CDR3 region of SEQ ID NO: 558;
94) a binding molecule comprising a) a light-chain variable region comprising a CDR1 region of SEQ ID NO: 559, a CDR2 region of SEQ ID NO: 560, and a CDR3 region of SEQ ID NO: 561, and b) a heavy-chain variable region comprising a CDR1 region of SEQ ID NO: 562, a CDR2 region of SEQ ID NO: 563, and a CDR3 region of SEQ ID NO: 564;
95) a binding molecule comprising a) a light-chain variable region comprising a CDR1 region of SEQ ID NO: 565, a CDR2 region of SEQ ID NO: 566, and a CDR3 region of SEQ ID NO: 567, and b) a heavy-chain variable region comprising a CDR1 region of SEQ ID NO: 568, a CDR2 region of SEQ ID NO: 569, and a CDR3 region of SEQ ID NO: 570;
96) a binding molecule comprising a) a light-chain variable region comprising a CDR1 region of SEQ ID NO: 571, a CDR2 region of SEQ ID NO: 572, and a CDR3 region of SEQ ID NO: 573, and b) a heavy-chain variable region comprising a CDR1 region of SEQ ID NO: 574, a CDR2 region of SEQ ID NO: 575, and a CDR3 region of SEQ ID NO: 576;
97) a binding molecule comprising a) a light-chain variable region comprising a CDR1 region of SEQ ID NO: 577, a CDR2 region of SEQ ID NO: 578, and a CDR3 region of SEQ ID NO: 579, and b) a heavy-chain variable region comprising a CDR1 region of SEQ ID NO: 580, a CDR2 region of SEQ ID NO: 581, and a CDR3 region of SEQ ID NO: 582;
98) a binding molecule comprising a) a light-chain variable region comprising a CDR1 region of SEQ ID NO: 583, a CDR2 region of SEQ ID NO: 584, and a CDR3 region of SEQ ID NO: 585, and b) a heavy-chain variable region comprising a CDR1 region of SEQ ID NO: 586, a CDR2 region of SEQ ID NO: 587, and a CDR3 region of SEQ ID NO: 588;
99) a binding molecule comprising a) a light-chain variable region comprising a CDR1 region of SEQ ID NO: 589, a CDR2 region of SEQ ID NO: 590, and a CDR3 region of SEQ ID NO: 591, and b) a heavy-chain variable region comprising a CDR1 region of SEQ ID NO: 592, a CDR2 region of SEQ ID NO: 593, and a CDR3 region of SEQ ID NO: 594;
100) a binding molecule comprising a) a light-chain variable region comprising a CDR1 region of SEQ ID NO: 595, a CDR2 region of SEQ ID NO: 596, and a CDR3 region of SEQ ID NO: 597, and b) a heavy-chain variable region comprising a CDR1 region of SEQ ID NO: 598, a CDR2 region of SEQ ID NO: 599, and a CDR3 region of SEQ ID NO: 600;
101) a binding molecule comprising a) a light-chain variable region comprising a CDR1 region of SEQ ID NO: 601, a CDR2 region of SEQ ID NO: 602, and a CDR3 region of SEQ ID NO: 603, and b) a heavy-chain variable region comprising a CDR1 region of SEQ ID NO: 604, a CDR2 region of SEQ ID NO: 605, and a CDR3 region of SEQ ID NO: 606;
102) a binding molecule comprising a) a light-chain variable region comprising a CDR1 region of SEQ ID NO: 607, a CDR2 region of SEQ ID NO: 608, and a CDR3 region of SEQ ID NO: 609, and b) a heavy-chain variable region comprising a CDR1 region of SEQ ID NO: 610, a CDR2 region of SEQ ID NO: 611, and a CDR3 region of SEQ ID NO: 612;
103) a binding molecule comprising a) a light-chain variable region comprising a CDR1 region of SEQ ID NO: 613, a CDR2 region of SEQ ID NO: 614, and a CDR3 region of SEQ ID NO: 615, and b) a heavy-chain variable region comprising a CDR1 region of SEQ ID NO: 616, a CDR2 region of SEQ ID NO: 617, and a CDR3 region of SEQ ID NO: 618;
104) a binding molecule comprising a) a light-chain variable region comprising a CDR1 region of SEQ ID NO: 619, a CDR2 region of SEQ ID NO: 620, and a CDR3 region of SEQ ID NO: 621, and b) a heavy-chain variable region comprising a CDR1 region of SEQ ID NO: 622, a CDR2 region of SEQ ID NO: 623, and a CDR3 region of SEQ ID NO: 624;
105) a binding molecule comprising a) a light-chain variable region comprising a CDR1 region of SEQ ID NO: 625, a CDR2 region of SEQ ID NO: 626, and a CDR3 region of SEQ ID NO: 627, and b) a heavy-chain variable region comprising a CDR1 region of SEQ ID NO: 628, a CDR2 region of SEQ ID NO: 629, and a CDR3 region of SEQ ID NO: 630;
106) a binding molecule comprising a) a light-chain variable region comprising a CDR1 region of SEQ ID NO: 631, a CDR2 region of SEQ ID NO: 632, and a CDR3 region of SEQ ID NO: 633, and b) a heavy-chain variable region comprising a CDR1 region of SEQ ID NO: 634, a CDR2 region of SEQ ID NO: 635, and a CDR3 region of SEQ ID NO: 636;
107) a binding molecule comprising a) a light-chain variable region comprising a CDR1 region of SEQ ID NO: 637, a CDR2 region of SEQ ID NO: 638, and a CDR3 region of SEQ ID NO: 639, and b) a heavy-chain variable region comprising a CDR1 region of SEQ ID NO: 640, a CDR2 region of SEQ ID NO: 641, and a CDR3 region of SEQ ID NO: 642;
108) a binding molecule comprising a) a light-chain variable region comprising a CDR1 region of SEQ ID NO: 643, a CDR2 region of SEQ ID NO: 644, and a CDR3 region of SEQ ID NO: 645, and b) a heavy-chain variable region comprising a CDR1 region of SEQ ID NO: 646, a CDR2 region of SEQ ID NO: 647, and a CDR3 region of SEQ ID NO: 648;
109) a binding molecule comprising a) a light-chain variable region comprising a CDR1 region of SEQ ID NO: 649, a CDR2 region of SEQ ID NO: 650, and a CDR3 region of SEQ ID NO: 651, and b) a heavy-chain variable region comprising a CDR1 region of SEQ ID NO: 652, a CDR2 region of SEQ ID NO: 653, and a CDR3 region of SEQ ID NO: 654;
110) a binding molecule comprising a) a light-chain variable region comprising a CDR1 region of SEQ ID NO: 655, a CDR2 region of SEQ ID NO: 656, and a CDR3 region of SEQ ID NO: 657, and b) a heavy-chain variable region comprising a CDR1 region of SEQ ID NO: 658, a CDR2 region of SEQ ID NO: 659, and a CDR3 region of SEQ ID NO: 660;
111) a binding molecule comprising a) a light-chain variable region comprising a CDR1 region of SEQ ID NO: 661, a CDR2 region of SEQ ID NO: 662, and a CDR3 region of SEQ ID NO: 663, and b) a heavy-chain variable region comprising a CDR1 region of SEQ ID NO: 664, a CDR2 region of SEQ ID NO: 665, and a CDR3 region of SEQ ID NO: 666;
112) a binding molecule comprising a) a light-chain variable region comprising a CDR1 region of SEQ ID NO: 667, a CDR2 region of SEQ ID NO: 668, and a CDR3 region of SEQ ID NO: 669, and b) a heavy-chain variable region comprising a CDR1 region of SEQ ID NO: 670, a CDR2 region of SEQ ID NO: 671, and a CDR3 region of SEQ ID NO: 672;
113) a binding molecule comprising a) a light-chain variable region comprising a CDR1 region of SEQ ID NO: 673, a CDR2 region of SEQ ID NO: 674, and a CDR3 region of SEQ ID NO: 675, and b) a heavy-chain variable region comprising a CDR1 region of SEQ ID NO: 676, a CDR2 region of SEQ ID NO: 677, and a CDR3 region of SEQ ID NO: 678;
114) a binding molecule comprising a) a light-chain variable region comprising a CDR1 region of SEQ ID NO: 679, a CDR2 region of SEQ ID NO: 680, and a CDR3 region of SEQ ID NO: 681, and b) a heavy-chain variable region comprising a CDR1 region of SEQ ID NO: 682, a CDR2 region of SEQ ID NO: 683, and a CDR3 region of SEQ ID NO: 684;
115) a binding molecule comprising a) a light-chain variable region comprising a CDR1 region of SEQ ID NO: 685, a CDR2 region of SEQ ID NO: 686, and a CDR3 region of SEQ ID NO: 687, and b) a heavy-chain variable region comprising a CDR1 region of SEQ ID NO: 688, a CDR2 region of SEQ ID NO: 689, and a CDR3 region of SEQ ID NO: 690;
116) a binding molecule comprising a) a light-chain variable region comprising a CDR1 region of SEQ ID NO: 691, a CDR2 region of SEQ ID NO: 692, and a CDR3 region of SEQ ID NO: 693, and b) a heavy-chain variable region comprising a CDR1 region of SEQ ID NO: 694, a CDR2 region of SEQ ID NO: 695, and a CDR3 region of SEQ ID NO: 696;
117) a binding molecule comprising a) a light-chain variable region comprising a CDR1 region of SEQ ID NO: 697, a CDR2 region of SEQ ID NO: 698, and a CDR3 region of SEQ ID NO: 699, and b) a heavy-chain variable region comprising a CDR1 region of SEQ ID NO: 700, a CDR2 region of SEQ ID NO: 701, and a CDR3 region of SEQ ID NO: 702;
118) a binding molecule comprising a) a light-chain variable region comprising a CDR1 region of SEQ ID NO: 703, a CDR2 region of SEQ ID NO: 704, and a CDR3 region of SEQ ID NO: 705, and b) a heavy-chain variable region comprising a CDR1 region of SEQ ID NO: 706, a CDR2 region of SEQ ID NO: 707, and a CDR3 region of SEQ ID NO: 708;
119) a binding molecule comprising a) a light-chain variable region comprising a CDR1 region of SEQ ID NO: 709, a CDR2 region of SEQ ID NO: 710, and a CDR3 region of SEQ ID NO: 711, and b) a heavy-chain variable region comprising a CDR1 region of SEQ ID NO: 712, a CDR2 region of SEQ ID NO: 713, and a CDR3 region of SEQ ID NO: 714;
120) a binding molecule comprising a) a light-chain variable region comprising a CDR1 region of SEQ ID NO: 715, a CDR2 region of SEQ ID NO: 716, and a CDR3 region of SEQ ID NO: 717, and b) a heavy-chain variable region comprising a CDR1 region of SEQ ID NO: 718, a CDR2 region of SEQ ID NO: 719, and a CDR3 region of SEQ ID NO: 720;
121) a binding molecule comprising a) a light-chain variable region comprising a CDR1 region of SEQ ID NO: 721, a CDR2 region of SEQ ID NO: 722, and a CDR3 region of SEQ ID NO: 723, and b) a heavy-chain variable region comprising a CDR1 region of SEQ ID NO: 724, a CDR2 region of SEQ ID NO: 725, and a CDR3 region of SEQ ID NO: 726;
122) a binding molecule comprising a) a light-chain variable region comprising a CDR1 region of SEQ ID NO: 727, a CDR2 region of SEQ ID NO: 728, and a CDR3 region of SEQ ID NO: 729, and b) a heavy-chain variable region comprising a CDR1 region of SEQ ID NO: 730, a CDR2 region of SEQ ID NO: 731, and a CDR3 region of SEQ ID NO: 732;
123) a binding molecule comprising a) a light-chain variable region comprising a CDR1 region of SEQ ID NO: 733, a CDR2 region of SEQ ID NO: 734, and a CDR3 region of SEQ ID NO: 735, and b) a heavy-chain variable region comprising a CDR1 region of SEQ ID NO: 736, a CDR2 region of SEQ ID NO: 737, and a CDR3 region of SEQ ID NO: 738;
124) a binding molecule comprising a) a light-chain variable region comprising a CDR1 region of SEQ ID NO: 739, a CDR2 region of SEQ ID NO: 740, and a CDR3 region of SEQ ID NO: 741, and b) a heavy-chain variable region comprising a CDR1 region of SEQ ID NO: 742, a CDR2 region of SEQ ID NO: 743, and a CDR3 region of SEQ ID NO: 744;
125) a binding molecule comprising a) a light-chain variable region comprising a CDR1 region of SEQ ID NO: 745, a CDR2 region of SEQ ID NO: 746, and a CDR3 region of SEQ ID NO: 747, and b) a heavy-chain variable region comprising a CDR1 region of SEQ ID NO: 748, a CDR2 region of SEQ ID NO: 749, and a CDR3 region of SEQ ID NO: 750;
126) a binding molecule comprising a) a light-chain variable region comprising a CDR1 region of SEQ ID NO: 751, a CDR2 region of SEQ ID NO: 752, and a CDR3 region of SEQ ID NO: 753, and b) a heavy-chain variable region comprising a CDR1 region of SEQ ID NO: 754, a CDR2 region of SEQ ID NO: 755, and a CDR3 region of SEQ ID NO: 756;
127) a binding molecule comprising a) a light-chain variable region comprising a CDR1 region of SEQ ID NO: 757, a CDR2 region of SEQ ID NO: 758, and a CDR3 region of SEQ ID NO: 759, and b) a heavy-chain variable region comprising a CDR1 region of SEQ ID NO: 760, a CDR2 region of SEQ ID NO: 761, and a CDR3 region of SEQ ID NO: 762;
128) a binding molecule comprising a) a light-chain variable region comprising a CDR1 region of SEQ ID NO: 763, a CDR2 region of SEQ ID NO: 764, and a CDR3 region of SEQ ID NO: 765, and b) a heavy-chain variable region comprising a CDR1 region of SEQ ID NO: 766, a CDR2 region of SEQ ID NO: 767, and a CDR3 region of SEQ ID NO: 768;
129) a binding molecule comprising a) a light-chain variable region comprising a CDR1 region of SEQ ID NO: 769, a CDR2 region of SEQ ID NO: 770, and a CDR3 region of SEQ ID NO: 771, and b) a heavy-chain variable region comprising a CDR1 region of SEQ ID NO: 772, a CDR2 region of SEQ ID NO: 773, and a CDR3 region of SEQ ID NO: 774;
130) a binding molecule comprising a) a light-chain variable region comprising a CDR1 region of SEQ ID NO: 775, a CDR2 region of SEQ ID NO: 776, and a CDR3 region of SEQ ID NO: 777, and b) a heavy-chain variable region comprising a CDR1 region of SEQ ID NO: 778, a CDR2 region of SEQ ID NO: 779, and a CDR3 region of SEQ ID NO: 780;
131) a binding molecule comprising a) a light-chain variable region comprising a CDR1 region of SEQ ID NO: 781, a CDR2 region of SEQ ID NO: 782, and a CDR3 region of SEQ ID NO: 783, and b) a heavy-chain variable region comprising a CDR1 region of SEQ ID NO: 784, a CDR2 region of SEQ ID NO: 785, and a CDR3 region of SEQ ID NO: 786;
132) a binding molecule comprising a) a light-chain variable region comprising a CDR1 region of SEQ ID NO: 787, a CDR2 region of SEQ ID NO: 788, and a CDR3 region of SEQ ID NO: 789, and b) a heavy-chain variable region comprising a CDR1 region of SEQ ID NO: 790, a CDR2 region of SEQ ID NO: 791, and a CDR3 region of SEQ ID NO: 792;
133) a binding molecule comprising a) a light-chain variable region comprising a CDR1 region of SEQ ID NO: 793, a CDR2 region of SEQ ID NO: 794, and a CDR3 region of SEQ ID NO: 795, and b) a heavy-chain variable region comprising a CDR1 region of SEQ ID NO: 796, a CDR2 region of SEQ ID NO: 797, and a CDR3 region of SEQ ID NO: 798;
134) a binding molecule comprising a) a light-chain variable region comprising a CDR1 region of SEQ ID NO: 799, a CDR2 region of SEQ ID NO: 800, and a CDR3 region of SEQ ID NO: 801, and b) a heavy-chain variable region comprising a CDR1 region of SEQ ID NO: 802, a CDR2 region of SEQ ID NO: 803, and a CDR3 region of SEQ ID NO: 804;
135) a binding molecule comprising a) a light-chain variable region comprising a CDR1 region of SEQ ID NO: 805, a CDR2 region of SEQ ID NO: 806, and a CDR3 region of SEQ ID NO: 807, and b) a heavy-chain variable region comprising a CDR1 region of SEQ ID NO: 808, a CDR2 region of SEQ ID NO: 809, and a CDR3 region of SEQ ID NO: 810;
136) a binding molecule comprising a) a light-chain variable region comprising a CDR1 region of SEQ ID NO: 811, a CDR2 region of SEQ ID NO: 812, and a CDR3 region of SEQ ID NO: 813, and b) a heavy-chain variable region comprising a CDR1 region of SEQ ID NO: 814, a CDR2 region of SEQ ID NO: 815, and a CDR3 region of SEQ ID NO: 816;
137) a binding molecule comprising a) a light-chain variable region comprising a CDR1 region of SEQ ID NO: 817, a CDR2 region of SEQ ID NO: 818, and a CDR3 region of SEQ ID NO: 819, and b) a heavy-chain variable region comprising a CDR1 region of SEQ ID NO: 820, a CDR2 region of SEQ ID NO: 821, and a CDR3 region of SEQ ID NO: 822;
138) a binding molecule comprising a) a light-chain variable region comprising a CDR1 region of SEQ ID NO: 823, a CDR2 region of SEQ ID NO: 824, and a CDR3 region of SEQ ID NO: 825, and b) a heavy-chain variable region comprising a CDR1 region of SEQ ID NO: 826, a CDR2 region of SEQ ID NO: 827, and a CDR3 region of SEQ ID NO: 828;
139) a binding molecule comprising a) a light-chain variable region comprising a CDR1 region of SEQ ID NO: 829, a CDR2 region of SEQ ID NO: 830, and a CDR3 region of SEQ ID NO: 831, and b) a heavy-chain variable region comprising a CDR1 region of SEQ ID NO: 832, a CDR2 region of SEQ ID NO: 833, and a CDR3 region of SEQ ID NO: 834;
140) a binding molecule comprising a) a light-chain variable region comprising a CDR1 region of SEQ ID NO: 835, a CDR2 region of SEQ ID NO: 836, and a CDR3 region of SEQ ID NO: 837, and b) a heavy-chain variable region comprising a CDR1 region of SEQ ID NO: 838, a CDR2 region of SEQ ID NO: 839, and a CDR3 region of SEQ ID NO: 840;
141) a binding molecule comprising a) a light-chain variable region comprising a CDR1 region of SEQ ID NO: 841, a CDR2 region of SEQ ID NO: 842, and a CDR3 region of SEQ ID NO: 843, and b) a heavy-chain variable region comprising a CDR1 region of SEQ ID NO: 844, a CDR2 region of SEQ ID NO: 845, and a CDR3 region of SEQ ID NO: 846;
142) a binding molecule comprising a) a light-chain variable region comprising a CDR1 region of SEQ ID NO: 847, a CDR2 region of SEQ ID NO: 848, and a CDR3 region of SEQ ID NO: 849, and b) a heavy-chain variable region comprising a CDR1 region of SEQ ID NO: 850, a CDR2 region of SEQ ID NO: 851, and a CDR3 region of SEQ ID NO: 852;
143) a binding molecule comprising a) a light-chain variable region comprising a CDR1 region of SEQ ID NO: 853, a CDR2 region of SEQ ID NO: 854, and a CDR3 region of SEQ ID NO: 855, and b) a heavy-chain variable region comprising a CDR1 region of SEQ ID NO: 856, a CDR2 region of SEQ ID NO: 857, and a CDR3 region of SEQ ID NO: 858;
144) a binding molecule comprising a) a light-chain variable region comprising a CDR1 region of SEQ ID NO: 859, a CDR2 region of SEQ ID NO: 860, and a CDR3 region of SEQ ID NO: 861, and b) a heavy-chain variable region comprising a CDR1 region of SEQ ID NO: 862, a CDR2 region of SEQ ID NO: 863, and a CDR3 region of SEQ ID NO: 864;
145) a binding molecule comprising a) a light-chain variable region comprising a CDR1 region of SEQ ID NO: 865, a CDR2 region of SEQ ID NO: 866, and a CDR3 region of SEQ ID NO: 867, and b) a heavy-chain variable region comprising a CDR1 region of SEQ ID NO: 868, a CDR2 region of SEQ ID NO: 869, and a CDR3 region of SEQ ID NO: 870;
146) a binding molecule comprising a) a light-chain variable region comprising a CDR1 region of SEQ ID NO: 871, a CDR2 region of SEQ ID NO: 872, and a CDR3 region of SEQ ID NO: 873, and b) a heavy-chain variable region comprising a CDR1 region of SEQ ID NO: 874, a CDR2 region of SEQ ID NO: 875, and a CDR3 region of SEQ ID NO: 876;
147) a binding molecule comprising a) a light-chain variable region comprising a CDR1 region of SEQ ID NO: 877, a CDR2 region of SEQ ID NO: 878, and a CDR3 region of SEQ ID NO: 879, and b) a heavy-chain variable region comprising a CDR1 region of SEQ ID NO: 880, a CDR2 region of SEQ ID NO: 881, and a CDR3 region of SEQ ID NO: 882;
148) a binding molecule comprising a) a light-chain variable region comprising a CDR1 region of SEQ ID NO: 883, a CDR2 region of SEQ ID NO: 884, and a CDR3 region of SEQ ID NO: 885, and b) a heavy-chain variable region comprising a CDR1 region of SEQ ID NO: 886, a CDR2 region of SEQ ID NO: 887, and a CDR3 region of SEQ ID NO: 888;
149) a binding molecule comprising a) a light-chain variable region comprising a CDR1 region of SEQ ID NO: 889, a CDR2 region of SEQ ID NO: 890, and a CDR3 region of SEQ ID NO: 891, and b) a heavy-chain variable region comprising a CDR1 region of SEQ ID NO: 892, a CDR2 region of SEQ ID NO: 893, and a CDR3 region of SEQ ID NO: 894;
150) a binding molecule comprising a) a light-chain variable region comprising a CDR1 region of SEQ ID NO: 895, a CDR2 region of SEQ ID NO: 896, and a CDR3 region of SEQ ID NO: 897, and b) a heavy-chain variable region comprising a CDR1 region of SEQ ID NO: 898, a CDR2 region of SEQ ID NO: 899, and a CDR3 region of SEQ ID NO: 900;
151) a binding molecule comprising a) a light-chain variable region comprising a CDR1 region of SEQ ID NO: 901, a CDR2 region of SEQ ID NO: 902, and a CDR3 region of SEQ ID NO: 903, and b) a heavy-chain variable region comprising a CDR1 region of SEQ ID NO: 904, a CDR2 region of SEQ ID NO: 905, and a CDR3 region of SEQ ID NO: 906;
152) a binding molecule comprising a) a light-chain variable region comprising a CDR1 region of SEQ ID NO: 907, a CDR2 region of SEQ ID NO: 908, and a CDR3 region of SEQ ID NO: 909, and b) a heavy-chain variable region comprising a CDR1 region of SEQ ID NO: 910, a CDR2 region of SEQ ID NO: 911, and a CDR3 region of SEQ ID NO: 912;
153) a binding molecule comprising a) a light-chain variable region comprising a CDR1 region of SEQ ID NO: 913, a CDR2 region of SEQ ID NO: 914, and a CDR3 region of SEQ ID NO: 915, and b) a heavy-chain variable region comprising a CDR1 region of SEQ ID NO: 916, a CDR2 region of SEQ ID NO: 917, and a CDR3 region of SEQ ID NO: 918;
154) a binding molecule comprising a) a light-chain variable region comprising a CDR1 region of SEQ ID NO: 919, a CDR2 region of SEQ ID NO: 920, and a CDR3 region of SEQ ID NO: 921, and b) a heavy-chain variable region comprising a CDR1 region of SEQ ID NO: 922, a CDR2 region of SEQ ID NO: 923, and a CDR3 region of SEQ ID NO: 924;
155) a binding molecule comprising a) a light-chain variable region comprising a CDR1 region of SEQ ID NO: 925, a CDR2 region of SEQ ID NO: 926, and a CDR3 region of SEQ ID NO: 927, and b) a heavy-chain variable region comprising a CDR1 region of SEQ ID NO: 928, a CDR2 region of SEQ ID NO: 929, and a CDR3 region of SEQ ID NO: 930;
156) a binding molecule comprising a) a light-chain variable region comprising a CDR1 region of SEQ ID NO: 931, a CDR2 region of SEQ ID NO: 932, and a CDR3 region of SEQ ID NO: 933, and b) a heavy-chain variable region comprising a CDR1 region of SEQ ID NO: 934, a CDR2 region of SEQ ID NO: 935, and a CDR3 region of SEQ ID NO: 936;
157) a binding molecule comprising a) a light-chain variable region comprising a CDR1 region of SEQ ID NO: 937, a CDR2 region of SEQ ID NO: 938, and a CDR3 region of SEQ ID NO: 939, and b) a heavy-chain variable region comprising a CDR1 region of SEQ ID NO: 940, a CDR2 region of SEQ ID NO: 941, and a CDR3 region of SEQ ID NO: 942;
158) a binding molecule comprising a) a light-chain variable region comprising a CDR1 region of SEQ ID NO: 943, a CDR2 region of SEQ ID NO: 944, and a CDR3 region of SEQ ID NO: 945, and b) a heavy-chain variable region comprising a CDR1 region of SEQ ID NO: 946, a CDR2 region of SEQ ID NO: 947, and a CDR3 region of SEQ ID NO: 948;
159) a binding molecule comprising a) a light-chain variable region comprising a CDR1 region of SEQ ID NO: 949, a CDR2 region of SEQ ID NO: 950, and a CDR3 region of SEQ ID NO: 951, and b) a heavy-chain variable region comprising a CDR1 region of SEQ ID NO: 952, a CDR2 region of SEQ ID NO: 953, and a CDR3 region of SEQ ID NO: 954;
160) a binding molecule comprising a) a light-chain variable region comprising a CDR1 region of SEQ ID NO: 955, a CDR2 region of SEQ ID NO: 956, and a CDR3 region of SEQ ID NO: 957, and b) a heavy-chain variable region comprising a CDR1 region of SEQ ID NO: 958, a CDR2 region of SEQ ID NO: 959, and a CDR3 region of SEQ ID NO: 960;
161) a binding molecule comprising a) a light-chain variable region comprising a CDR1 region of SEQ ID NO: 961, a CDR2 region of SEQ ID NO: 962, and a CDR3 region of SEQ ID NO: 963, and b) a heavy-chain variable region comprising a CDR1 region of SEQ ID NO: 964, a CDR2 region of SEQ ID NO: 965, and a CDR3 region of SEQ ID NO: 966;
162) a binding molecule comprising a) a light-chain variable region comprising a CDR1 region of SEQ ID NO: 967, a CDR2 region of SEQ ID NO: 968, and a CDR3 region of SEQ ID NO: 969, and b) a heavy-chain variable region comprising a CDR1 region of SEQ ID NO: 970, a CDR2 region of SEQ ID NO: 971, and a CDR3 region of SEQ ID NO: 972;
163) a binding molecule comprising a) a light-chain variable region comprising a CDR1 region of SEQ ID NO: 973, a CDR2 region of SEQ ID NO: 974, and a CDR3 region of SEQ ID NO: 975, and b) a heavy-chain variable region comprising a CDR1 region of SEQ ID NO: 976, a CDR2 region of SEQ ID NO: 977, and a CDR3 region of SEQ ID NO: 978;
164) a binding molecule comprising a) a light-chain variable region comprising a CDR1 region of SEQ ID NO: 979, a CDR2 region of SEQ ID NO: 980, and a CDR3 region of SEQ ID NO: 981, and b) a heavy-chain variable region comprising a CDR1 region of SEQ ID NO: 982, a CDR2 region of SEQ ID NO: 983, and a CDR3 region of SEQ ID NO: 984;
165) a binding molecule comprising a) a light-chain variable region comprising a CDR1 region of SEQ ID NO: 985, a CDR2 region of SEQ ID NO: 986, and a CDR3 region of SEQ ID NO: 987, and b) a heavy-chain variable region comprising a CDR1 region of SEQ ID NO: 988, a CDR2 region of SEQ ID NO: 989, and a CDR3 region of SEQ ID NO: 990;
166) a binding molecule comprising a) a light-chain variable region comprising a CDR1 region of SEQ ID NO: 991, a CDR2 region of SEQ ID NO: 992, and a CDR3 region of SEQ ID NO: 993, and b) a heavy-chain variable region comprising a CDR1 region of SEQ ID NO: 994, a CDR2 region of SEQ ID NO: 995, and a CDR3 region of SEQ ID NO: 996;
167) a binding molecule comprising a) a light-chain variable region comprising a CDR1 region of SEQ ID NO: 997, a CDR2 region of SEQ ID NO: 998, and a CDR3 region of SEQ ID NO: 999, and b) a heavy-chain variable region comprising a CDR1 region of SEQ ID NO: 1,000, a CDR2 region of SEQ ID NO: 1,001, and a CDR3 region of SEQ ID NO: 1,002;
168) a binding molecule comprising a) a light-chain variable region comprising a CDR1 region of SEQ ID NO: 1,003, a CDR2 region of SEQ ID NO: 1,004, and a CDR3 region of SEQ ID NO: 1,005, and b) a heavy-chain variable region comprising a CDR1 region of SEQ ID NO: 1,006, a CDR2 region of SEQ ID NO: 1,007, and a CDR3 region of SEQ ID NO: 1,008;
169) a binding molecule comprising a) a light-chain variable region comprising a CDR1 region of SEQ ID NO: 1,009, a CDR2 region of SEQ ID NO: 1,010, and a CDR3 region of SEQ ID NO: 1,011, and b) a heavy-chain variable region comprising a CDR1 region of SEQ ID NO: 1,012, a CDR2 region of SEQ ID NO: 1,013, and a CDR3 region of SEQ ID NO: 1,014;
170) a binding molecule comprising a) a light-chain variable region comprising a CDR1 region of SEQ ID NO: 1,015, a CDR2 region of SEQ ID NO: 1,016, and a CDR3 region of SEQ ID NO: 1,017, and b) a heavy-chain variable region comprising a CDR1 region of SEQ ID NO: 1,018, a CDR2 region of SEQ ID NO: 1,019, and a CDR3 region of SEQ ID NO: 1,020;
171) a binding molecule comprising a) a light-chain variable region comprising a CDR1 region of SEQ ID NO: 1,021, a CDR2 region of SEQ ID NO: 1,022, and a CDR3 region of SEQ ID NO: 1,023, and b) a heavy-chain variable region comprising a CDR1 region of SEQ ID NO: 1,024, a CDR2 region of SEQ ID NO: 1,025, and a CDR3 region of SEQ ID NO: 1,026;
172) a binding molecule comprising a) a light-chain variable region comprising a CDR1 region of SEQ ID NO: 1,027, a CDR2 region of SEQ ID NO: 1,028, and a CDR3 region of SEQ ID NO: 1,029, and b) a heavy-chain variable region comprising a CDR1 region of SEQ ID NO: 1,030, a CDR2 region of SEQ ID NO: 1,031, and a CDR3 region of SEQ ID NO: 1,032;
173) a binding molecule comprising a) a light-chain variable region comprising a CDR1 region of SEQ ID NO: 1,033, a CDR2 region of SEQ ID NO: 1,034, and a CDR3 region of SEQ ID NO: 1,035, and b) a heavy-chain variable region comprising a CDR1 region of SEQ ID NO: 1,036, a CDR2 region of SEQ ID NO: 1,037, and a CDR3 region of SEQ ID NO: 1,038;
174) a binding molecule comprising a) a light-chain variable region comprising a CDR1 region of SEQ ID NO: 1,039, a CDR2 region of SEQ ID NO: 1,040, and a CDR3 region of SEQ ID NO: 1,041, and b) a heavy-chain variable region comprising a CDR1 region of SEQ ID NO: 1,042, a CDR2 region of SEQ ID NO: 1,043, and a CDR3 region of SEQ ID NO: 1,044;
175) a binding molecule comprising a) a light-chain variable region comprising a CDR1 region of SEQ ID NO: 1,045, a CDR2 region of SEQ ID NO: 1,046, and a CDR3 region of SEQ ID NO: 1,047, and b) a heavy-chain variable region comprising a CDR1 region of SEQ ID NO: 1,048, a CDR2 region of SEQ ID NO: 1,049, and a CDR3 region of SEQ ID NO: 1,050;
176) a binding molecule comprising a) a light-chain variable region comprising a CDR1 region of SEQ ID NO: 1,051, a CDR2 region of SEQ ID NO: 1,052, and a CDR3 region of SEQ ID NO: 1,053, and b) a heavy-chain variable region comprising a CDR1 region of SEQ ID NO: 1,054, a CDR2 region of SEQ ID NO: 1,055, and a CDR3 region of SEQ ID NO: 1,056;
177) a binding molecule comprising a) a light-chain variable region comprising a CDR1 region of SEQ ID NO: 1,057, a CDR2 region of SEQ ID NO: 1,058, and a CDR3 region of SEQ ID NO: 1,059, and b) a heavy-chain variable region comprising a CDR1 region of SEQ ID NO: 1,060, a CDR2 region of SEQ ID NO: 1,061, and a CDR3 region of SEQ ID NO: 1,062;
178) a binding molecule comprising a) a light-chain variable region comprising a CDR1 region of SEQ ID NO: 1,063, a CDR2 region of SEQ ID NO: 1,064, and a CDR3 region of SEQ ID NO: 1,065, and b) a heavy-chain variable region comprising a CDR1 region of SEQ ID NO: 1,066, a CDR2 region of SEQ ID NO: 1,067, and a CDR3 region of SEQ ID NO: 1,068;
179) a binding molecule comprising a) a light-chain variable region comprising a CDR1 region of SEQ ID NO: 1,069, a CDR2 region of SEQ ID NO: 1,070, and a CDR3 region of SEQ ID NO: 1,071, and b) a heavy-chain variable region comprising a CDR1 region of SEQ ID NO: 1,072, a CDR2 region of SEQ ID NO: 1,073, and a CDR3 region of SEQ ID NO: 1,074;
180) a binding molecule comprising a) a light-chain variable region comprising a CDR1 region of SEQ ID NO: 1,075, a CDR2 region of SEQ ID NO: 1,076, and a CDR3 region of SEQ ID NO: 1,077, and b) a heavy-chain variable region comprising a CDR1 region of SEQ ID NO: 1,078, a CDR2 region of SEQ ID NO: 1,079, and a CDR3 region of SEQ ID NO: 1,080;
181) a binding molecule comprising a) a light-chain variable region comprising a CDR1 region of SEQ ID NO: 1,081, a CDR2 region of SEQ ID NO: 1,082, and a CDR3 region of SEQ ID NO: 1,083, and b) a heavy-chain variable region comprising a CDR1 region of SEQ ID NO: 1,084, a CDR2 region of SEQ ID NO: 1,085, and a CDR3 region of SEQ ID NO: 1,086;
182) a binding molecule comprising a) a light-chain variable region comprising a CDR1 region of SEQ ID NO: 1,087, a CDR2 region of SEQ ID NO: 1,088, and a CDR3 region of SEQ ID NO: 1,089, and b) a heavy-chain variable region comprising a CDR1 region of SEQ ID NO: 1,090, a CDR2 region of SEQ ID NO: 1,091, and a CDR3 region of SEQ ID NO: 1,092;
183) a binding molecule comprising a) a light-chain variable region comprising a CDR1 region of SEQ ID NO: 1,093, a CDR2 region of SEQ ID NO: 1,094, and a CDR3 region of SEQ ID NO: 1,095, and b) a heavy-chain variable region comprising a CDR1 region of SEQ ID NO: 1,096, a CDR2 region of SEQ ID NO: 1,097, and a CDR3 region of SEQ ID NO: 1,098;
184) a binding molecule comprising a) a light-chain variable region comprising a CDR1 region of SEQ ID NO: 1,099, a CDR2 region of SEQ ID NO: 1,100, and a CDR3 region of SEQ ID NO: 1,101, and b) a heavy-chain variable region comprising a CDR1 region of SEQ ID NO: 1,102, a CDR2 region of SEQ ID NO: 1,103, and a CDR3 region of SEQ ID NO: 1,104;
185) a binding molecule comprising a) a light-chain variable region comprising a CDR1 region of SEQ ID NO: 1,105, a CDR2 region of SEQ ID NO: 1,106, and a CDR3 region of SEQ ID NO: 1,107, and b) a heavy-chain variable region comprising a CDR1 region of SEQ ID NO: 1,108, a CDR2 region of SEQ ID NO: 1,109, and a CDR3 region of SEQ ID NO: 1,110;
186) a binding molecule comprising a) a light-chain variable region comprising a CDR1 region of SEQ ID NO: 1,111, a CDR2 region of SEQ ID NO: 1,112, and a CDR3 region of SEQ ID NO: 1,113, and b) a heavy-chain variable region comprising a CDR1 region of SEQ ID NO: 1,114, a CDR2 region of SEQ ID NO: 1,115, and a CDR3 region of SEQ ID NO: 1,116;
187) a binding molecule comprising a) a light-chain variable region comprising a CDR1 region of SEQ ID NO: 1,117, a CDR2 region of SEQ ID NO: 1,118, and a CDR3 region of SEQ ID NO: 1,119, and b) a heavy-chain variable region comprising a CDR1 region of SEQ ID NO: 1,120, a CDR2 region of SEQ ID NO: 1,121, and a CDR3 region of SEQ ID NO: 1,122;
188) a binding molecule comprising a) a light-chain variable region comprising a CDR1 region of SEQ ID NO: 1,123, a CDR2 region of SEQ ID NO: 1,124, and a CDR3 region of SEQ ID NO: 1,125, and b) a heavy-chain variable region comprising a CDR1 region of SEQ ID NO: 1,126, a CDR2 region of SEQ ID NO: 1,127, and a CDR3 region of SEQ ID NO: 1,128;
189) a binding molecule comprising a) a light-chain variable region comprising a CDR1 region of SEQ ID NO: 1,129, a CDR2 region of SEQ ID NO: 1,130, and a CDR3 region of SEQ ID NO: 1,131, and b) a heavy-chain variable region comprising a CDR1 region of SEQ ID NO: 1,132, a CDR2 region of SEQ ID NO: 1,133, and a CDR3 region of SEQ ID NO: 1,134; and
190) a binding molecule comprising a) a light-chain variable region comprising a CDR1 region of SEQ ID NO: 1,135, a CDR2 region of SEQ ID NO: 1,136, and a CDR3 region of SEQ ID NO: 1,137, and b) a heavy-chain variable region comprising a CDR1 region of SEQ ID NO: 1,138, a CDR2 region of SEQ ID NO: 1,139, and a CDR3 region of SEQ ID NO: 1,140.

37. The binding molecule according to claim 36, wherein the binding molecule competes with any one binding molecule selected from the group consisting of the following binding molecules 1) to 49):
1) a binding molecule comprising a) a light-chain variable region comprising a CDR1 region of SEQ ID NO: 7, a CDR2 region of SEQ ID NO: 8, and a CDR3 region of SEQ ID NO: 9, and b) a heavy-chain variable region comprising a CDR1 region of SEQ ID NO: 10, a CDR2 region of SEQ ID NO: 11, and a CDR3 region of SEQ ID NO: 12;
2) a binding molecule comprising a) a light-chain variable region comprising a CDR1 region of SEQ ID NO: 13, a CDR2 region of SEQ ID NO: 14, and a CDR3 region of SEQ ID NO: 15, and b) a heavy-chain variable region comprising a CDR1 region of SEQ ID NO: 16, a CDR2 region of SEQ ID NO: 17, and a CDR3 region of SEQ ID NO: 18;
3) a binding molecule comprising a) a light-chain variable region comprising a CDR1 region of SEQ ID NO: 67, a CDR2 region of SEQ ID NO: 68, and a CDR3 region of SEQ ID NO: 69, and b) a heavy-chain variable region comprising a CDR1 region of SEQ ID NO: 70, a CDR2 region of SEQ ID NO: 71, and a CDR3 region of SEQ ID NO: 72;
4) a binding molecule comprising a) a light-chain variable region comprising a CDR1 region of SEQ ID NO: 85, a CDR2 region of SEQ ID NO: 86, and a CDR3 region of SEQ ID NO: 87, and b) a heavy-chain variable region comprising a CDR1 region of SEQ ID NO: 88, a CDR2 region of SEQ ID NO: 89, and a CDR3 region of SEQ ID NO: 90;
5) a binding molecule comprising a) a light-chain variable region comprising a CDR1 region of SEQ ID NO: 91, a CDR2 region of SEQ ID NO: 92, and a CDR3 region of SEQ ID NO: 93, and b) a heavy-chain variable region comprising a CDR1 region of SEQ ID NO: 94, a CDR2 region of SEQ ID NO: 95, and a CDR3 region of SEQ ID NO: 96;
6) a binding molecule comprising a) a light-chain variable region comprising a CDR1 region of SEQ ID NO: 103, a CDR2 region of SEQ ID NO: 104, and a CDR3 region of SEQ ID NO: 105, and b) a heavy-chain variable region comprising a CDR1 region of SEQ ID NO: 106, a CDR2 region of SEQ ID NO: 107, and a CDR3 region of SEQ ID NO: 108;
7) a binding molecule comprising a) a light-chain variable region comprising a CDR1 region of SEQ ID NO: 109, CDR2 region of SEQ ID NO: 110, and CDR3 region of SEQ ID NO: 111, and b) a heavy-chain variable region comprising a CDR1 region of SEQ ID NO: 112, a CDR2 region of SEQ ID NO: 113, and a CDR3 region of SEQ ID NO: 114;
8) a binding molecule comprising a) a light-chain variable region comprising a CDR1 region of SEQ ID NO: 115, a CDR2 region of SEQ ID NO: 116, and a CDR3 region of SEQ ID NO: 117, and b) a heavy-chain variable region comprising a CDR1 region of SEQ ID NO: 118, a CDR2 region of SEQ ID NO: 119, and a CDR3 region of SEQ ID NO: 120;
9) a binding molecule comprising a) a light-chain variable region comprising a CDR1 region of SEQ ID NO: 127, a CDR2 region of SEQ ID NO: 128, and a CDR3 region of SEQ ID NO: 129, and b) a heavy-chain variable region comprising a CDR1 region of SEQ ID NO: 130, a CDR2 region of SEQ ID NO: 131, and a CDR3 region of SEQ ID NO: 132;
10) a binding molecule comprising a) a light-chain variable region comprising a CDR1 region of SEQ ID NO: 133, a CDR2 region of SEQ ID NO: 134, and a CDR3 region of SEQ ID NO: 135, and b) a heavy-chain variable region comprising a CDR1 region of SEQ ID NO: 136, a CDR2 region of SEQ ID NO: 137, and a CDR3 region of SEQ ID NO: 138;
11) a binding molecule comprising a) a light-chain variable region comprising a CDR1 region of SEQ ID NO: 139, a CDR2 region of SEQ ID NO: 140, and a CDR3 region of SEQ ID NO: 141, and b) a heavy-chain variable region comprising a CDR1 region of SEQ ID NO: 142, a CDR2 region of SEQ ID NO: 143, and a CDR3 region of SEQ ID NO: 144;
12) a binding molecule comprising a) a light-chain variable region comprising a CDR1 region of SEQ ID NO: 151, a CDR2 region of SEQ ID NO: 152, and a CDR3 region of SEQ ID NO: 153, and b) a heavy-chain variable region comprising a CDR1 region of SEQ ID NO: 154, a CDR2 region of SEQ ID NO: 155, and a CDR3 region of SEQ ID NO: 156;
13) a binding molecule comprising a) a light-chain variable region comprising a CDR1 region of SEQ ID NO: 181, a CDR2 region of SEQ ID NO: 182, and a CDR3 region of SEQ ID NO: 183, and b) a heavy-chain variable region comprising a CDR1 region of SEQ ID NO: 184, a CDR2 region of SEQ ID NO: 185, and a CDR3 region of SEQ ID NO: 186;
14) a binding molecule comprising a) a light-chain variable region comprising a CDR1 region of SEQ ID NO: 187, a CDR2 region of SEQ ID NO: 188, and a CDR3 region of SEQ ID NO: 189, and b) a heavy-chain variable region comprising a CDR1 region of SEQ ID NO: 190, a CDR2 region of SEQ ID NO: 191, and a CDR3 region of SEQ ID NO: 192;
15) a binding molecule comprising a) a light-chain variable region comprising a CDR1 region of SEQ ID NO: 193, a CDR2 region of SEQ ID NO: 194, and a CDR3 region of SEQ ID NO: 195, and b) a heavy-chain variable region comprising a CDR1 region of SEQ ID NO: 196, a CDR2 region of SEQ ID NO: 197, and a CDR3 region of SEQ ID NO: 198;
16) a binding molecule comprising a) a light-chain variable region comprising a CDR1 region of SEQ ID NO: 199, a CDR2 region of SEQ ID NO: 200, and a CDR3 region of SEQ ID NO: 201, and b) a heavy-chain variable region comprising a CDR1 region of SEQ ID NO: 202, a CDR2 region of SEQ ID NO: 203, and a CDR3 region of SEQ ID NO: 204;
17) a binding molecule comprising a) a light-chain variable region comprising a CDR1 region of SEQ ID NO: 217, a CDR2 region of SEQ ID NO: 218, and a CDR3 region of SEQ ID NO: 219, and b) a heavy-chain variable region comprising a CDR1 region of SEQ ID NO: 220, a CDR2 region of SEQ ID NO: 221, and a CDR3 region of SEQ ID NO: 222;
18) a binding molecule comprising a) a light-chain variable region comprising a CDR1 region of SEQ ID NO: 253, a CDR2 region of SEQ ID NO: 254, and a CDR3 region of SEQ ID NO: 255, and b) a heavy-chain variable region comprising a CDR1 region of SEQ ID NO: 256, a CDR2 region of SEQ ID NO: 257, and a CDR3 region of SEQ ID NO: 258;
19) a binding molecule comprising a) a light-chain variable region comprising a CDR1 region of SEQ ID NO: 265, a CDR2 region of SEQ ID NO: 266, and a CDR3 region of SEQ ID NO: 267, and b) a heavy-chain variable region comprising a CDR1 region of SEQ ID NO: 268, a CDR2 region of SEQ ID NO: 269, and a CDR3 region of SEQ ID NO: 270;
20) a binding molecule comprising a) a light-chain variable region comprising a CDR1 region of SEQ ID NO: 283, a CDR2 region of SEQ ID NO: 284, and a CDR3 region of SEQ ID NO: 285, and b) a heavy-chain variable region comprising a CDR1 region of SEQ ID NO: 286, a CDR2 region of SEQ ID NO: 287, and a CDR3 region of SEQ ID NO: 288;
21) a binding molecule comprising a) a light-chain variable region comprising a CDR1 region of SEQ ID NO: 295, a CDR2 region of SEQ ID NO: 296, and a CDR3 region of SEQ ID NO: 297, and b) a heavy-chain variable region comprising a CDR1 region of SEQ ID NO: 298, a CDR2 region of SEQ ID NO: 299, and a CDR3 region of SEQ ID NO: 300;
22) a binding molecule comprising a) a light-chain variable region comprising a CDR1 region of SEQ ID NO: 301, a CDR2 region of SEQ ID NO: 302, and a CDR3 region of SEQ ID NO: 303, and b) a heavy-chain variable region comprising a CDR1 region of SEQ ID NO: 304, a CDR2 region of SEQ ID NO: 305, and a CDR3 region of SEQ ID NO: 306;
23) a binding molecule comprising a) a light-chain variable region comprising a CDR1 region of SEQ ID NO: 313, a CDR2 region of SEQ ID NO: 314, and a CDR3 region of SEQ ID NO: 315, and b) a heavy-chain variable region comprising a CDR1 region of SEQ ID NO: 316, a CDR2 region of SEQ ID NO: 317, and a CDR3 region of SEQ ID NO: 318;
24) a binding molecule comprising a) a light-chain variable region comprising a CDR1 region of SEQ ID NO: 319, a CDR2 region of SEQ ID NO: 320, and a CDR3 region of SEQ ID NO: 321, and b) a heavy-chain variable region comprising a CDR1 region of SEQ ID NO: 322, a CDR2 region of SEQ ID NO: 323, and a CDR3 region of SEQ ID NO: 324;
25) a binding molecule comprising a) a light-chain variable region comprising a CDR1 region of SEQ ID NO: 325, a CDR2 region of SEQ ID NO: 326, and a CDR3 region of SEQ ID NO: 327, and b) a heavy-chain variable region comprising a CDR1 region of SEQ ID NO: 328, a CDR2 region of SEQ ID NO: 329, and a CDR3 region of SEQ ID NO: 330;
26) a binding molecule comprising a) a light-chain variable region comprising a CDR1 region of SEQ ID NO: 331, a CDR2 region of SEQ ID NO: 332, and a CDR3 region of SEQ ID NO: 333, and b) a heavy-chain variable region comprising a CDR1 region of SEQ ID NO: 334, a CDR2 region of SEQ ID NO: 335, and a CDR3 region of SEQ ID NO: 336;
27) a binding molecule comprising a) a light-chain variable region comprising a CDR1 region of SEQ ID NO: 349, a CDR2 region of SEQ ID NO: 350, and a CDR3 region of SEQ ID NO: 351, and b) a heavy-chain variable region comprising a CDR1 region of SEQ ID NO: 352, a CDR2 region of SEQ ID NO: 353, and a CDR3 region of SEQ ID NO: 354;
28) a binding molecule comprising a) a light-chain variable region comprising a CDR1 region of SEQ ID NO: 355, a CDR2 region of SEQ ID NO: 356, and a CDR3 region of SEQ ID NO: 357, and b) a heavy-chain variable region comprising a CDR1 region of SEQ ID NO: 358, a CDR2 region of SEQ ID NO: 359, and a CDR3 region of SEQ ID NO: 360;
29) a binding molecule comprising a) a light-chain variable region comprising a CDR1 region of SEQ ID NO: 361, a CDR2 region of SEQ ID NO: 362, and a CDR3 region of SEQ ID NO: 363, and b) a heavy-chain variable region comprising a CDR1 region of SEQ ID NO: 364, a CDR2 region of SEQ ID NO: 365, and a CDR3 region of SEQ ID NO: 366;
30) a binding molecule comprising a) a light-chain variable region comprising a CDR1 region of SEQ ID NO: 391, a CDR2 region of SEQ ID NO: 392, and a CDR3 region of SEQ ID NO: 393, and b) a heavy-chain variable region comprising a CDR1 region of SEQ ID NO: 394, a CDR2 region of SEQ ID NO: 395, and a CDR3 region of SEQ ID NO: 396;
31) a binding molecule comprising a) a light-chain variable region comprising a CDR1 region of SEQ ID NO: 409, a CDR2 region of SEQ ID NO: 410, and a CDR3 region of SEQ ID NO: 411, and b) a heavy-chain variable region comprising a CDR1 region of SEQ ID NO: 412, a CDR2 region of SEQ ID NO: 413, and a CDR3 region of SEQ ID NO: 414;
32) a binding molecule comprising a) a light-chain variable region comprising a CDR1 region of SEQ ID NO: 439, a CDR2 region of SEQ ID NO: 440, and a CDR3 region of SEQ ID NO: 441, and b) a heavy-chain variable region comprising a CDR1 region of SEQ ID NO: 442, a CDR2 region of SEQ ID NO: 443, and a CDR3 region of SEQ ID NO: 444;
33) a binding molecule comprising a) a light-chain variable region comprising a CDR1 region of SEQ ID NO: 445, a CDR2 region of SEQ ID NO: 446, and a CDR3 region of SEQ ID NO: 447, and b) a heavy-chain variable region comprising a CDR1 region of SEQ ID NO: 448, a CDR2 region of SEQ ID NO: 449, and a CDR3 region of SEQ ID NO: 450;
34) a binding molecule comprising a) a light-chain variable region comprising a CDR1 region of SEQ ID NO: 451, a CDR2 region of SEQ ID NO: 452, and a CDR3 region of SEQ ID NO: 453, and b) a heavy-chain variable region comprising a CDR1 region of SEQ ID NO: 454, a CDR2 region of SEQ ID NO: 455, and a CDR3 region of SEQ ID NO: 456;
35) a binding molecule comprising a) a light-chain variable region comprising a CDR1 region of SEQ ID NO: 481, a CDR2 region of SEQ ID NO: 482, and a CDR3 region of SEQ ID NO: 483, and b) a heavy-chain variable region comprising a CDR1 region of SEQ ID NO: 484, a CDR2 region of SEQ ID NO: 485, and a CDR3 region of SEQ ID NO: 486;
36) a binding molecule comprising a) a light-chain variable region comprising a CDR1 region of SEQ ID NO: 487, a CDR2 region of SEQ ID NO: 488, and a CDR3 region of SEQ ID NO: 489, and b) a heavy-chain variable region comprising a CDR1 region of SEQ ID NO: 490, a CDR2 region of SEQ ID NO: 491, and a CDR3 region of SEQ ID NO: 492;
37) a binding molecule comprising a) a light-chain variable region comprising a CDR1 region of SEQ ID NO: 493, a CDR2 region of SEQ ID NO: 494, and a CDR3 region of SEQ ID NO: 495, and b) a heavy-chain variable region comprising a CDR1 region of SEQ ID NO: 496, a CDR2 region of SEQ ID NO: 497, and a CDR3 region of SEQ ID NO: 498;
38) a binding molecule comprising a) a light-chain variable region comprising a CDR1 region of SEQ ID NO: 505, a CDR2 region of SEQ ID NO: 506, and a CDR3 region of SEQ ID NO: 507, and b) a heavy-chain variable region comprising a CDR1 region of SEQ ID NO: 508, a CDR2 region of SEQ ID NO: 509, and a CDR3 region of SEQ ID NO: 510;
39) a binding molecule comprising a) a light-chain variable region comprising a CDR1 region of SEQ ID NO: 511, a CDR2 region of SEQ ID NO: 512, and a CDR3 region of SEQ ID NO: 513, and b) a heavy-chain variable region comprising a CDR1 region of SEQ ID NO: 514, a CDR2 region of SEQ ID NO: 515, and a CDR3 region of SEQ ID NO: 516;
40) a binding molecule comprising a) a light-chain variable region comprising a CDR1 region of SEQ ID NO: 517, a CDR2 region of SEQ ID NO: 518, and a CDR3 region of SEQ ID NO: 519, and b) a heavy-chain variable region comprising a CDR1 region of SEQ ID NO: 520, a CDR2 region of SEQ ID NO: 521, and a CDR3 region of SEQ ID NO: 522;
41) a binding molecule comprising a) a light-chain variable region comprising a CDR1 region of SEQ ID NO: 553, a CDR2 region of SEQ ID NO: 554, and a CDR3 region of SEQ ID NO: 555, and b) a heavy-chain variable region comprising a CDR1 region of SEQ ID NO: 556, a CDR2 region of SEQ ID NO: 557, and a CDR3 region of SEQ ID NO: 558;
42) a binding molecule comprising a) a light-chain variable region comprising a CDR1 region of SEQ ID NO: 559, a CDR2 region of SEQ ID NO: 560, and a CDR3 region of SEQ ID NO: 561, and b) a heavy-chain variable region comprising a CDR1 region of SEQ ID NO: 562, a CDR2 region of SEQ ID NO: 563, and a CDR3 region of SEQ ID NO: 564;
43) a binding molecule comprising a) a light-chain variable region comprising a CDR1 region of SEQ ID NO: 565, a CDR2 region of SEQ ID NO: 566, and a CDR3 region of SEQ ID NO: 567, and b) a heavy-chain variable region comprising a CDR1 region of SEQ ID NO: 568, a CDR2 region of SEQ ID NO: 569, and a CDR3 region of SEQ ID NO: 570;
44) a binding molecule comprising a) a light-chain variable region comprising a CDR1 region of SEQ ID NO: 571, a CDR2 region of SEQ ID NO: 572, and a CDR3 region of SEQ ID NO: 573, and b) a heavy-chain variable region comprising a CDR1 region of SEQ ID NO: 574, a CDR2 region of SEQ ID NO: 575, and a CDR3 region of SEQ ID NO: 576;
45) a binding molecule comprising a) a light-chain variable region comprising a CDR1 region of SEQ ID NO: 583, a CDR2 region of SEQ ID NO: 584, and a CDR3 region of SEQ ID NO: 585, and b) a heavy-chain variable region comprising a CDR1 region of SEQ ID NO: 586, a CDR2 region of SEQ ID NO: 587, and a CDR3 region of SEQ ID NO: 588;
46) a binding molecule comprising a) a light-chain variable region comprising a CDR1 region of SEQ ID NO: 607, a CDR2 region of SEQ ID NO: 608, and a CDR3 region of SEQ ID NO: 609, and b) a heavy-chain variable region comprising a CDR1 region of SEQ ID NO: 610, a CDR2 region of SEQ ID NO: 611, and a CDR3 region of SEQ ID NO: 612;
47) a binding molecule comprising a) a light-chain variable region comprising a CDR1 region of SEQ ID NO: 613, a CDR2 region of SEQ ID NO: 614, and a CDR3 region of SEQ ID NO: 615, and b) a heavy-chain variable region comprising a CDR1 region of SEQ ID NO: 616, a CDR2 region of SEQ ID NO: 617, and a CDR3 region of SEQ ID NO: 618;
48) a binding molecule comprising a) a light-chain variable region comprising a CDR1 region of SEQ ID NO: 619, a CDR2 region of SEQ ID NO: 620, and a CDR3 region of SEQ ID NO: 621, and b) a heavy-chain variable region comprising a CDR1 region of SEQ ID NO: 622, a CDR2 region of SEQ ID NO: 623, and a CDR3 region of SEQ ID NO: 624; and
49) a binding molecule comprising a) a light-chain variable region comprising a CDR1 region of SEQ ID NO: 643, a CDR2 region of SEQ ID NO: 644, and a CDR3 region of SEQ ID NO: 645, and b) a heavy-chain variable region comprising a CDR1 region of SEQ ID NO: 646, a CDR2 region of SEQ ID NO: 647, and a CDR3 region of SEQ ID NO: 648.

38. A neutralizing binding molecule that binds to a spike protein (S protein) on a surface of coronavirus, wherein the binding molecule is any one selected from the group consisting of the following binding molecules 1) to 49):
1) a binding molecule comprising a) a light-chain variable region comprising a CDR1 region of SEQ ID NO: 7, a CDR2 region of SEQ ID NO: 8, and a CDR3 region of SEQ ID NO: 9, and b) a heavy-chain variable region comprising a CDR1 region of SEQ ID NO: 10, a CDR2 region of SEQ ID NO: 11, and a CDR3 region of SEQ ID NO: 12;
2) a binding molecule comprising a) a light-chain variable region comprising a CDR1 region of SEQ ID NO: 13, a CDR2 region of SEQ ID NO: 14, and a CDR3 region of SEQ ID NO: 15, and b) a heavy-chain variable region comprising a CDR1 region of SEQ ID NO: 16, a CDR2 region of SEQ ID NO: 17, and a CDR3 region of SEQ ID NO: 18;
3) a binding molecule comprising a) a light-chain variable region comprising a CDR1 region of SEQ ID NO: 67, a CDR2 region of SEQ ID NO: 68, and a CDR3 region of SEQ ID NO: 69, and b) a heavy-chain variable region comprising a CDR1 region of SEQ ID NO: 70, a CDR2 region of SEQ ID NO: 71, and a CDR3 region of SEQ ID NO: 72;
4) a binding molecule comprising a) a light-chain variable region comprising a CDR1 region of SEQ ID NO: 85, a CDR2 region of SEQ ID NO: 86, and a CDR3 region of SEQ ID NO: 87, and b) a heavy-chain variable region comprising a CDR1 region of SEQ ID NO: 88, a CDR2 region of SEQ ID NO: 89, and a CDR3 region of SEQ ID NO: 90;
5) a binding molecule comprising a) a light-chain variable region comprising a CDR1 region of SEQ ID NO: 91, a CDR2 region of SEQ ID NO: 92, and a CDR3 region of SEQ ID NO: 93, and b) a heavy-chain variable region comprising a CDR1 region of SEQ ID NO: 94, a CDR2 region of SEQ ID NO: 95, and a CDR3 region of SEQ ID NO: 96;
6) a binding molecule comprising a) a light-chain variable region comprising a CDR1 region of SEQ ID NO: 103, a CDR2 region of SEQ ID NO: 104, and a CDR3 region of SEQ ID NO: 105, and b) a heavy-chain variable region comprising a CDR1 region of SEQ ID NO: 106, a CDR2 region of SEQ ID NO: 107, and a CDR3 region of SEQ ID NO: 108;
7) a binding molecule comprising a) a light-chain variable region comprising a CDR1 region of SEQ ID NO: 109, CDR2 region of SEQ ID NO: 110, and CDR3 region of SEQ ID NO: 111, and b) a heavy-chain variable region comprising a CDR1 region of SEQ ID NO: 112, a CDR2 region of SEQ ID NO: 113, and a CDR3 region of SEQ ID NO: 114;
8) a binding molecule comprising a) a light-chain variable region comprising a CDR1 region of SEQ ID NO: 115, a CDR2 region of SEQ ID NO: 116, and a CDR3 region of SEQ ID NO: 117, and b) a heavy-chain variable region comprising a CDR1 region of SEQ ID NO: 118, a CDR2 region of SEQ ID NO: 119, and a CDR3 region of SEQ ID NO: 120;
9) a binding molecule comprising a) a light-chain variable region comprising a CDR1 region of SEQ ID NO: 127, a CDR2 region of SEQ ID NO: 128, and a CDR3 region of SEQ ID NO: 129, and b) a heavy-chain variable region comprising a CDR1 region of SEQ ID NO: 130, a CDR2 region of SEQ ID NO: 131, and a CDR3 region of SEQ ID NO: 132;
10) a binding molecule comprising a) a light-chain variable region comprising a CDR1 region of SEQ ID NO: 133, a CDR2 region of SEQ ID NO: 134, and a CDR3 region of SEQ ID NO: 135, and b) a heavy-chain variable region comprising a CDR1 region of SEQ ID NO: 136, a CDR2 region of SEQ ID NO: 137, and a CDR3 region of SEQ ID NO: 138;
11) a binding molecule comprising a) a light-chain variable region comprising a CDR1 region of SEQ ID NO: 139, a CDR2 region of SEQ ID NO: 140, and a CDR3 region of SEQ ID NO: 141, and b) a heavy-chain variable region comprising a CDR1 region of SEQ ID NO: 142, a CDR2 region of SEQ ID NO: 143, and a CDR3 region of SEQ ID NO: 144;
12) a binding molecule comprising a) a light-chain variable region comprising a CDR1 region of SEQ ID NO: 151, a CDR2 region of SEQ ID NO: 152, and a CDR3 region of SEQ ID NO: 153, and b) a heavy-chain variable region comprising a CDR1 region of SEQ ID NO: 154, a CDR2 region of SEQ ID NO: 155, and a CDR3 region of SEQ ID NO: 156;
13) a binding molecule comprising a) a light-chain variable region comprising a CDR1 region of SEQ ID NO: 181, a CDR2 region of SEQ ID NO: 182, and a CDR3 region of SEQ ID NO: 183, and b) a heavy-chain variable region comprising a CDR1 region of SEQ ID NO: 184, a CDR2 region of SEQ ID NO: 185, and a CDR3 region of SEQ ID NO: 186;
14) a binding molecule comprising a) a light-chain variable region comprising a CDR1 region of SEQ ID NO: 187, a CDR2 region of SEQ ID NO: 188, and a CDR3 region of SEQ ID NO: 189, and b) a heavy-chain variable region comprising a CDR1 region of SEQ ID NO: 190, a CDR2 region of SEQ ID NO: 191, and a CDR3 region of SEQ ID NO: 192;
15) a binding molecule comprising a) a light-chain variable region comprising a CDR1 region of SEQ ID NO: 193, a CDR2 region of SEQ ID NO: 194, and a CDR3 region of SEQ ID NO: 195, and b) a heavy-chain variable region comprising a CDR1 region of SEQ ID NO: 196, a CDR2 region of SEQ ID NO: 197, and a CDR3 region of SEQ ID NO: 198;
16) a binding molecule comprising a) a light-chain variable region comprising a CDR1 region of SEQ ID NO: 199, a CDR2 region of SEQ ID NO: 200, and a CDR3 region of SEQ ID NO: 201, and b) a heavy-chain variable region comprising a CDR1 region of SEQ ID NO: 202, a CDR2 region of SEQ ID NO: 203, and a CDR3 region of SEQ ID NO: 204;
17) a binding molecule comprising a) a light-chain variable region comprising a CDR1 region of SEQ ID NO: 217, a CDR2 region of SEQ ID NO: 218, and a CDR3 region of SEQ ID NO: 219, and b) a heavy-chain variable region comprising a CDR1 region of SEQ ID NO: 220, a CDR2 region of SEQ ID NO: 221, and a CDR3 region of SEQ ID NO: 222;
18) a binding molecule comprising a) a light-chain variable region comprising a CDR1 region of SEQ ID NO: 253, a CDR2 region of SEQ ID NO: 254, and a CDR3 region of SEQ ID NO: 255, and b) a heavy-chain variable region comprising a CDR1 region of SEQ ID NO: 256, a CDR2 region of SEQ ID NO: 257, and a CDR3 region of SEQ ID NO: 258;
19) a binding molecule comprising a) a light-chain variable region comprising a CDR1 region of SEQ ID NO: 265, a CDR2 region of SEQ ID NO: 266, and a CDR3 region of SEQ ID NO: 267, and b) a heavy-chain variable region comprising a CDR1 region of SEQ ID NO: 268, a CDR2 region of SEQ ID NO: 269, and a CDR3 region of SEQ ID NO: 270;
20) a binding molecule comprising a) a light-chain variable region comprising a CDR1 region of SEQ ID NO: 283, a CDR2 region of SEQ ID NO: 284, and a CDR3 region of SEQ ID NO: 285, and b) a heavy-chain variable region comprising a CDR1 region of SEQ ID NO: 286, a CDR2 region of SEQ ID NO: 287, and a CDR3 region of SEQ ID NO: 288;
21) a binding molecule comprising a) a light-chain variable region comprising a CDR1 region of SEQ ID NO: 295, a CDR2 region of SEQ ID NO: 296, and a CDR3 region of SEQ ID NO: 297, and b) a heavy-chain variable region comprising a CDR1 region of SEQ ID NO: 298, a CDR2 region of SEQ ID NO: 299, and a CDR3 region of SEQ ID NO: 300;
22) a binding molecule comprising a) a light-chain variable region comprising a CDR1 region of SEQ ID NO: 301, a CDR2 region of SEQ ID NO: 302, and a CDR3 region of SEQ ID NO: 303, and b) a heavy-chain variable region comprising a CDR1 region of SEQ ID NO: 304, a CDR2 region of SEQ ID NO: 305, and a CDR3 region of SEQ ID NO: 306;
23) a binding molecule comprising a) a light-chain variable region comprising a CDR1 region of SEQ ID NO: 313, a CDR2 region of SEQ ID NO: 314, and a CDR3 region of SEQ ID NO: 315, and b) a heavy-chain variable region comprising a CDR1 region of SEQ ID NO: 316, a CDR2 region of SEQ ID NO: 317, and a CDR3 region of SEQ ID NO: 318;
24) a binding molecule comprising a) a light-chain variable region comprising a CDR1 region of SEQ ID NO: 319, a CDR2 region of SEQ ID NO: 320, and a CDR3 region of SEQ ID NO: 321, and b) a heavy-chain variable region comprising a CDR1 region of SEQ ID NO: 322, a CDR2 region of SEQ ID NO: 323, and a CDR3 region of SEQ ID NO: 324;
25) a binding molecule comprising a) a light-chain variable region comprising a CDR1 region of SEQ ID NO: 325, a CDR2 region of SEQ ID NO: 326, and a CDR3 region of SEQ ID NO: 327, and b) a heavy-chain variable region comprising a CDR1 region of SEQ ID NO: 328, a CDR2 region of SEQ ID NO: 329, and a CDR3 region of SEQ ID NO: 330;
26) a binding molecule comprising a) a light-chain variable region comprising a CDR1 region of SEQ ID NO: 331, a CDR2 region of SEQ ID NO: 332, and a CDR3 region of SEQ ID NO: 333, and b) a heavy-chain variable region comprising a CDR1 region of SEQ ID NO: 334, a CDR2 region of SEQ ID NO: 335, and a CDR3 region of SEQ ID NO: 336;
27) a binding molecule comprising a) a light-chain variable region comprising a CDR1 region of SEQ ID NO: 349, a CDR2 region of SEQ ID NO: 350, and a CDR3 region of SEQ ID NO: 351, and b) a heavy-chain variable region comprising a CDR1 region of SEQ ID NO: 352, a CDR2 region of SEQ ID NO: 353, and a CDR3 region of SEQ ID NO: 354;
28) a binding molecule comprising a) a light-chain variable region comprising a CDR1 region of SEQ ID NO: 355, a CDR2 region of SEQ ID NO: 356, and a CDR3 region of SEQ ID NO: 357, and b) a heavy-chain variable region comprising a CDR1 region of SEQ ID NO: 358, a CDR2 region of SEQ ID NO: 359, and a CDR3 region of SEQ ID NO: 360;
29) a binding molecule comprising a) a light-chain variable region comprising a CDR1 region of SEQ ID NO: 361, a CDR2 region of SEQ ID NO: 362, and a CDR3 region of SEQ ID NO: 363, and b) a heavy-chain variable region comprising a CDR1 region of SEQ ID NO: 364, a CDR2 region of SEQ ID NO: 365, and a CDR3 region of SEQ ID NO: 366;
30) a binding molecule comprising a) a light-chain variable region comprising a CDR1 region of SEQ ID NO: 391, a CDR2 region of SEQ ID NO: 392, and a CDR3 region of SEQ ID NO: 393, and b) a heavy-chain variable region comprising a CDR1 region of SEQ ID NO: 394, a CDR2 region of SEQ ID NO: 395, and a CDR3 region of SEQ ID NO: 396;
31) a binding molecule comprising a) a light-chain variable region comprising a CDR1 region of SEQ ID NO: 409, a CDR2 region of SEQ ID NO: 410, and a CDR3 region of SEQ ID NO: 411, and b) a heavy-chain variable region comprising a CDR1 region of SEQ ID NO: 412, a CDR2 region of SEQ ID NO: 413, and a CDR3 region of SEQ ID NO: 414;
32) a binding molecule comprising a) a light-chain variable region comprising a CDR1 region of SEQ ID NO: 439, a CDR2 region of SEQ ID NO: 440, and a CDR3 region of SEQ ID NO: 441, and b) a heavy-chain variable region comprising a CDR1 region of SEQ ID NO: 442, a CDR2 region of SEQ ID NO: 443, and a CDR3 region of SEQ ID NO: 444;
33) a binding molecule comprising a) a light-chain variable region comprising a CDR1 region of SEQ ID NO: 445, a CDR2 region of SEQ ID NO: 446, and a CDR3 region of SEQ ID NO: 447, and b) a heavy-chain variable region comprising a CDR1 region of SEQ ID NO: 448, a CDR2 region of SEQ ID NO: 449, and a CDR3 region of SEQ ID NO: 450;
34) a binding molecule comprising a) a light-chain variable region comprising a CDR1 region of SEQ ID NO: 451, a CDR2 region of SEQ ID NO: 452, and a CDR3 region of SEQ ID NO: 453, and b) a heavy-chain variable region comprising a CDR1 region of SEQ ID NO: 454, a CDR2 region of SEQ ID NO: 455, and a CDR3 region of SEQ ID NO: 456;
35) a binding molecule comprising a) a light-chain variable region comprising a CDR1 region of SEQ ID NO: 481, a CDR2 region of SEQ ID NO: 482, and a CDR3 region of SEQ ID NO: 483, and b) a heavy-chain variable region comprising a CDR1 region of SEQ ID NO: 484, a CDR2 region of SEQ ID NO: 485, and a CDR3 region of SEQ ID NO: 486;
36) a binding molecule comprising a) a light-chain variable region comprising a CDR1 region of SEQ ID NO: 487, a CDR2 region of SEQ ID NO: 488, and a CDR3 region of SEQ ID NO: 489, and b) a heavy-chain variable region comprising a CDR1 region of SEQ ID NO: 490, a CDR2 region of SEQ ID NO: 491, and a CDR3 region of SEQ ID NO: 492;
37) a binding molecule comprising a) a light-chain variable region comprising a CDR1 region of SEQ ID NO: 493, a CDR2 region of SEQ ID NO: 494, and a CDR3 region of SEQ ID NO: 495, and b) a heavy-chain variable region comprising a CDR1 region of SEQ ID NO: 496, a CDR2 region of SEQ ID NO: 497, and a CDR3 region of SEQ ID NO: 498;
38) a binding molecule comprising a) a light-chain variable region comprising a CDR1 region of SEQ ID NO: 505, a CDR2 region of SEQ ID NO: 506, and a CDR3 region of SEQ ID NO: 507, and b) a heavy-chain variable region comprising a CDR1 region of SEQ ID NO: 508, a CDR2 region of SEQ ID NO: 509, and a CDR3 region of SEQ ID NO: 510;
39) a binding molecule comprising a) a light-chain variable region comprising a CDR1 region of SEQ ID NO: 511, a CDR2 region of SEQ ID NO: 512, and a CDR3 region of SEQ ID NO: 513, and b) a heavy-chain variable region comprising a CDR1 region of SEQ ID NO: 514, a CDR2 region of SEQ ID NO: 515, and a CDR3 region of SEQ ID NO: 516;
40) a binding molecule comprising a) a light-chain variable region comprising a CDR1 region of SEQ ID NO: 517, a CDR2 region of SEQ ID NO: 518, and a CDR3 region of SEQ ID NO: 519, and b) a heavy-chain variable region comprising a CDR1 region of SEQ ID NO: 520, a CDR2 region of SEQ ID NO: 521, and a CDR3 region of SEQ ID NO: 522;
41) a binding molecule comprising a) a light-chain variable region comprising a CDR1 region of SEQ ID NO: 553, a CDR2 region of SEQ ID NO: 554, and a CDR3 region of SEQ ID NO: 555, and b) a heavy-chain variable region comprising a CDR1 region of SEQ ID NO: 556, a CDR2 region of SEQ ID NO: 557, and a CDR3 region of SEQ ID NO: 558;
42) a binding molecule comprising a) a light-chain variable region comprising a CDR1 region of SEQ ID NO: 559, a CDR2 region of SEQ ID NO: 560, and a CDR3 region of SEQ ID NO: 561, and b) a heavy-chain variable region comprising a CDR1 region of SEQ ID NO: 562, a CDR2 region of SEQ ID NO: 563, and a CDR3 region of SEQ ID NO: 564;
43) a binding molecule comprising a) a light-chain variable region comprising a CDR1 region of SEQ ID NO: 565, a CDR2 region of SEQ ID NO: 566, and a CDR3 region of SEQ ID NO: 567, and b) a heavy-chain variable region comprising a CDR1 region of SEQ ID NO: 568, a CDR2 region of SEQ ID NO: 569, and a CDR3 region of SEQ ID NO: 570;
44) a binding molecule comprising a) a light-chain variable region comprising a CDR1 region of SEQ ID NO: 571, a CDR2 region of SEQ ID NO: 572, and a CDR3 region of SEQ ID NO: 573, and b) a heavy-chain variable region comprising a CDR1 region of SEQ ID NO: 574, a CDR2 region of SEQ ID NO: 575, and a CDR3 region of SEQ ID NO: 576;
45) a binding molecule comprising a) a light-chain variable region comprising a CDR1 region of SEQ ID NO: 583, a CDR2 region of SEQ ID NO: 584, and a CDR3 region of SEQ ID NO: 585, and b) a heavy-chain variable region comprising a CDR1 region of SEQ ID NO: 586, a CDR2 region of SEQ ID NO: 587, and a CDR3 region of SEQ ID NO: 588;
46) a binding molecule comprising a) a light-chain variable region comprising a CDR1 region of SEQ ID NO: 607, a CDR2 region of SEQ ID NO: 608, and a CDR3 region of SEQ ID NO: 609, and b) a heavy-chain variable region comprising a CDR1 region of SEQ ID NO: 610, a CDR2 region of SEQ ID NO: 611, and a CDR3 region of SEQ ID NO: 612;
47) a binding molecule comprising a) a light-chain variable region comprising a CDR1 region of SEQ ID NO: 613, a CDR2 region of SEQ ID NO: 614, and a CDR3 region of SEQ ID NO: 615, and b) a heavy-chain variable region comprising a CDR1 region of SEQ ID NO: 616, a CDR2 region of SEQ ID NO: 617, and a CDR3 region of SEQ ID NO: 618;
48) a binding molecule comprising a) a light-chain variable region comprising a CDR1 region of SEQ ID NO: 619, a CDR2 region of SEQ ID NO: 620, and a CDR3 region of SEQ ID NO: 621, and b) a heavy-chain variable region comprising a CDR1 region of SEQ ID NO: 622, a CDR2 region of SEQ ID NO: 623, and a CDR3 region of SEQ ID NO: 624; and
49) a binding molecule comprising a) a light-chain variable region comprising a CDR1 region of SEQ ID NO: 643, a CDR2 region of SEQ ID NO: 644, and a CDR3 region of SEQ ID NO: 645, and b) a heavy-chain variable region comprising a CDR1 region of SEQ ID NO: 646, a CDR2 region of SEQ ID NO: 647, and a CDR3 region of SEQ ID NO: 648.

39. A binding molecule that binds to a spike protein (S protein) on a surface of SARS-coronavirus-2 (SARS-CoV-2), wherein the binding molecule comprises a light-chain variable region comprising LC CDR1, LC CDR2 and LC CDR3, and a heavy-chain variable region comprising HC CDR1, HC CDR2 and HC CDR3, and is any one selected from the group consisting of the following binding molecules 1) to 49):
1) a binding molecule in which
the LC CDR1 comprises the sequence SGSX₁SNIGX₂NTVN, where X₁ is S or T, and X₂ is S or T,
the LC CDR2 comprises the sequence X₃X₄NX₅RPS, where X₃ is S or T, X4 is N or D, and X₅ is Q or R,
the LC CDR3 comprises the sequence AX₆X₇DDX₈LX₉X₁₀X₁₁X₁₂, where X₆ is A or S, X₇ is W or R, X₈ is S or R, X₉ is Nor S, X₁₀ is A or G, X₁₁ is S, L, V, Y, P, A or T, X₁₂ is L or V,
the HC CDR1 comprises the sequence GYYWS,
the HC CDR2 comprises the sequence EINHSGSTNYNPSLKS, and
the HC CDR3 comprises the sequence GRYSSNWYEAWTPRGIGMDV;
2) a binding molecule in which
the LC CDR1 comprises the sequence X₁₃GSX₁₄SX₁₅IX₁₆X₁₇X₁₈X₁₉VS, where X₁₃ is S or T, X₁₄ is S or N, X₁₅ is Nor S, X₁₆ is G or E, X₁₇ is Nor S, X₁₈ is Nor S, and X₁₉ is Y or F,
the LC CDR2 comprises the sequence X₂₀NX₂₁X₂₂RPS, where X₂₀ is D or R, X₂₁ is N or S, and X₂₂ is K, R, Q or V,
the LC CDR3 comprises the sequence X₂₃X₂₄WDX₂₅SLSX₂₆X₂₇V or GTWDSSLSAYWV, where X₂₃ is G or A, X₂₄ is T or A, X₂₅ is S, N, D or T, X₂₆ is A, G or S, and X₂₇ is W or Y,
the HC CDR1 comprises the sequence X₂₈YYMH, where X₂₈ is S or G,
the HC CDR2 comprises the sequence X₂₉INRSGGSTIYAQTFQX₃₀, where X₂₉ is I or V, X₃₀ is G or S, and
the HC CDR3 comprises the sequence GGRHSLDX₃₁, where X₃₁ is V or A;
3) a binding molecule in which
the LC CDR1 comprises the sequence RASQSVX₃₂X₃₃X₃₄X₃₅A, where X₃₂ is S, N or R, X₃₃ is S or N, X₃₄ is Y or N, and X₃₅ is L or S,
the LC CDR2 comprises the sequence X₃₆ASX₃₇RAT, where X₃₆ is D or G, and X₃₇ is N, T or S,
the LC CDR3 comprises the sequence X₃₈QX₃₉X₄₀X₄₁X₄₂PX₄₃T or QQYGSSPSIT, where X₃₈ is Q or H, X₃₉ is Y or H, X₄₀ is G, N, Y or H, X₄₁ is S, N, T, Q or R, X₄₂ is S, W or V, and X₄₃ is L, F, I or Q,
the HC CDR1 comprises the sequence X₄₄SSYYWG, where X₄₄ is S or G,
the HC CDR2 comprises the sequence X₄₅IX₄₆YSGSTYYNPSLKS, where X₄₅ is S or N, and X₄₆ is Y or F, and
the HC CDR3 comprises the sequence GSRGYYDX₄₇LTGYSTGGFDY, where X₄₇ is F or I;
4) a binding molecule in which
the LC CDR1 comprises the sequence RASQSVX₄₈X₄₉X₅₀YLA, where X₄₈ is S or G, X₄₉ is S or R, and X₅₀ is S or N,
the LC CDR2 comprises the sequence X₅₁ASX₅₂RAT, where X₅₁ is G or D, and X₅₂ is S or T,
the LC CDR3 comprises the sequence QQYGX₅₃SPX₅₄X₅₅, where X₅₃ is S or T, X₅₄ is L or I, and X₅₅ is T or A,
the HC CDR1 comprises the sequence SX₅₆X₅₇YYWX₅₈, where X₅₆ is S or G, X₅₇ is S or G, and X₅₈ is G or S,
the HC CDR2 comprises the sequence X₅₉IX₆₀YSGSTYYNPSLKS, where X₅₉ is S, Y or N, and X₆₀ is Y or F, and
the HC CDR3 comprises the sequence GSRGYYDX₆₁LTGYSTGGFDY, where X₆₁ is F or I;
5) a binding molecule in which
the LC CDR1 comprises the sequence TGTSSX₆₂VGX₆₃YX₆₄X₆₅VS, where X₆₂ is N or D, X₆₃ is D or G, X₆₄ is N or S, and X₆₅ is Y or H,
the LC CDR2 comprises the sequence X₆₆VX₆₇X₆₈RPS, where X₆₆ is D or E, X₆₇ is T or S, and X₆₈ is N or D,
the LC CDR3 comprises the sequence SSYTSGSTPVV or NSYTSSSTWV,
the HC CDR1 comprises the sequence SSSYYWG,
the HC CDR2 comprises the sequence NIFYSGSTYYNPSLKS, and
the HC CDR3 comprises the sequence GSRGYYDILTGYSTGGFDY;
6) a binding molecule in which
the LC CDR1 comprises the sequence SGX₆₉X₇₀SNIGSNX₇₁VX₇₂, where X₆₉ is S or L, X₇₀ is S or T, X₇₁ is Y, T or F, and X₇₂ is Y, H or N,
the LC CDR2 comprises the sequence X₇₃NX₇₄X₇₅RPS, where X₇₃ is R or S, X₇₄ is N or D, and X₇₅ is Q or E,
the LC CDR3 comprises the sequence X₇₆X₇₇WDDX₇₈LX₇₉GX₈₀V, where X₇₆ is A or S, X₇₇ is A or T, X₇₈ is S or T, X₇₉ is S, N or V, and X₈₀ is R or K,
the HC CDR1 comprises the sequence SYAIS,
the HC CDR2 comprises the sequence GIIPIFGTANYAQX₈₁FQX₈₂, where X₈₁ is K or R, and X₈₂ is G or D, and
the HC CDR3 comprises the sequence DGVVVPAVMYDTTDPYYYGMDV;
7) a binding molecule in which
the LC CDR1 comprises the sequence TGTSSDVGGYNYVS,
the LC CDR2 comprises the sequence X₈₃ VSNRPS, where X₈₃ is D or E,
the LC CDR3 comprises the sequence SSYAGSNYVV or SSYTSSSTX₈₄X₈₅V, where X₈₄ is L or R, and X₈₅ is G or Y,
the HC CDR1 comprises the sequence SYAMX₈₆, where X₈₆ is S or N,
the HC CDR2 comprises the sequence AISGSGGTTYYADSVKG, and
the HC CDR3 comprises the sequence APSDLSFIWTGYYSEYYFDY;
8) a binding molecule in which
the LC CDR1 comprises the sequence RASQSVSSSYLA,
the LC CDR2 comprises the sequence GASSRAT,
the LC CDR3 comprises the sequence QQYGSSPX₈₇T or QQYGSSPALT, where X₈₇ is Y or F,
the HC CDR1 comprises the sequence SSPMH,
the HC CDR2 comprises the sequence VISYX₈₈GSNKYYADSVKG, where X₈₈ is D or G, and
the HC CDR3 comprises the sequence EIALNNYYGMDV;
9) a binding molecule in which
the LC CDR1 comprises the sequence TGX₈₉X₉₀SDX₉₁GGYNX₉₂X₉₃S, where X₈₉ is N or T, X₉₀ is R or S, X₉₁ is I or V, X₉₂ is S or Y, and X₉₃ is L or V,
the LC CDR2 comprises the sequence DVX₉₄NRPS, where X₉₄ is N or S,
the LC CDR3 comprises the sequence X₉₅SYTSX₉₆X₉₇TWV, where X₉₅ is N or S, X₉₆ is K or S, and X₉₇ is N or S,
the HC CDR1 comprises the sequence SSPMH,
the HC CDR2 comprises the sequence VISYDGSNKYYADSVKG, and
the HC CDR3 comprises the sequence EIALNNYYGMDV;
10) a binding molecule in which
the LC CDR1 comprises the sequence SGSSSNIGNNYVS or GGNNIGSKSVH,
the LC CDR2 comprises the sequence DX₉₈X₉₉X₁₀₀RPS, where X₉₈ is N or D, X₉₉ is N or S, and X₁₀₀ is K or D,
the LC CDR3 comprises the sequence GTWDSSLSAGWV or X₁₀₁X₁₀₂WDSSX₁₀₃X₁₀₄X₁₀₅VV, where X₁₀₁ is G or Q, X₁₀₂ is T or V, X₁₀₃ is L or S, X₁₀₄ is S or D, and X₁₀₅ is A or H,
the HC CDR1 comprises the sequence DYGMH,
the sequence HC CDR2 comprises the sequence AISYDGSNKYYADSVKG, and
the HC CDR3 comprises the sequence DSGLYGSGWSTYQYYAMDV;
11) a binding molecule in which
the LC CDR1 comprises the sequence X₁₀₆GTX₁₀₇SDVGGYNYVS, where X₁₀₆ is T or S, and X₁₀₇ is S or N,
the LC CDR2 comprises the sequence DVX₁₀₈KRPS, where X₁₀₈ is S or T,
the LC CDR3 comprises the sequence SSYTSSX₁₀₉TWV or SSYTSSSTPWV, where X₁₀₉ is S or R,
the HC CDR1 comprises the sequence X₁₁₀YAMH, where X₁₁₀ is S or G,
the HC CDR2 comprises the sequence VISYDGSHKNYADSVKG, and
the HC CDR3 comprises the sequence DNCGGDCGGGMDV;
12) a binding molecule in which
the LC CDR1 comprises the sequence SGSSSNIGSNX₁₁₁VN or TGSSSNIGAGYDVH, where X₁₁₁ is A or T,
the LC CDR2 comprises the sequence X₁₁₂NSX₁₁₃RPS, where X₁₁₂ is G or S, and X₁₁₃ isN or Y,
the LC CDR3 comprises the sequence QSYDSX₁₁₄LX₁₁₅X₁₁₆X₁₁₇X₁₁₈, where X₁₁₄ is S or A, X₁₁₅ is S or R, X₁₁₆ is D or G, X₁₁₇ is V or P, and X₁₁₈ is V or L,
the HC CDR1 comprises the sequence SYGIS,
the HC CDR2 comprises the sequence GIIPILATTKFAQKFQG, and
the HC CDR3 comprises the sequence GWGLRLFGEFSVWFDP;
13) a binding molecule in which
the LC CDR1 comprises the sequence RASQSVSSYLA,
the LC CDR2 comprises the sequence DASNRAT,
the LC CDR3 comprises the sequence QQRRNWPPX₁₁₉X₁₂₀T, where X₁₁₉ is V or R, and X₁₂₀ is L or I,
the HC CDR1 comprises the sequence SYAIS,
the HC CDR2 comprises the sequence GIIPIFGTANYAQKFQG, and
the HC CDR3 comprises the sequence VEGYDSSGYYLDY;
14) a binding molecule in which
the LC CDR1 comprises the sequence X₁₂₁GSX₁₂₂SNIGX₁₂₃NX₁₂₄VX₁₂₅, where X₁₂₁ is S or T, X₁₂₂ is S or R, X₁₂₃ is S or I, X₁₂₄ is T or S, and X₁₂₅ is S or N,
the LC CDR2 comprises the sequence SNNQRPS,
the LC CDR3 comprises the sequence AAWDDSLNGX₁₂₆V, where X₁₂₆ is V or Y,
the HC CDR1 comprises the sequence GYYWS,
the HC CDR2 comprises the sequence EINHSGSTNYNPSLKSG, and
the HC CDR3 comprises the sequence RYSSNWYEAWTPRGIGMDV;
15) a binding molecule in which
the LC CDR1 comprises the sequence TGSSSNIGAGYDVH or SGSSSNIGSNTVN,
the LC CDR2 comprises the sequence X₁₂₇NX₁₂₈X₁₂₉RPS, where X₁₂₇ is G or S, X₁₂₈ is S or N, and X₁₂₉ is N or Q,
the LC CDR3 comprises the sequence AAWDDSLX₁₃₀GX₁₃₁V, where X₁₃₀ is G or N, and X₁₃₁ is P or W,
the HC CDR1 comprises the sequence TYGMH,
the HC CDR2 comprises the sequence VISYDGFNKYYADSVKG, and
the HC CDR3 comprises the sequence SLGGNYYYGMDV;
16) a binding molecule in which
the LC CDR1 comprises the sequence TGTSSDIGGYNYVS or GGNNIGSKSVH,
the LC CDR2 comprises the sequence X₁₃₂X₁₃₃SKRPS, where X₁₃₂ is A or Q, and X₁₃₃ is V or D,
the LC CDR3 comprises the sequence SSYTSSSGTLNV or QAWDSSTV,
the HC CDR1 comprises the sequence TYGMH,
the HC CDR2 comprises the sequence VISYDGFNKYYADSVKG, and
the HC CDR3 comprises the sequence SLGGNYYYGMDV;
17) a binding molecule in which
the LC CDR1 comprises the sequence RASQSVSX₁₃₄X₁₃₅LA, where X₁₃₄ is S or T, and X₁₃₅ is Y or F,
the LC CDR2 comprises the sequence DASNRAT,
the LC CDR3 comprises the sequence QQRSNWPPX₁₃₆X₁₃₇T, where X₁₃₆ is A, R, K or M, and X₁₃₇ is L, I or Y,
the HC CDR1 comprises the sequence SYAIX₁₃₈, where X₁₃₈ is S or I,
the HC CDR2 comprises the sequence GIIPIFGTANYAQKFQG, and
the HC CDR3 comprises the sequence VX₁₃₉GYDX₁₄₀SGYYQX₁₄₁Y, where X₁₃₉ is T or S, X₁₄₀ is S or G, and X₁₄₁ is D or E;
18) a binding molecule in which
the LC CDR1 comprises the sequence TGTSX₁₄₂DX₁₄₃GX₁₄₄YNYVS, where X₁₄₂ is T or S, X₁₄₃ is I or V, and X₁₄₄ is R or A,
the LC CDR2 comprises the sequence DVSKRPS,
the LC CDR3 comprises the sequence SYTSSSTYA or NSYTSSSTWV,
the HC CDR1 comprises the sequence EVAIH,
the HC CDR2 comprises the sequence GFDPEDGETSYAQKFQG, and
the HC CDR3 comprises the sequence GPVLGLSKWLEFDP;
19) a binding molecule in which
the LC CDR1 comprises the sequence SGSSSNIGNNYVS,
the LC CDR2 comprises the sequence DNNKRPS,
the LC CDR3 comprises the sequence X₁₄₅X₁₄₆WDX₁₄₇SLSX₁₄₈X₁₄₉X₁₅₀, where X₁₄₅ is G or E, X₁₄₆ is T or A, X₁₄₇ is S or T, X₁₄₈ is A or D, X₁₄₉ is V or G, and X₁₅₀ is V or L,
the HC CDR1 comprises the sequence RYAMS,
the HC CDR2 comprises the sequence AISGSGGX₁₅₁TYYADSVKG, where X₁₅₁ is S or T, and
the HC CDR3 comprises the sequence GPRGQDYGDYGFLDY;
20) a binding molecule in which
the LC CDR1 comprises the sequence SGSSSNIGSNTVN,
the LC CDR2 comprises the sequence X₁₅₂NNX₁₅₃RPS, where X₁₅₂ is S, T or N, and X₁₅₃ is Q or R,
the LC CDR3 comprises the sequence AAWDDSLX₁₅₄GX₁₅₅V or AAWDDSLNGX₁₅₆VV, where X₁₅₄ is N or S, X₁₅₅ is L, P, W or V, and X₁₅₆ is S or H,
the HC CDR1 comprises the sequence SX₁₅₇WIX₁₅₈, where X₁₅₇ is H or Y, and X₁₅₈ is A, V or G,
the HC CDR2 comprises the sequence X₁₅₉IYPGDSDSRYSPSFQG, where X₁₅₉ is I or T, and
the HC CDR3 comprises the sequence GPNX₁₆₀YNWFDS, where X₁₆₀ is L or I;
21) a binding molecule in which
the LC CDR1 comprises the sequence RASQX₁₆₁X₁₆₂X₁₆₃X₁₆₄X₁₆₅X₁₆₆A, where X₁₆₁ is S or G, X₁₆₂ is V or I, X₁₆₃ is S or R, X₁₆₄ is S or N, X₁₆₅ is Y, S or D, and X₁₆₆ is L or I,
the LC CDR2 comprises the sequence DX₁₆₇SX₁₆₈X₁₆₉X₁₇₀T or AASRLES, where X₁₆₇ is A or T, X₁₆₈ is N or T, X₁₆₉ is R or L, and X₁₇₀ is A or E,
the LC CDR3 comprises the sequence QQYYX₁₇₁TPX₁₇₂T, where X₁₇₁ is S or T, and X₁₇₂ is F or I,
the HC CDR1 comprises the sequence DYYIQ,
the HC CDR2 comprises the sequence WINPNSGDTNYAHKFQG, and
the HC CDR3 comprises the sequence GGSYYNVMYWFDP;
22) a binding molecule in which
the LC CDR1 comprises the sequence X₁₇₃ASQX₁₇₄ISSYLN, where X₁₇₃ is R or Q, and X₁₇₄ is S or D,
the LC CDR2 comprises the sequence X₁₇₅ASSLX₁₇₆S, where X₁₇₅ is K or A, and X₁₇₆ is E or Q,
the LC CDR3 comprises the sequence QQSYSTWT,
the HC CDR1 comprises the sequence SYAMH,
the HC CDR2 comprises the sequence VISYDGSNKYYADSVKG, and
the HC CDR3 comprises the sequence ARGGSYI,YGMDV;
23) a binding molecule in which
the LC CDR1 comprises the sequence SGSX₁₇₇SNIGX₁₇₈NYVX₁₇₉, where X₁₇₇ is S or G, X₁₇₈ is N or S, and X₁₇₉ is S or H,
the LC CDR2 comprises the sequence X₁₈₀NX₁₈₁X₁₈₂RPS, where X₁₈₀ is D or R, X₁₈₁ is N or S, and X₁₈₂ is K or G,
the LC CDR3 comprises the sequence AAWDDSLX₁₈₃GWV, where X₁₈₃ is N or S,
the HC CDR1 comprises the sequence SYAIS,
the HC CDR2 comprises the sequence GIIPMFGTTNYAQKFQG, and
the HC CDR3 comprises the sequence DPTSRSTYYYYSGSYYP;
24) a binding molecule in which
the LC CDR1 comprises the sequence SGSSSNIGNNYVS or TGTRGDIGAYDGVS,
the LC CDR2 comprises the sequence DNNKRPS or AVTQRPS,
the LC CDR3 comprises the sequence AAWDDSLNGVV or SSYTSSSSWV,
the HC CDR1 comprises the sequence RYAMS,
the HC CDR2 comprises the sequence AISGSGGSTYYADSVKG, and
the HC CDR3 comprises the sequence GPRGQDYGDYGFLDY;
25) a binding molecule in which
the LC CDR1 comprises the sequence TGSSSNIGAGYDVH or SGSSSNIGNNYVS,
the LC CDR2 comprises the sequence X₁₈₄NX₁₈₅X₁₈₆RPS, where X₁₈₄ is G or D, X₁₈₅ is S or N, and X₁₈₆ is N or K,
the LC CDR3 comprises the sequence QSYDSSLSGLWV or QSYDSSLSAWV,
the HC CDR1 comprises the sequence SNYMS,
the HC CDR2 comprises the sequence VIYPGGSTFFADSVQG, and
the HC CDR3 comprises the sequence SYDFLTDYTDAFDI;
26) a binding molecule in which
the LC CDR1 comprises the sequence TGTX₁₈₇X₁₈₈DX₁₈₉GX₁₉₀YX₁₉₁X₁₉₂VS, where X₁₈₇ is S or R, X₁₈₈ is S or G, X₁₈₉ is V or I, X₁₉₀ is G or A, X₁₉₁ is N or D, and X₁₉₂ is Y or G,
the LC CDR2 comprises the sequence X₁₉₃VX₁₉₄X₁₉₅RPS, where X₁₉₃ is D or A, X₁₉₄ is SorT, and X₁₉₅ is K or Q,
the LC CDR3 comprises the sequence SSYTX₁₉₆SSX₁₉₇WV, where X₁₉₆ is T or S, and X₁₉₇ is T or S,
the HC CDR1 comprises the sequence ELSX₁₉₈H, where X₁₉₈ is I or M,
the HC CDR2 comprises the sequence GFDPEDX₁₉₉ETIYAQKFQG, where X₁₉₉ is G or A, and
the HC CDR3 comprises the sequence SPAX₂₀₀X₂₀₁X₂₀₂X₂₀₃X₂₀₄WFDP, where X₂₀₀ is V or I, X₂₀₁ is T or I, X₂₀₂ is T or R, X₂₀₃ is A or V, and X₂₀₄ is G or D;
27) a binding molecule in which
the LC CDR1 comprises the sequence SGSSSNIGX₂₀₅NX₂₀₆VX₂₀₇, where X₂₀₅ is S or N, X₂₀₆ is T or Y, and X₂₀₇ is N or S,
the LC CDR2 comprises the sequence X₂₀₈NNX₂₀₉RPS, where X₂₀₈ is S or D, and X₂₀₉ is Q or K,
the LC CDR3 comprises the sequence AAWDDSLX₂₁₀GPV, where X₂₁₀ is N or S,
the HC CDR1 comprises the sequence X₂₁₁YAMS, where X₂₁₁ is S or R,
the HC CDR2 comprises the sequence AISGSGGX₂₁₂TYYADSVKG, where X₂₁₂ is D or S, and
the HC CDR3 comprises the sequence VRYYDFWSGWDVMDV;
28) a binding molecule in which
the LC CDR1 comprises the sequence TGTSX₂₁₃DVGX₂₁₄YNX₂₁₅VS, where X₂₁₃ is T or S, X₂₁₄ is G or S, and X₂₁₅ is Y or L,
the LC CDR2 comprises the sequence DVSX₂₁₆RPS, where X₂₁₆ is K or N,
the LC CDR3 comprises the sequence SSYTSSSTLV,
the HC CDR1 comprises the sequence NYGMH,
the HC CDR2 comprises the sequence VMSYDGSNKYYADSVKG, and
the HC CDR3 comprises the sequence PDDSSGYYPDY;
29) a binding molecule in which
the LC CDR1 comprises the sequence TGTSSDVGGYNYVS,
the LC CDR2 comprises the sequence DVX₂₁₇KRPS, where X₂₁₇ is G or S,
the LC CDR3 comprises the sequence SSYTSSSTX₂₁₈V, where X₂₁₈ is W OR L,
the HC CDR1 comprises the sequence X₂₁₉YAIS, where X₂₁₉ is F or R,
the HC CDR2 comprises the sequence GIIPX₂₂₀FGX₂₂₁X₂₂₂X₂₂₃YAQX₂₂₄FQD, where X₂₂₀ is L or I, X₂₂₁ is T or K, X₂₂₂ is A or V, X₂₂₃ is K or N, and X₂₂₄ is R or K, and
the HC CDR3 comprises the sequence GGWIYRGNWFDP or GGSTWYGGNWFDP;
30) a binding molecule in which
the LC CDR1 comprises the sequence TGSSSNIGAGYDVH,
the LC CDR2 comprises the sequence GNSNRPS,
the LC CDR3 comprises the sequence QSYDSSLSGX₂₂₅WV, where X₂₂₅ is P or S,
the HC CDR1 comprises the sequence RYAIX₂₂₆, where X₂₂₆ is N or S,
the HC CDR2 comprises the sequence GIIPX₂₂₇X₂₂₈GTX₂₂₉X₂₃₀YX₂₃₁QKFQG, where X₂₂₇ is L or I, X₂₂₈ is L or F, X₂₂₉ is A or G, X₂₃₀ is D or N, and X₂₃₁ is P or A, and
the HC CDR3 comprises the sequence TLYYYDRSGNARTDDYFDH or SLYYYDRSGYPISEDYFDY;
31) a binding molecule in which
the LC CDR1 comprises the sequence TGTSSDVGX₂₃₂YNYVS, where X₂₃₂ is N or G,
the LC CDR2 comprises the sequence DVSX₂₃₃RPS, where X₂₃₃ is N or K,
the LC CDR3 comprises the sequence SSYTSSX₂₃₄TX₂₃₅V, where X₂₃₄ is G or S, and X₂₃₅ is L or Y,
the HC CDR1 comprises the sequence ELSIH,
the HC CDR2 comprises the sequence GFDPEX₂₃₆X₂₃₇ETIX₂₃₈AQX₂₃₉FQG, where X₂₃₆ is N or D, X₂₃₇ is G or A, X₂₃₈ is H or Y, and X₂₃₉ is R or K, and
the HC CDR3 comprises the sequence SX₂₄₀X₂₄₁X₂₄₂X₂₄₃X₂₄₄AX₂₄₅WFDP, where X₂₄₀ is T or P, X₂₄₁ is P or A, X₂₄₂ is M or V, X₂₄₃ is I or T, X₂₄₄ is R or T, and X₂₄₅ is S or G;
32) a binding molecule in which
the LC CDR1 comprises the sequence TGSSSNIGAGYDVH,
the LC CDR2 comprises the sequence GNSNRPS,
the LC CDR3 comprises the sequence QSYDSSLSGWV,
the HC CDR1 comprises the sequence NYGIS,
the HC CDR2 comprises the sequence X₂₄₆ISX₂₄₇X₂₄₈NGNTX₂₄₉YAQKLQG, where X₂₄₆ is G or W, X₂₄₇ is S or A, X₂₄₈ is H or Y, and X₂₄₉ is K or N, and
the HC CDR3 comprises the sequence EGGYYYGSGSYYNPRFAFDI or PRGYSGYGSNWYF;
33) a binding molecule in which
the LC CDR1 comprises the sequence RASQSVSSNLA or RASQGISNSL,
the LC CDR2 comprises the sequence X₂₅₀AX₂₅₁X₂₅₂RX₂₅₃X₂₅₄, where X₂₅₀ is W or A, X₂₅₁ is S or A, X₂₅₂ is T or S, X₂₅₃ is E or L, and X₂₅₄ is S or E,
the LC CDR3 comprises the sequence LQYFTIPWT or SQQYYSTPYT,
the HC CDR1 comprises the sequence NAWMX₂₅₅, where X₂₅₅ is S or T,
the HC CDR2 comprises the sequence RIKTKTDGGTTDYAAPVKG, and
the HC CDR3 comprises the sequence X₂₅₆SX₂₅₇PDY, where X₂₅₅ is F or H, and X₂₅₇ is T or R;
34) a binding molecule in which
the LC CDR1 comprises the sequence RASQSVSSX₂₅₈LA, where X₂₅₈ is Y or F,
the LC CDR2 comprises the sequence DASNRAT,
the LC CDR3 comprises the sequence QQYGSSPPIT or QQRSNWPPSIT,
the HC CDR1 comprises the sequence SYAIS,
the HC CDR2 comprises the sequence GIIPIFGTX₂₅₉NYAQKFQG, where X₂₅₉ is T or A, and
the HC CDR3 comprises the sequence DPTGWYSGYFDY or VGTYDSSGYSFDY;
35) a binding molecule in which
the LC CDR1 comprises the sequence GGNNIGSKSVH,
the LC CDR2 comprises the sequence YDSDRPS,
the LC CDR3 comprises the sequence QVWDSSSDHWV,
the HC CDR1 comprises the sequence DYAMS or SYYIH,
the HC CDR2 comprises the sequence VISGSGGSTSNADSVKG or FINPSDVTTYYAQKFQG, and
the HC CDR3 comprises the sequence VYCGDDCYPVVGTPGDAFDI or SRIAPTEDFFDY;
36) a binding molecule in which
the LC CDR1 comprises the sequence SSSTGAVTRGHFPN or SGSSSNIGNNYVS,
the LC CDR2 comprises the sequence STSNKHS or DNNKRPS,
the LC CDR3 comprises the sequence LLYDAGAPGWV or GAWDSSLSTPNW,
the HC CDR1 comprises the sequence SYPMH,
the HC CDR2 comprises the sequence VISYDGSLKYYVDSVKG, and
the HC CDR3 comprises the sequence DSSCSGGSCFDY;
37) a binding molecule in which
the LC CDR1 comprises the sequence SGSSSNIGSNTVN,
the LC CDR2 comprises the sequence SNNQRPS,
the LC CDR3 comprises the sequence AAWDDSLNGWV,
the HC CDR1 comprises the sequence X₂₆₀X₂₆₁AMX₂₆₂, where X₂₆₀ is S or N, X₂₆₁ is F or Y, and X₂₆₂ is H or N,
the HC CDR2 comprises the sequence X₂₆₃ISX₂₆₄X₂₆₅GX₂₆₆X₂₆₇X₂₆₈YYADSVKG, where X₂₆₃ is V or T, X₂₆₄ is F or G, X₂₆₅ is D or S, X₂₆₆ is S or G, X₂₆₇ is N or S, and X₂₆₈ is K or T, and
the HC CDR3 comprises the sequence GDYYGSGSYYNPSPFFDY or GYALNP;
38) a binding molecule in which
the LC CDR1 comprises the sequence CRASQSVSSSYLA,
the LC CDR2 comprises the sequence GASSRAT,
the LC CDR3 comprises the sequence QQYGSSPX₂₆₉T, where X₂₆₉ is Y or P,
the HC CDR1 comprises the sequence SSPMH or NYVIN,
the HC CDR2 comprises the sequence VISYDGSNKYYADSVKG or GFIPVFGIADYAQKFQG, and
the HC CDR3 comprises the sequence EIALNNYYGMDV or SGLFDWLLPRSRHRDYFDY;
39) a binding molecule in which
the LC CDR1 comprises the sequence RASQSISSYLN,
the LC CDR2 comprises the sequence AASSLQS,
the LC CDR3 comprises the sequence QQSYSTPLT,
the HC CDR1 comprises the sequence SNYMX₂₇₀, where X₂₇₀ is T or S,
the HC CDR2 comprises the sequence X₂₇₁IYPGGSTX₂₇₂X₂₇₃ADSVX₂₇₄G, where X₂₇₁ is I or V, X₂₇₂ is Y or F, X₂₇₃ is Y or F, and X₂₇₄ is K or Q, and
the HC CDR3 comprises the sequence DLPLTGTTLDY or SYDFLTDYTDAFDI;
40) a binding molecule in which
the LC CDR1 comprises the sequence RASQSISSYLN,
the LC CDR2 comprises the sequence AASSLQS,
the LC CDR3 comprises the sequence QQSYSTPX₂₇₅T, where X₂₇₅ is F or L,
the HC CDR1 comprises the sequence SX₂₇₆AX₂₇₇X₂₇₈, where X₂₇₆ is Y or S, X₂₇₇ is M or I, and X₂₇₈ is H or N,
the HC CDR2 comprises the sequence VISYDGINKYYADSVKG or GIIPIFGTVNYAQKFQG, and
the HC CDR3 comprises the sequence X₂₇₉X₂₈₀X₂₈₁X₂₈₂X₂₈₃X₂₈₄X₂₈₅YGMDV, where X₂₇₉ is A or D, X₂₈₀ is L or H, X₂₈₁ is G or I, X₂₈₂ is G or V, X₂₈₃ is N or S, X₂₈₄ is Y or P, and X₂₈₅ is Y or L;
41) a binding molecule in which
the LC CDR1 comprises the sequence TGSSSNIGAGYDVH,
the LC CDR2 comprises the sequence GNSNRPS,
the LC CDR3 comprises the sequence QSYDSSLSGPNWV or QSYDSRLRAVV,
the HC CDR1 comprises the sequence SYAIS or TQYLH,
the HC CDR2 comprises the sequence X₂₈₆IX₂₈₇PX₂₈₈X₂₈₉GX₂₉₀X₂₉₁X₂₉₂YAQKFQG, where X₂₈₆ is G or I, X₂₈₇ is I or N, X₂₈₈ is F or Y, X₂₈₉ is F or G, X₂₉₀ is T or S, X₂₉₁ is S or T, and X₂₉₂ is N or T, and
the HC CDR3 comprises the sequence GQFSDSSGYQHPYYDYGMD or SPSGYYGDYEGDAFDI;
42) a binding molecule in which
the LC CDR1 comprises the sequence TGTSSDVGGYNYVS,
the LC CDR2 comprises the sequence DVSX₂₉₃RPS, where X₂₉₃ is K or N,
the LC CDR3 comprises the sequence SSYTSSSTLV,
the HC CDR1 comprises the sequence RGDYWG or THALS,
the HC CDR2 comprises the sequence SIYHSGSTYYNPSLKS or GIIPIFGPADYAQKFQG, and
the HC CDR3 comprises the sequence HGTSGYYYPNWFDP or GLSLGFCSAGSCYDYLDY;
43) a binding molecule in which
the LC CDR1 comprises the sequence TGSSSNIGAGYDVH,
the LC CDR2 comprises the sequence GNSNRPS,
the LC CDR3 comprises the sequence QSYDSSLSGX₂₉₄V, where X₂₉₄ is V or P,
the HC CDR1 comprises the sequence DYAMH or SYDIS,
the HC CDR2 comprises the sequence GISWNSGTIGYADSVKG or GIIPILGIPNYAQKFQG, and
the HC CDR3 comprises the sequence EELGPYSNRWYSSSDGMDV or SRGYSGYGANWYFDL;
44) a binding molecule in which
the LC CDR1 comprises the sequence TGTSSDVGGYNX₂₉₅VS, where X₂₉₅ is Y or N,
the LC CDR2 comprises the sequence DVSX₂₉₆RPS, where X₂₉₆ is N or K,
the LC CDR3 comprises the sequence SSYTSSSPWV,
the HC CDR1 comprises the sequence SYAX₂₉₇S, where X₂₉₇ is M or I,
the HC CDR2 comprises the sequence GIX₂₉₈X₂₉₉X₃₀₀X₃₀₁GX₃₀₂TX₃₀₃YAX₃₀₄X₃₀₅X₃₀₆X₃₀₇G, where X₂₉₅ is S or I, X₂₉₉ is G or P, X₃₀₀ is S or M, X₃₀₁ is G or F, X₃₀₂ is S or T, X₃₀₃ is Y or N, X₃₀₄ is D or Q, X₃₀₅ is S or K, X₃₀₆ is V or F, and X₃₀₇ is K or Q, and
the HC CDR3 comprises the sequence GVVTADWYFDL or DPTSRSTYYYYSGSYYP;
45) a binding molecule in which
the LC CDR1 comprises the sequence SGSSSNIGX₃₀₈NYVS, where X₃₀₈ is R or N,
the LC CDR2 comprises the sequence DNNX₃₀₉RPS, where X₃₀₉ is R or K,
the LC CDR3 comprises the sequence GTWDSSLSAVX₃₁₀, where X₃₁₀ is A or V,
the HC CDR1 comprises the sequence SYX₃₁₁X₃₁₂X₃₁₃, where X₃₁₁ is Y or A, X₃₁₂ is M or I, and X₃₁₃ is H or I,
the HC CDR2 comprises the sequence X₃₁₄IX₃₁₅PX₃₁₆X₃₁₇GX₃₁₈X₃₁₉X₃₂₀YAQKFQG, where X₃₁₄ is I or G, X₃₁₅ is N or I, X₃₁₆ is S or I, X₃₁₇ is G or F, X₃₁₈ is S or T, X₃₁₉ is T or A, and X₃₂₀ is S or N, and
the HC CDR3 comprises the sequence GGDHGMDV or VTGYDSSGYYQDY;
46) a binding molecule in which
the LC CDR1 comprises the sequence SGSSSNIGNNYVS,
the LC CDR2 comprises the sequence DNNKRPS,
the LC CDR3 comprises the sequence GTWDSSLSAX₃₂₁V, where X₃₂₁ is G or V,
the HC CDR1 comprises the sequence SX₃₂₂X₃₂₃IX₃₂₄, where X₃₂₂ is N or Y, X₃₂₃ is W or V, and X₃₂₄ is A or S,
the HC CDR2 comprises the sequence X₃₂₅IX₃₂₆PX₃₂₇X₃₂₈X₃₂₉X₃₃₀X₃₃₁X₃₃₂X₃₃₃X₃₃₄X₃₃₅X₃₃₆FQG, where X₃₂₅ is I or R, X₃₂₆ is Y or I, X₃₂₇ is G or I, X₃₂₈ is D or F, X₃₂₉ is S or G, X₃₃₀ is D or T, X₃₃₁ is T or V, X₃₃₂ is R or K, X₃₃₃ is N or Y, X₃₃₄ is S or A, X₃₃₅ is P or Q, and X₃₃₆ is S or K, and
the HC CDR3 comprises the sequence LAYFHPQRNGGYEYYFDY or DLPGDSRDGYNYDAFDI;
47) a binding molecule in which
the LC CDR1 comprises the sequence SGSSSX₃₃₇X₃₃₈GSNX₃₃₉VN, where X₃₃₇ is N or D, X₃₃₈ is V or I, and X₃₃₉ is I or T,
the LC CDR2 comprises the sequence X₃₄₀NNQRPS, where X₃₄₀ is N or S,
the LC CDR3 comprises the sequence AAWDDSLNX₃₄₁WV, where X₃₄₁ is A or G,
the HC CDR1 comprises the sequence NHWIA or NYAIN,
the HC CDR2 comprises the sequence X₃₄₂IX₃₄₃PX₃₄₄X₃₄₅X₃₄₆X₃₄₇X₃₄₈X₃₄₉YX₃₅₀X₃₅₁X₃₅₂FQG, where X₃₄₂ is I or G, X₃₄₃ is Y or I, X₃₄₄ is Y or I, X₃₄₅ is D or F, X₃₄₆ is S or G, X₃₄₇ is D or T, X₃₄₈ is T or A, X₃₄₉ is K or N, X₃₅₀ is S or A, X₃₅₁ is P or Q, and X₃₅₂ is S or K, and
the HC CDR3 comprises the sequence NPTVTNWFDS or DRYPGYYDILTGQIGTGERNAMDV;
48) a binding molecule in which
the LC CDR1 comprises the sequence SGSSSNIGX₃₅₃NYVX₃₅₄, where X₃₅₃ is N or S, and X₃₅₄ is S or Y,
the LC CDR2 comprises the sequence X₃₅₅NNX₃₅₆RPS, where X₃₅₅ is D or R, and X₃₅₆ is K or Q,
the LC CDR3 comprises the sequence X₃₅₇X₃₅₈WDX₃₅₉SLSX₃₆₀X₃₆₁X₃₆₂, where X₃₅₇ is G or A, X₃₅₈ is T or A, X₃₅₉ is S or D, X₃₆₀ is A or G, X₃₆₁ is G or W, and X₃₆₂ is P or V,
the HC CDR1 comprises the sequence TSGVGVG or ELPIH,
the HC CDR2 comprises the sequence LIDWDDNKYYTTSLKT or RFDPEDGETIYAQNFQG, and
the HC CDR3 comprises the sequence X₃₆₃X₃₆₄X₃₆₅X₃₆₆X₃₆₇X₃₆₈X₃₆₉X₃₇₀X₃₇₁X₃₇₂YYYYGMDV, where X₃₆₃ is I or D, X₃₆₄ is P or L, X₃₆₅ is G or T, X₃₆₆ is F or T, X₃₆₇ is L or V, X₃₆₈ is R or T, X₃₆₉ is Y or N, X₃₇₀ is R or P, X₃₇₁ is N or L, and X₃₇₂ is R or N; and
49) a binding molecule in which
the LC CDR1 comprises the sequence SGSSSNIGX₃₇₃NYVX₃₇₄, where X₃₇₃ is N or S, and X₃₇₄ is S or N,
the LC CDR2 comprises the sequence X₃₇₅NNX₃₇₆RPS, where X₃₇₅ is D or S, and X₃₇₆ is K or Q,
the LC CDR3 comprises the sequence X₃₇₇TWDX₃₇₈SLX₃₇₉X₃₈₀X₃₈₁V, where X₃₇₇ is G or A, X₃₇₈ is S or D, X₃₇₉ is S or N, X₃₈₀ is A or G, and X₃₈₁ is G or Q,
the HC CDR1 comprises the sequence DYX₃₈₂MX₃₈₃, where X₃₈₂ is G or W, and X₃₈₃ is H or S,
the HC CDR2 comprises the sequence X₃₈₄IX₃₈₅X₃₈₆DGSX₃₈₇KYYX₃₈₈DSVKG, where X₃₈₄ is A or N, X₃₈₅ is S or K, X₃₈₆ is Y or E, X₃₈₇ is N or E, and X₃₈₈ is A or V, and
the HC CDR3 comprises the sequence DSGLYGSGWSTYQYYAMDV or GYSGNF.

40. The binding molecule according to claim 39, wherein the binding molecule has an IC₅₀ value of 200 ng/ml or less, as evaluated according to a plaque reduction neutralization test (PRNT) method for SARS-coronavirus-2 (SARS-CoV-2), or
a minimum concentration value of the binding molecule that neutralizes 100 TCID50 of virus for SARS-coronavirus-2 (SARS-CoV-2) is 2 µg/ml or less, or
the binding molecule is able to bind to a receptor-binding domain (RBD) of the spike protein of SARS-coronavirus-2 with an equilibrium dissociation constant (K_{D}) of 1.0×10⁻⁸M or less.
